# EUROPÄISCHE PATENTSCHRIFT

(11) **EP 1 347 969 B1**
(45) Veröffentlichungstag und Bekanntmachung des Hinweises auf die Patenterteilung: **23.03.2005**
(21) Anmeldenummer: 01990536.3
(22) Anmeldetag: 10.12.2001
(51) Int. Cl.: C07D 413/06, C07D 417/06, A01N 43/76, A01N 43/78

(54) **5-'(PYRAZOL-4-YL)CARBONYL!BENZAZOLONE ALS HERBIZIDE**
5- (PYRAZOL-4-YL)CARBONYL]BENZAZOLONE AS HERBICIDE
5- (PYRAZOL-4-YL)CARBONYL]BENZAZOLONES EN TANT QU'HERBICIDES

(30) Priorität: 11.12.2000 DE 10061657
(43) Veröffentlichungstag der Anmeldung: 01.10.2003
(73) Patentinhaber: BASF AKTIENGESELLSCHAFT, 67056 Ludwigshafen (DE)
(72) Erfinder: MAYER, Guido, 67161 Gönnheim (DE); MISSLITZ, Ulf, 67433 Neustadt (DE); BAUMANN, Ernst, 67373 Dudenhofen (DE); VON DEYN, Wolfgang, 67435 Neustadt (DE); KUDIS, Steffen, 68167 Mannheim (DE); HOFMANN, Michael, 67059 Ludwigshafen (DE); VOLK, Thorsten, 64297 Darmstadt (DE); WITSCHEL, Matthias, 67098 Bad Dürkheim (DE); ZAGAR, Cyrill, 68167 Mannheim (DE); LANDES, Andreas, 67354 Römerberg (DE); LANGEMANN, Klaus, 34270 Schauenburg (DE)
(74) Vertreter: Pohl, Michael, Dr.
(86) Internationale Anmeldenummer: PCT/EP2001/014476
(87) Internationale Veröffentlichungsnummer: WO 2002/048140

(56) Entgegenhaltungen:
- WO-A-00/68228

## Beschreibung

Die vorliegende Erfindung betrifft 5-[(Pyrazol-4-yl)carbonyl]benzazolone (im Folgenden Benzazolone), Mittel, die derartige Verbindungen enthalten, sowie die Verwendung der Benzazolone oder Mittel, die diese enthalten, zur Schadpflanzenbekämpfung.

Aus der WO 96/05197 sind Saccharin-Derivate mit herbizider Wirkung bekannt, die am Benzolkern des Saccharingerüstes mit einem (5-Hydroxypyrazol-4-yl)carbonyl-Rest substituiert sind. Die WO 97/30993 und die WO 97/09327 beschreiben Dioxothiochroman-Derivate und Dihydrobenzothiophen-Derivate mit herbizider Wirkung, die ebenfalls am Benzolkern der Schwefel-Heterocyclen einen (5-Hydroxypyrazol-4-yl)carbonyl-Rest aufweisen.

Aus der WO 97/08164 sind unter anderem benzkondensierte Derivate des γ-Butyrolactams mit herbizider Wirkung bekannt, die ebenfalls einen (5-Hydroxypyrazol-4-yl)carbonyl-Rest aufweisen.

Die JP 10130627 beschreibt u. a. Herbizide auf der Basis von pyrazol-Derivaten der allgemeinen Formel (a), 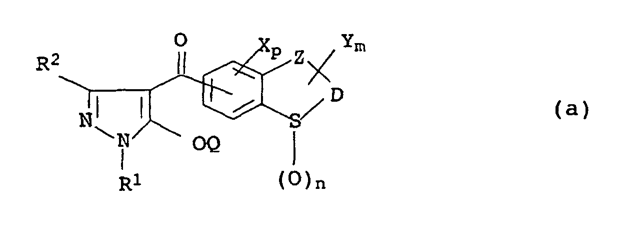 worin R¹ für C₁-C₄-Alkyl oder C₂-C₄-Alkenyl, R² vorzugsweise für Wasserstoff oder Methyl, Q vorzugsweise für Wasserstoff, n-Propylsulfonyl, Tolylsulfonyl oder Cyclohexylsulfonyl stehen. X steht für C₁-C₄-(Halogen)alkyl, C₂-C₄-Alkoxyalkyl, Halogen, C₁-C₄-(Halo)alkoxy. Z steht für O, SOᵣ oder NR³ mit r = 0 bis 2,
wobei R³ für Wasserstoff, C₁-C₄-(Halogen)alkyl, C₁-C₄-(Halogen)alkylsulfonyl oder C₁-C₄-Alkylcarbonyl steht. Y steht für Sauerstoff oder einen Substituenten an der Alkylenkette. D steht für eine substituierte oder unsubstituierte C₁-C₃-Alkylenkette und p für eine ganze Zahl von 0 bis 3. Die Variable m steht für eine ganze Zahl von null bis sechs und n für eine ganze Zahl von null bis zwei.

Die herbiziden Eigenschaften der aus den genannten Druckschriften bekannten Verbindungen sowie deren Verträglichkeiten gegenüber Kulturpflanzen vermögen jedoch nur bedingt die Anforderungen an Herbizide zu befriedigen.

Aus der PCT/EP 00/04040 sind 4-(3',4'-Heterocyclylbenzoyl)pyrazole bekannt. Als heterocyclische Reste werden unter anderem Benzoxazolyl, Benzimidazolyl, Benzthiazolyl und Benztriazinyl genannt. Die Verbindungen weisen Herbizid-Wirkung auf.

Der vorliegenden Erfindung liegt somit die Aufgabe zugrunde neue Verbindungen mit herbizider Wirkung bereitzustellen, die vorzugsweise eine höhere Wirksamkeit als die herbiziden Substanzen des Standes der Technik und/oder eine bessere Selektivität gegenüber Schadpflanzen aufweisen.

Es wurde nun überraschenderweise gefunden, dass diese Aufgabe durch 5-[(Pyrazol-4-yl)carbonyl]benzazolone der nachstehend definierten, allgemeinen Formel I gelöst wird.

Demnach betrifft die vorliegende Erfindung Pyrazolylcarbonylbenzazolone der allgemeinen Formel I, 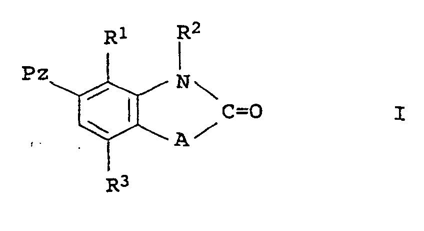 worin A, R¹, R², R³ und Pz die nachfolgend angegebenen Bedeutungen aufweisen,
- A: O, S, SO, SO₂ oder NR⁶;
- R¹: Halogen, C₁-C₄-Alkyl, C₁-C₄-Halogenalkyl, C₁-C₄-Hydroxyalkyl, C₁-C₄-Alkoxy-C₁-C₄-alkyl, C₁-C₄-Halogenalkoxy-C₁-C₄-alkyl, C₁-C₄-Halogenalkylthio-C₁-C₄-alkyl, C₁-C₄-Alkylthio-C₁-C₄-alkyl, C₁-C₄-Alkylsulfonyl-C₁-C₄-alkyl, C₁-C₄-Halogenalkylsulfonyl-C₁-C₄-alkyl, C₁-C₄-Alkoxy, C₁-C₄-Hydroxyalkoxy, C₁-C₄-Halogenalkoxy, C₁-C₄-Alkoxy-C₁-C₄-alkoxy-, C₁-C₄-Halogenalkoxy-C₁-C₄-alkoxy, C₁-C₄-Alkylthio, C₁-C₄-Halogenalkylthio, C₁-C₄-Alkylsulfonyl, C₁-C₄-Halogenalkylsulfonyl;
- R²: Hydroxy, Nitro, Amino, C₁-C₆-Alkylamino, Di-(C₁-C₆-alkyl)amino, Cyano, CHO, C₁-C₆-Alkoxy, C₁-C₆-Alkyl, C₁-C₆-Hydroxyalkyl, C₁-C₆-Halogenalkyl, C₁-C₆-Alkoxy-C₁-C₆-alkyl, C₁-C₆-Halogenalkoxy-C₁-C₆-alkyl, C₂-C₆-Alkenyl, C₂-C₆-Halogenalkenyl, C₁-C₆-Halogenalkoxy-C₂-C₆-alkenyl, C₁-C₆-Alkoxy-C₂-C₆-alkenyl, C₂-C₆-Alkinyl, C₂-C₆-Halogenalkinyl, C₁-C₆-Halogenalkoxy-C₂-C₆-alkinyl, C₁-C₆-Alkoxy-C₂-C₆-alkinyl, C₁-C₆-Alkylsulfonyl, C₁-C₆-Halogenalkylsulfonyl, (C₁-C₆-Alkyl)carbonyl, C₁-C₆-Halogenalkylcarbonyl, Hydroxycarbonyl-C₁-C₄-alkyl, C₁-C₆-Alkoxycarbonyl-C₁-C₆-alkyl,
ein Rest der Formel C(O)OR⁴, CON(R⁵)₂ oder C(=NOR^{4a})R^{4b}, Aryl, Aryl-C₁-C₄-alkyl, Arylsulfonyl, Arylcarbonyl, C₃-C₆-Cycloalkyl, C₃-C₆-Cycloalkenyl,
3-, 4-, 5-, 6- oder 7-gliedriges Heterocyclyl, 3-, 4-, 5-, 6- oder 7-gliedriges Heterocyclyl-C₁-C₆-alkyl,
wobei jeder Aryl-, Cycloalkyl-, Cycloalkenyl- und jeder Heterocyclylrest unsubstituiert sein kann oder ein, zwei, drei oder vier Substituenten tragen kann, jeweils ausgewählt unter Halogen, C₁-C₄-Halogenalkyl, C₁-C₄-Alkoxy und C₁-C₄-Halogenalkoxy;
- R³: Wasserstoff, C₁-C₄-Alkyl, C₁-C₄-Halogenalkyl, C₁-C₄-Alkoxy-C₁-C₄-alkyl, C₁-C₄-Halogenalkoxy-C₁-C₄-alkyl oder Halogen;
worin
R⁴ C₁-C₆-Alkyl, C₁-C₆-Halogenalkyl, C₁-C₆-Alkoxy-C₁-C₆-alkyl, C₁-C₆-Halogenalkoxy-C₁-C₆-alkyl, C₂-C₆-Alkenyl, C₂-C₆-Halogenalkenyl, C₁-C₆-Halogenalkoxy-C₂-C₆-alkenyl, C₁-C₆-Alkoxy-C₂-C₆-alkenyl, C₂-C₆-Alkinyl, C₂-C₆-Halogenalkinyl, C₁-C₆-Halogenalkoxy-C₂-C₆-alkinyl, C₁-C₆-Alkoxy-C₂-C₆-alkinyl, bedeutet;
R^{4a}, R^{4b} unabhängig voneinander die für R⁴ genannten Bedeutungen aufweisen können und R^{4b} für Wasserstoff stehen kann;
R⁵ unabhängig voneinander für Wasserstoff, C₁-C₆-Alkyl, C₁-C₆-Halogenalkyl, C₁-C₆-Alkoxy-C₁-C₆-alkyl, (C₁-C₆-Halogenalkoxy)-C₁-C₆-alkyl, C₂-C₆-Alkenyl, C₂-C₆-Halogenalkenyl, C₁-C₆-Halogenalkyl-C₂-C₆-alkenyl, C₁-C₆-Halogenalkoxy-C₂-C₆-alkenyl, C₁-C₆-Alkoxy-C₂-C₆-alkenyl, C₂-C₆-Alkinyl, C₂-C₆-Halogenalkinyl, C₁-C₆-Halogenalkyl-C₂-C₆-alkinyl, C₁-C₆-Halogenalkoxy-C₂-C₆-alkinyl, C₁-C₆-Alkoxy-C₂-C₆-alkinyl stehen, oder zusammen einen 3- bis 7-gliedrigen Heterocyclus bilden, der gegebenenfalls partiell oder vollständig halogeniert ist und/oder einen, zwei oder drei Substituenten, ausgewählt unter C₁-C₆-Alkyl, C₁-C₆-Halogenalkyl, C₁-C₆-Alkoxy, C₁-C₆-Halogenalkoxy tragen können;
R⁶ Wasserstoff, C₁-C₄-Alkyl, C₁-C₄-Halogenalkyl, C₁-C₄-Haloalkoxy-C₁-C₄-alkyl, C₁-C₄-Alkoxy-C₁-C₄-alkyl bedeutet; und
- Pz: für einen Rest der Formel IIa oder IIb steht, 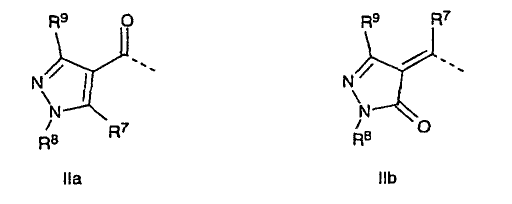
worin die Variablen R⁷, R⁸ und R⁹ folgende Bedeutung haben:
- R⁷: Hydroxy, Mercapto, Halogen, OR¹⁰, SR¹⁰, SOR¹¹, SO₂R¹¹, OSO₂R¹¹, P(O)R¹²R¹³, OP(O)R¹²R¹³, P(S)R¹²R¹³, OP(S)R¹²R¹³, NR¹⁴R¹⁵, ONR¹⁴R¹⁵ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R⁸: Wasserstoff, C₁-C₆-Alkyl, C₃-C₆-Cycloalkyl, C₁-C₆-Halogenalkyl, Hydroxy, C₁-C₆-Alkoxy oder C₁-C₆-Halogenalkoxy;
- R⁹: Wasserstoff, Halogen, C₁-C₆-Alkyl, C₁-C₆-Halogenalkyl, Hydroxy, C₁-C₆-Alkoxy, C₁-C₆-Halogenalkoxy, C₁-C₆-Alkylthio oder C₁-C₆-Halogenalkylthio;
wobei
R¹⁰ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, C₂-C₆-Alkenylcarbonyl, C₂-C₆ Alkinylcarbonyl, C₃-C₆-Cycloalkylcarbonyl, C₁-C₆ Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆ Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆ Alkinyl)-N-(C₁-C₆-alkoxy)-aminocarbonyl, Di-(C₁-C₆-alkyl)-aminothiocarbonyl oder C₁-C₆-Alkoxyimino-C₁-C₆-alkyl, wobei die genannten Alkyl-, Cycloalkyl- und Alkoxyreste partiell oder vollständig halogeniert sein können und/oder eine, zwei oder drei der folgenden Gruppen tragen können: Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Phenyl-C₁-C₆-alkyl, Phenylcarbonyl-C₁-C₆-alkyl, Phenylcarbonyl, Phenoxycarbonyl, Phenyloxythiocarbonyl, Phenylaminocarbonyl, N-(C₁-C₆-Alkyl)-N-(phenyl)-aminocarbonyl, Phenyl-C₂-C₆-alkenylcarbonyl, Heterocyclyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, Heterocyclylaminocarbonyl, N-(C₁-C₆-Alkyl)-N-(heterocyclyl)-aminocarbonyl, oder Heterocyclyl-C₂-C₆-alkenylcarbonyl, wobei der Phenyl- und der Heterocyclyl-Rest der 18 letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
R¹¹ C₁-C₆-Alkyl, C₃-C₆-Alkenyl, C₃-C₆-Alkinyl oder C₃-C₆-Cycloalkyl, wobei die vier genannten Reste partiell oder vollständig halogeniert sein können und/oder eine, zwei oder drei der folgenden Gruppen tragen können: Cyano, C₁-C₄-Alkoxy, C₁-C₄-Halogenalkoxy, C₁-C₄-Alkylthio, C₁-C₄-Halogenalkylthio, C₁-C₄-Alkylcarbonyl, C₁-C₄-Alkoxycarbonyl oder C₁-C₄-Halogenalkoxycarbonyl;
Phenyl, Phenyl-C₁-C₆-alkyl, Heterocyclyl oder Heterocyclyl-C₁-C₆-alkyl, wobei der Phenyl- und der Heterocyclyl-Rest der letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy, C₁-C₄-Halogenalkoxy oder C₁-C₄-Alkoxycaxbonyl;
R¹², R¹³ unabhängig voneinander Wasserstoff, Hydroxy, C₁-C₆-Alkyl, C₁-C₆-Alkoxy, C₁-C₆-Alkylthio, Phenyl, Phenyl-C₁-C₄-alkyl oder Phenoxy, wobei die drei letztgenannten Substituenten partiell oder vollständig halogeniert sein können und/oder einen, zwei oder drei der folgenden Reste tragen können: Nitro, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl;
R¹⁴ Wasserstoff, C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-alkyl)-amino oder C₁-C₆-Alkylcarbonylamino, wobei die genannten Alkyl-, Cycloalkyl- und Alkoxyreste partiell oder vollständig halogeniert sein können und/oder einen, zwei oder drei Reste der folgenden Gruppe tragen können: Cyano, C₁-C₄-Alkoxycarbonyl, C₁-C₄-Alkylaminocarbonyl, Di-(C₁-C₄-alkyl)-aminocarbonyl oder C₃-C₆-Cycloalkyl;
Phenyl, Phenyl-C₁-C₄-alkyl, Phenylcarbonyl, Heterocyclyl, Heterocyclyl-C₁-C₄-alkyl oder Heterocyclylcarbonyl, wobei der Phenyl- oder Heterocyclyl-Rest der sechs letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy; und
R¹⁵ Wasserstoff, C₁-C₆-Alkyl oder C₃-C₆-Alkenyl, C₃-C₆-Alkinyl; bedeutet,
sowie deren landwirtschaftlich brauchbaren Salze.

Ferner wurden herbizide Mittel gefunden, die Pyrazolyl-Derivate der Formel I enthalten und eine sehr gute herbizide Wirkung besitzen. Außerdem wurden Verfahren zur Bekämpfung von unerwünschtem Pflanzenwuchs mit den Pyrazolyl-Derivaten der Formel I gefunden.

Die Verbindungen der Formel I können je nach Substitutionsmuster ein oder mehrere Chiralitätszentren enthalten und liegen dann als Enantiomere oder Diastereomerengemische vor. Gegenstand der Erfindung sind sowohl die reinen Enantiomere oder Diastereomere als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₉-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Im Falle von R⁷ = Hydroxy oder Mercapto {Z = O,S} steht IIa auch stellvertretend für die tautomeren Formen IIa' und IIa" 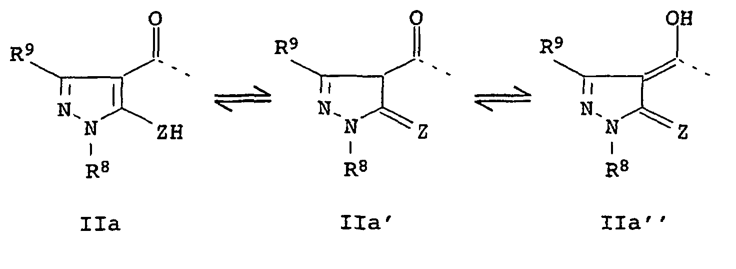 bzw. IIb auch stellvertretend für die tautomeren Formen IIb' und IIb".

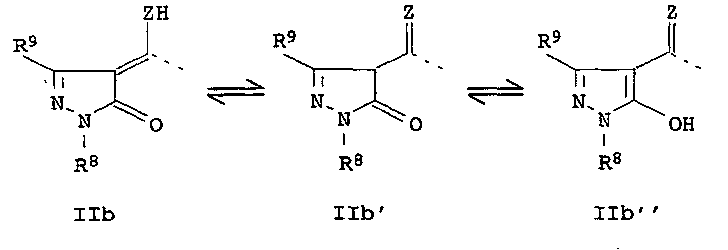

Die für die Substituenten R¹ bis R¹⁵ oder als Reste an Phenyl- und Heterocyclyl-Resten genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylamino-, N,N-Dialkylamino-, N-Halogenalkylamino-, N-Alkoxyamino-, N-Alkoxy-N-alkylamino-, N-Alkylcarbonylamino-, Alkylcarbonyl-, Halogenalkylcarbonyl, Alkoxycarbonyl-, Halogenalkoxycarbonyl, Alkylthiocarbonyl-, Alkylcarbonyloxy-, Alkylaminocarbonyl-, Dialkylaminocarbonyl-, Dialkylaminothiocarbonyl-, Alkoxyalkyl-, Hydroxyalkoxyalkyl, Alkoxyiminoalkyl-, Phenylalkylcarbonyl, Heterocyclylalkylcarbonyl, Phenylalkenylcarbonyl-, Heterocyclylalkenylcarbonyl-, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl-, Alkenyloxy-, Alkinyloxy, Alkandiyl-, Alkendiyl-, Alkadiendiyl- oder Alkindiyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl für: z. B. Methyl, Ethyl, Propyl, 1-Methylethyl, Butyl, 1-Methylpropyl, 2-Methylpropyl oder 1,1-Dimethylethyl;
- C₁-C₄-Halogenalkyl für: einen C₁-C₄-Alkylrest, wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Alkyl sowie die Alkylteile von C₁-C₆ Alkylamino, Di-(C₁-C₆-alkyl)amino, N(C₁-C₆-Alkoxy)-N-(C₁-C₆-alkyl)-aminocarbonyl, N-(C₃-C₆-Alkenyl)-N-(C₁-C₆-alkyl)-aminocarbonyl, (C₃-C₆ Alkinyl)-N-(C₁-C₆-alkyl)-aminocarbonyl, N-(C₁-C₆-Alkyl)-N-phenylaminocarbonyl sowie N-(C₁-C₆ Alkyl)-N-heterocyclylaminocarbonyl: C₁-C₄ Alkyl, wie voranstehend genannt, sowie z. B. n-Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₆-Halogenalkyl sowie die Halogenalkylteile von N-C₁-C₆-Halogenalkylamino: C₁-C₄-Halogenalkyl, wie vorstehend genannt, sowie für 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy sowie die Alkoxyteile von Hydroxy-C₁-C₄-alkoxy-C₁-C₄-alkyl, C₁-C₄-Alkoxy-C₁-C₄-alkoxycarbonyl für: z. B. Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy sowie die Alkoxyteile von C₁-C₆-Alkoxy-C₃-C₆-alkenyl, C₁-C₆ Alkoxy-C₃-C₆-alkinyl, C₁-C₆-Alkoxy-C₂-C₆-alkenyl, C₁-C₆ Alkoxy-C₂-C₆-alkinyl, N-C₁-C₆-Alkoxyamino, N-(C₁-C₆-Alkoxy)-N-(C₁-C₆-alkyl)amino, C₁-C₆ Alkoxyimino-C₁-C₆-alkyl, (C₁-C₆-Alkoxy)-(C₁-C₆-alkylthio)-methyl, N-(C₁-C₆ Alkoxy)-N-(C₁-C₆-alkyl)-aminocarbonyl, N-(C₃-C₆-Alkenyl)-(C₁-C₆-alkoxy)-aminocarbonyl und N-(C₃-C₆-Alkinyl)-N-(C₁-C₆-alkoxy)-aminocarbonyl für: C₁-C₄ Alkoxy, wie voranstehend genannt, sowie z. B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy sowie die Halogenalkoxyteile von C₁-C₄-Halogenalkoxy-C₁-C₄-alkyl, C₁-C₄-Halogenalkoxy-C₁-C₄-alkoxy, C₁-C₄-Halogenalkoxy-C₁-C₆-alkyl, C₁-C₄-Halogenalkoxy-C₁-C₆-alkoxy, C₁-C₄-Halogenalkoxy-C₃-C₆-alkenyl, C₁-C₄-Halogenalkoxy-C₃-C₆-alkinyl für: einen C₁-C₄-Alkoxyrest, wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Di-chlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy für: C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio (C₁-C₄-Alkylsulfanyl: C₁-C₄-Alkyl-S-) für: z.B. Methylthio, Ethylthio, Propylthio, 1-Methylethylthio, Butylthio, 1-Methylpropylthio, 2-Methylpropylthio oder 1,1-Dimethylethylthio;
- C₁-C₆-Alkylthio sowie die Alkylthioteile von C₁-C₆-Alkylthiocarbonyl für: C₁-C₄ Alkylthio, wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₄-Halogenalkylthio sowie die Halogenalkylthioteile von C₁-C₄-Halogenalkylthio-C₁-C₄-alkyl für: einen C₁-C₄-Alkylthiorest, wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio., 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Tri-chlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;
- C₁-C₆-Halogenalkylthio für: C₁-C₄-Halogenalkylthio, wie vorstehend genannt, sowie 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₄-Alkylsulfinyl (C₁-C₄-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl oder 1,1-Dimethylethylsulfinyl;
- C₁-C₆-Alkylsulfinyl: C₁-C₄-Alkylsulfinyl, wie vorstehend genannt, sowie Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
- C₁-C₄-Halogenalkylsulfinyl: C₁-C₄-Alkylsulfinylrest, wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl oder Nonafluorbutylsulfinyl;
- C₁-C₆-Halogenalkylsulfinyl: C₁-C₄-Halogenalkylsulfinyl, wie vorstehend genannt, sowie 5-Fluorpentylsulfinyl, 5-chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₄-Alkylsulfonyl (C₁-C₄ Alkyl-S(=O)₂-) sowie die Alkylsulfonylteile von C₁-C₄-Alkylsulfonyl-C₁-C₄-Alkyl für: z. B. Methylsulfonyl, Ethylsulfonyl, Propylsulfonyl, 1-Methylethylsulfanyl, Butylsulfonyl, 1-Methylpropylsulfonyl, 2-Methylpropylsulfonyl oder 1,1-Dimethylethylsulfonyl;
- C₁-C₆-Alkylsulfonyl: C₁-C₄-Alkylsulfonyl, wie vorstehend genannt, sowie Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl;
- C₁-C₄-Halogenalkylsulfonyl einen C₁-C₄ Alkylsulfonylrest, wie voranstehend genannt, der partiell oder vollständig durch Fluor, Chlor, Brom und/oder Iod substituiert ist, sowie für die Halogenalkylsulfonyl von C₁-C₄-Halogenalkylsulfonyl-C₁-C₄-alkyl für: z. B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl;
- C₁-C₆-Halogenalkysulfonyl sowie für die Halogenalkylsulfonylteile von C₁-C₆-Halogenalkylsulfonyl-C₁-C₆-alkyl für: C₁-C₄-Halogenalkylsulfonyl, wie vorstehend genannt, sowie 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₁-C₆-Alkylamino: Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- Di-(C₁-C₄-alkyl)amino, also z.B. N,N-Dimethylamino, N,N-Di-ethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Di-methylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl)amino;
- Di-(C₁-C₆-alkyl)amino: Di-(C₁-C₄-alkyl)amino wie vorstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylamino
- C₁-C₄ Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von C₁-C₆-Alkylcarbonylamino: C₁-C₄ Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 2-Ethyl-1-methylpropylcarbonyl oder 1-Ethyl-2-methylpropylcarbonyl;
- C₁-C₄-Halogenalkylcarbonyl: einen C₁-C₄-Alkylcarbonylrest, wie vorstehend genannt, der partiell oder vollständig durch Fluor, Chlor, Brom und/oder Iod substituiert ist, also z.B. Chloracetyl, Dichloracetyl, Trichloracetyl, Fluoracetyl, Difluoracetyl, Trifluoracetyl, Chlorfluoracetyl, Dichlor-fluoracetyl, Chlordifluoracetyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 2-Fluorpropylcarbonyl, 3-Fluorpropylcarbonyl, 2,2-Difluorpropylcarbonyl, 2,3-Di-fluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, 2,2,3,3,3-Pentafluorpropylcarbonyl, Heptafluorpropylcarbonyl, 1-(Fluormethyl)-2-fluorethylcarbonyl, 1-(Chlormethyl)-2-chlorethylcarbonyl, 1-(Brommethyl)-2-bromethylcarbonyl, 4-Fluorbutylcarbonyl, 4-Chlorbutylcarbonyl, 4-Brombutylcarbonyl oder Nonafluorbutylcarbonyl;
- C₁-C₆-Halogenalkylcarbonyl: einen C₁-C₄-Halogenalkylcarbonylrest wie voranstehend genannt, sowie 5-Fluorpentylcarbonyl, 5-Chlorpentylcarbonyl, 5-Brompentylcarbonyl, Perfluorpentylcarbonyl, 6-Fluorhexylcarbonyl, 6-Chlorhexylcarbonyl, 6-Bromhexylcarbonyl oder Perfluorhexylcarbonyl;
- C₁-C₄ Alkoxycarbonyl also z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- C₁-C₆-Alkoxycarbonyl: sowie die Alkoxycarbonylteile von C₁-C₆-Alkoxycarbonyl-C₁-C₆-alkyl für C₁-C₄ Alkoxycarbonyl,wie vorstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₄-Halogenalkoxycarbonyl: einen C₁-C₄-Alkoxycarbonylrest, wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl oder 4-Iodbutoxycarbonyl;
- C₁-C₆-Halogenalkoxycarbonyl: einen C₁-C₄-Halogenalkoxycarbonylrest wie voranstehend genannt, sowie 5-Fluorpentoxycarbonyl, 5-Chlorpentoxycarbonyl, 5-Brompentoxycarbonyl, 6-Fluorhexoxycarbonyl, 6-Chlorhexoxycarbonyl oder 6-Bromhexoxycarbonyl;
- (C₁-C₄-Alkyl)carbonyloxy: Acetyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy oder 1,1-Dimethylethylcarbonyloxy;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₆ Alkylamino)carbonyl: (C₁-C₄ Alkylamino)carbonyl, wie vorstehend genannt, sowie z. B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocärbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di-(C₁-C₄-alkyl)-aminocarbonyl: z. B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)-aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)-aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1,1,2-trimethylpropyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexylaminocarbonyl;
- Di-(C₁-C₆-alkyl)-aminothiocarbonyl: z.B. N,N-Dimethylaminothiocarbonyl, N,N-Diethylaminothiocarbonyl, N,N-Di-(1-methylethyl)aminothiocarbonyl, N,N-Dipropylaminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylaminothiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(2,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothiocarbonyl, N-Methyl-N-(1-ethylpropyl)-aminothiocarbonyl, N-Methyl-N-hexylaminothiocarbonyl, N-Methyl-N-(1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothiocarbonyl, N-Methyl-N-(1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dixnethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N Methyl-N-(1-ethyl-1-methylpropyl)-aminothiocarbonyl, N-Methyl-N-(1-ethyl-2-methylpropyl)-aminothiocarbonyl, N-Ethyl-N-pentylaminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)-aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethylpropyl)-aminothiocarbonyl, N-Ethyl-N-(1,2-dimethylpropyl)-aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-aminothiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-14-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexylaminothiocarbonyl, N-Butyl-N-hexylaminothiocarbonyl,-N-Pentyl-N-hexylaminothiocarbonyl oder N,N-Dihexylaminothiocarbonyl;
- C₁-C₆-Hydroxyalkyl: durch ein bis drei OH-Gruppen substituiertes C₁-C₆-Alkyl, z. B Hydroxymethyl, 1-Hydroxyethyl, 2-Hydroxyethyl, 1,2-Bishydroxyethyl, 1-Hydroxypropyl, 2-Hydroxypropyl, 3-Hydroxypropyl, 4-Hydroxybutyl, 2,2-Dimethyl-3-hydroxypropyl;
- Phenyl-C₁-C₆-alkyl: durch einen Phenylrest substituiertes C₁-C₆-Alkyl, z. B. Benzyl, 1-Phenylethyl und 2-Phenylethyl, wobei der Phenylrest in der angegebenen Weise teilweise oder vollständig halogeniert sein kann oder einen bis drei der für Phenyl oben angegebenen Substituenten aufweisen kann; Heterocyclyl-C₁-C₆-alkyl steht dementsprechend für ein durch einen Heterocyclylrest substituiertes C₁-C₆-Alkyl;
- C₁-C₆-Alkoxy-C₁-C₆-alkyl: durch C₁-C₆-Alkoxy, wie vorstehend genannt, substituiertes C₁-C₆-Alkyl, also z.B. Methoxymethyl, Ethoxymethyl, Propoxymethyl, (1-Methylethoxy)methyl, Butoxymethyl, (1-Methylpropoxy)methyl, (2-Methylpropoxy)-methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)-propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(Prop-oxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(Propoxy)butyl, 4-(1-Methylethoxy)butyl, 4-(Butoxy)-butyl, 4-(1-Methylpropoxy)butyl, 4-(2-Methylpropoxy)butyl oder 4-(1,1-Dimethylethoxy)butyl;
- C₁-C₆-Alkoxy-C₁-C₆-alkoxy, sowie die Alkoxyalkoxyteile von C₁-C₆ Alkoxy-C₁-C₆-alkoxycarbonyl: durch C₁-C₆-Alkoxy, wie vorstehend genannt, substituiertes C₁-C₆-Alkoxy, also z.B. für Methoxymethoxy, Ethoxymethoxy, Propoxymethoxy, (1-Methylethoxy)methoxy, Butoxymethoxy, (1-Methylpropoxy)methoxy, (2-Methylpropoxy)methoxy, (1,1-Dimethylethoxy)methoxy, 2-(Methoxy)ethoxy, 2-(Ethoxy)ethoxy, 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)-propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy oder 4-(1,1-Dimethylethoxy)butoxy;
- C₃-C₆-Alkenyl, sowie die Alkenylteile von C₃-C₆-Alkenylcarbonyl, C₃-C₆-Alkenyloxy, C₃-C₆-Alkenyloxycarbonyl, C₃-C₆-Alkenylaminocarbonyl, N-(C₃-C₆-Alkenyl)-N-(C₁-C₆-alkyl)aminocarbonyl, N-(C₃-C₆-Alkenyl)-N-(C₁-C₆-alkoxy)aminocarbonyl: z. B. Prop-1-en-3-yl, But-1-en-4-yl, 1-Methyl-prop-2-en-1-yl, 2-Methyl-prop-2-en-1-yl, 2-Buten-1-yl, 1-Penten-3-yl, 1-Penten-4-yl, 2-Penten-4-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en-1-yl, 1-Ethyl-prop-2-en-1-yl, Hex-3-en-1-yl, Hex-4-en-1-yl, Hex-5-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1 yl, 1,1-Dimethyl-but-3-en-1-yl, 1,2-Dimethyl-but-2-en-1-yl, 1,2-Dimethyl-but-3-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methyl-prop-2-en-1-yl;
- C₂-C₆ Alkenyl, sowie die Alkenylteile von C₂-C₆ Alkenylcarbonyl, Phenyl-C₂-C₆-alkenylcarbonyl und Heterocyclyl-C₂-C₆-alkenylcarbonyl: C₃-C₆-Alkenyl, wie voranstehend genannt, sowie Ethenyl;
- C₃-C₆-Halogenalkenyl: einen C₃-C₆-Alkenylrest, wie vorstehend genannt, der partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Tri-chlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl, sowie die Alkinylteile von C₃-C₆-Alkinylcarbonyl, C₃-C₆-Alkinyloxy, C₃-C₆-Alkinyloxycarbony, C₃-C₆-Alkinylaminocarbonyl, N-(C₃-C₆ Alkinyl) N--(C₁-C₆-alkyl)-aminocarbonyl, N-(C₃-C₆ Alkinyl)-N-(C₁-C₆-alkoxy)aminocarbonyl: z.B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methyl-pent-1-in-5-yl, 4-Methyl-pent-2-in-4-yl oder 4-Methyl-pent-2-in-5-yl;
- C₂-C₆-Alkinyl, sowie die Alkinylteile von C₂-C₆-Alkinylcarbonyl: C₃-C₆-Alkinyl, wie voranstehend genannt, sowie Ethinyl;
- C₃-C₆-Halogenalkinyl: einen C₃-C₆-Alkinylrest, wie vorstehend genannt, der partiell oder vollständig durch Fluor, Chlor, Brom und/oder Iod substituiert ist, also z. B. 1,1-Difluor-prop-2-in-1-yl, 3-Iod-prop-2-in-1-yl, 4-Fluorbut-2-in-1-yl, 4-Chlorbut-2-in-1-yl, 1,1-Difluorbut-2-in-1-yl, 4-Iod-but-3-in-1-yl, 5-Fluorpent-3-in-1-yl, 5-Iod-pent-4-in-1-yl, 6-Fluor-hex-4-in-1-yl oder 6-Iod-hex-5-in-1-yl;
- C₁-C₆-Alkandiyl: Methandiyl, Ethan-1,1-diyl, Ethan-1,2-diyl, Propan-1,1-diyl, Propan-1,2-diyl, Propan-1,3-diyl, Propan-2,2-diyl, Butan-1,1-diyl, Butan-1,2-diyl, Butan-1,3-diyl, Butan-1,4-diyl, 2-Methyl-propan-1,3-diyl, 2-Methyl-propan-1,2-diyl, 2-Methyl-propan-1,1-diyl, 1-Methyl-propan-1,2-diyl, 1-Methyl-propan-2,2-diyl, 1-Methyl-propan-1,1-diyl, Pentan-1,1-diyl, Pentan-1,2-diyl, Pentan-1,3-diyl, Pentan-1,5-diyl, Pentan-2,3-diyl, Pentan-2,2-diyl, 1-Methyl-butan-1,1-diyl, 1-Methyl-butan-1,2-diyl, 1-Methyl-butan-1,3-diyl, 1-Methyl-butan-1,4-diyl, 2-Methyl-butan-1,1-diyl, 2-Methyl-butan-1,2-diyl, 2-Methyl-butan-1,3-diyl, 2-Methyl-butan-1,4-diyl, 2,2-Dimethyl-propan-1,1-diyl, 2,2-Dimethyl-propan-1,3-diyl, 1,1-Dimethyl-propan-1,3-diyl, 1,1-Dimethyl-propan-1,2-diyl, 2,3-Dimethyl-propan-1,3-diyl, 2,3-Dimethyl-propan-1,2-diyl, 1,3-Dimethyl-propan-1,3-diyl, Hexan-1,1-diyl, Hexan-1,2-diyl, Hexan-1,3-diyl, Hexan-1,4-diyl-, Hexan-1,5-diyl, Hexan-1,6-diyl, Hexan-2,5-diyl, 2-Methyl-pentan-1,1-diyl, 1-Methyl-pentan-1,2-diyl, 1-Methyl-pentan-1,3-diyl, 1-Methyl-pentan-1,4-diyl, 1-Methyl-pentan-1,5-diyl, 2-Methyl-pentan-1,1-diyl, 2-Methyl-pentan-1,2-diyl, 2-Methyl-pentan-1,3-diyl, 2-Methyl-pentan-1,4-diyl, 2-Methyl-pentan-1,5-diyl, 3-Methyl-pentan-1,1-diyl, 3-Methyl-pentan-1,2-diyl, 3-Methyl-pentan-1,3-diyl, 3-Methyl-pentan-1,4-diyl, 3-Methyl-pentan-1,5-diyl, 1,1-Dimethyl-butan-1,2-diyl, 1,1-Dimethyl-butan-1,3-diyl, 1,1-Dimethyl-butan-1,4-diyl, 1,2-Dimethyl-butan-1,1-diyl, 1,2-Dimethyl-butan-1,2-diyl, 1,2-Dimethyl-butan-1,3-diyl, 1,2-Dimethyl-butan-1,4-diyl, 1,3-Dimethyl-butan-1,1-diyl, 1,3-Dimethyl-butan-1,2-diyl, 1,3-Dimethyl-butan-1,3-diyl, 1,3-Dimethyl-butan-1,4-diyl, 1-Ethyl-butan-1,1-diyl, 1-Ethyl-butan-1,2-diyl, 1-Ethyl-butan-1,3-diyl, 1-Ethyl-butan-1,4-diyl, 2-Ethyl-butan-1,1-diyl, 2-Ethyl-butan-1,2-diyl, 2-Ethyl-butan-1,3-diyl, 2-Ethyl-butan-1,4-diyl, 2-Ethyl-butan-2,3-diyl, 2,2-Dimethyl-butan-1,1-diyl, 2,2-Dimethyl-butan-1,3-diyl, 2,2-Dimethyl-butan-1,4-diyl, 1-Isopropyl-propan-1,1-diyl, 1-Isopropyl-propan-1,2-diyl, 1-Isopropyl-propan-1,3-diyl, 2-Isopropyl-propan-1,1-diyl, 2-Isopropyl-propan-1,2-diyl, 2-Isopropyl-propan-1,3-diyl, 1,2,3-Trimethyl-propan-1,1-diyl, 1,2,3-Trimethyl-propan-1,2-diyl oder 1,2,3-Trimethyl-propan-1,3-diyl;
- C₂-C₆-Alkendiyl: Ethen-1,1-diyl, Ethen-1,2-diyl, 1-Propen-1,1-diyl, 1-Propen-1,2-diyl, 1-Propen-1,3-diyl, 2-Propen-1,1-diyl, 2-Propen-1,2-diyl, 2-Propen-1,3-diyl, 1-Buten-1,1-diyl, 1-Buten-1,2-diyl, 1-Buten-1,3-diyl, 1-Buten-1,4-diyl, 2-Buten-1,1-diyl, 2-Buten-1,2-diyl, 2-Buten-1,3-diyl, 2-Buten-1,4-diyl, 3-Buten-1,1-diyl, 3-Buten-1,2-diyl, 3-Buten-1,3-diyl, 3-Buten-1,4-diyl, 1-Methyl-1-propen-1,2-diyl, 1-Methyl-1-propen-1,3-diyl, 1-Methyl-2-propen-1,1-diyl, 1-Methyl-2-propen-1,2-diyl, 1-Methyl-2-propen-1,3-diyl, 2-Methyl-1,1-propen-1,1-diyl, 2-Methyl-1-propen-1,3-dzyl, 3-Buten-1,1-diyl, 3-Buten-1,2-diyl, 3-Buten-1,3-diyl, 3-Buten-1,4-diyl, 1-Penten-1,1-diyl, 1-Penten-1,2-diyl, 1-Penten-1,3-diyl, 1-Penten-1,4-diyl, 1-Penten-1,5-diyl, 1-Hexen-1,1-diyl, 1-Hexen-1,2-diyl, 1-Hexen-1,3-diyl, 1-Hexen-1,4-diyl, 1-Hexen-1,5-diyl oder 1-Hexen-1,6-diyl;
- C₂-C₆-Alkadiendiyl: 1,3-Butadien-1,1-diyl, 1,3-Butadien-1,2-diyl, 1,3-Butadien-1,3-diyl, 1,3-Butadien-1,4-diyl, 1,3-Pen-tadien-1,1-diyl, 1,3-Pentadien-1,2-diyl, 1,3-Pentadien-1,3-diyl, 1,3-Pentadien-1,4-diyl, 1,3-Pentadien-1,5-diyl, 2,4-Pentadien-1,1-diyl, 2,4-Pentadien-1,2-diyl, 2,4-Pentadien-1,3-diyl, 2,4-Pentadien-1,4-diyl, 2,4-Pentadien-1,5-diyl, 1-Methyl-1,3-butadien-1,4-diyl, 1,3-Hexadien-1,1-diyl, 1,3-Hexadien-1,2-diyl, 1,3-Hexadien-1,3-diyl, 1,3-Hexadien-1,4-diyl, 1,3-Hexadien-1,5-diyl, 1,3-Hexadien-1,6-diyl, 1-Methyl-1,3-pentadien-1,2-diyl, 1-Methyl-1,3-pentadien-1,3-diyl, 1-Methyl-1,3-pentadien-1,4-diyl oder 1-Methyl-1,3-pentadien-1,5-diyl;
- C₂-C₆-Alkindiyl: Ethin-1,2-diyl, 1-Propin-1,3-diyl, 2-Propin-1,1-diyl, 2-Propin-1,3-diyl, 1-Butin-1,3-diyl, 1-Butin-1,4-diyl, 2-Butin-1,1-diyl, 2-Butin-1,4-diyl, 1-Methyl-2-propin-1,1-diyl, 1-Methyl-2-propin-1,3-diyl, 1-Pentin-1,3-diyl, 1-Pentin-1,4-diyl, 1-Pentin-1,5-diyl, 2-Pentin-1,1-diyl, 2-Pentin-1,4-diyl, 2-Pentin-1,5-diyl, 3-Pentin-1,1-diyl, 3-Pentin-1,2-diyl, 3-Pentin-1,5-diyl, 4-Pentin-1,1-diyl, 4-Pentin-1,2-diyl, 4-Pentin-1,3-diyl, 4-Pentin-1,5-diyl, 1-Hexin-1,3-diyl, 1-Hexin-1,4-diyl, 1-Hexin-1,5-diyl, 1-Hexin-1,6-diyl, 2-Hexin-1,1-diyl, 2-Hexin-1,4-diyl, 2-Hexin-1,5-diyl, 2-Hexin-1,6-diyl, 3-Hexin-1,1-diyl, 3-Hexin-1,2-diyl, 3-Hexin-1,5-diyl, 3-Hexin-1,6-diyl, 4-Hexin-1,1-diyl, 4-Hexin-1,2-diyl, 4-Hexin-1,3-diyl, 4-Hexin-1,6-diyl, 5-Hexin-1,1-diyl, 5-Hexin-1,2-diyl, 5-Hexin-1,3-diyl, 5-Hexin-1,4-diyl oder 5-Hexin-1,6-diyl;
- C₃-C₆-Cycloalkyl, sowie die Cycloalkylteile von C₃-C₆-Cycloalkylamino und C₃-C₆-Cycloalkylcarbonyl: z B. Cyclopropyl, Cyclobutyl, Cyclopentyl oder Cyclohexyl;
- Unter Aryl versteht man carbocyclische aromatische Verbindungen wie beispielsweise Phenyl oder Naphthyl;
- Unter einem 3- bis 7-gliedrigen Heterocyclyl versteht man einen gesättigten, partiell gesättigten oder ungesättigten 3-, 4-, 5-, 6- oder 7-gliedrigen, heterocyclischen Ring, der ein, zwei, drei oder vier gleiche oder verschiedene Heteroatome, ausgewählt aus folgender Gruppe: Sauerstoff, Schwefel oder Stickstoff, enthält, also z. B.
   C-gebundene 5-gliedrige Ringe wie:
   Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3 yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 2-Furyl, 3-Furyl, 2-Thienyl, 3-Thienyl, Pyrrol-2-yl, Pyrrol-3-yl, Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydro-isothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4 yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5 yl, 2,5-Dihydro-1H-pyrazol-3-yl, 2,5-Dihydro-1H pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4 yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4 yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2 yl, 1,3-Dioxol-4-yl, 1,3-Dithiol-2-yl, 1,3-Dithiol-4-yl, 1,3-Oxathiol-2-yl, 1,3-Oxathiol-4 yl, 1,3-Oxathiol-5-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadiazolin-3 yl, 1,2,4-Δ²-Oxadiazolin-5-yl, 1,2,4-Δ³-Oxadiazolin-3-yl, 1,2,4-Δ³-oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,3,2-Dioxathiolan-4-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-DithiazoL-5 yl, 2H-1,3,4-Dithiazol-5 yl, 2H-1,3,4-Oxathiazol-5-yl, 1,2,3-Oxadiazol-4-yl, 1,2,3-Oxadiazol-5-yl, 1,2,4-Oxadiazol-3-yl, 1,2,4-Oxadiazol-5 yl, 1,3,4-Oxadiazol-2 yl, 1,2,3-Thiadiazol-4 yl, 1,2,3-Thiadiazol-5-yl, 1,2,4-Thiadiazol-3-yl, 1,2,4-Thiadiazol-5-yl, 1,3,4-Thiadiazol-2-yl, 1,2,3-Triazol-4-yl, 1,2,4-Triazol-3-yl, Tetrazol-5-yl
   C-gebundene 6-gliedrige Ringe wie:
   Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5 yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, Pyridin-2-yl, Pyridin-3-yl, Pyridin-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-oxathian-2-yl, 1,3-Oxathian-4-yl, 1,3-Oxathian-5 yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl, 2H-5, 6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5 Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6 yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-2,4-4xazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5 yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl, 3,4-Dihydropyrimidin-6-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl oder 1,2,4,5-Tetra-zin-3-yl;
   N-gebundene 5-gliedrige Ringe wie:
   Tetrahydropyrrol-1-yl, 2,3-Dihydro-1H-pyrrol-1-yl, 2,5-Dihydro-1H-pyrrol-1-yl, Pyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, Pyrazol-1-yl, Imidazol-1-yl, 1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4 yl, 1,2,4-Δ⁵-Thiadiazolin-2 yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2, 4-Δ³-Triazolin-1-yl, 1,2,4-Δ¹-Triazolin-4 yl, 1,2,3-Triazol-1-yl, 1,2,4-Triazol-1-yl, Tetrazol-1-yl;
   N-gebundene 6-gliedrige Ringe wie:
   Piperidin-1-yl, 1,2,3,4-Tetrahydropyridin-1-yl, 1,2,5,6-Tetrahydropyridin-1-yl, 1,4-Dihydropyridin-1-yl, 1,2-Dihydropyridin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl (Morpholinyl), Tetrahydro-1,2-oxazin-2-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihydro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
   sowie N-gebundene cyclische Imide wie:
   Phthalsäureimid, Tetrahydrophthalsäureimid, Succinimid, Maleinimid, Glutarimid, 5-Oxo-triazolin-1-yl, 5-Oxo-1,3,4-oxadiazolin-4-yl oder 2,4-Dioxo-(1H,3H)-pyrimidin-3-yl;
wobei gegebenenfalls der Schwefel der genannten Heterocyclen zu S=O oder S(=O)₂ oxidiert sein kann
und wobei mit einem ankondensierten Phenylring oder mit einem C₃-C₆-Carbocyclus oder mit einem weiteren 5- bis 6-gliedrigen Heterocyclus ein bicyclisches Ringsystem ausgebildet werden kann.

Alle Phenylringe bzw. Heterocyclylreste sowie alle Phenylkomponenten in Phenoxy, Phenylalkyl, Phenylcarbonylalkyl, Phenylcarbonyl, Phenylalkenylcarbonyl, Phenoxycarbonyl, Phenyloxythiocarbonyl, Phenylaminocarbonyl und N-Alkyl-N-phenylaminocarbonyl, Phenylsulfonyl oder Phenoxysulfonyl bzw. Heterocyclylkomponenten in Heterocyclyloxy, Heterocyclylalkyl, Heterocyclylcarbonylalkyl, Heterocyclylcarbonyl, Heterocyclyloxythiocarbonyl, Heterocyclylalkenylcarbonyl, Heterocyclyloxycarbonyl, Heterocyclylaminocarbonyl, N-Alkyl-N-heterocyclylaminocarbonyl, Heterocyclylsulfonyl oder Heterocyclyloxysulfonyl sind, soweit nicht anders angegeben, vorzugsweise unsubstituiert oder tragen ein, zwei oder drei Halogenatome und/oder eine Nitrogruppe, einen Cyanorest und/oder einen oder zwei Methyl-, Trifluormethyl-, Methoxy- oder Trifluormethoxysubstituenten.

Im Hinblick auf die Verwendung der erfindungsgemäßen Verbindungen der Formel I als Herbizide haben die Variablen A, R¹ bis R¹⁵ vorzugsweise folgende Bedeutungen, und zwar jeweils für sich alleine oder in Kombination:
- A: S, SO₂, O oder NR⁶ mit den für R⁶ zuvor genannten Bedeutungen. Vorzugsweise ist R⁶ nicht Wasserstoff. Insbesondere steht R⁶ für C₁-C₄-Alkyl und ganz besonders bevorzugt für Methyl. In einer ersten Gruppe besonders bevorzugter Verbindungen I steht A für O oder NR⁶, worin R⁶ die zuvor genannten Bedeutungen aufweist. In einer anderen Gruppe besonders bevorzugter Verbindungen steht A für S oder SO₂ steht.
- R¹: Halogen, C₁-C₄-Alkyl, C₁-C₄-Alkoxy, insbesondere Methyl, Methoxy oder Chlor.
- R²: die vorgenannten Bedeutungen, insbesondere C₁-C₄-Alkyl, C₁-C₄-Halogenalkyl, Hydroxy, C₁-C₄-Alkoxy, C₁-C₄-Alkylsulfonyl, C₁-C₄-Halogenalkylsulfonyl, Hydroxycarbonyl-C₁-C₄-alkyl, C(=N-O(-C₁-C₄-Alkyl))-(C₁-C₄-Alkyl), NH₂, C₁-C₄-Alkylamino, Di-(C₁-C₄-alkyl)amino, Phenyl, Phenylcarbonyl oder Benzyl, wobei die Phenylringe der drei letztgenannten Substituenten durch ein oder zwei Halogenatome, Methoxy- oder Methylgruppen substituiert sein können, Cyano, C₁-C₄-Alkylcarbonyl, C₁-C₄-Halogenalkylcarbonyl, C₁-C₄-Alkoxycarbonyl, C₁-C₄-Alkoxycarbonyl-C₁-C₄-alkyl, C₃-C₆-Alkenyl, C₃-C₆-Alkinyl, 1,3-Dioxolan-2-yl, 1,3-Dithiolan-2-yl, 1,3-Dioxan-2-yl, 1,3-Dithian-2-yl, Oxazolin-2-yl, Oxazolidin-2-yl, 4,5-Dihydroisoxazol-3-yl, 4,5-Dihydroisoxazol-4-yl, Isoxazol-3-yl, Isoxazol-4-yl, Methyl- oder Ethyl, das durch einen der vorgenannten Heterocyclen substistuiert ist wie in (1,3-Dioxolan-2-yl)methyl, (1,3-Dithiolan-2-yl)methyl, (1,3-Dioxan-2-yl)methyl, (1,3-Dithian-2-yl)methyl, (Oxazolin-2-yl)methyl, (Oxazolidin-2-yl)methyl, (4,5-Dihydroisoxazol-3-yl)methyl, (4,5-Dihydroisoxazol-4-yl)methyl, (Isoxazol-3-yl)methyl, (Isoxazol-4-yl)methyl, 2-(1,3-Dioxolan-2-yl)ethyl, 2-(1,3-Dithiolan-2-yl)ethyl, 2-(1,3-Dioxan-2-yl)ethyl, 2-(1,3-Dithian-2-yl)ethyl, 2-(Oxazolin-2-yl)ethyl, 2-(Oxazolidin-2-yl)ethyl, 2-(4,5-Dihydroisoxazol-3-yl)ethyl, (4,5-Dihydroisoxazol-4-yl)ethyl, 2-(Isoxazol-3-yl)ethyl, 2-(Isoxazol-4-yl)ethyl, wobei die vorgenannten Heterocyclen 1-, 2- oder 3-fach mit C₁-C₄-Alkyl, insbesondere Methyl, substituiert sein können;
speziell C₁-C₄-Alkyl, C₃-C₆-Alkenyl, Methoxymethyl, CF₃, CHF₂, CN, OH, OCH₃, NH₂, NHCH₃, N(CH₃)₂, C₁-C₄-Alkoxycarbonyl, Acetyl, Trifluoracetyl, C(=NOCH₃)CH₃, SO₂CH₃, SO₂CF₃, CH₂CO₂H, C₁-C₄-Alkoxycarbonyl-C₁-C₄-alkyl, Cyclopropyl, Cyclopentyl, Cyclohexyl, 4,5-Dihydroisoxazol-3-yl, 4,5-Dihydroisoxazol-4-yl, Isoxazol-3-yl, Isoxazol-4-yl, wobei die vorgenannten Heterocyclen einfach mit Methyl substituiert sein können, Phenyl, Benzyl, Benzoyl, 2-Pyridyl.
Besonders bevorzugt steht R² für C₁-C₄-Alkyl oder C₃-C₆-Alkenyl. Besonders bevorzugt sind auch solche Verbindungen I, worin R² für C₃-C₆-Alkinyl, (1,3-Dioxolan-2-yl)methyl, 2-(1,3-Dioxolan-2-yl)ethyl oder für Benzyl steht, worin der Phenylring unsubstituiert sein kann oder 1 oder 2 Substituenten, ausgewählt unter Halogen, Methyl oder Methoxy, aufweisen kann.
- R³: C₁-C₄-Alkyl, Halogen und insbesondere Wasserstoff.

In ganz besonders bevorzugten Verbindungen I steht A für O oder S, R¹ für Methyl, R³ für Wasserstoff und R² für einen von Wasserstoff verschiedenen Substituenten, insbesondere für einen der oben als bevorzugt angegebenen Substituenten und speziell für C₁-C₆-Alkyl, C₃-C₆-Alkinyl, C₃-C₆-Alkenyl, (1,3-Dioxolan-2-yl)methyl, 2-(1,3-Dioxolan-2-yl)ethyl oder für Benzyl, worin der Phenylring unsubstituiert sein kann oder 1 oder 2 Substituenten, ausgewählt unter Halogen, Methyl oder Methoxy, aufweisen kann.
- R⁷: Hydroxy, Halogen, OR¹⁰, SR¹⁰, SO₂R¹¹, OSO₂R¹¹, NR¹⁴R¹⁵, ONR¹⁴R¹⁵ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy; insbesondere Hydroxy, OR¹⁰ und OSO₂R¹¹, speziell Hydroxy, C₁-C₄-Alkyloxy, O-CH₂-Phenyl, Phenylcarbonyloxy, 2-, 3- oder 4-Fluorphenylcarbonyloxy, Cyclopropylcarbonyloxy, C₁-C₄-Sulfonyloxy, Phenylsulfonyloxy und 2-, 3- oder 4-Methylphenylsulfonyloxy;
- R⁸: C₁-C₆-Alkyl, C₃-C₆-Cycloalkyl, insbesondere C₁-C₄-Alkyl und Cyclopropyl; und
- R⁹: Wasserstoff oder C₁-C₄-Alkyl, insbesondere Wasserstoff oder C₁-C₄-Alkyl.

Eine wichtige Gruppe von Verbindungen sind solche, in denen R⁸ für eine C₃-C₆-Cycloalkylgruppe steht, insbesondere wenn A für S, SO oder SO₂ steht.

Unter den als bevorzugt angegebenen Pyrazolyl-Derivaten der allgemeinen Formel I sind wiederum solche Verbindungen bevorzugt, worin Pz in Formel I für eine Gruppe der allgemeinen Formel IIa steht. Hierunter sind wiederum Verbindungen der allgemeinen Formel I besonders bevorzugt, worin die Variablen R⁷, R⁸ und R⁹ in Formel IIa für sich alleine, und besonders bevorzugt in Kombination miteinander, die folgenden Bedeutungen aufweisen:
- R⁷: Hydroxy, Halogen, OR¹⁰, SR¹⁰, SO₂R¹¹, OSO₂R¹¹, NR¹⁴R¹⁵, ONR¹⁴R¹⁵ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy, insbesondere Hydroxy, OR¹⁰ und OSO₂R¹¹;
- R⁸: C₁-C₄-Alkyl, C₃-C₆-Cycloalkyl; insbesondere Cyclopropyl;
- R⁹: Wasserstoff oder C₁-C₄-Alkyl.

Unter den Verbindungen der allgemeinen Formel I, worin Pz für einen Pyrazolylrest der allgemeinen Formel IIa steht, sind solche Verbindungen ganz besonders bevorzugt, worin die Variablen R⁷, R⁸ und R⁹ gemeinsam die folgenden Bedeutungen aufweisen:
- R⁷: für Hydroxy, C₁-C₄-Alkyloxy, Acetoxy, O-CH₂-Phenyl, Phenylcarbonyloxy, 2-, 3- oder 4-Fluorphenylcarbonyloxy, Cyclopropylcarbonyloxy, C₁-C₄-Sulfonyloxy, Phenylsulfonyloxy und 2-, 3- oder 4-Methylphenylsulfonyloxy;
- R⁸: für C₁-C₄-Alkyl oder Cyclopropyl und
- R⁹: für Wasserstoff oder C₁-C₄-Alkyl stehen.

Beispiele für besonders bevorzugte Reste der allgemeinen Formel IIa sind die in Tabelle 1 angegebenen Reste IIa1 bis IIa90.

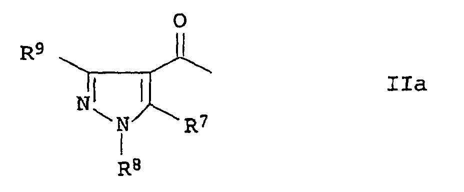

**Tabelle 1**

| IIa | R⁷ | R⁸ | R⁹ |
|---|---|---|---|
| IIa1 | OH | CH₃ | H |
| IIa2 | OCH₃ | CH₃ | H |
| IIa3 | OCH₂-C₆H₅ | CH₃ | H |
| IIa4 | OC(O)CH₃ | CH₃ | H |
| IIa5 | OC(O)C₆H₅ | CH₃ | H |
| IIa6 | OC(O)-(3-C₆H₄F) | CH₃ | H |
| IIa7 | OS(O)₂CH₃ | CH₃ | H |
| IIa8 | OS(O)₂-(4-C₆H₄CH₃) | CH₃ | H |
| IIa9 | OH | C₂H₅ | H |
| IIa10 | OCH₃ | C₂H₅ | H |
| IIa11 | OCH₂-C₆H₅ | C₂H₅ | H |
| IIa12 | OC(O)CH₃ | C₂H₅ | H |
| IIa13 | OC(O)C₆H₅ | C₂H₅ | H |
| IIa14 | OC(O)-(3-C₆H₄F) | C₂H₅ | H |
| IIa15 | OS(O)₂CH₃ | C₂H₅ | H |
| IIa16 | OS(O)₂-(4-C₆H₄CH₃) | C₂H₅ | H |
| IIa17 | OH | i-C₃H₇ | H |
| IIa18 | OCH₃ | i-C₃H₇ | H |
| IIa19 | OCH₂-C₆H₅ | i-C₃H₇ | H |
| IIa20 | OC(O)CH₃ | i-C₃H₇ | H |
| IIa21 | OC(O)C₆H₅ | i-C₃H₇ | H |
| IIa22 | OC(O)-(3-C₆H₄F) | i=C₃H₇ | H |
| IIa23 | OS(O)₂CH₃ | i-C₃H₇ | H |
| IIa24 | OS(O)₂-(4-C₆H₄CH₃) | i-C₃H₇ | H |
| IIa25 | OH | t-C₄H₉ | H |
| IIa26 | OCH₃ | t-G₄H₉ | H |
| IIa27 | OCH₂-C₆H₅ | t-C₄H₉ | H |
| IIa28 | OC(O)CH₃ | t-C₄H₉ | H |
| IIa29 | OC(O)C₆H₅ | t-C₄H₉ | H |
| IIa30 | OC(O)-(3-C₆H₄F) | t-C₄H₉ | H |
| IIa31 | OS(O)₂CH₃ | t-C₄H₉ | H |
| IIa32 | OS(O)₂-(4-C₆H₄CH₃) | t-C₄H₉ | H |
| IIa33 | OH | CH₃ | CH₃ |
| IIa34 | OCH₃ | CH₃ | CH₃ |
| IIa35 | OCH₂-C₆H₅ | CH₃ | CH₃ |
| IIa36 | OC(O)CH₃ | CH₃ | CH₃ |
| IIa37 | OC(O)C₆H₅ | CH₃ | CH₃ |
| IIa38 | OC(O)-(3-C₆H₄F) | CH₃ | CH₃ |
| IIa39 | OS(O)₂CH₃ | CH₃ | CH₃ |
| IIa40 | OS(O)₂-(4-C₆H₄CH₃) | CH₃ | CH₃ |
| IIa41 | OH | C₂H₅ | CH₃ |
| IIa42 | OCH₃ | C₂H₅ | CH₃ |
| IIa43 | OCH₂-C₆H₅ | C₂H₅ | CH₃ |
| IIa44 | OC(O)CH₃ | C₂H₅ | CH₃ |
| IIa45 | OC(O)C₆H₅ | C₂H₅ | CH₃ |
| IIa46 | OC(O)-(3-C₆H₄F) | C₂H₅ | CH₃ |
| IIa47 | OS(O)₂CH₃ | C₂H₅ | CH₃ |
| IIa48 | OS(O)₂-(4-C₆H₄CH₃) | C₂H₅ | CH₃ |
| IIa49 | OH | i-C₃H₇ | CH₃ |
| IIa50 | OCH₃ | i-C₃H₇ | CH₃ |
| IIa51 | OCH₂-C₆H₅ | i-C₃H₇ | CH₃ |
| IIa52 | OC(O)CH₃ | i-C₃H₇ | CH₃ |
| IIa53 | OC(O)C₆H₅ | i-C₃H₇ | CH₃ |
| IIa54 | OC(O)-(3-C₆H₄F) | i-C₃H₇ | CH₃ |
| IIa55 | OS(O)₂CH₃ | i-C₃H₇ | CH₃ |
| IIa56 | OS(O)₂-(4-C₆H₄CH₃) | i-C₃H₇ | CH₃ |
| IIa57 | OH | t-C₄H₉ | CH₃ |
| IIa58 | OCH₃ | t-C₄H₉ | CH₃ |
| IIa59 | OCH₂-C₆H₅ | t-C₄H₉ | CH₃ |
| IIa60 | OC(O)CH₃ | t-C₄H₉ | CH₃ |
| IIa61 | OC(O)C₆H₅ | t-C₄H₉ | CH₃ |
| IIa62 | OC(O)-(3-C₆H₄F) | t-C₄H₉ | CH₃ |
| IIa63 | OS(O)₂CH₃ | t-C₄H₉ | CH₃ |
| IIa64 | OS(O)₂-(4-C₆H₄CH₃) | t-C₄H₉ | CH₃ |
| IIa65 | OH | c-C₃H₅ | CH₃ |
| IIa66 | OCH₃ | C-C₃H₅ | CH₃ |
| IIa67 | OCH₂-C₆H₅ | C-C₃H₅ | CH₃ |
| IIa68 | OC(O)CH₃ | C-C₃H₅ | CH₃ |
| IIa69 | OC(O)C₆H₅ | C-C₃H₅ | CH₃ |
| IIa70 | OC(O)-(3-C₆H₄F) | c-C₃H₅ | CH₃ |
| IIa71 | OS(O)₂CH₃ | c-C₃H₅ | CH₃ |
| IIa72 | OS(O)₂-(4-C₆H₄CH₃) | c-C₃H₅ | CH₃ |
| IIa73 | OH | c-C₃H₅ | H |
| IIa74 | OCH₃ | c-C₃H₅ | H |
| IIa75 | OCH₂-C₆H₅ | c-C₃H₅ | H |
| IIa76 | OC(O)CH₃ | c-C₃H₅ | H |
| IIa77 | OC(O)C₆H₅ | c-C₃H₅ | H |
| IIa78 | OC(O)-(3-C₆H₄F) | c-C₃H₅ | H |
| IIa79 | OS(O)₂CH₃ | c-C₃H₅ | H |
| IIa80 | OS(O)₂-(4-C₆H₄CH₃) | c-C₃H₅ | H |
| IIa81 | OC(O)c-C₃H₅ | CH₃ | H |
| IIa82 | OC(O)-c-C₃H₅ | C₂H₅ | H |
| IIa83 | OC(O)-c-C₃H₅ | i-C₃H₇ | H |
| IIa84 | OC(O)-c-C₃H₅ | t-C₄H₉ | H |
| IIa85 | OC(O)-c-C₃H₅ | c-C₃H₅ | H |
| IIa86 | OC(O)-c-C₃H₅ | CH₃ | CH₃ |
| IIa87 | OC(O)-c-C₃H₅ | C₂H₅ | CH₃ |
| IIa88 | OC(O)-c-C₃H₅ | i-C₃H₇ | CH₃ |
| IIa89 | OC(O)-c-C₃H₅ | t-C₄H₉ | CH₃ |
| IIa90 | OC(O)-c-C₃H₅ | c-C₃H₅ | CH₃ |
| i-C₃H₇: Isopropyl | | | |
| c-C₃H₅: Cyclopropyl | | | |
| t-C₄H₉: tertiär-Butyl | | | |
| C₆H₅: Phenyl | | | |
| 3-C₆H₄F: 3-Fluorphenyl | | | |
| 4-C₆H₄CH₃: 4-Methylphenyl | | | |

Beispiele für erfindungsgemäße besonders bevorzugte Verbindungen der allgemeinen Formel I sind die in den nachstehend angegebenen Tabellen 2 bis 25 angegebenen Benzazolonylcarbonylpyrazole I, worin R¹, R², R³ und A jeweils die in einer Zeile der Tabelle A angegebenen Bedeutungen aufweisen.
- Tabelle 2: Verbindungen I-1a.1 bis I-1a.3960 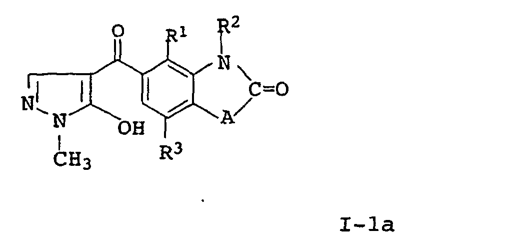
- Tabelle 3: Verbindungen I-1b.1 bis I-1b.3960 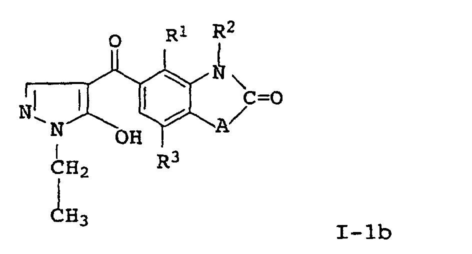
- Tabelle 4: Verbindungen I-1c.1 bis I-1c.3960 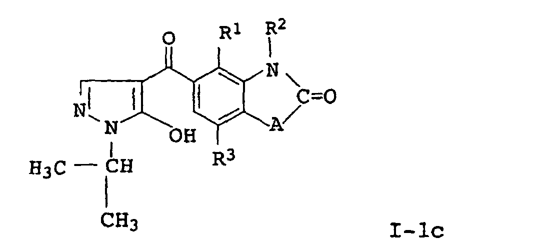
- Tabelle 5: Verbindungen I-1d.1 bis I-1d.3960 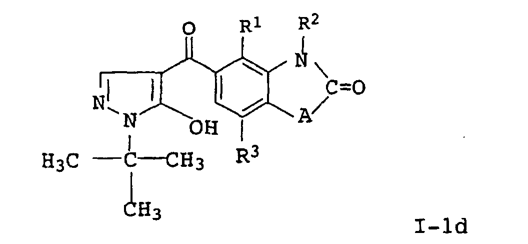
- Tabelle 6: Verbindungen I-1e.1 bis I-1e.3960 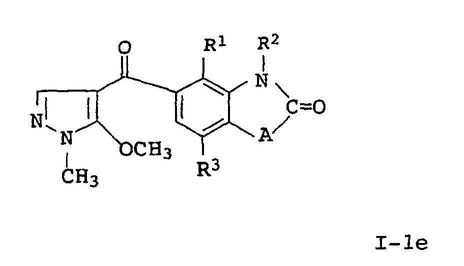
- Tabelle 7: Verbindungen I-1f.1 bis I-1f.3960 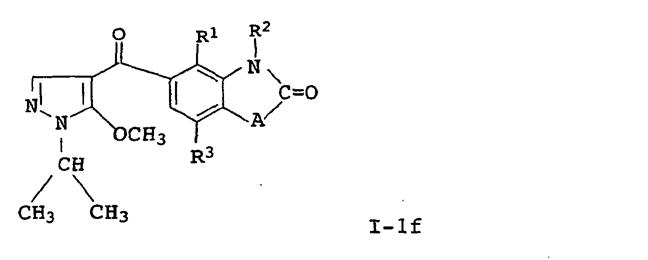
- Tabelle 8: Verbindungen I-1g.1 bis I-1g.3960 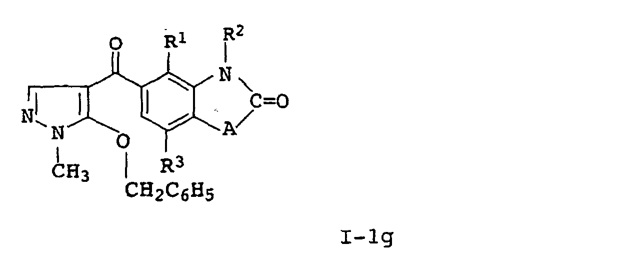
- Tabelle 9: Verbindungen I-1h.1 bis I-1h.3960 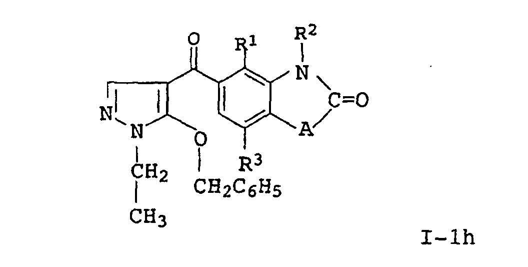
- Tabelle 10: Verbindungen I-1i.1 bis I-1i.3960 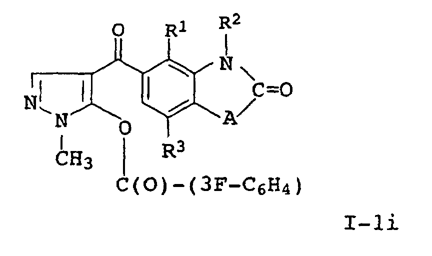
- Tabelle 11: Verbindungen I-1k.1 bis I-1k.3960 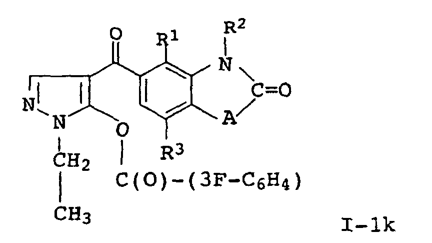
- Tabelle 12: Verbindungen I-1l.1 bis I-1l.3960 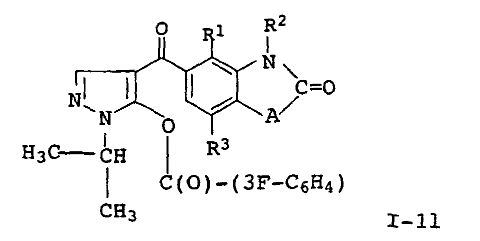
- Tabelle 13: Verbindungen I-1m.1 bis I-1m.3960 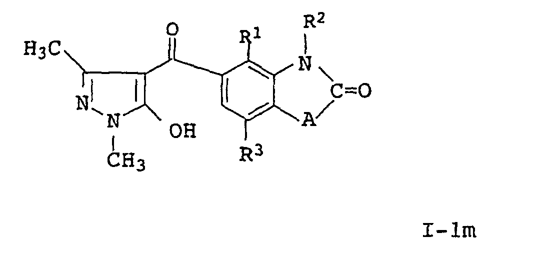
- Tabelle 14: Verbindungen I-1n.1 bis I-1n.3960 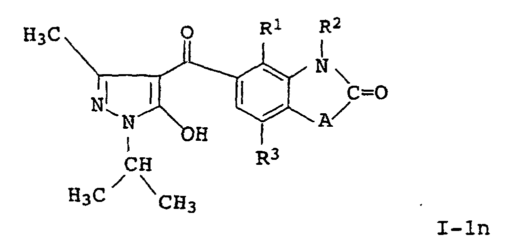
- Tabelle 15: Verbindungen I-1o.1 bis I-1o.3960 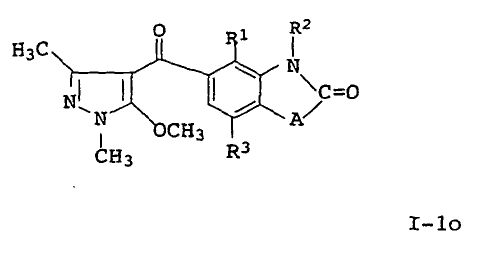
- Tabelle 16: Verbindungen I-1p.1 bis I-1p.3960 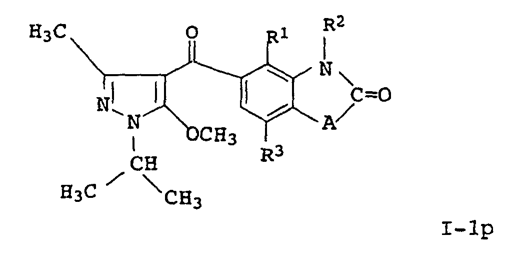
- Tabelle 17: Verbindungen I-1q.1 bis I-1q.3960 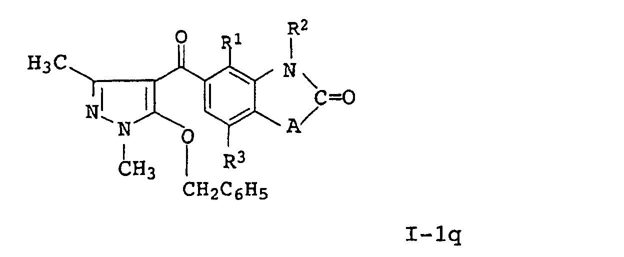
- Tabelle 18: Verbindungen I-1r.1 bis I-1r.3960 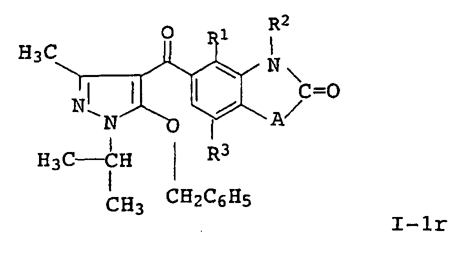
- Tabelle 19: Verbindungen I-1s.1 bis I-1s.3960 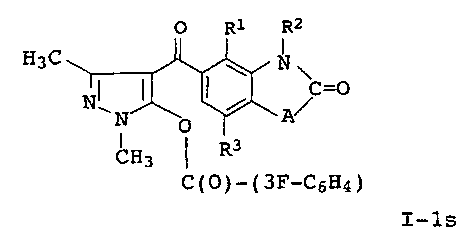
- Tabelle 20: Verbindungen I-1t.1 bis I-1t.3960 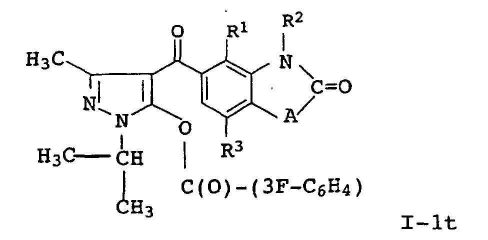
- Tabelle 21: Verbindungen I-1u.1 bis I-1u.3960 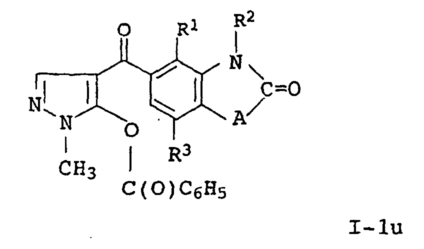
- Tabelle 22: Verbindungen I-1v.1 bis I-1v.3960 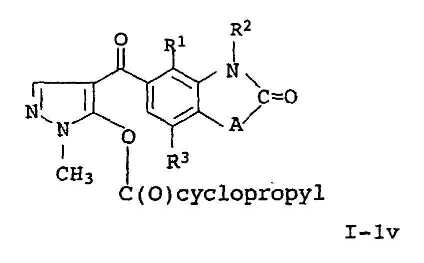
- Tabelle 23: Verbindungen I-1w.1 bis I-1w.3960 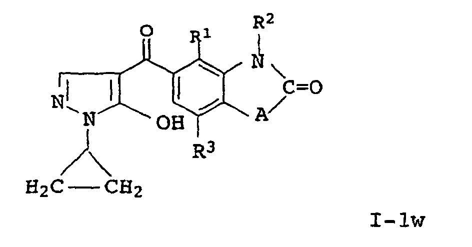
- Tabelle 24: Verbindungen I-1x.1 bis I-1x.3960 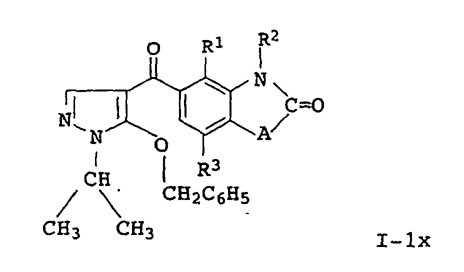
- Tabelle 25: Verbindungen I-1y.1 bis I-1y.3960

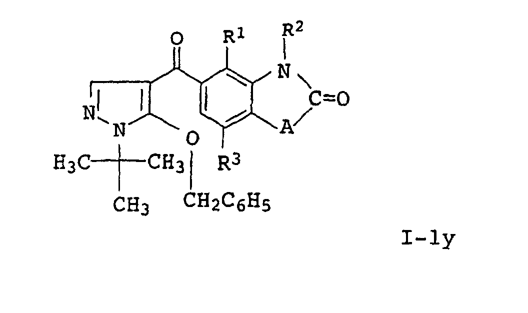

**Tabelle A:**

| Besonders bevorzugte Kombinationen von R¹, R², R³ und A | | | | |
|---|---|---|---|---|
| Nr. | R¹ | R² | R³ | A |
| 2 | CH₃ | CH₃ | H | O |
| 3 | CH₃ | C₂H₅ | H | O |
| 4 | CH₃ | n-C₃H₇ | H | O |
| 5 | CH₃ | i-C₃H₇ | H | O |
| 6 | CH₃ | n-C₄H₉ | H | O |
| 7 | CH₃ | i-C₄H₉ | H | O |
| 8 | CH₃ | s-C₄H₉ | H | O |
| 9 | CH₃ | t-C₄H₉ | H | O |
| 10 | CH₃ | CH₂OCH₃ | H | O |
| 11 | CH₃ | CF₃ | H | O |
| 12 | CH₃ | CF₂H | H | O |
| 13 | CH₃ | CN | H | O |
| 14 | CH₃ | OH | H | O |
| 15 | CH₃ | OCH₃ | H | O |
| 16 | CH₃ | NH₂ | H | O |
| 17 | CH₃ | NHCH₃ | H | O |
| 18 | CH₃ | N(CH₃)₂ | H | O |
| 19 | CH₃ | CO₂CH₃ | H | O |
| 20 | CH₃ | CO₂C₂H₅ | H | O |
| 21 | CH₃ | C(O)CH₃ | H | O |
| 22 | CH₃ | C(O)CF₃ | H | O |
| 23 | CH₃ | C(=NOCH₃)CH₃ | H | O |
| 24 | CH₃ | SO₂CH₃ | H | O |
| 25 | CH₃ | SO₂CF₃ | H | O |
| 26 | CH₃ | CH₂CO₂H | H | O |
| 27 | CH₃ | CH₂COOCH₃ | H | O |
| 28 | CH₃ | CH₂COOC₂H₅ | H | O |
| 29 | CH₃ | Prop-1-en-3-yl | H | O |
| 30 | CH₃ | trans-But-2-en-1-yl | H | O |
| 31 | CH₃ | cis-But-2-en-1-yl | H | O |
| 32 | CH₃ | cis-3-Methyl-but-2-en-1-yl | H | O |
| 33 | CH₃ | Cyclopropyl | H | O |
| 34 | CH₃ | Cyclopentyl | H | O |
| 35 | CH₃ | Cyclohexyl | H | O |
| 36 | CH₃ | 4,5-Dihydroisoxazol-3-yl | H | O |
| 37 7 | CH₃ | 4-Methyl-4,5-dihydroisoxazol-3-yl | H | O |
| 38 | CH₃ | Isoxazol-3-yl | H | O |
| 39 | CH₃ | 4-Methylisoxazol-3-yl | H | O |
| 40 | CH₃ | 4,5-Dihydroisoxazol-4-yl | H | O |
| 41 | CH₃ | 3-Methyl-4,5-dihydroisoxazol-4-yl | H | O |
| 42 | CH₃ | Isoxazol-4-yl | H | O |
| 43 | CH₃ | 3-Methylisoxazol-4-yl | H | O |
| 44 | CH₃ | Phenyl | H | O |
| 45 | CH₃ | Benzyl | H | O |
| 46 | CH₃ | Benzoyl | H | O |
| 47 | CH₃ | 2-Pyridyl | H | O |
| 49 | CH₃ | CH₃ | CH₃ | O |
| 50 | CH₃ | C₂H₅ | CH₃ | O |
| 51 | CH₃ | n-C₃H₇ | CH₃ | O |
| 52 | CH₃ | i-C₃H₇ | CH₃ | O |
| 53 | CH₃ | n-C₄H₉ | CH₃ | O |
| 54 | CH₃ | i-C₄H₉ | CH₃ | O |
| 55 | CH₃ | s-C₄H₉ | CH₃ | O |
| 56 | CH₃ | t-C₄H₉ | CH₃ | O |
| 57 | CH₃ | CH₂OCH₃ | CH₃ | O |
| 58 | CH₃ | CF₃ | CH₃ | O |
| 59 | CH₃ | CF₂H | CH₃ | O |
| 60 | CH₃ | CN | CH₃ | O |
| 61 | CH₃ | OH | CH₃ | O |
| 62 | CH₃ | OCH₃ | CH₃ | O |
| 63 | CH₃ | NH₂ | CH₃ | O |
| 64 | CH₃ | NHCH₃ | CH₃ | O |
| 65 | CH₃ | N(CH₃)₂ | CH₃ | O |
| 66 | CH₃ | CO₂CH₃ | CH₃ | O |
| 67 | CH₃ | CO₂C₂H₅ | CH₃ | O |
| 68 | CH₃ | C(O)CH₃ | CH₃ | O |
| 69 | CH₃ | C(O)CF₃ | CH₃ | O |
| 70 | CH₃ | C(=NOCH₃)CH₃ | CH₃ | O |
| 71 | CH₃ | SO₂CH₃ | CH₃ | O |
| 72 | CH₃ | SO₂CF₃ | CH₃ | O |
| 73 | CH₃ | CH₂CO₂H | CH₃ | O |
| 74 | CH₃ | CH₂COOCH₃ | CH₃ | O |
| 75 | CH₃ | CH₂COOC₂H₅ | CH₃ | O |
| 76 | CH₃ | Prop-1-en-3-yl | CH₃ | O |
| 77 | CH₃ | trans-But-2-en-1-yl | CH₃ | O |
| 78 | CH₃ | cis-But-2-en-1-yl | CH₃ | O |
| 79 | CH₃ | cis-3-Methyl-but-2-en-1-yl | CH₃ | O |
| 80 | CH₃ | Cyclopropyl | CH₃ | O |
| 81 | CH₃ | Cyclopentyl | CH₃ | O |
| 82 | CH₃ | Cyclohexyl | CH₃ | O |
| 83 | CH₃ | 4,5-Dihydroisoxazol-3-yl | CH₃ | O |
| 84 | CH₃ | 4-Methyl-4,5-dihydroisoxazol-3-yl | CH₃ | O |
| 85 | CH₃ | Isoxazol-3-yl | CH₃ | O |
| 86 | CH₃ | 4-Methylisoxazol-3-yl | CH₃ | O |
| 87 | CH₃ | 4,5-Dihydroisoxazol-4-yl | CH₃ | O |
| 88 | CH₃ | 3-Methyl-4,5-dihydroisoxazol-4-yl | CH₃ | O |
| 89 | CH₃ | Isoxazol-4-yl | CH₃ | O |
| 90 | CH₃ | 3-Methylisoxazol-4-yl | CH₃ | O |
| 91 | CH₃ | Phenyl | CH₃ | O |
| 92 | CH₃ | Benzyl | CH₃ | O |
| 93 | CH₃ | Benzoyl | CH₃ | O |
| 94 | CH₃ | 2-Pyridyl | CH₃ | O |
| 96 | CH₃ | CH₃ | Cl | O |
| 97 | CH₃ | C₂H₅ | Cl | O |
| 98 | CH₃ | n-C₃H₇ | Cl | O |
| 99 | CH₃ | i-C₃H₇ | Cl | O |
| 100 | CH₃ | n-C₄H₉ | Cl | O |
| 101 | CH₃ | i-C₄H₉ | Cl | O |
| 102 | CH₃ | s-C₄H₉ | Cl | O |
| 103 | CH₃ | t-C₄H₉ | Cl | O |
| 104 | CH₃ | CH₂OCH₃ | Cl | O |
| 105 | CH₃ | CF₃ | Cl | O |
| 106 | CH₃ | CF₂H | Cl | O |
| 107 | CH₃ | CN | Cl | O |
| 108 | CH₃ | OH | Cl | O |
| 109 | CH₃ | OCH₃ | Cl | O |
| 110 | CH₃ | NH₂ | Cl | O |
| 111 | CH₃ | NHCH₃ | Cl | O |
| 112 | CH₃ | N(CH₃)₂ | Cl | O |
| 113 | CH₃ | CO₂CH₃ | Cl | O |
| 114 | CH₃ | CO₂C₂H₅ | Cl | O |
| 115 | CH₃ | C(O)CH₃ | Cl | O |
| 116 | CH₃ | C(O)CF₃ | Cl | O |
| 117 | CH₃ | C(=NOCH₃)CH₃ | Cl | O |
| 118 | CH₃ | SO₂CH₃ | Cl | O |
| 119 | CH₃ | SO₂CF₃ | Cl | O |
| 120 | CH₃ | CH₂CO₂H | Cl | O |
| 121 | CH₃ | CH₂COOCH₃ | Cl | O |
| 122 | CH₃ | CH₂COOC₂H₅ | Cl | O |
| 123 | CH₃ | Prop-1-en-3-yl | Cl | O |
| 124 | CH₃ | trans-But-2-en-1-yl | Cl | O |
| 125 | CH₃ | cis-But-2-en-1-yl | Cl | O |
| 126 | CH₃ | cis-3-Methyl-but-2-en-1-yl | Cl | O |
| 127 | CH₃ | Cyclopropyl | Cl | O |
| 128 | CH₃ | Cyclopentyl | Cl | O |
| 129 | CH₃ | Cyclohexyl | Cl | O |
| 130 | CH₃ | 4,5-Dihydroisoxazol-3-yl | Cl | O |
| 131 | CH₃ | 4-Methyl-4,5-dihydroisoxazol-3-yl | Cl | O |
| 132 | CH₃ | Isoxazol-3-yl | Cl | O |
| 133 | CH₃ | 4-Methylisoxazol-3-yl | Cl | O |
| 134 | CH₃ | 4,5-Dihydroisoxazol-4-yl | Cl | O |
| 135 | CH₃ | 3-Methyl-4,5-dihydroisoxazol-4-yl | Cl | O |
| 136 | CH₃ | Isoxazol-4-yl | Cl | O |
| 137 | CH₃ | 3-Methylisoxazol-4-yl | Cl | O |
| 138 | CH₃ | Phenyl | Cl | O |
| 139 | CH₃ | Benzyl | Cl | O |
| 140 | CH₃ | Benzoyl | Cl | O |
| 141 | CH₃ | 2-Pyridyl | Cl | O |
| 143 | Cl | CH₃ | H | O |
| 144 | Cl | C₂H₅ | H | O |
| 145 | Cl | n-C₃H₇ | H | O |
| 146 | Cl | i-C₃H₇ | H | O |
| 147 | Cl | n-C₄H₉ | H | O |
| 148 | Cl | i-C₄H₉ | H | O |
| 149 | Cl | s-C₄H₉ | H | O |
| 150 | Cl | t-C₄H₉ | H | O |
| 151 | Cl | CH₂OCH₃ | H | O |
| 152 | Cl | CF₃ | H | O |
| 153 | Cl | CF₂H | H | O |
| 154 | Cl | CN | H | O |
| 155 | Cl | OH | H | O |
| 156 | Cl | OCH₃ | H | O |
| 157 | Cl | NH₂ | H | O |
| 158 | Cl | NHCH₃ | H | O |
| 159 | Cl | N(CH₃)₂ | H | O |
| 160 | Cl | CO₂CH₃ | H | O |
| 161 | Cl | CO₂C₂H₅ | H | O |
| 162 | Cl | C(O)CH₃ | H | O |
| 163 | Cl | C(O)CF₃ | H | O |
| 164 | Cl | C(=NOCH₃)CH₃ | H | O |
| 165 | Cl | SO₂CH₃ | H | O |
| 166 | Cl | SO₂CF₃ | H | O |
| 167 | Cl | CH₂CO₂H | H | O |
| 168 | Cl | CH₂COOCH₃ | H | O |
| 169 | Cl | CH₂COOC₂H₅ | H | O |
| 170 | Cl | Prop-1-en-3-yl | H | O |
| 171 | Cl | trans-But-2-en-1-yl | H | O |
| 172 | Cl | cis-But-2-en-1-yl | H | O |
| 173 | Cl | cis-3-Methyl-but-2-en-1-yl | H | O |
| 174 | Cl | Cyclopropyl | H | O |
| 175 | Cl | Cyclopentyl | H | O |
| 176 | Cl | Cyclohexyl | H | O |
| 177 | Cl | 4,5-Dihydroisoxazol-3-yl | H | O |
| 178 | Cl | 4-Methyl-4,5-dihydroisoxazol-3-yl | H | O |
| 179 | Cl | Isoxazol-3-yl | H | O |
| 180 | Cl | 4-Methylisoxazol-3-yl | H | O |
| 181 | Cl | 4,5-Dihydroisoxazol-4-yl | H | O |
| 182 | Cl | 3-Methyl-4,5-dihydroisoxazol-4-yl | H | O |
| 183 | Cl | Isoxazol-4-yl | H | O |
| 184 | Cl | 3-Methylisoxazol-4-yl | H | O |
| 185 | Cl | Phenyl | H | O |
| 186 | Cl | Benzyl | H | O |
| 187 | Cl | Benzoyl | H | O |
| 188 | Cl | 2-Pyridyl | H | O |
| 190 | Cl | CH₃ | CH₃ | O |
| 191 | Cl | C₂H₅ | CH₃ | O |
| 192 | Cl | n-C₃H₇ | CH₃ | O |
| 193 | Cl | i-C₃H₇ | CH₃ | O |
| 194 | Cl | n-C₄H₉ | CH₃ | O |
| 195 | Cl | i-C₄H₉ | CH₃ | O |
| 196 | Cl | s-C₄H₉ | CH₃ | O |
| 197 | Cl | t-C₄H₉ | CH₃ | O |
| 198 | Cl | CH₂OCH₃ | CH₃ | O |
| 199 | Cl | CF₃ | CH₃ | O |
| 200 | Cl | CF₂H | CH₃ | O |
| 201 | Cl | CN | CH₃ | O |
| 202 | Cl | OH | CH₃ | O |
| 203 | Cl | OCH₃ | CH₃ | O |
| 204 | Cl | NH₂ | CH₃ | O |
| 205 | Cl | NHCH₃ | CH₃ | O |
| 206 | Cl | N(CH₃)₂ | CH₃ | O |
| 207 | Cl | CO₂CH₃ | CH₃ | O |
| 208 | Cl | CO₂C₂H₅ | CH₃ | O |
| 209 | Cl | C(O)CH₃ | CH₃ | O |
| 210 | Cl | C(O)CF₃ | CH₃ | O |
| 211 | Cl | C(=NOCH₃)CH₃ | CH₃ | O |
| 212 | Cl | SO₂CH₃ | CH₃ | O |
| 213 | Cl | SO₂CF₃ | CH₃ | O |
| 214 | Cl | CH₂CO₂H | CH₃ | O |
| 215 | Cl | CH₂COOCH₃ | CH₃ | O |
| 216 | Cl | CH₂COOC₂H₅ | CH₃ | O |
| 217 | Cl | Prop-1-en-3-yl | CH₃ | O |
| 218 | Cl | trans-But-2-en-1-yl | CH₃ | O |
| 219 | Cl | cis-But-2-en-1-yl | CH₃ | O |
| 220 | Cl | cis-3-Methyl-but-2-en-1-yl | CH₃ | O |
| 221 | Cl | Cyclopropyl | CH₃ | O |
| 222 | Cl | Cyclopentyl | CH₃ | O |
| 223 | Cl | Cyclohexyl | CH₃ | O |
| 224 | Cl | 4,5-Dihydroisoxazol-3-yl | CH₃ | O |
| 225 | Cl | 4-Methyl-4,5-dihydroisoxazol-3-yl | CH₃ | O |
| 226 | Cl | Isoxazol-3-yl | CH₃ | O |
| 227 | Cl | 4-Methylisoxazol-3-yl | CH₃ | O |
| 228 | Cl | 4,5-Dihydroisoxazol-4-yl | CH₃ | O |
| 229 | Cl | 3-Methyl-4,5-dihydroisoxazol-4-yl | CH₃ | O |
| 230 | Cl | Isoxazol-4-yl | CH₃ | O |
| 231 | Cl | 3-Methylisoxazol-4-yl | CH₃ | O |
| 232 | Cl | Phenyl | CH₃ | O |
| 233 | Cl | Benzyl | CH₃ | O |
| 234 | Cl | Benzoyl | CH₃ | O |
| 235 | Cl | 2-Pyridyl | CH₃ | O |
| 237 | Cl | CH₃ | Cl | O |
| 238 | Cl | C₂H₅ | Cl | O |
| 239 | Cl | n-C₃H₇ | Cl | O |
| 240 | Cl | i-C₃H₇ | Cl | O |
| 241 | Cl | n-C₄H₉ | Cl | O |
| 242 | Cl | i-C₄H₉ | Cl | O |
| 243 | Cl | s-C₄H₉ | Cl | O |
| 244 | Cl | t-C₄H₉ | Cl | O |
| 245 | Cl | CH₂OCH₃ | Cl | O |
| 246 | Cl | CF₃ | Cl | O |
| 247 | Cl | CF₂H | Cl | O |
| 248 | Ck | CN | Cl | O |
| 249 | Cl | OH | Cl | O |
| 250 | Cl | OCH₃ | Cl | O |
| 251 | Cl | NH₂ | Cl | O |
| 252 | Cl | NHCH₃ | Cl | O |
| 253 | Cl | N(CH₃)₂ | Cl | O |
| 254 | Cl | CO₂CH₃ | Cl | O |
| 255 | Cl | CO₂C₂H₅ | Cl | O |
| 256 | Cl | C(O)CH₃ | Cl | O |
| 257 | Cl | C(O)CF₃ | Cl | O |
| 258 | Cl | C(=NOCH₃)CH₃ | Cl | O |
| 259 | Cl | SO₂CH₃ | Cl | O |
| 260 | Cl | SO₂CF₃ | Cl | O |
| 261 | Cl | CH₂CO₂H | Cl | O |
| 262 | Cl | CH₂COOCH₃ | Cl | O |
| 263 | Cl | CH₂COOC₂H₅ | Cl | O |
| 264 | Cl | Prop-1-en-3-yl | Cl | O |
| 265 | Cl | trans-But-2-en-1-yl | Cl | O |
| 266 | Cl | cis-But-2-en-1-yl | Cl | O |
| 267 | Cl | cis-3-Methyl-but-2-en-1-yl | Cl | O |
| 268 | Cl | Cyclopropyl | Cl | O |
| 269 | Cl | Cyclopentyl | Cl | O |
| 270 | Cl | Cyclohexyl | Cl | O |
| 271 | Cl | 4,5-Dihydroisoxazol-3-yl | Cl | O |
| 272 | Cl | 4-Methyl-4,5-dihydroisoxazol-3-yl | Cl | O |
| 273 | Cl | Isoxazol-3-yl | Cl | O |
| 274 | Cl | 4-Methylisoxazol-3-yl | Cl | O |
| 275 | Cl | 4,5-Dihydroisoxazol-4-yl | Cl | O |
| 276 | Cl | 3-Methyl-4,5-dihydroisoxazol-4-yl | Cl | O |
| 277 | Cl | Isoxazol-4-yl | Cl | O |
| 278 | Cl | 3-Methylisoxazol-4-yl | Cl | O |
| 279 | Cl | Phenyl | Cl | O |
| 280 | Cl | Benzyl | Cl | O |
| 281 | Cl | Benzoyl | Cl | O |
| 282 | Cl | 2-Pyridyl | Cl | O |
| 284 | OCH₃ | CH₃ | H | O |
| 285 | OCH₃ | C₂H₅ | H | O |
| 286 | OCH₃ | n-C₃H₇ | H | O |
| 287 | OCH₃ | i-C₃H₇ | H | O |
| 288 | OCH₃ | n-C₄H₉ | H | O |
| 289 | OCH₃ | i-C₄H₉ | H | O |
| 290 | OCH₃ | s-C₄H₉ | H | O |
| 291 | OCH₃ | t-C₄H₉ | H | O |
| 292 | OCH₃ | CH₂OCH₃ | H | O |
| 293 | OCH₃ | CF₃ | H | O |
| 294 | OCH₃ | CF₂H | H | O |
| 295 | OCH₃ | CN | H | O |
| 296 | OCH₃ | OH | H | O |
| 297 | OCH₃ | OCH₃ | H | O |
| 298 | OCH₃ | NH₂ | H | O |
| 299 | OCH₃ | NHCH₃ | H | O |
| 300 | OCH₃ | N(CH₃)₂ | H | O |
| 301 | OCH₃ | CO₂CH₃ | H | O |
| 302 | OCH₃ | CO₂C₂H₅ | H | O |
| 303 | OCH₃ | C(O)CH₃ | H | O |
| 304 | OCH₃ | C(O)CF₃ | H | O |
| 305 | OCH₃ | C(=NOCH₃)CH₃ | H | O |
| 306 | OCH₃ | SO₂CH₃ | H | O |
| 307 | OCH₃ | SO₂CF₃ | H | O |
| 308 | OCH₃ | CH₂CO₂H | H | O |
| 309 | OCH₃ | CH₂COOCH₃ | H | O |
| 310 | OCH₃ | CH₂COOC₂H₅ | H | O |
| 311 | OCH₃ | Prop-1-en-3-yl | H | O |
| 312 | OCH₃ | trans-But-2-en-1-yl | H | O |
| 313 | OCH₃ | cis-But-2-en-1-yl | H | O |
| 314 | OCH₃ | cis-3-Methyl-but-2-en-1-yl | H | O |
| 315 | OCH₃ | Cyclopropyl | H | O |
| 316 | OCH₃ | Cyclopentyl | H | O |
| 317 | OCH₃ | Cyclohexyl | H | O |
| 318 | OCH₃ | 4,5-Dihydroisoxazol-3-yl | H | O |
| 319 | OCH₃ | 4-Methyl-4,5-dihydroisoxazol-3-yl | H | O |
| 320 | OCH₃ | Isoxazol-3-yl | H | O |
| 321 | OCH₃ | 4-Methylisoxazol-3-yl | H | O |
| 322 | OCH₃ | 4,5-Dihydroisoxazol-4-yl | H | O |
| 323 | OCH₃ | 3-Methyl-4,5-dihydroisoxazol-4-yl | H | O |
| 324 | OCH₃ | Isoxazol-4-yl | H | O |
| 325 | OCH₃ | 3-Methylisoxazol-4-yl | H | O |
| 326 | OCH₃ | Phenyl | H | O |
| 327 | OCH₃ | Benzyl | H | O |
| 328 | OCH₃ | Benzoyl | H | O |
| 329 | OCH₃ | 2-Pyridyl | H | O |
| 331 | OCH₃ | CH₃ | CH₃ | O |
| 332 | OCH₃ | C₂H₅ | CH₃ | O |
| 333 | OCH₃ | n-C₃H₇ | CH₃ | O |
| 334 | OCH₃ | i-C₃H₇ | CH₃ | O |
| 335 | OCH₃ | n-C₄H₉ | CH₃ | O |
| 336 | OCH₃ | i-C₄H₉ | CH₃ | O |
| 337 | OCH₃ | s-C₄H₉ | CH₃ | O |
| 338 | OCH₃ | t-C₄H₉ | CH₃ | O |
| 339 | OCH₃ | CH₂OCH₃ | CH₃ | O |
| 340 | OCH₃ | CF₃ | CH₃ | O |
| 341 | OCH₃ | CF₂H | CH₃ | O |
| 342 | OCH₃ | CN | CH₃ | O |
| 343 | OCH₃ | OH | CH₃ | O |
| 344 | OCH₃ | OCH₃ | CH₃ | O |
| 345 | OCH₃ | NH₂ | CH₃ | O |
| 346 | OCH₃ | NHCH₃ | CH₃ | O |
| 347 | OCH₃ | N(CH₃)₂ | CH₃ | O |
| 348 | OCH₃ | CO₂CH₃ | CH₃ | O |
| 349 | OCH₃ | CO₂C₂H₅ | CH₃ | O |
| 350 | OCH₃ | C(O)CH₃ | CH₃ | O |
| 351 | OCH₃ | C(O)CF₃ | CH₃ | O |
| 352 | OCH₃ | C(=NOCH₃)CH₃ | CH₃ | O |
| 353 | OCH₃ | SO₂CH₃ | CH₃ | O |
| 354 | OCH₃ | SO₂CF₃ | CH₃ | O |
| 355 | OCH₃ | CH₂CO₂H | CH₃ | O |
| 356 | OCH₃ | CH₂COOCH₃ | CH₃ | O |
| 357 | OCH₃ | CH₂COOC₂H₅ | CH₃ | O |
| 358 | OCH₃ | Prop-1-en-3-yl | CH₃ | O |
| 359 | OCH₃ | trans-But-2-en-1-yl | CH₃ | O |
| 360 | OCH₃ | cis-But-2-en-1-yl | CH₃ | O |
| 361 | OCH₃ | cis-3-Methyl-but-2-en-1-yl | CH₃ | O |
| 362 | OCH₃ | Cyclopropyl | CH₃ | O |
| 363 | OCH₃ | Cyclopentyl | CH₃ | O |
| 364 | OCH₃ | Cyclohexyl | CH₃ | O |
| 365 | OCH₃ | 4,5-Dihydroisoxazol-3-yl | CH₃ | O |
| 366 | OCH₃ | 4-Methyl-4,5-dihydroisoxazol-3-yl | CH₃ | O |
| 367 | OCH₃ | Isoxazol-3-yl | CH₃ | O |
| 368 | OCH₃ | 4-Methylisoxazol-3-yl | CH₃ | O |
| 369 | OCH₃ | 4,5-Dihydroisoxazol-4-yl | CH₃ | O |
| 370 | OCH₃ | 3-Methyl-4,5-dihydroisoxazol-4-yl | CH₃ | O |
| 371 | OCH₃ | Isoxazol-4-yl | CH₃ | O |
| 372 | OCH₃ | 3-Methylisoxazol-4-yl | CH₃ | O |
| 373 | OCH₃ | Phenyl | CH₃ | O |
| 374 | OCH₃ | Benzyl | CH₃ | O |
| 375 | OCH₃ | Benzoyl | CH₃ | O |
| 376 | OCH₃ | 2-Pyridyl | CH₃ | O |
| 378 | OCH₃ | CH₃ | Cl | O |
| 379 | OCH₃ | C₂H₅ | Cl | O |
| 380 | OCH₃ | n-C₃H₇ | Cl | O |
| 381 | OCH₃ | i-C₃H₇ | Cl | O |
| 382 | OCH₃ | n-C₄H₉ | Cl | O |
| 383 | OCH₃ | i-C₄H₉ | Cl | O |
| 384 | OCH₃ | s-C₄H₉ | Cl | O |
| 385 | OCH₃ | t-C₄H₉ | Cl | O |
| 386 | OCH₃ | CH₂OCH₃ | Cl | O |
| 387 | OCH₃ | CF₃ | Cl | O |
| 388 | OCH₃ | CF₂H | Cl | O |
| 389 | OCH₃ | CN | Cl | O |
| 390 | OCH₃ | OH | Cl | O |
| 391 | OCH₃ | OCH₃ | Cl | O |
| 392 | OCH₃ | NH₂ | Cl | O |
| 393 | OCH₃ | NHCH₃ | Cl | O |
| 394 | OCH₃ | N(CH₃)₂ | Cl | O |
| 395 | OCH₃ | CO₂CH₃ | Cl | O |
| 396 | OCH₃ | CO₂C₂H₅ | Cl | O |
| 397 | OCH₃ | C(O)CH₃ | Cl | O |
| 398 | OCH₃ | C(O)CF₃ | Cl | O |
| 399 | OCH₃ | C(=NOCH₃)CH₃ | Cl | O |
| 400 | OCH₃ | SO₂CH₃ | Cl | O |
| 401 | OCH₃ | SO₂CF₃ | Cl | O |
| 402 | OCH₃ | CH₂CO₂H | Cl | O |
| 403 | OCH₃ | CH₂COOCH₃ | Cl | O |
| 404 | OCH₃ | CH₂COOC₂H₅ | Cl | O |
| 405 | OCH₃ | Prop-1-en-3-yl | Cl | O |
| 406 | OCH₃ | trans-But-2-en-1-yl | Cl | O |
| 407 | OCH₃ | cis-But-2-en-1-yl | Cl | O |
| 408 | OCH₃ | cis-3-Methyl-but-2-en-1-yl | Cl | O |
| 409 | OCH₃ | Cyclopropyl | Cl | O |
| 410 | OCH₃ | Cyclopentyl | Cl | O |
| 411 | OCH₃ | Cyclohexyl | Cl | O |
| 412 | OCH₃ | 4,5-Dihydroisoxazol-3-yl | Cl | O |
| 413 | OCH₃ | 4-Methyl-4,5-dihydroisoxazol-3-yl | Cl | O |
| 414 | OCH₃ | Isoxazol-3-yl | Cl | O |
| 415- | OCH₃ | 4-Methylisoxazol-3-yl | Cl | O |
| 416 | OCH₃ | 4,5-Dihydroisoxazol-4-yl | Cl | O |
| 417 | OCH₃ | 3-Methyl-4,5-dihydroisoxazol-4-yl | Cl | O |
| 418 | OCH₃ | Isoxazol-4-yl | Cl | O |
| 419 | OCH₃ | 3-Methylisoxazol-4-yl | Cl | O |
| 420 | OCH₃ | Phenyl | Cl | O |
| 421 | OCH₃ | Benzyl | Cl | O |
| 422 | OCH₃ | Benzoyl | Cl | O |
| 423 | OCH₃ | 2-Pyridyl | Cl | O |
| 425 | OCF₃ | CH₃ | H | O |
| 426 | OCF₃ | C₂H₅ | H | O |
| 427 | OCF₃ | n-C₃H₇ | H | O |
| 428 | OCF₃ | i-C₃H₇ | H | O |
| 429 | OCF₃ | n-C₄H₉ | H | O |
| 430 | OCF₃ | i-C₄H₉ | H | O |
| 431 | OCF₃ | s-C₄H₉ | H | O |
| 432 | OCF₃ | t-C₄H₉ | H | O |
| 433 | OCF₃ | CH₂OCH₃ | H | O |
| 434 | OCF₃ | CF₃ | H | O |
| 435 | OCF₃ | CF₂H | H | O |
| 436 | OCF₃ | CN | H | O |
| 437 | OCF₃ | OH | H | O |
| 438 | OCF₃ | OCH₃ | H | O |
| 439 | OCF₃ | NH₂ | H | O |
| 440 | OCF₃ | NHCH₃ | H | O |
| 441 | OCF₃ | N(CH₃)₂ | H | O |
| 442 | OCF₃ | CO₂CH₃ | H | O |
| 443 | OCF₃ | CO₂C₂H₅ | H | O |
| 444 | OCF₃ | C(O)CH₃ | H | O |
| 445 | OCF₃ | C(O)CF₃ | H | O |
| 446 | OCF₃ | C(=NOCH₃)CH₃ | H | O |
| 447 | OCF₃ | SO₂CH₃ | H | O |
| 448 | OCF₃ | SO₂CF₃ | H | O |
| 449 | OCF₃ | CH₂CO₂H | H | O |
| 450 | OCF₃ | CH₂COOCH₃ | H | O |
| 451 | OCF₃ | CH₂COOC₂H₅ | H | O |
| 452 | OCF₃ | Prop-1-en-3-yl | H | O |
| 453 | OCF₃ | trans-But-2-en-1-yl | H | O |
| 454 | OCF₃ | cis-But-2-en-1-yl | H | O |
| 455 | OCF₃ | cis-3-Methyl-but-2-en-1-yl | H | O |
| 456 | OCF₃ | Cyclopropyl | H | O |
| 457 | OCF₃ | Cyclopentyl | H | O |
| 458 | OCF₃ | Cyclohexyl | H | O |
| 459 | OCF₃ | 4,5-Dihydroisoxazol-3-yl | H | O |
| 460 | OCF₃ | 4-Methyl-4,5-dihydroisoxazol-3-yl | H | O |
| 461 | OCF₃ | Isoxazol-3-yl | H | O |
| 462 | OCF₃ | 4-Methylisoxazol-3-yl | H | O |
| 463 | OCF₃ | 4,5-Dihydroisoxazol-4-yl | H | O |
| 464 | OCF₃ | 3-Methyl-4,5-dihydroisoxazol-4-yl | H | O |
| 465 | OCF₃ | Isoxazol-4-yl | H | O |
| 466 | OCF₃ | 3-Methylisoxazol-4-yl | H | O |
| 467 | OCF₃ | Phenyl | H | O |
| 468 | OCF₃ | Benzyl | H | O |
| 469 | OCF₃ | Benzoyl | H | O |
| 470 | OCF₃ | 2-Pyridyl | H | O |
| 472 | OCF₃ | CH₃ | CH₃ | O |
| 473 | OCF₃ | C₂H₅ | CH₃ | O |
| 474 | OCF₃ | n-C₃H₇ | CH₃ | O |
| 475 | OCF₃ | i-C₃H₇ | CH₃ | O |
| 476 | OCF₃ | n-C₄H₉ | CH₃ | O |
| 477 | OCF₃ | i-C₄H₉ | CH₃ | O |
| 478 | OCF₃ | s-C₄H₉ | CH₃ | O |
| 479 | OCF₃ | t-C₄H₉ | CH₃ | O |
| 480 | OCF₃ | CH₂OCH₃ | CH₃ | O |
| 481 | OCF₃ | CF₃ | CH₃ | O |
| 482 | OCF₃ | CF₂H | CH₃ | O |
| 483 | OCF₃ | CN | CH₃ | O |
| 484 | OCF₃ | OH | CH₃ | O |
| 485 | OCF₃ | OCH₃ | CH₃ | O |
| 486 | OCF₃ | NH₂ | CH₃ | O |
| 487 | OCF₃ | NHCH₃ | CH₃ | O |
| 488 | OCF₃ | N(CH₃)₂ | CH₃ | O |
| 489 | OCF₃ | CO₂CH₃ | CH₃ | O |
| 490 | OCF₃ | CO₂C₂H₅ | CH₃ | O |
| 491 | OCF₃ | C(O)CH₃ | CH₃ | O |
| 492 | OCF₃ | C(O)CF₃ | CH₃ | O |
| 493 | OCF₃ | C(=NOCH₃)CH₃ | CH₃ | O |
| 494 | OCF₃ | SO₂CH₃ | CH₃ | O |
| 495 | OCF₃ | SO₂CF₃ | CH₃ | O |
| 496 | OCF₃ | CH₂CO₂H | CH₃ | O |
| 497 | OCF₃ | CH₂COOCH₃ | CH₃ | O |
| 498 | OCF₃ | CH₂COOC₂H₅ | CH₃ | O |
| 499 | OCF₃ | Prop-1-en-3-yl | CH₃ | O |
| 500 | OCF₃ | trans-But-2-en-1-yl | CH₃ | O |
| 501 | OCF₃ | cis-But-2-en-1-yl | CH₃ | O |
| 502 | OCF₃ | cis-3-Methyl-but-2-en-1-yl | CH₃ | O |
| 503 | OCF₃ | Cyclopropyl | CH₃ | O |
| 504 | OCF₃ | Cyclopentyl | CH₃ | O |
| 505 | OCF₃ | Cyclohexyl | CH₃ | O |
| 506 | OCF₃ | 4,5-Dihydroisoxazol-3-yl | CH₃ | O |
| 507 | OCF₃ | 4-Methyl-4,5-dihydroisoxazol-3-yl | C_{H3} | O |
| 508 | OCF₃ | Isoxazol-3-yl | CH₃ | O |
| 509 | OCF₃ | 4-Methylisoxazol-3-yl | CH₃ | O |
| 510 | OCF₃ | 4,5-Dihydroisoxazol-4-yl | CH₃ | O |
| 511 | OCF₃ | 3-Methyl-4,5-dihydroisoxazol-4-yl | CH₃ | O |
| 512 | OCF₃ | Isoxazol-4-yl | CH₃ | O |
| 513 | OCF₃ | 3-Methylisoxazol-4-yl | CH₃ | O |
| 514 | OCF₃ | Phenyl | CH₃ | O |
| 515 | OCF₃ | Benzyl | CH₃ | O |
| 516 | OCF₃ | Benzoyl | CH₃ | O |
| 517 | OCF₃ | 2-Pyridyl | CH₃ | O |
| 519 | OCF₃ | CH₃ | Cl | O |
| 520 | OCF₃ | C₂H₅ | Cl | O |
| 521 | OCF₃ | n-C₃H₇ | Cl | O |
| 522 | OCF₃ | i-C₃H₇ | Cl | O |
| 523 | OCF₃ | n-C₄H₉ | Cl | O |
| 524 | OCF₃ | i-C₄H₉ | Cl | O |
| 525 | OCF₃ | s-C₄H₉ | Cl | O |
| 526 | OCF₃ | t-C₄H₉ | Cl | O |
| 527 | OCF₃ | CH₂OCH₃ | Cl | O |
| 528 | OCF₃ | CF₃ | Cl | O |
| 529 | OCF₃ | CF₂H | Cl | O |
| 530 | OCF₃ | CN | Cl | O |
| 531 | OCF₃ | OH | Cl | O |
| 532 | OCF₃ | OCH₃ | Cl | O |
| 533 | OCF₃ | NH₂ | Cl | O |
| 534 | OCF₃ | NHCH₃ | Cl | O |
| 535 | OCF₃ | N(CH₃)₂ | Cl | O |
| 536 | OCF₃ | CO₂CH₃ | Cl | O |
| 537 | OCF₃ | CO₂C₂H₅ | Cl | O |
| 538 | OCF₃ | C(O)CH₃ | Cl | O |
| 539 | OCF₃ | C(O)CF₃ | Cl | O |
| 540 | OCF₃ | C(=NOCH₃)CH₃ | Cl | O |
| 541 | OCF₃ | SO₂CH₃ | Cl | O |
| 542 | OCF₃ | SO₂CF₃ | Cl | O |
| 543 | OCF₃ | CH₂CO₂H | Cl | O |
| 544 | OCF₃ | CH₂COOCH₃ | Cl | O |
| 545 | OCF₃ | CH₂COOC₂H₅ | Cl | O |
| 546 | OCF₃ | Prop-1-en-3-yl | Cl | O |
| 547 | OCF₃ | trans-But-2-en-1-yl | Cl | O |
| 548 | OCF₃ | cis-But-2-en-1-yl | Cl | O |
| 549 | OCF3 | cis-3-Methyl-but-2-en-1-yl | Cl | O |
| 550 | OCF₃ | Cyclopropyl | Cl | O |
| 551 | OCF₃ | Cyclopentyl | Cl | O |
| 552 | OCF₃ | Cyclohexyl | Cl | O |
| 553 | OCF₃ | 4,5-Dihydroisoxazol-3-yl | Cl | O |
| 554 | OCF₃ | 4-Methyl-4,5-dihydroisoxazol-3-yl | Cl | O |
| 555 | OCF₃ | Isoxazol-3-yl | Cl | O |
| 556 | OCF₃ | 4-Methylisoxazol-3-yl | Cl | O |
| 557 | OCF₃ | 4,5-Dihydroisoxazol-4-yl | Cl | O |
| 558 | OCF₃ | 3-Methyl-4,5-dihydroisoxazol-4-yl | Cl | O |
| 559 | OCF₃ | Isoxazol-4-yl | Cl | O |
| 560 | OCF₃ | 3-Methylisoxazol-4-yl | Cl | O |
| 561 | OCF₃ | Phenyl | Cl | O |
| 562 | OCF₃ | Benzyl | Cl | O |
| 563 | OCF₃ | Benzoyl | Cl | O |
| 564 | OCF₃ | 2-Pyridyl | Cl | O |
| 566 | SCH₃ | CH₃ | H | O |
| 567 | SCH₃ | C₂H₅ | H | O |
| 568 | SCH₃ | n-C₃H₇ | H | O |
| 569 | SCH₃ | i-C₃H₇ | H | O |
| 570 | SCH₃ | n-C₄H₉ | H | O |
| 571 | SCH₃ | i-C₄H₉ | H | O |
| 572 | SCH₃ | s-C₄H₉ | H | O |
| 573 | SCH₃ | t-C₄H₉ | H | O |
| 574 | SCH₃ | CH₂OCH₃ | H | O |
| 575 | SCH₃ | CF₃ | H | O |
| 576 | SCH₃ | CF₂H | H | O |
| 577 | SCH₃ | CN | H | O |
| 578 | SCH₃ | OH | H | O |
| 579 | SCH₃ | OCH₃ | H | O |
| 580 | SCH₃ | NH₂ | H | O |
| 581 | SCH₃ | NHCH₃ | H | O |
| 582 | SCH₃ | N(CH₃)₂ | H | O |
| 583 | SCH₃ | CO₂CH₃ | H | O |
| 584 | SCH₃ | CO₂C₂H₅ | H | O |
| 585 | SCH₃ | C(O)CH₃ | H | O |
| 586 | SCH₃ | C(O)CF₃ | H | O |
| 587 | SCH₃ | C(=NOCH₃)CH₃ | H | O |
| 588 | SCH₃ | SO₂CH₃ | H | O |
| 589 | SCH₃ | SO₂CF₃ | H | O |
| 590 | SCH₃ | CH₂CO₂H | H | O |
| 591 | SCH₃ | CH₂COOCH₃ | H | O |
| 592 | SCH₃ | CH₂COOC₂H₅ | H | O |
| 593 | SCH₃ | Prop-1-en-3-yl | H | O |
| 594 | SCH₃ | trans-But-2-en-1-yl | H | O |
| 595 | SCH₃ | cis-But-2-en-1-yl | H | O |
| 596 | SCH₃ | cis-3-Methyl-but-2-en-1-yl | H | O |
| 597 | SCH₃ | Cyclopropyl | H | O |
| 598 | SCH₃ | Cyclopentyl | H | O |
| 599 | SCH₃ | Cyclohexyl | H | O |
| 600 | SCH₃ | 4,5-Dihydroisoxazol-3-yl | H | O |
| 601 | SCH₃ | 4-Methyl-4,5-dihydroisoxazol-3-yl | H | O |
| 602 | SCH₃ | Isoxazol-3-yl | H | O |
| 603 | SCH₃ | 4-Methylisoxazol-3-yl | H | O |
| 604 | SCH₃ | 4,5-Dihydroisoxazol-4-yl | H | O |
| 605 | SCH₃ | 3-Methyl-4,5-dihydroisoxazol-4-yl | H | O |
| 606 | SCH₃ | Isoxazol-4-yl | H | O |
| 607 | SCH₃ | 3-Methylisoxazol-4-yl | H | O |
| 608 | SCH₃ | Phenyl | H | O |
| 609 | SCH₃ | Benzyl | H | O |
| 610 | SCH₃ | Benzoyl | H | O |
| 611 | SCH₃ | 2-Pyridyl | H | O |
| 613 | SCH₃ | CH₃ | CH₃ | O |
| 614 | SCH₃ | C₂H₅ | CH₃ | O |
| 615 | SCH₃ | n-C₃H₇ | CH₃ | O |
| 616 | SCH₃ | i-C₃H₇ | CH₃ | O |
| 617 | SCH₃ | n-C₄H₉ | CH₃ | O |
| 618 | SCH₃ | i-C₄H₉ | CH₃ | O |
| 619 | SCH₃ | s-C₄H₉ | CH₃ | O |
| 620 | SCH₃ | t-C₄H₉ | CH₃ | O |
| 621 | SCH₃ | CH₂OCH₃ | CH₃ | O |
| 622 | SCH₃ | CF₃ | CH₃ | O |
| 623 | SCH₃ | CF₂H | CH₃ | O |
| 624 | SCH₃ | CN | CH₃ | O |
| 625 | SCH₃ | OH | CH₃ | O |
| 626 | SCH₃ | OCH₃ | CH₃ | O |
| 627 | SCH₃ | NH₂ | CH₃ | O |
| 628 | SCH₃ | NHCH₃ | CH₃ | O |
| 629 | SCH₃ | N(CH₃)₂ | CH₃ | O |
| 630 | SCH₃ | CO₂CH₃ | CH₃ | O |
| 631 | SCH₃ | CO₂C₂H₅ | CH₃ | O |
| 632 | SCH₃ | C(O)CH₃ | CH₃ | O |
| 633 | SCH₃ | C(O)CF₃ | CH₃ | O |
| 634 | SCH₃ | C(=NOCH₃)CH₃ | CH₃ | O |
| 635 | SCH₃ | SO₂CH₃ | CH₃ | O |
| 636 | SCH₃ | SO₂CF₃ | CH₃ | O |
| 637 | SCH₃ | CH₂CO₂H | CH₃ | O |
| 638 | SCH₃ | CH₂COOCH₃ | CH₃ | O |
| 639 | SCH₃ | CH₂COOC₂H₅ | CH₃ | O |
| 640 | SCH₃ | Prop-1-en-3-yl | CH₃ | O |
| 641 | SCH₃ | trans-But-2-en-1-yl | CH₃ | O |
| 642 | SCH₃ | cis-But-2-en-1-yl | CH₃ | O |
| 643 | SCH₃ | cis-3-Methyl-but-2-en-1-yl | CH₃ | O |
| 644 | SCH₃ | Cyclopropyl | CH₃ | O |
| 645 | SCH₃ | Cyclopentyl | CH₃ | O |
| 646 | SCH₃ | Cyclohexyl | CH₃ | O |
| 647 | SCH₃ | 4,5-Dihydroisoxazol-3-yl | CH₃ | O |
| 648 | SCH₃ | 4-Methyl-4,5-dihydroisoxazol-3-yl | CH₃ | O |
| 649 | SCH₃ | Isoxazol-3-yl | CH₃ | O |
| 650 | SCH₃ | 4-Methylisoxazol-3-yl | CH₃ | O |
| 651 | SCH₃ | 4,5-Dihydroisoxazol-4-yl | CH₃ | O |
| 652 | SCH₃ | 3-Methyl-4,5-dihydroisoxazol-4-yl | CH₃ | O |
| 653 | SCH₃ | Isoxazol-4-yl | CH₃ | O |
| 654 | SCH₃ | 3-Methylisoxazol-4-yl | CH₃ | O |
| 655 | SCH₃ | Phenyl | CH₃ | O |
| 656 | SCH₃ | Benzyl | CH₃ | O |
| 657 | SCH₃ | Benzoyl | CH₃ | O |
| 658 | SCH₃ | 2-Pyridyl | CH₃ | O |
| 660 | SCH₃ | CH₃ | Cl | O |
| 661 | SCH₃ | C₂H₅ | Cl | O |
| 662 | SCH₃ | n-C₃H₇ | Cl | O |
| 663 | SCH₃ | i-C₃H₇ | Cl | O |
| 664 | SCH₃ | n-C₄H₉ | Cl | O |
| 665 | SCH₃ | i-C₄H₉ | Cl | O |
| 666 | SCH₃ | s-C₄H₉ | Cl | O |
| 667 | SCH₃ | t-C₄H₉ | Cl | O |
| 668 | SCH₃ | CH₂OCH₃ | Cl | O |
| 669 | SCH₃ | CF₃ | Cl | O |
| 670 | SCH₃ | CF₂H | Cl | O |
| 671 | SCH₃ | CN | Cl | O |
| 672 | SCH₃ | OH | Cl | O |
| 673 | SCH₃ | OCH₃ | Cl | O |
| 674 | SCH₃ | NH₂ | Cl | O |
| 675 | SCH₃ | NHCH₃ | Cl | O |
| 676 | SCH₃ | N(CH₃)₂ | Cl | O |
| 677 | SCH₃ | CO₂CH₃ | Cl | O |
| 678 | SCH₃ | CO₂C₂H₅ | Cl | O |
| 679 | SCH₃ | C(O)CH₃ | Cl | O |
| 680 | SCH₃ | C(O)CF₃ | Cl | O |
| 681 | SCH₃ | C(=NOCH₃)CH₃ | Cl | O |
| 682 | SCH₃ | SO₂CH₃ | Cl | O |
| 683 | SCH₃ | SO₂CF₃ | Cl | O |
| 684 | SCH₃ | CH₂CO₂H | Cl | O |
| 685 | SCH₃ | CH₂COOCH₃ | Cl | O |
| 686 | SCH₃ | CH₂COOC₂H₅ | Cl | O |
| 687 | SCH₃ | Prop-1-en-3-yl | Cl | O |
| 688 | SCH₃ | trans-But-2-en-1-yl | Cl | O |
| 689 | SCH₃ | cis-But-2-en-1-yl | Cl | O |
| 690 | SCH₃ | cis-3-Methyl-but-2-en-1-yl | Cl | O |
| 691 | SCH₃ | Cyclopropyl | Cl | O |
| 692 | SCH₃ | Cyclopentyl | Cl | O |
| 693 | SCH₃ | Cyclohexyl | Cl | O |
| 694 | SCH₃ | 4,5-Dihydroisoxazol-3-yl | Cl | O |
| 695 | SCH₃ | 4-Methyl-4,5-dihydroisoxazol-3-yl | Cl | O |
| 696 | SCH₃ | Isoxazol-3-yl | Cl | O |
| 697 | SCH₃ | 4-Methylisoxazol-3-yl | Cl | O |
| 698 | SCH₃ | 4,5-Dihydroisoxazol-4-yl | Cl | O |
| 699 | SCH₃ | 3-Methyl-4,5-dihydroisoxazol-4-yl | Cl | O |
| 700 | SCH₃ | Isoxazol-4-yl | Cl | O |
| 701 | SCH₃ | 3-Methylisoxazol-4-yl | Cl | O |
| 702 | SCH₃ | Phenyl | Cl | O |
| 703 | SCH₃ | Benzyl | Cl | O |
| 704 | SCH₃ | Benzoyl | Cl | O |
| 705 | SCH₃ | 2-Pyridyl | Cl | O |
| 707 | SO₂CH₃ | CH₃ | H | O |
| 708 | SO₂CH₃ | C₂H₅ | H | O |
| 709 | SO₂CH₃ | n-C₃H₇ | H | O |
| 710 | SO₂CH₃ | i-C₃H₇ | H | O |
| 711 | SO₂CH₃ | n-C₄H₉ | H | O |
| 712 | SO₂CH₃ | i-C₄H₉ | H | O |
| 713 | SO₂CH₃ | s-C₄H₉ | H | O |
| 714 | SO₂CH₃ | t-C₄H₉ | H | O |
| 715 | SO₂CH₃ | CH₂OCH₃ | H | O |
| 716 | SO₂CH₃ | CF₃ | H | O |
| 717 | SO₂CH₃ | CF₂H | H | O |
| 718 | SO₂CH₃ | CN | H | O |
| 719 | SO₂CH₃ | OH | H | O |
| 720 | SO₂CH₃ | OCH₃ | H | O |
| 721 | SO₂CH₃ | NH₂ | H | O |
| 722 | SO₂CH₃ | NHCH₃ | H | O |
| 723 | SO₂CH₃ | N(CH₃)₂ | H | O |
| 724 | SO₂CH₃ | CO₂CH₃ | H | O |
| 725 | SO₂CH₃ | CO₂C₂H₅ | H | O |
| 726 | SO₂CH₃ | C(O)CH₃ | H | O |
| 727 | SO₂CH₃ | C(O)CF₃ | H | O |
| 728 | SO₂CH₃ | C(=NOCH₃)CH₃ | H | O |
| 729 | SO₂CH₃ | SO₂CH₃ | H | O |
| 730 | SO₂CH₃ | SO₂CF₃ | H | O |
| 731 | SO₂CH₃ | CH₂CO₂H | H | O |
| 732 | SO₂CH₃ | CH₂COOCH₃ | H | O |
| 733 | SO₂CH₃ | CH₂COOC₂H₅ | H | O |
| 734 | SO₂CH₃ | Prop-1-en-3-yl | H | O |
| 735 | SO₂CH₃ | trans-But-2-en-1-yl | H | O |
| 736 | SO₂CH₃ | cis-But-2-en-1-yl | H | O |
| 737 | SO₂CH₃ | cis-3-Methyl-but-2-en-1-yl | H | O |
| 738 | SO₂CH₃ | Cyclopropyl | H | O |
| 739 | SO₂CH₃ | Cyclopentyl | H | O |
| 740 | SO₂CH₃ | Cyclohexyl | H | O |
| 741 | SO₂CH₃ | 4,5-Dihydroisoxazol-3-yl | H | O |
| 742 | SO₂CH₃ | 4-Methyl-4,5-dihydroisoxazol-3-yl | H | O |
| 743 | SO₂CH₃ | Isoxazol-3-yl | H | O |
| 744 | SO₂CH₃ | 4-Methylisoxazol-3-yl | H | O |
| 745 | SO₂CH₃ | 4,5-Dihydroisoxazol-4-yl | H | O |
| 746 | SO₂CH₃ | 3-Methyl-4,5-dihydroisoxazol-4-yl | H | O |
| 747 | SO₂CH₃ | Isoxazol-4-yl | H | O |
| 748 | SO₂CH₃ | 3-Methylisoxazol-4-yl | H | O |
| 749 | SO₂CH₃ | Phenyl | H | O |
| 750 | SO₂CH₃ | Benzyl | H | O |
| 751 | SO₂CH₃ | Benzoyl | H | O |
| 752 | SO₂CH₃ | 2-Pyridyl | H | O |
| 754 | SO₂CH₃ | CH₃ | CH₃ | O |
| 755 | SO₂CH₃ | C₂H₅ | CH₃ | O |
| 756 | SO₂CH₃ | n-C₃H₇ | CH₃ | O |
| 757 | SO₂CH₃ | i-C₃H₇ | CH₃ | O |
| 758 | SO₂CH₃ | n-C₄H₉ | CH₃ | O |
| 759 | SO₂CH₃ | i-C₄H₉ | CH₃ | O |
| 760 | SO₂CH₃ | s-C₄H₉ | CH₃ | O |
| 761 | SO₂CH₃ | t-C₄H₉ | CH₃ | O |
| 762 | SO₂CH₃ | CH₂OCH₃ | CH₃ | O |
| 763 | SO₂CH₃ | CF₃ | CH₃ | O |
| 764 | SO₂CH₃ | CF₂H | CH₃ | O |
| 765 | SO₂CH₃ | CN | CH₃ | O |
| 766 | SO₂CH₃ | OH | CH₃ | O |
| 767 | SO₂CH₃ | OCH₃ | CH₃ | O |
| 768 | SO₂CH₃ | NH₂ | CH₃ | O |
| 769 | SO₂CH₃ | NHCH₃ | CH₃ | O |
| 770 | SO₂CH₃ | N(CH₃)₂ | CH₃ | O |
| 771 | SO₂CH₃ | CO₂CH₃ | CH₃ | O |
| 772 | SO₂CH₃ | CO₂C₂H₅ | CH₃ | O |
| 773 | SO₂CH₃ | C(O)CH₃ | CH₃ | O |
| 774 | SO₂CH₃ | C(O)CF₃ | CH₃ | O |
| 775 | SO₂CH₃ | C(=NOCH₃)CH₃ | CH₃ | O |
| 776 | SO₂CH₃ | SO₂CH₃ | CH₃ | O |
| 777 | SO₂CH₃ | SO₂CF₃ | CH₃ | O |
| 778 | SO₂CH₃ | CH₂CO₂H | CH₃ | O |
| 779 | SO₂CH₃ | CH₂COOCH₃ | CH₃ | O |
| 780 | SO₂CH₃ | CH₂COOC₂H₅ | CH₃ | O |
| 781 | SO₂CH₃ | Prop-1-en-3-yl | CH₃ | O |
| 782 | SO₂CH₃ | trans-But-2-en-1-yl | CH₃ | O |
| 783 | SO₂CH₃ | cis-But-2-en-1-yl | CH₃ | O |
| 784 | SO₂CH₃ | cis-3-Methyl-but-2-en-1-yl | CH₃ | O |
| 785 | SO₂CH₃ | Cyclopropyl | CH₃ | O |
| 786 | SO₂CH₃ | Cyclopentyl | CH₃ | O |
| 787 | SO₂CH₃ | Cyclohexyl | CH₃ | O |
| 788 | SO₂CH₃ | 4,5-Dihydroisoxazol-3-yl | CH₃ | O |
| 789 | SO₂CH₃ | 4-Methyl-4,5-dihydroisoxazol-3-yl | CH₃ | O |
| 790 | SO₂CH₃ | Isoxazol-3-yl | CH₃ | O |
| 791 | SO₂CH₃ | 4-Methylisoxazol-3-yl | CH₃ | O |
| 792 | SO₂CH₃ | 4,5-Dihydroisoxazol-4-yl | CH₃ | O |
| 793 | SO₂CH₃ | 3-Methyl-4,5-dihydroisoxazol-4-yl | CH₃ | O |
| 794 | SO₂CH₃ | Isoxazol-4-yl | CH₃ | O |
| 795 | SO₂CH₃ | 3-Methylisoxazol-4-yl | CH₃ | O |
| 796 | SO₂CH₃ | Phenyl | CH₃ | O |
| 797 | SO₂CH₃ | Benzyl | CH₃ | O |
| 798 | SO₂CH₃ | Benzoyl | CH₃ | O |
| 799 | SO₂CH₃ | 2-Pyridyl | CH₃ | O |
| 801 | SO₂CH₃ | CH₃ | Cl | O |
| 802 | SO₂CH₃ | C₂H₅ | Cl | O |
| 803 | SO₂CH₃ | n-C₃H₇ | Cl | O |
| 804 | SO₂CH₃ | i-C₃H₇ | Cl | O |
| 805 | SO₂CH₃ | n-C₄H₉ | Cl | O |
| 806 | SO₂CH₃ | i-C₄H₉ | Cl | O |
| 807 | SO₂CH₃ | s-C₄H₉ | Cl | O |
| 808 | SO₂CH₃ | t-C₄H₉ | Cl | O |
| 809 | SO₂CH₃ | CH₂OCH₃ | Cl | O |
| 810 | SO₂CH₃ | CF₃ | Cl | O |
| 811 | SO₂CH₃ | CF₂H | Cl | O |
| 812 | SO₂CH₃ | CN | Cl | O |
| 813 | SO₂CH₃ | OH | Cl | O |
| 814 | SO₂CH₃ | OCH₃ | Cl | O |
| 815 | SO₂CH₃ | NH₂ | Cl | O |
| 816 | SO₂CH₃ | NHCH₃ | Cl | O |
| 817 | SO₂CH₃ | N(CH₃)₂ | Cl | O |
| 818 | SO₂CH₃ | CO₂CH₃ | Cl | O |
| 819 | SO₂CH₃ | CO₂C₂H₅ | Cl | O |
| 820 | SO₂CH₃ | C(O)CH₃ | Cl | O |
| 821 | SO₂CH₃ | C(O)CF₃ | Cl | O |
| 822 | SO₂CH₃ | C(=NOCH₃)CH₃ | Cl | O |
| 823 | SO₂CH₃ | SO₂CH₃ | Cl | O |
| 824 | SO₂CH₃ | SO₂CF₃ | Cl | O |
| 825 | SO₂CH₃ | CH₂CO₂H | Cl | O |
| 826 | SO₂CH₃ | CH₂COOCH₃ | Cl | O |
| 827 | SO₂CH₃ | CH₂COOC₂H₅ | Cl | O |
| 828 | SO₂CH₃ | Prop-1-en-3-yl | Cl | O |
| 829 | SO₂CH₃ | trans-But-2-en-1-yl | Cl | O |
| 830 | SO₂CH₃ | cis-But-2-en-1-yl | Cl | O |
| 831 | SO₂CH₃ | cis-3-Methyl-but-2-en-1-yl | Cl | O |
| 832 | SO₂CH₃ | Cyclopropyl | Cl | O |
| 833 | SO₂CH₃ | Cyclopentyl | Cl | O |
| 834 | SO₂CH₃ | Cyclohexyl | Cl | O |
| 835 | SO₂CH₃ | 4,5-Dihydroisoxazol-3-yl | Cl | O |
| 836 | SO₂CH₃ | 4-Methyl-4,5-dihydroisoxazol-3-yl | Cl | O |
| 837 | SO₂CH₃ | Isoxazol-3-yl | Cl | O |
| 838 | SO₂CH₃ | 4-Methylisoxazol-3-yl | Cl | O |
| 839 | SO₂CH₃ | 4,5-Dihydroisoxazol-4-yl | Cl | O |
| 840 | SO₂CH₃ | 3-Methyl-4,5-dihydroisoxazol-4-yl | Cl | O |
| 841 | SO₂CH₃ | Isoxazol-4-yl | Cl | O |
| 842 | SO₂CH₃ | 3-Methylisoxazol-4-yl | Cl | O |
| 843 | SO₂CH₃ | Phenyl | Cl | O |
| 844 | SO₂CH₃ | Benzyl | CL | O |
| 845 | SO₂CH₃ | Benzoyl | Cl | O |
| 846 | SO₂CH₃ | 2-Pyridyl | Cl | O |
| 848 | CF₃ | CH₃ | H | O |
| 849 | CF₃ | C₂H₅ | H | O |
| 850 | CF₃ | n-C₃H₇ | H | O |
| 851 | CF₃ | i-C₃H₇ | H | O |
| 852 | CF₃ | n-C₄H₉ | H | O |
| 853 | CF₃ | i-C₄H₉ | H | O |
| 854 | CF₃ | s-C₄H₉ | H | O |
| 855 | CF₃ | t-C₄H₉ | H | O |
| 856 | CF₃ | CH₂OCH₃ | H | O |
| 857 | CF₃ | CF₃ | H | O |
| 858 | CF₃ | CF₂H | H | O |
| 859 | CF₃ | CN | H | O |
| 860 | CF₃ | OH | H | O |
| 861 | CF₃ | OCH₃ | H | O |
| 862 | CF₃ | NH₂ | H | O |
| 863 | CF₃ | NHCH₃ | H | O |
| 864 | CF₃ | N(CH₃)₂ | H | O |
| 865 | CF₃ | CO₂CH₃ | H | O |
| 866 | CF₃ | CO₂C₂H₅ | H | O |
| 867 | CF₃ | C(O)CH₃ | H | O |
| 868 | CF₃ | C(O)CF₃ | H | O |
| 869 | CF₃ | C(=NOCH₃)CH₃ | H | O |
| 870 | CF₃ | SO₂CH₃ | H | O |
| 871 | CF₃ | SO₂CF₃ | H | O |
| 872 | CF₃ | CH₂CO₂H | H | O |
| 873 | CF₃ | CH₂COOCH₃ | H | O |
| 874 | CF₃ | CH₂COOC₂H₅ | H | O |
| 875 | CF₃ | Prop-1-en-3-yl | H | O |
| 876 | CF₃ | trans-But-2-en-1-yl | H | O |
| 877 | CF₃ | cis-But-2-en-1-yl | H | O |
| 878 | CF₃ | cis-3-Methyl-but-2-en-1-yl | H | O |
| 879 | CF₃ | Cyclopropyl | H | O |
| 880 | CF₃ | Cyclopentyl | H | O |
| 881 | CF₃ | Cyclohexyl | H | O |
| 882 | CF₃ | 4,5-Dihydroisoxazol-3-yl | H | O |
| 883 | CF₃ | 4-Methyl-4,5-dihydroisoxazol-3-yl | H | O |
| 884 | CF₃ | Isoxazol-3-yl | H | O |
| 885 | CF₃ | 4-Methylisoxazol-3-yl | H | O |
| 886 | CF₃ | 4,5-Dihydroisoxazol-4-yl | H | O |
| 887 | CF₃ | 3-Methyl-4,5-dihydroisoxazol-4-yl | H | O |
| 888 | CF₃ | Isoxazol-4-yl | H | O |
| 889 | CF₃ | 3-Methylisoxazol-4-yl | H | O |
| 890 | CF₃ | Phenyl | H | O |
| 891 | CF₃ | Benzyl | H | O |
| 892 | CF₃ | Benzoyl | H | O |
| 893 | CF₃ | 2-Pyridyl | H | O |
| 895 | CF₃ | CH₃ | CH₃ | O |
| 896 | CF₃ | C₂H₅ | CH₃ | O |
| 897 | CF₃ | n-C₃H₇ | CH₃ | O |
| 898 | CF₃ | i-C₃H₇ | CH₃ | O |
| 899 | CF₃ | n-C₄H₉ | CH₃ | O |
| 900 | CF₃ | i-C₄H₉ | CH₃ | O |
| 901 | CF₃ | s-C₄H₉ | CH₃ | O |
| 902 | CF₃ | t-C₄H₉ | CH₃ | O |
| 903 | CF₃ | CH₂OCH₃ | CH₃ | O |
| 904 | CF₃ | CF₃ | CH₃ | O |
| 905 | CF₃ | CF₂H | CH₃ | O |
| 906 | CF₃ | CN | CH₃ | O |
| 907 | CF₃ | OH | CH₃ | O |
| 908 | CF₃ | OCH₃ | CH₃ | O |
| 909 | CF₃ | NH₂ | CH₃ | O |
| 910 | CF₃ | NHCH₃ | CH₃ | O |
| 911 | CF₃ | N(CH₃)₂ | CH₃ | O |
| 912 | CF₃ | CO₂CH₃ | CH₃ | O |
| 913 | CF₃ | CO₂C₂H₅ | CH₃ | O |
| 914 | CF₃ | C(O)CH₃ | CH₃ | O |
| 915 | CF₃ | C(O) CF₃ | CH₃ | O |
| 916 | CF₃ | C(=NOCH₃)CH₃ | CH₃ | O |
| 917 | CF₃ | SO₂CH₃ | CH₃ | O |
| 918 | CF₃ | SO₂CF₃ | CH₃ | O |
| 919 | CF₃ | CH₂CO₂H | CH₃ | O |
| 920 | CF₃ | CH₂COOCH₃ | CH₃ | O |
| 921 | CF₃ | CH₂COOC₂H₅ | CH₃ | O |
| 922 | CF₃ | Prop-1-en-3-yl | CH₃ | O |
| 923 | CF₃ | trans-But-2-en-1-yl | CH₃ | O |
| 924 | CF₃ | cis-But-2-en-1-yl | CH₃ | O |
| 925 | CF₃ | cis-3-Methyl-but-2-en-1-yl | CH₃ | O |
| 926 | CF₃ | Cyclopropyl | CH₃ | O |
| 927 | CF₃ | Cyclopentyl | CH₃ | O |
| 928 | CF₃ | Cyclohexyl | CH₃ | O |
| 929 | CF₃ | 4,5-Dihydroisoxazol-3-yl | CH₃ | O |
| 930 | CF₃ | 4-Methyl-4,5-dihydroisoxazol-3-yl | CH₃ | O |
| 931 | CF₃ | Isoxazol-3-yl | CH₃ | O |
| 932 | CF₃ | 4-Methylisoxazol-3-yl | CH₃ | O |
| 933 | CF₃ | 4,5-Dihydroisoxazol-4-yl | CH₃ | O |
| 934 | CF₃ | 3-Methyl-4,5-dihydroisoxazol-4-yl | CH₃ | O |
| 935 | CF₃ | Isoxazol-4-yl | CH₃ | O |
| 936 | CF₃ | 3-Methylisoxazol-4-yl | CH₃ | O |
| 937 | CF₃ | Phenyl | CH₃ | O |
| 938 | CF₃ | Benzyl | CH₃ | O |
| 939 | CF₃ | Benzoyl | CH₃ | O |
| 940 | CF₃ | 2-Pyridyl | CH₃ | O |
| 942 | CF₃ | CH₃ | Cl | O |
| 943 | CF₃ | C₂H₅ | Cl | O |
| 944 | CF₃ | n-C₃H₇ | Cl | O |
| 945 | CF₃ | i-C₃H₇ | Cl | O |
| 946 | CF₃ | n-C₄H₉ | Cl | O |
| 947 | CF₃ | i-C₄H₉ | Cl | O |
| 948 | CF₃ | s-C₄H₉ | Cl | O |
| 949 | CF₃ | t-C₄H₉ | Cl | O |
| 950 | CF₃ | CH₂OCH₃ | Cl | O |
| 951 | CF₃ | CF₃ | Cl | O |
| 952 | CF₃ | CF₂H | Cl | O |
| 953 | CF₃ | CN | Cl | O |
| 954 | CF₃ | OH | Cl | O |
| 955 | CF₃ | OCH₃ | Cl | O |
| 956 | CF₃ | NH₂ | Cl | O |
| 957 | CF₃ | NHCH₃ | Cl | O |
| 958 | CF₃ | N(CH₃)₂ | Cl | O |
| 959 | CF₃ | CO₂CH₃ | Cl | O |
| 960 | CF₃ | CO₂C₂H₅ | Cl | O |
| 961 | CF₃ | C(O)CH₃ | Cl | O |
| 962 | CF₃ | C(O)CF₃ | Cl | O |
| 963 | CF₃ | C(=NOCH₃)CH₃ | Cl | O |
| 964 | CF₃ | SO₂CH₃ | Cl | O |
| 965 | CF₃ | SO₂CF₃ | Cl | O |
| 966 | CF₃ | CH₂CO₂H | Cl | O |
| 967 | CF₃ | CH₂COOCH₃ | Cl | O |
| 968 | CF₃ | CH₂COOC₂H₅ | Cl | O |
| 969 | CF₃ | Prop-1-en-3-yl | Cl | O |
| 970 | CF₃ | trans-But-2-en-1-yl | Cl | O |
| 971 | CF₃ | cis-But-2-en-1-yl | Cl | O |
| 972 | CF₃ | cis-3-Methyl-but-2-en-1-yl | Cl | O |
| 973 | CF₃ | Cyclopropyl | Cl | O |
| 974 | CF₃ | Cyclopentyl | Cl | O |
| 975 | CF₃ | Cyclohexyl | Cl | O |
| 976 | CF₃ | 4,5-Dihydroisoxazol-3-yl | Cl | O |
| 977 | CF₃ | 4-Methyl-4,5-dihydroisoxazol-3-yl | Cl | O |
| 978 | CF₃ | Isoxazol-3-yl | Cl | O |
| 979 | CF₃ | 4-Methylisoxazol-3-yl | Cl | O |
| 980 | CF₃ | 4,5-Dihydroisoxazol-4-yl | Cl | O |
| 981 | CF₃ | 3-Methyl-4,5-dihydroisoxazol-4-yl | Cl | O |
| 982 | CF₃ | Isoxazol-4-yl | Cl | O |
| 983 | CF₃ | 3-Methylisoxazol-4-yl | Cl | O |
| 984 | CF₃ | Phenyl | Cl | O |
| 985 | CF₃ | Benzyl | Cl | O |
| 986 | CF₃ | Benzoyl | Cl | O |
| 987 | CF₃ | 2-Pyridyl | Cl | O |
| 989 | C₂H₅ | CH₃ | H | O |
| 990 | C₂H₅ | C₂H₅ | H | O |
| 991 | C₂H₅ | n-C₃H₇ | H | O |
| 992 | C₂H₅ | i-C₃H₇ | H | O |
| 993 | C₂H₅ | n-C₄H₉ | H | O |
| 994 | C₂H₅ | i-C₄H₉ | H | O |
| 995 | C₂H₅ | s-C₄H₉ | H | O |
| 996 | C₂H₅ | t-C₄H₉ | H | O |
| 997 | C₂H₅ | CH₂OCH₃ | H | O |
| 998 | C₂H₅ | CF₃ | H | O |
| 999 | C₂H₅ | CF₂H | H | O |
| 1000 | C₂H₅ | CN | H | O |
| 1001 | C₂H₅ | OH | H | O |
| 1002 | C₂H₅ | OCH₃ | H | O |
| 1003 | C₂H₅ | NH₂ | H | O |
| 1004 | C₂H₅ | NHCH₃ | H | O |
| 1005 | C₂H₅ | N(CH₃)₂ | H | O |
| 1006 | C₂H₅ | CO₂CH₃ | H | O |
| 1007 | C₂H₅ | CO₂C₂H₅ | H | O |
| 1008 | C₂H₅ | C(O)CH₃ | H | O |
| 1009 | C₂H₅ | C(O)CF₃ | H | O |
| 1010 | C₂H₅ | C(=NOCH₃)CH₃ | H | O |
| 1011 | C₂H₅ | SO₂CH₃ | H | O |
| 1012 | C₂H₅ | SO₂CF₃ | H | O |
| 1013 | C₂H₅ | CH₂CO₂H | H | O |
| 1014 | C₂H₅ | CH₂COOCH₃ | H | O |
| 1015 | C₂H₅ | CH₂COOC₂H₅ | H | O |
| 1016 | C₂H₅ | Prop-1-en-3-yl | H | O |
| 1017 | C₂H₅ | trans-But-2-en-1-yl | H | O |
| 1018 | C₂H₅ | cis-But-2-en-1-yl | H | O |
| 1019 | C₂H₅ | cis-3-Methyl-but-2-en-1-yl | H | O |
| 1020 | C₂H₅ | Cyclopropyl | H | O |
| 1021 | C₂H₅ | Cyclopentyl | H | O |
| 1022 | C₂H₅ | Cyclohexyl | H | O |
| 1023 | C₂H₅ | 4,5-Dihydroisoxazol-3-yl | H | O |
| 1024 | C₂H₅ | 4-Methyl-4,5-dihydroisoxazol-3-yl | H | O |
| 1025 | C₂H₅ | Isoxazol-3-yl | H | O |
| 1026 | C₂H₅ | 4-Methylisoxazol-3-yl | H | O |
| 1027 | C₂H₅ | 4,5-Dihydroisoxazol-4-yl | H | O |
| 1028 | C₂H₅ | 3-Methyl-4,5-dihydroisoxazol-4-yl | H | O |
| 1029 | C₂H₅ | Isoxazol-4-yl | H | O |
| 1030 | C₂H₅ | 3-Methylisoxazol-4-yl | H | O |
| 1031 | C₂H₅ | Phenyl | H | O |
| 1032 | C₂H₅ | Benzyl | H | O |
| 1033 | C₂H₅ | Benzoyl | H | O |
| 1034 | C₂H₅ | 2-Pyridyl | H | O |
| 1036 | C₂H₅ | CH₃ | CH₃ | O |
| 1037 | C₂H₅ | C₂H₅ | CH₃ | O |
| 1038 | C₂H₅ | n-C₃H₇ | CH₃ | O |
| 1039 | C₂H₅ | i-C₃H₇ | CH₃ | O |
| 1040 | C₂H₅ | n-C₄H₉ | CH₃ | O |
| 1041 | C₂H₅ | i-C₄H₉ | CH₃ | O |
| 1042 | C₂H₅ | s-C₄H₉ | CH₃ | O |
| 1043 | C₂H₅ | t-C₄H₉ | CH₃ | O |
| 1044 | C₂H₅ | CH₂OCH₃ | CH₃ | O |
| 1045 | C₂H₅ | CF₃ | CH₃ | O |
| 1046 | C₂H₅ | CF₂H | CH₃ | O |
| 1047 | C₂H₅ | CN | CH₃ | O |
| 1048 | C₂H₅ | OH | CH₃ | O |
| 1049 | C₂H₅ | OCH₃ | CH₃ | O |
| 1050 | C₂H₅ | NH₂ | CH₃ | O |
| 1051 | C₂H₅ | NHCH₃ | CH₃ | O |
| 1052 | C₂H₅ | N(CH₃)₂ | CH₃ | O |
| 1053 | C₂H₅ | CO₂CH₃ | CH₃ | O |
| 1054 | C₂H₅ | CO₂C₂H₅ | CH₃ | O |
| 1055 | C₂H₅ | C(O)CH₃ | CH₃ | O |
| 1056 | C₂H₅ | C(O)CF₃ | CH₃ | O |
| 1057 | C₂H₅ | C(=NOCH₃)CH₃ | CH₃ | O |
| 1058 | C₂H₅ | SO₂CH₃ | CH₃ | O |
| 1059 | C₂H₅ | SO₂CF₃ | CH₃ | O |
| 1060 | C₂H₅ | CH₂CO₂H | CH₃ | O |
| 1061 | C₂H₅ | CH₂COOCH₃ | CH₃ | O |
| 1062 | C₂H₅ | CH₂COOC₂H₅ | CH₃ | O |
| 1063 | C₂H₅ | Prop-1-en-3-yl | CH₃ | O |
| 1064 | C₂H₅ | trans-But-2-en-1-yl | CH₃ | O |
| 1065 | C₂H₅ | cis-But-2-en-1-yl | CH₃ | O |
| 1066 | C₂H₅ | cis-3-Methyl-but-2-en-1-yl | CH₃ | O |
| 1067 | C₂H₅ | Cyclopropyl | CH₃ | O |
| 1068 | C₂H₅ | Cyclopentyl | CH₃ | O |
| 1069 | C₂H₅ | Cyclohexyl | CH₃ | O |
| 1070 | C₂H₅ | 4,5-Dihydroisoxazol-3-yl | CH₃ | O |
| 1071 | C₂H₅ | 4-Methyl-4,5-dihydroisoxazol-3-yl | CH₃ | O |
| 1072 | C₂H₅ | Isoxazol-3-yl | CH₃ | O |
| 1073 | C₂H₅ | 4-Methylisoxazol-3-yl | CH₃ | O |
| 1074 | C₂H₅ | 4,5-Dihydroisoxazol-4-yl | CH₃ | O |
| 1075 | C₂H₅ | 3-Methyl-4,5-dihydroisoxazol-4-yl | CH₃ | O |
| 1076 | C₂H₅ | Isoxazol-4-yl | CH₃ | O |
| 1077 | C₂H₅ | 3-Methylisoxazol-4-yl | CH₃ | O |
| 1078 | C₂H₅ | Phenyl | CH₃ | O |
| 1079 | C₂H₅ | Benzyl | CH₃ | O |
| 1080 | C₂H₅ | Benzoyl | CH₃ | O |
| 1081 | C₂H₅ | 2-Pyridyl | CH₃ | O |
| 1083 | C₂H₅ | CH₃ | Cl | O |
| 1084 | C₂H₅ | C₂H₅ | Cl | O |
| 1085 | C₂H₅ | n-C₃H₇ | Cl | O |
| 1086 | C₂H₅ | i-C₃H₇ | Cl | O |
| 1087 | C₂H₅ | n-C₄H₉ | Cl | O |
| 1088 | C₂H₅ | i-C₄H₉ | Cl | O |
| 1089 | C₂H₅ | s-C₄H₉ | Cl | O |
| 1090 | C₂H₅ | t-C₄H₉ | Cl | O |
| 1091 | C₂H₅ | CH₂OCH₃ | Cl | O |
| 1092 | C₂H₅ | CF₃ | Cl | O |
| 1093 | C₂H₅ | CF₂H | Cl | O |
| 1094 | C₂H₅ | CN | Cl | O |
| 1095 | C₂H₅ | OH | Cl | O |
| 1096 | C₂H₅ | OCH₃ | Cl | O |
| 1097 | C₂H₅ | NH₂ | Cl | O |
| 1098 | C₂H₅ | NHCH₃ | Cl | O |
| 1099 | C₂H₅ | N(CH₃)₂ | Cl | O |
| 1100 | C₂H₅ | CO₂CH₃ | Cl | O |
| 1101 | C₂H₅ | CO₂C₂H₅ | Cl | O |
| 1102 | C₂H₅ | C(O)CH₃ | Cl | O |
| 1103 | C₂H₅ | C(O)CF₃ | Cl | O |
| 1104 | C₂H₅ | C(=NOCH₃)CH₃ | Cl | O |
| 1105 | C₂H₅ | SO₂CH₃ | Cl | O |
| 1106 | C₂H₅ | SO₂CF₃ | Cl | O |
| 1107 | C₂H₅ | CH₂CO₂H | Cl | O |
| 1108 | C₂H₅ | CH₂COOCH₃ | Cl | O |
| 1109 | C₂H₅ | CH₂COOC₂H₅ | Cl | O |
| 1110 | C₂H₅ | Prop-1-en-3-yl | Cl | O |
| 1111 | C₂H₅ | trans-But-2-en-1-yl | Cl | O |
| 1112 | C₂H₅ | cis-But-2-en-1-yl | Cl | O |
| 1113 | C₂H₅ | cis-3-Methyl-but-2-en-1-yl | Cl | O |
| 1114 | C₂H₅ | Cyclopropyl | Cl | O |
| 1115 | C₂H₅ | Cyclopentyl | Cl | O |
| 1116 | C₂H₅ | Cyclohexyl | Cl | O |
| 1117 | C₂H₅ | 4,5-Dihydroisoxazol-3-yl | Cl | O |
| 1118 | C₂H₅ | 4-Methyl-4,5-dihydroisoxazol-3-yl | Cl | O |
| 1119 | C₂H₅ | Isoxazol-3-yl | Cl | O |
| 1120 | C₂H₅ | 4-Methylisoxazol-3-yl | Cl | O |
| 1121 | C₂H₅ | 4,5-Dihydroisoxazol-4-yl | Cl | O |
| 1122 | C₂H₅ | 3-Methyl-4,5-dihydroisoxazol-4-yl | Cl | O |
| 1123 | C₂H₅ | Isoxazol-4-yl | Cl | O |
| 1124 | C₂H₅ | 3-Methylisoxazol-4-yl | Cl | O |
| 1125 | C₂H₅ | Phenyl | Cl | O |
| 1126 | C₂H₅ | Benzyl | Cl | O |
| 1127 | C₂H₅ | Benzoyl | Cl | O |
| 1128 | C₂H₅ | 2-Pyridyl | Cl | O |
| 1129 | CH₃ | CH₃ | H | S |
| 1131 | CH₃ | C₂H₅ | H | S |
| 1132 | CH₃ | n-C₃H₇ | H | S |
| 1133 | CH₃ | i-C₃H₇ | H | S |
| 1134 | CH₃ | n-C₄H₉ | H | S |
| 1135 | CH₃ | i-C₄H₉ | H | S |
| 1136 | CH₃ | s-C₄H₉ | H | S |
| 1137 | CH₃ | t-C₄H₉ | H | S |
| 1138 | CH₃ | CH₂OCH₃ | H | S |
| 1139 | CH₃ | CF₃ | H | S |
| 1140 | CH₃ | CF₂H | H | S |
| 1141 | CH₃ | CN | H | S |
| 1142 | CH₃ | OH | H | S |
| 1143 | CH₃ | OCH₃ | H | S |
| 1144 | CH₃ | NH₂ | H | S |
| 1145 | CH₃ | NHCH₃ | H | S |
| 1146 | CH₃ | N(CH₃)₂ | H | S |
| 1147 | CH₃ | CO₂CH₃ | H | S |
| 1148 | CH₃ | CO₂C₂H₅ | H | S |
| 1149 | CH₃ | C(O)CH₃ | H | S |
| 1150 | CH₃ | C(O)CF₃ | H | S |
| 1151 | CH₃ | C(=NOCH₃)CH₃ | H | S |
| 1152 | CH₃ | SO₂CH₃ | H | S |
| 1153 | CH₃ | SO₂CF₃ | H | S |
| 1154 | CH₃ | CH₂CO₂H | H | S |
| 1155 | CH₃ | CH₂COOCH₃ | H | S |
| 1156 | CH₃ | CH₂COOC₂H₅ | H | S |
| 1157 | CH₃ | Prop-1-en-3-yl | H | S |
| 1158 | CH₃ | trans-But-2-en-1-yl | H | S |
| 1159 | CH₃ | cis-But-2-en-1-yl | H | S |
| 1160 | CH₃ | cis-3-Methyl-but-2-en-1-yl | H | S |
| 1161 | CH₃ | Cyclopropyl | H | S |
| 1162 | CH₃ | Cyclopentyl | H | S |
| 1163 | CH₃ | Cyclohexyl | H | S |
| 1164 | CH₃ | 4,5-Dihydroisoxazol-3-yl | H | S |
| 1165 | CH₃ | 4-Methyl-4,5-dihydroisoxazol-3-yl | H | S |
| 1166 | CH₃ | Isoxazol-3-yl | H | S |
| 1167 | CH₃ | 4-Methylisoxazol-3-yl | H | S |
| 1168 | CH₃ | 4,5-Dihydroisoxazol-4-yl | H | S |
| 1169 | CH₃ | 3-Methyl-4,5-dihydroisoxazol-4-yl | H | S |
| 1170 | CH₃ | Isoxazol-4-yl | H | S |
| 1171 | CH₃ | 3-Methylisoxazol-4-yl | H | S |
| 1172 | CH₃ | Phenyl | H | S |
| 1173 | CH₃ | Benzyl | H | S |
| 1174 | CH₃ | Benzoyl | H | S |
| 1175 | CH₃ | 2-Pyridyl | H | S |
| 1177 | CH₃ | CH₃ | CH₃ | S |
| 1178 | CH₃ | C₂H₅ | CH₃ | S |
| 1179 | CH₃ | n-C₃H₇ | CH₃ | S |
| 1180 | CH₃ | i-C₃H₇ | CH₃ | S |
| 1181 | CH₃ | n-C₄H₉ | CH₃ | S |
| 1182 | CH₃ | i-C₄H₉ | CH₃ | S |
| 1183 | CH₃ | s-C₄H₉ | CH₃ | S |
| 1184 | CH₃ | t-C₄H₉ | CH₃ | S |
| 1185 | CH₃ | CH₂OCH₃ | CH₃ | S |
| 1186 | CH₃ | CF₃ | CH₃ | S |
| 1187 | CH₃ | CF₂H | CH₃ | S |
| 1188 | CH₃ | CN | CH₃ | S |
| 1189 | CH₃ | OH | CH₃ | S |
| 1190 | CH₃ | OCH₃ | CH₃ | S |
| 1191 | CH₃ | NH₂ | CH₃ | S |
| 1192 | CH₃ | NHCH₃ | CH₃ | S |
| 1193 | CH₃ | N(CH₃)₂ | CH₃ | S |
| 1194 | CH₃ | CO₂CH₃ | CH₃ | S |
| 1195 | CH₃ | CO₂C₂H₅ | CH₃ | S |
| 1196 | CH₃ | C(O)CH₃ | CH₃ | S |
| 1197 | CH₃ | C(O)CF₃ | CH₃ | S |
| 1198 | CH₃ | C(=NOCH₃)CH₃ | CH₃ | S |
| 1199 | CH₃ | SO₂CH₃ | CH₃ | S |
| 1200 | CH₃ | SO₂CF₃ | CH₃ | S |
| 1201 | CH₃ | CH₂CO₂H | CH₃ | S |
| 1202 | CH₃ | CH₂COOCH₃ | CH₃ | S |
| 1203 | CH₃ | CH₂COOC₂H₅ | CH₃ | S |
| 1204 | CH₃ | Prop-1-en-3-yl | CH₃ | S |
| 1205 | CH₃ | trans-But-2-en-1-yl | CH₃ | S |
| 1206 | CH₃ | cis-But-2-en-1-yl | CH₃ | S |
| 1207 | CH₃ | cis-3-Methyl-but-2-en-1-yl | CH₃ | S |
| 1208 | CH₃ | Cyclopropyl | CH₃ | S |
| 1209 | CH₃ | Cyclopentyl | CH₃ | S |
| 1210 | CH₃ | Cyclohexyl | CH₃ | S |
| 1211 | CH₃ | 4,5-Dihydroisoxazol-3-yl | CH₃ | S |
| 1212 | CH₃ | 4-Methyl-4,5-dihydroisoxazol-3-yl | CH₃ | S |
| 1213 | CH₃ | Isoxazol-3-yl | CH₃ | S |
| 1214 | CH₃ | 4-Methylisoxazol-3-yl | CH₃ | S |
| 1215 | CH₃ | 4,5-Dihydroisoxazol-4-yl | CH₃ | S |
| 1216 | CH₃ | 3-Methyl-4,5-dihydroisoxazol-4-yl | CH₃ | S |
| 1217 | CH₃ | Isoxazol-4-yl | CH₃ | S |
| 1218 | CH₃ | 3-Methylisoxazol-4-yl | CH₃ | S |
| 1219 | CH₃ | Phenyl | CH₃ | S |
| 1220 | CH₃ | Benzyl | CH₃ | S |
| 1221 | CH₃ | Benzoyl | CH₃ | S |
| 1222 | CH₃ | 2-Pyridyl | CH₃ | S |
| 1224 | CH₃ | CH₃ | Cl | S |
| 1225 | CH₃ | C₂H₅ | Cl | S |
| 1226 | CH₃ | n-C₃H₇ | Cl | S |
| 1227 | CH₃ | i-C₃H₇ | Cl | S |
| 1228 | CH₃ | n-C₄H₉ | Cl | S |
| 1229 | CH₃ | i-C₄H₉ | Cl | S |
| 1230 | CH₃ | s-C₄H₉ | Cl | S |
| 1231 | CH₃ | t-C₄H₉ | Cl | S |
| 1232 | CH₃ | CH₂OCH₃ | Cl | S |
| 1233 | CH₃ | CF₃ | Cl | S |
| 1234 | CH₃ | CF₂H | Cl | S |
| 1235 | CH₃ | CN | Cl | S |
| 1236 | CH₃ | OH | Cl | S |
| 1237 | CH₃ | OCH₃ | Cl | S |
| 1238 | CH₃ | NH₂ | Cl | S |
| 1239 | CH₃ | NHCH₃ | Cl | S |
| 1240 | CH₃ | N(CH₃)₂ | Cl | S |
| 1241 | CH₃ | CO₂CH₃ | Cl | S |
| 1242 | CH₃ | CO₂C₂H₅ | Cl | S |
| 1243 | CH₃ | C(O)CH₃ | Cl | S |
| 1244 | CH₃ | C(O)CF₃ | Cl | S |
| 1245 | CH₃ | C(=NOCH₃)CH₃ | Cl | S |
| 1246 | CH₃ | SO₂CH₃ | Cl | S |
| 1247 | CH₃ | SO₂CF₃ | Cl | S |
| 1248 | CH₃ | CH₂CO₂H | Cl | S |
| 1249 | CH₃ | CH₂COOCH₃ | Cl | S |
| 1250 | CH₃ | CH₂COOC₂H₅ | Cl | S |
| 1251 | CH₃ | Prop-1-en-3-yl | Cl | S |
| 1252 | CH₃ | trans-But-2-en-1-yl | Cl | S |
| 1253 | CH₃ | cis-But-2-en-1-yl | Cl | S |
| 1254 | CH₃ | cis-3-Methyl-but-2-en-1-yl | Cl | S |
| 1255 | CH₃ | Cyclopropyl | Cl | S |
| 1256 | CH₃ | Cyclopentyl | Cl | S |
| 1257 | CH₃ | Cyclohexyl | Cl | S |
| 1258 | CH₃ | 4,5-Dihydroisoxazol-3-yl | Cl | S |
| 1259 | CH₃ | 4-Methyl-4,5-dihydroisoxazol-3-yl | Cl | S |
| 1260 | CH₃ | Isoxazol-3-yl | Cl | S |
| 1261 | CH₃ | 4-Methylisoxazol-3-yl | Cl | S |
| 1262 | CH₃ | 4,5-Dihydroisoxazol-4-yl | Cl | S |
| 1263 | CH₃ | 3-Methyl-4,5-dihydroisoxazol-4-yl | Cl | S |
| 1264 | CH₃ | Isoxazol-4-yl | Cl | S |
| 1265 | CH₃ | 3-Methylisoxazol-4-yl | Cl | S |
| 1266 | CH₃ | Phenyl | Cl | S |
| 1267 | CH₃ | Benzyl | Cl | S |
| 1268 | CH₃ | Benzoyl | Cl | S |
| 1269 | CH₃ | 2-Pyridyl | Cl | S |
| 1271 | Cl | CH₃ | H | S |
| 1272 | Cl | C₂H₅ | H | S |
| 1273 | Cl | n-C₃H₇ | H | S |
| 1274 | Cl | i-C₃H₇ | H | S |
| 1275 | Cl | n-C₄H₉ | H | S |
| 1276 | Cl | i-C₄H₉ | H | S |
| 1277 | Cl | s-C₄H₉ | H | S |
| 1278 | Cl | t-C₄H₉ | H | S |
| 1279 | Cl | CH₂OCH₃ | H | S |
| 1280 | Cl | CF₃ | H | S |
| 1281 | Cl | CF₂H | H | S |
| 1282 | Cl | CN | H | S |
| 1283 | Cl | OH | H | S |
| 1284 | Cl | OCH₃ | H | S |
| 1285 | Cl | NH₂ | H | S |
| 1286 | Cl | NHCH₃ | H | S |
| 1287 | Cl | N(CH₃)₂ | H | S |
| 1288 | Cl | CO₂CH₃ | H | S |
| 1289 | Cl | CO₂C₂H₅ | H | S |
| 1290 | Cl | C(O)CH₃ | H | S |
| 1291 | Cl | C(O)CF₃ | H | S |
| 1292 | Cl | C(=NOCH₃)CH₃ | H | S |
| 1293 | Cl | SO₂CH₃ | H | S |
| 1294 | Cl | SO₂CF₃ | H | S |
| 1295 | Cl | CH₂CO₂H | H | S |
| 1296 | Cl | CH₂COOCH₃ | H | S |
| 1297 | Cl | CH₂COOC₂H₅ | H | S |
| 1298 | Cl | Prop-1-en-3-yl | H | S |
| 1299 | Cl | trans-But-2-en-1-yl | H | S |
| 1300 | Cl | cis-But-2-en-1-yl | H | S |
| 1301 | Cl | cis-3-Methyl-but-2-en-1-yl | H | S |
| 1302 | Cl | Cyclopropyl | H | S |
| 1303 | Cl | Cyclopentyl | H | S |
| 1304 | Cl | Cyclohexyl | H | S |
| 1305 | Cl | 4,5-Dihydroisoxazol-3-yl | H | S |
| 1306 | Cl | 4-Methyl-4,5-dihydroisoxazol-3-yl | H | S |
| 1307 | Cl | Isoxazol-3-yl | H | S |
| 1308 | Cl | 4-Methylisoxazol-3-yl | H | S |
| 1309 | Cl | 4,5-Dihydroisoxazol-4-yl | H | S |
| 1310 | Cl | 3-Methyl-4,5-dihydroisoxazol-4-yl | H | S |
| 1311 | Cl | Isoxazol-4-yl | H | S |
| 1312 | Cl | 3-Methylisoxazol-4-yl | H | S |
| 1313 | Cl | Phenyl | H | S |
| 1314 | Cl | Benzyl | H | S |
| 1315 | Cl | Benzoyl | H | S |
| 1316 | Cl | 2-Pyridyl | H | S |
| 1318 | Cl | CH₃ | CH₃ | S |
| 1319 | Cl | C₂H₅ | CH₃ | S |
| 1320 | Cl | n-C₃H₇ | CH₃ | S |
| 1321 | Cl | i-C₃H₇ | CH₃ | S |
| 1322 | Cl | n-C₄H₉ | CH₃ | S |
| 1323 | Cl | i-C₄H₉ | CH₃ | S |
| 1324 | Cl | s-C₄H₉ | CH₃ | S |
| 1325 | Cl | t-C₄H₉ | CH₃ | S |
| 1326 | Cl | CH₂OCH₃ | CH₃ | S |
| 1327 | Cl | CF₃ | CH₃ | S |
| 1328 | Cl | CF₂H | CH₃ | S |
| 1329 | Cl | CN | CH₃ | S |
| 1330 | Cl | OH | CH₃ | S |
| 1331 | Cl | OCH₃ | CH₃ | S |
| 1332 | Cl | NH₂ | CH₃ | S |
| 1333 | Cl | NHCH₃ | CH₃ | S |
| 1334 | Cl | N(CH₃)₂ | CH₃ | S |
| 1335 | Cl | CO₂CH₃ | CH₃ | S |
| 1336 | Cl | CO₂C₂H₅ | CH₃ | S |
| 1337 | Cl | C(O)CH₃ | CH₃ | S |
| 1338 | Cl | C(O)CF₃ | CH₃ | S |
| 1339 | Cl | C(=NOCH₃)CH₃ | CH₃ | S |
| 1340 | Cl | SO₂CH₃ | CH₃ | S |
| 1341 | Cl | SO₂CF₃ | CH₃ | S |
| 1342 | Cl | CH₂CO₂H | CH₃ | S |
| 1343 | Cl | CH₂COOCH₃ | CH₃ | S |
| 1344 | Cl | CH₂COOC₂H₅ | CH₃ | S |
| 1345 | Cl | Prop-1-en-3-yl | CH₃ | S |
| 1346 | Cl | trans-But-2-en-1-yl | CH₃ | S |
| 1347 | Cl | cis-But-2-en-1-yl | CH₃ | S |
| 1348 | Cl | cis-3-Methyl-but-2-en-1-yl | CH₃ | S |
| 1349 | Cl | Cyclopropyl | CH₃ | S |
| 1350 | Cl | Cyclopentyl | CH₃ | S |
| 1351 | Cl | Cyclohexyl | CH₃ | S |
| 1352 | Cl | 4,5-Dihydroisoxazol-3-yl | CH₃ | S |
| 1353 | Cl | 4-Methyl-4,5-dihydroisoxazol-3-yl | CH₃ | S |
| 1354 | Cl | Isoxazol-3-yl | CH₃ | S |
| 1355 | Cl | 4-Methylisoxazol-3-yl | CH₃ | S |
| 1356 | Cl | 4,5-Dihydroisoxazol-4-yl | CH₃ | S |
| 1357 | Cl | 3-Methyl-4,5-dihydroisoxazol-4-yl | CH₃ | S |
| 1358 | Cl | Isoxazol-4-yl | CH₃ | S |
| 1359 | Cl | 3-Methylisoxazol-4-yl | CH₃ | S |
| 1360 | Cl | Phenyl | CH₃ | S |
| 1361 | Cl | Benzyl | CH₃ | S |
| 1362 | Cl | Benzoyl | CH₃ | S |
| 1363 | Cl | 2-Pyridyl | CH₃ | S |
| 1365 | Cl | CH₃ | Cl | S |
| 1366 | Cl | C₂H₅ | Cl | S |
| 1367 | Cl | n-C₃H₇ | Cl | S |
| 1368 | Cl | i-C₃H₇ | Cl | S |
| 1369 | Cl | n-C₄H₉ | Cl | S |
| 1370 | Cl | i-C₄H₉ | Cl | S |
| 1371 | Cl | s-C₄H₉ | Cl | S |
| 1372 | Cl | t-C₄H₉ | Cl | S |
| 1373 | Cl | CH₂OCH₃ | Cl | S |
| 1374 | Cl | CF₃ | Cl | S |
| 1375 | Cl | CF₂H | Cl | S |
| 1376 | Cl | CN | Cl | S |
| 1377 | Cl | OH | Cl | S |
| 1378 | Cl | OCH₃ | Cl | S |
| 1379 | Cl | NH₂ | Cl | S |
| 1380 | Cl | NHCH₃ | Cl | S |
| 1381 | Cl | N(CH₃)₂ | Cl | S |
| 1382 | Cl | CO₂CH₃ | Cl | S |
| 1383 | Cl | CO₂C₂H₅ | Cl | S |
| 1384 | Cl | C(O)CH₃ | Cl | S |
| 1385 | Cl | C(O)CF₃ | Cl | S |
| 1386 | Cl | C(=NOCH₃)CH₃ | Cl | S |
| 1387 | Cl | SO₂CH₃ | Cl | S |
| 1388 | Cl | SO₂CF₃ | Cl | S |
| 1389 | Cl | CH₂CO₂H | Cl | S |
| 1390 | Cl | CH₂COOCH₃ | Cl | S |
| 1391 | Cl | CH₂COOC₂H₅ | Cl | S |
| 1392 | Cl | Prop-1-en-3-yl | Cl | S |
| 1393 | Cl | trans-But-2-en-1-yl | Cl | S |
| 1394 | Cl | cis-But-2-en-1-yl | Cl | S |
| 1395 | Cl | cis-3-Methyl-but-2-en-1-yl | Cl | S |
| 1396 | Cl | Cyclopropyl | Cl | S |
| 1397 | Cl | Cyclopentyl | Cl | S |
| 1398 | Cl | Cyclohexyl | Cl | S |
| 1399 | Cl | 4,5-Dihydroisoxazol-3-yl | Cl | S |
| 1400 | Cl | 4-Methyl-4,5-dihydroisoxazol-3-yl | Cl | S |
| 1401 | Cl | Isoxazol-3-yl | Cl | S |
| 1402 | Cl | 4-Methylisoxazol-3-yl | Cl | S |
| 1403 | Cl | 4,5-Dihydroisoxazol-4-yl | Cl | S |
| 1404 | Cl | 3-Methyl-4,5-dihydroisoxazol-4-yl | Cl | S |
| 1405 | Cl | Isoxazol-4-yl | Cl | S |
| 1406 | Cl | 3-Methylisoxazol-4-yl | Cl | S |
| 1407 | Cl | Phenyl | Cl | S |
| 1408 | Cl | Benzyl | Cl | S |
| 1409 | Cl | Benzoyl | Cl | S |
| 1410 | Cl | 2-Pyridyl | Cl | S |
| 1412 | OCH₃ | CH₃ | H | S |
| 1413 | OCH₃ | C₂H₅ | H | S |
| 1414 | OCH₃ | n-C₃H₇ | H | S |
| 1415 | OCH₃ | i-C₃H₇ | H | S |
| 1416 | OCH₃ | n-C₄H₉ | H | S |
| 1417 | OCH₃ | i-C₄H₉ | H | S |
| 1418 | OCH₃ | s-C₄H₉ | H | S |
| 1419 | OCH₃ | t-C₄H₉ | H | S |
| 1420 | OCH₃ | CH₂OCH₃ | H | S |
| 1421 | OCH₃ | CF₃ | H | S |
| 1422 | OCH₃ | CF₂H | H | S |
| 1423 | OCH₃ | CN | H | S |
| 1424 | OCH₃ | OH | H | S |
| 1425 | OCH₃ | OCH₃ | H | S |
| 1426 | OCH₃ | NH₂ | H | S |
| 1427 | OCH₃ | NHCH₃ | H | S |
| 1428 | OCH₃ | N(CH₃)₂ | H | S |
| 1429 | OCH₃ | CO₂CH₃ | H | S |
| 1430 | OCH₃ | CO₂C₂H₅ | H | S |
| 1431 | OCH₃ | C(O)CH₃ | H | S |
| 1432 | OCH₃ | C(O)CF₃ | H | S |
| 1433 | OCH₃ | C(=NOCH₃)CH₃ | H | S |
| 1434 | OCH₃ | SO₂CH₃ | H | S |
| 1435 | OCH₃ | SO₂CF₃ | H | S |
| 1436 | OCH₃ | CH₂CO₂H | H | S |
| 1437 | OCH₃ | CH₂COOCH₃ | H | S |
| 1438 | OCH₃ | CH₂COOC₂H₅ | H | S |
| 1439 | OCH₃ | Prop-1-en-3-yl | H | S |
| 1440 | OCH₃ | trans-But-2-en-1-yl | H | S |
| 1441 | OCH₃ | cis-But-2-en-1-yl | H | S |
| 1442 | OCH₃ | cis-3-Methyl-but-2-en-1-yl | H | S |
| 1443 | OCH₃ | Cyclopropyl | H | S |
| 1444 | OCH₃ | Cyclopentyl | H | S |
| 1445 | OCH₃ | Cyclohexyl | H | S |
| 1446 | OCH₃ | 4,5-Dihydroisoxazol-3-yl | H | S |
| 1447 | OCH₃ | 4-Methyl-4,5-dihydroisoxazol-3-yl | H | S |
| 1448 | OCH₃ | Isoxazol-3-yl | H | S |
| 1449 | OCH₃ | 4-Methylisoxazol-3-yl | H | S |
| 1450 | OCH₃ | 4,5-Dihydroisoxazol-4-yl | H | S |
| 1451 | OCH₃ | 3-Methyl-4,5-dihydroisoxazol-4-yl | H | S |
| 1452 | OCH₃ | Isoxazol-4-yl | H | S |
| 1453 | OCH₃ | 3-Methylisoxazol-4-yl | H | S |
| 1454 | OCH₃ | Phenyl | H | S |
| 1455 | OCH₃ | Benzyl | H | S |
| 1456 | OCH₃ | Benzoyl | H | S |
| 1457 | OCH₃ | 2-Pyridyl | H | S |
| 1459 | OCH₃ | CH₃ | CH₃ | S |
| 1460 | OCH₃ | C₂H₅ | CH₃ | S |
| 1461 | OCH₃ | n-C₃H₇ | CH₃ | S |
| 1462 | OCH₃ | i-C₃H₇ | CH₃ | S |
| 1463 | OCH₃ | n-C₄H₉ | CH₃ | S |
| 1464 | OCH₃ | i-C₄H₉ | CH₃ | S |
| 1465 | OCH₃ | s-C₄H₉ | CH₃ | S |
| 1466 | OCH₃ | t-C₄H₉ | CH₃ | S |
| 1467 | OCH₃ | CH₂OCH₃ | CH₃ | S |
| 1468 | OCH₃ | CF₃ | CH₃ | S |
| 1469 | OCH₃ | CF₂H | CH₃ | S |
| 1470 | OCH₃ | CN | CH₃ | S |
| 1471 | OCH₃ | OH | CH₃ | S |
| 1472 | OCH₃ | OCH₃ | CH₃ | S |
| 1473 | OCH₃ | NH₂ | CH₃ | S |
| 1474 | OCH₃ | NHCH₃ | CH₃ | S |
| 1475 | OCH₃ | N(CH₃)₂ | CH₃ | S |
| 1476 | OCH₃ | CO₂CH₃ | CH₃ | S |
| 1477 | OCH₃ | CO₂C₂H₅ | CH₃ | S |
| 1478 | OCH₃ | C(O)CH₃ | CH₃ | S |
| 1479 | OCH₃ | C(O)CF₃ | CH₃ | S |
| 1480 | OCH₃ | C(=NOCH₃)CH₃ | CH₃ | S |
| 1481 | OCH₃ | SO₂CH₃ | CH₃ | S |
| 1482 | OCH₃ | SO₂CF₃ | CH₃ | S |
| 1483 | OCH₃ | CH₂CO₂H | CH₃ | S |
| 1484 | OCH₃ | CH₂COOCH₃ | CH₃ | S |
| 1485 | OCH₃ | CH₂COOC₂H₅ | CH₃ | S |
| 1486 | OCH₃ | Prop-1-en-3-yl | CH₃ | S |
| 1487 | OCH₃ | trans-But-2-en-1-yl | CH₃ | S |
| 1488 | OCH₃ | cis-But-2-en-1-yl | CH₃ | S |
| 1489 | OCH₃ | cis-3-Methyl-but-2-en-1-yl | CH₃ | S |
| 1490 | OCH₃ | Cyclopropyl | CH3 | S |
| 1491 | OCH₃ | Cyclopentyl | CH₃ | S |
| 1492 | OCH₃ | Cyclohexyl | CH₃ | S |
| 1493 | OCH₃ | 4,5-Dihydroisoxazol-3-yl | CH₃ | S |
| 1494 | OCH₃ | 4-Methyl-4,5-dihydroisoxazol-3-yl | CH₃ | S |
| 1495 | OCH₃ | Isoxazol-3-yl | CH₃ | S |
| 1496 | OCH₃ | 4-Methylisoxazol-3-yl | CH₃ | S |
| 1497 | OCH₃ | 4,5-Dihydroisoxazol-4-yl | CH₃ | S |
| 1498 | OCH₃ | 3-Methyl-4,5-dihydroisoxazol-4-yl | CH₃ | S |
| 1499 | OCH₃ | Isoxazol-4-yl | CH₃ | S |
| 1500 | OCH₃ | 3-Methylisoxazol-4-yl | CH₃ | S |
| 1501 | OCH₃ | Phenyl | CH₃ | S |
| 1502 | OCH₃ | Benzyl | CH₃ | S |
| 1503 | OCH₃ | Benzoyl | CH₃ | S |
| 1504 | OCH₃ | 2-Pyridyl | CH₃ | S |
| 1506 | OCH₃ | CH₃ | Cl | S |
| 1507 | OCH₃ | C₂H₅ | Cl | S |
| 1508 | OCH₃ | n-C₃H₇ | Cl | S |
| 1509 | OCH₃ | i-C₃H₇ | Cl | S |
| 1510 | OCH₃ | n-C₄H₉ | Cl | S |
| 1511 | OCH₃ | i-C₄H₉ | Cl | S |
| 1512 | OCH₃ | s-C₄H₉ | Cl | S |
| 1513 | OCH₃ | t-C₄H₉ | Cl | S |
| 1514 | OCH₃ | CH₂OCH₃ | Cl | S |
| 1515 | OCH₃ | CF₃ | Cl | S |
| 1516 | OCH₃ | CF₂H | Cl | S |
| 1517 | OCH₃ | CN | Cl | S |
| 1518 | OCH₃ | OH | Cl | S |
| 1519 | OCH₃ | OCH₃ | Cl | S |
| 1520 | OCH₃ | NH₂ | Cl | S |
| 1521 | OCH₃ | NHCH₃ | Cl | S |
| 1522 | OCH₃ | N(CH₃)₂ | Cl | S |
| 1523 | OCH₃ | CO₂CH₃ | Cl | S |
| 1524 | OCH₃ | CO₂C₂H₅ | Cl | S |
| 1525 | OCH₃ | C(O)CH₃ | Cl | S |
| 1526 | OCH₃ | C(O)CF₃ | Cl | S |
| 1527 | OCH₃ | C(=NOCH₃)CH₃ | Cl | S |
| 1528 | OCH₃ | SO₂CH₃ | Cl | S |
| 1529 | OCH₃ | SO₂CF₃ | Cl | S |
| 1530 | OCH₃ | CH₂CO₂H | Cl | S |
| 1531 | OCH₃ | CH₂COOCH₃ | Cl | S |
| 1532 | OCH₃ | CH₂COOC₂H₅ | Cl | S |
| 1533 | OCH₃ | Prop-1-en-3-yl | Cl | S |
| 1534 | OCH₃ | trans-But-2-en-1-yl | Cl | S |
| 1535 | OCH₃ | cis-But-2-en-1-yl | Cl | S |
| 1536 | OCH₃ | cis-3-Methyl-but-2-en-1-yl | Cl | S |
| 1537 | OCH₃ | Cyclopropyl | Cl | S |
| 1538 | OCH₃ | Cyclopentyl | Cl | S |
| 1539 | OCH₃ | Cyclohexyl | Cl | S |
| 1540 | OCH₃ | 4,5-Dihydroisoxazol-3-yl | Cl | S |
| 1541 | OCH₃ | 4-Methyl-4,5-dihydroisoxazol-3-yl | Cl | S |
| 1542 | OCH₃ | Isoxazol-3-yl | Cl | S |
| 1543 | OCH₃ | 4-Methylisoxazol-3-yl | Cl | S |
| 1544 | OCH₃ | 4,5-Dihydroisoxazol-4-yl | Cl | S |
| 154-5 | OCH₃ | 3-Methyl-4,5-dihydroisoxazol-4-yl | Cl | S |
| 1546 | OCH₃ | Isoxazol-4-yl | Cl | S |
| 1547 | OCH₃ | 3-Methylisoxazol-4-yl | Cl | S |
| 1548 | OCH₃ | Phenyl | Cl | S |
| 1549 | OCH₃ | Benzyl | Cl | S |
| 1550 | OCH₃ | Benzoyl | Cl | S |
| 1551 | OCH₃ | 2-Pyridyl | Cl | S |
| 1553 | OCF₃ | CH₃ | H | S |
| 1554 | OCF₃ | C₂H₅ | H | S |
| 1555 | OCF₃ | n-C₃H₇ | H | S |
| 1556 | OCF₃ | i-C₃H₇ | H | S |
| 1557 | OCF₃ | n-C₄H₉ | H | S |
| 1558 | OCF₃ | i-C₄H₉ | H | S |
| 1559 | OCF₃ | s-C₄H₉ | H | S |
| 1560 | OCF₃ | t-C₄H₉ | H | S |
| 1561 | OCF₃ | CH₂OCH₃ | H | S |
| 1562 | OCF₃ | CF₃ | H | S |
| 1563 | OCF₃ | CF₂H | H | S |
| 1564 | OCF₃ | CN | H | S |
| 1565 | OCF₃ | OH | H | S |
| 1566 | OCF₃ | OCH₃ | H | S |
| 1567 | OCF₃ | NH₂ | H | S |
| 1568 | OCF₃ | NHCH₃ | H | S |
| 1569 | OCF₃ | N(CH₃)₂ | H | S |
| 1570 | OCF₃ | CO₂CH₃ | H | S |
| 1571 | OCF₃ | CO₂C₂H₅ | H | S |
| 1572 | OCF₃ | C(O)CH₃ | H | S |
| 1573 | OCF₃ | C(O)CF₃ | H | S |
| 1574 | OCF₃ | C(=NOCH₃)CH₃ | H | S |
| 1575 | OCF₃ | SO₂CH₃ | H | S |
| 1576 | OCF₃ | SO₂CF₃ | H | S |
| 1577 | OCF₃ | CH₂CO₂H | H | S |
| 1578 | OCF₃ | CH₂COOCH₃ | H | S |
| 1579 | OCF₃ | CH₂COOC₂H₅ | H | S |
| 1580 | OCF₃ | Prop-1-en-3-yl | H | S |
| 1581 | OCF₃ | trans-But-2-en-1-yl | H | S |
| 1582 | OCF₃ | cis-But-2-en-1-yl | H | S |
| 1583 | OCF₃ | cis-3-Methyl-but-2-en-1-yl | H | S |
| 1584 | OCF₃ | Cyclopropyl | H | S |
| 1585 | OCF₃ | Cyclopentyl | H | S |
| 1586 | OCF₃ | Cyclohexyl | H | S |
| 1587 | OCF₃ | 4,5-Dihydroisoxazol-3-yl | H | S |
| 1588 | OCF₃ | 4-Methyl-4,5-dihydroisoxazol-3-yl | H | S |
| 1589 | OCF₃ | Isoxazol-3-yl | H | S |
| 1590 | OCF₃ | 4-Methylisoxazol-3-yl | H | S |
| 1591 | OCF₃ | 4,5-Dihydroisoxazol-4-yl | H | S |
| 1592 | OCF₃ | 3-Methyl-4,5-dihydroisoxazol-4-yl | H | S |
| 1593 | OCF₃ | Isoxazol-4-yl | H | S |
| 1594 | OCF₃ | 3-Methylisoxazol-4-yl | H | S |
| 1595 | OCF₃ | Phenyl | H | S |
| 1596 | OCF₃ | Benzyl | H | S |
| 1597 | OCF₃ | Benzoyl | H | S |
| 1598 | OCF₃ | 2-Pyridyl | H | S |
| 1600 | OCF₃ | CH₃ | CH₃ | S |
| 1601 | OCF₃ | C₂H₅ | CH₃ | S |
| 1602 | OCF₃ | n-C₃H₇ | CH₃ | S |
| 1603 | OCF₃ | i-C₃H₇ | CH₃ | S |
| 1604 | OCF₃ | n-C₄H₉ | CH₃ | S |
| 1605 | OCF₃ | i-C₄H₉ | CH₃ | S |
| 1606 | OCF₃ | s-C₄H₉ | CH₃ | S |
| 1607 | OCF₃ | t-C₄H₉ | CH₃ | S |
| 1608 | OCF₃ | CH₂OCH₃ | CH₃ | S |
| 1609 | OCF₃ | CF₃ | CH₃ | S |
| 1610 | OCF₃ | CF₂H | CH₃ | S |
| 1611 | OCF₃ | CN | CH₃ | S |
| 1612 | OCF₃ | OH | CH₃ | S |
| 1613 | OCF₃ | OCH₃ | CH₃ | S |
| 1614 | OCF₃ | NH₂ | CH₃ | S |
| 1615 | OCF₃ | NHCH₃ | CH₃ | S |
| 1616 | OCF₃ | N(CH₃)₂ | CH₃ | S |
| 1617 | OCF₃ | CO₂CH₃ | CH₃ | S |
| 1618 | OCF₃ | CO₂C₂H₅ | CH₃ | S |
| 1619 | OCF₃ | C(O)CH₃ | CH₃ | S |
| 1620 | OCF₃ | C(O)CF₃ | CH₃ | S |
| 1621 | OCF₃ | C(=NOCH₃)CH₃ | CH₃ | S |
| 1622 | OCF₃ | SO₂CH₃ | CH₃ | S |
| 1623 | OCF₃ | SO₂CF₃ | CH₃ | S |
| 1624 | OCF₃ | CH₂CO₂H | CH₃ | S |
| 1625 | OCF₃ | CH₂COOCH₃ | CH₃ | S |
| 1626 | OCF₃ | CH₂COOC₂H₅ | CH₃ | S |
| 1627 | OCF₃ | Prop-1-en-3-yl | CH₃ | S |
| 1628 | OCF₃ | trans-But-2-en-1-yl | CH₃ | S |
| 1629 | OCF₃ | cis-But-2-en-1-yl | CH₃ | S |
| 1630 | OCF₃ | cis-3-Methyl-but-2-en-1-yl | CH₃ | S |
| 1631 | OCF₃ | Cyclopropyl | CH₃ | S |
| 1632 | OCF₃ | Cyclopentyl | CH₃ | S |
| 1633 | OCF₃ | Cyclohexyl | CH₃ | S |
| 1634 | OCF₃ | 4,5-Dihydroisoxazol-3-yl | CH₃ | S |
| 1635 | OCF₃ | 4-Methyl-4,5-dihydroisoxazol-3-yl | CH₃ | S |
| 1636 | OCF₃ | Isoxazol-3-yl | CH₃ | S |
| 1637 | OCF₃ | 4-Methylisoxazol-3-yl | CH₃ | S |
| 1638 | OCF₃ | 4,5-Dihydroisoxazol-4-yl | CH₃ | S |
| 1639 | OCF₃ | 3-Methyl-4,5-dihydroisoxazol-4-yl | CH₃ | S |
| 1640 | OCF₃ | Isoxazol-4-yl | CH₃ | S |
| 1641 | OCF₃ | 3-Methylisoxazol-4-yl | CH₃ | S |
| 1642 | OCF₃ | Phenyl | CH₃ | S |
| 1643 | OCF₃ | Benzyl | CH₃ | S |
| 1644 | OCF₃ | Benzoyl | CH₃ | S |
| 1645 | OCF₃ | 2-Pyridyl | CH₃ | S |
| 1647 | OCF₃ | CH₃ | Cl | S |
| 1648 | OCF₃ | C₂H₅ | Cl | S |
| 1649 | OCF₃ | n-C₃H₇ | Cl | S |
| 1650 | OCF₃ | i-C₃H₇ | Cl | S |
| 1651 | OCF₃ | n-C₄H₉ | Cl | S |
| 1652 | OCF₃ | i-C₄H₉ | Cl | S |
| 1653 | OCF₃ | s-C₄H₉ | Cl | S |
| 1654 | OCF₃ | t-C₄H₉ | Cl | S |
| 1655 | OCF₃ | CH₂OCH₃ | Cl | S |
| 1656 | OCF₃ | CF₃ | Cl | S |
| 1657 | OCF₃ | CF₂H | Cl | S |
| 1658 | OCF₃ | CN | Cl | S |
| 1659 | OCF₃ | OH | Cl | S |
| 1660 | OCF₃ | OCH₃ | Cl | S |
| 1661 | OCF₃ | NH₂ | Cl | S |
| 1662 | OCF₃ | NHCH₃ | Cl | S |
| 1663 | OCF₃ | N(CH₃)₂ | Cl | S |
| 1664 | OCF₃ | CO₂CH₃ | Cl | S |
| 1665 | OCF₃ | CO₂C₂H₅ | Cl | S |
| 1666 | OCF₃ | C(O)CH₃ | Cl | S |
| 1667 | OCF₃ | C(O)CF₃ | Cl | S |
| 1668 | OCF₃ | C(=NOCH₃)CH₃ | Cl | S |
| 1669 | OCF₃ | SO₂CH₃ | Cl | S |
| 1670 | OCF₃ | SO₂CF₃ | Cl | S |
| 1671 | OCF₃ | CH₂CO₂H | Cl | S |
| 1672 | OCF₃ | CH₂COOCH₃ | Cl | S |
| 1673 | OCF₃ | CH₂COOC₂H₅ | Cl | S |
| 1674 | OCF₃ | Prop-1-en-3-yl | Cl | S |
| 1675 | OCF₃ | trans-But-2-en-1-yl | Cl | S |
| 1676 | OCF₃ | cis-But-2-en-1-yl | Cl | S |
| 1677 | OCF₃ | cis-3-Methyl-but-2-en-1-yl | Cl | S |
| 1678 | OCF₃ | Cyclopropyl | Cl | S |
| 1679 | OCF₃ | Cyclopentyl | Cl | S |
| 1680 | OCF₃ | Cyclohexyl | Cl | S |
| 1681 | OCF₃ | 4,5-Dihydroisoxazol-3-yl | Cl | S |
| 1682 | OCF₃ | 4-Methyl-4,5-dihydroisoxazol-3-yl | Cl | S |
| 1683 | OCF₃ | Isoxazol-3-yl | Cl | S |
| 1684 | OCF₃ | 4-Methylisoxazol-3-yl | Cl | S |
| 1685 | OCF₃ | 4,5-Dihydroisoxazol-4-yl | Cl | S |
| 1686 | OCF₃ | 3-Methyl-4,5-dihydroisoxazol-4-yl | Cl | S |
| 1687 | OCF₃ | Isoxazol-4-yl | Cl | S |
| 1688 | OCF₃ | 3-Methylisoxazol-4-yl | Cl | S |
| 1689 | OCF₃ | Phenyl | Cl | S |
| 1690 | OCF₃ | Benzyl | Cl | S |
| 1691 | OCF₃ | Benzoyl | Cl | S |
| 1692 | OCF₃ | 2-Pyridyl | Cl | S |
| 1694 | SCH₃ | CH₃ | H | S |
| 1695 | SCH₃ | C₂H₅ | H | S |
| 1696 | SCH₃ | n-C₃H₇ | H | S |
| 1697 | SCH₃ | i-C₃H₇ | H | S |
| 1698 | SCH₃ | n-C₄H₉ | H | S |
| 1699 | SCH₃ | i-C₄H₉ | H | S |
| 1700 | SCH₃ | s-C₄H₉ | H | S |
| 1701 | SCH₃ | t-C₄H₉ | H | S |
| 1702 | SCH₃ | CH₂OCH₃ | H | S |
| 1703 | SCH₃ | CF₃ | H | S |
| 1704 | SCH₃ | CF₂H | H | S |
| 1705 | SCH₃ | CN | H | S |
| 1706 | SCH₃ | OH | H | S |
| 1707 | SCH₃ | OCH₃ | H | S |
| 1708 | SCH₃ | NH₂ | H | S |
| 1709 | SCH₃ | NHCH₃ | H | S |
| 1710 | SCH₃ | N(CH₃)₂ | H | S |
| 1711 | SCH₃ | CO₂CH₃ | H | S |
| 1712 | SCH₃ | CO₂C₂H₅ | H | S |
| 1713 | SCH₃ | C(O)CH₃ | H | S |
| 1714 | SCH₃ | C(O)CF₃ | H | S |
| 1715 | SCH₃ | C(=NOCH₃)CH₃ | H | S |
| 1716 | SCH₃ | SO₂CH₃ | H | S |
| 1717 | SCH₃ | SO₂CF₃ | H | S |
| 1718 | SCH₃ | CH₂CO₂H | H | S |
| 1719 | SCH₃ | CH₂COOCH₃ | H | S |
| 1720 | SCH₃ | CH₂COOC₂H₅ | H | S |
| 1721 | SCH₃ | Prop-1-en-3-yl | H | S |
| 1722 | SCH₃ | trans-But-2-en-1-yl | H | S |
| 1723 | SCH₃ | cis-But-2-en-1-yl | H | S |
| 1724 | SCH₃ | cis-3-Methyl-but-2-en-1-yl | H | S |
| 1725 | SCH₃ | Cyclopropyl | H | S |
| 1726 | SCH₃ | Cyclopentyl | H | S |
| 1727 | SCH₃ | Cyclohexyl | H | S |
| 1728 | SCH₃ | 4,5-Dihydroisoxazol-3-yl | H | S |
| 1729 | SCH₃ | 4-Methyl-4,5-dihydroisoxazol-3-yl | H | S |
| 1730 | SCH₃ | Isoxazol-3-yl | H | S |
| 1731 | SCH₃ | 4-Methylisoxazol-3-yl | H | S |
| 1732 | SCH₃ | 4,5-Dihydroisoxazol-4-yl | H | S |
| 1733 | SCH₃ | 3-Methyl-4,5-dihydroisoxazol-4-yl | H | S |
| 1734 | SCH₃ | Isoxazol-4-yl | H | S |
| 1735 | SCH₃ | 3-Methylisoxazol-4-yl | H | S |
| 1736 | SCH₃ | Phenyl | H | S |
| 1737 | SCH₃ | Benzyl | H | S |
| 1738 | SCH₃ | Benzoyl | H | S |
| 1739 | SCH₃ | 2-Pyridyl | H | S |
| 1741 | SCH3 | CH₃ | CH₃ | S |
| 1742 | SCH₃ | C₂H₅ | CH₃ | S |
| 1743 | SCH₃ | n-C₃H₇ | CH₃ | S |
| 1744 | SCH₃ | i-C₃H₇ | CH₃ | S |
| 1745 | SCH₃ | n-C₄H₉ | CH₃ | S |
| 1746 | SCH₃ | i-C₄H₉ | CH₃ | S |
| 1747 | SCH₃ | s-C₄H₉ | CH₃ | S |
| 1748 | SCH₃ | t-C₄H₉ | CH₃ | S |
| 1749 | SCH₃ | CH₂OCH₃ | CH₃ | S |
| 1750 | SCH₃ | CF₃ | CH₃ | S |
| 1751 | SCH₃ | CF₂H | CH₃ | S |
| 1752 | SCH₃ | CN | CH₃ | S |
| 1753 | SCH₃ | OH | CH₃ | S |
| 1754 | SCH₃ | OCH₃ | CH₃ | S |
| 1755 | SCH₃ | NH₂ | CH₃ | S |
| 1756 | SCH₃ | NHCH₃ | CH₃ | S |
| 1757 | SCH₃ | N(CH₃)₂ | CH₃ | S |
| 1758 | SCH₃ | CO₂CH₃ | CH₃ | S |
| 1759 | SCH₃ | CO₂C₂H₅ | CH₃ | S |
| 1760 | SCH₃ | C(O)CH₃ | CH₃ | S |
| 1761 | SCH₃ | C(O)CF₃ | CH₃ | S |
| 1762 | SCH₃ | C(=NOCH₃)CH₃ | CH₃ | S |
| 1763 | SCH₃ | SO₂CH₃ | CH₃ | S |
| 1764 | SCH₃ | SO₂CF₃ | CH₃ | S |
| 1765 | SCH₃ | CH₂CO₂H | CH₃ | S |
| 1766 | SCH₃ | CH₂COOCH₃ | CH₃ | S |
| 1767 | SCH₃ | CH₂COOC₂H₅ | CH₃ | S |
| 1768 | SCH₃ | Prop-1-en-3-yl | CH₃ | S |
| 1769 | SCH₃ | trans-But-2-en-1-yl | CH₃ | S |
| 1770 | SCH₃ | cis-But-2-en-1-yl | CH₃ | S |
| 1771 | SCH₃ | cis-3-Methyl-but-2-en-1-yl | CH₃ | S |
| 1772 | SCH₃ | Cyclopropyl | CH₃ | S |
| 1773 | SCH₃ | Cyclopentyl | CH₃ | S |
| 1774 | SCH₃ | Cyclohexyl | CH₃ | S |
| 1775 | SCH₃ | 4,5-Dihydroisoxazol-3-yl | CH₃ | S |
| 1776 | SCH₃ | 4-Methyl-4,5-dihydroisoxazol-3-yl | CH₃ | S |
| 1777 | SCH₃ | Isoxazol-3-yl | CH₃ | S |
| 1778 | SCH₃ | 4-Methylisoxazol-3-yl | CH₃ | S |
| 1779 | SCH₃ | 4,5-Dihydroisoxazol-4-yl | CH₃ | S |
| 1780 | SCH₃ | 3-Methyl-4,5-dihydroisoxazol-4-yl | CH₃ | S |
| 1781 | SCH₃ | Isoxazol-4-yl | CH₃ | S |
| 1782 | SCH₃ | 3-Methylisoxazol-4-yl | CH₃ | S |
| 1783 | SCH₃ | Phenyl | CH₃ | S |
| 1784 | SCH₃ | Benzyl | CH₃ | S |
| 1785 | SCH₃ | Benzoyl | CH₃ | S |
| 1786 | SCH₃ | 2-Pyridyl | CH₃ | S |
| 1788 | SCH₃ | CH₃ | Cl | S |
| 1789 | SCH₃ | C₂H₅ | Cl | S |
| 1790 | SCH₃ | n-C₃H₇ | Cl | S |
| 1791 | SCH₃ | i-C₃H₇ | Cl | S |
| 1792 | SCH₃ | n-C₄H₉ | Cl | S |
| 1793 | SCH₃ | i-C₄H₉ | Cl | S |
| 1794 | SCH₃ | s-C₄H₉ | Cl | S |
| 1795 | SCH₃ | t-C₄H₉ | Cl | S |
| 1796 | SCH₃ | CH₂OCH₃ | Cl | S |
| 1797 | SCH₃ | CF₃ | Cl | S |
| 1798 | SCH₃ | CF₂H | Cl | S |
| 1799 | SCH₃ | CN | Cl | S |
| 1800 | SCH₃ | OH | Cl | S |
| 1801 | SCH₃ | OCH₃ | Cl | S |
| 1802 | SCH₃ | NH₂ | Cl | S |
| 1803 | SCH₃ | NHCH₃ | Cl | S |
| 1804 | SCH₃ | N(CH₃)₂ | Cl | S |
| 1805 | SCH₃ | CO₂CH₃ | Cl | S |
| 1806 | SCH₃ | CO₂C₂H₅ | Cl | S |
| 1807 | SCH₃ | C(O)CH₃ | Cl | S |
| 1808 | SCH₃ | C(O)CF₃ | Cl | S |
| 1809 | SCH₃ | C(=NOCH₃)CH₃ | Cl | S |
| 1810 | SCH₃ | SO₂CH₃ | Cl | S |
| 1811 | SCH₃ | SO₂CF₃ | Cl | S |
| 1812 | SCH₃ | CH₂CO₂H | Cl | S |
| 1813 | SCH₃ | CH₂COOCH₃ | Cl | S |
| 1814 | SCH₃ | CH₂COOC₂H₅ | Cl | S |
| 1815 | SCH₃ | Prop-1-en-3-yl | Cl | S |
| 1816 | SCH₃ | trans-But-2-en-1-yl | Cl | S |
| 1817 | SCH₃ | cis-But-2-en-1-yl | Cl | S |
| 1818 | SCH₃ | cis-3-Methyl-but-2-en-1-yl | Cl | S |
| 1819 | SCH₃ | Cyclopropyl | Cl | S |
| 1820 | SCH₃ | Cyclopentyl | Cl | S |
| 1821 | SCH₃ | Cyclohexyl | Cl | S |
| 1822 | SCH₃ | 4,5-Dihydroisoxazol-3-yl | Cl | S |
| 1823 | SCH₃ | 4-Methyl-4,5-dihydroisoxazol-3-yl | Cl | S |
| 1824 | SCH₃ | Isoxazol-3-yl | Cl | S |
| 1825 | SCH₃ | 4-Methylisoxazol-3-yl | Cl | S |
| 1826 | SCH₃ | 4,5-Dihydroisoxazol-4-yl | Cl | S |
| 1827 | SCH₃ | 3-Methyl-4,5-dihydroisoxazol-4-yl | Cl | S |
| 1828 | SCH₃ | Isoxazol-4-yl | Cl | S |
| 1829 | SCH₃ | 3-Methylisoxazol-4-yl | Cl | S |
| 1830 | SCH₃ | Phenyl | Cl | S |
| 1831 | SCH₃ | Benzyl | Cl | S |
| 1832 | SCH₃ | Benzoyl | Cl | S |
| 1833 | SCH₃ | 2-Pyridyl | Cl | S |
| 1835 | SO₂CH₃ | CH₃ | H | S |
| 1836 | SO₂CH₃ | C₂H₅ | H | S |
| 1837 | SO₂CH₃ | n-C₃H₇ | H | S |
| 1838 | SO₂CH₃ | i-C₃H₇ | H | S |
| 1839 | SO₂CH₃ | n-C₄H₉ | H | S |
| 1840 | SO₂CH₃ | i-C₄H₉ | H | S |
| 1841 | SO₂CH₃ | s-C₄H₉ | H | S |
| 1842 | SO₂CH₃ | t-C₄H₉ | H | S |
| 1843 | SO₂CH₃ | CH₂OCH₃ | H | S |
| 1844 | SO₂CH₃ | CF₃ | H | S |
| 1845 | SO₂CH₃ | CF₂H | H | S |
| 1846 | SO₂CH₃ | CN | H | S |
| 1847 | SO₂CH₃ | OH | H | S |
| 1848 | SO₂CH₃ | OCH₃ | H | S |
| 1849 | SO₂CH₃ | NH₂ | H | S |
| 1850 | SO₂CH₃ | NHCH₃ | H | S |
| 1851 | SO₂CH₃ | N(CH₃)₂ | H | S |
| 1852 | SO₂CH₃ | CO₂CH₃ | H | S |
| 1853 | SO₂CH₃ | CO₂C₂H₅ | H | S |
| 1854 | SO₂CH₃ | C(O)CH₃ | H | S |
| 1855 | SO₂CH₃ | C(O)CF₃ | H | S |
| 1856 | SO₂CH₃ | C(=NOCH₃)CH₃ | H | S |
| 1857 | SO₂CH₃ | SO₂CH₃ | H | S |
| 1858 | SO₂CH₃ | SO₂CF₃ | H | S |
| 1859 | SO₂CH₃ | CH₂CO₂H | H | S |
| 1860 | SO₂CH₃ | CH₂COOCH₃ | H | S |
| 1861 | SO₂CH₃ | CH₂COOC₂H₅ | H | S |
| 1862 | SO₂CH₃ | Prop-1-en-3-yl | H | S |
| 1863 | SO₂CH₃ | trans-But-2-en-1-yl | H | S |
| 1864 | SO₂CH₃ | cis-But-2-en-1-yl | H | S |
| 1865 | SO₂CH₃ | cis-3-Methyl-but-2-en-1-yl | H | S |
| 1866 | SO₂CH₃ | Cyclopropyl | H | S |
| 1867 | SO₂CH₃ | Cyclopentyl | H | S |
| 1868 | SO₂CH₃ | Cyclohexyl | H | S |
| 1869 | SO₂CH₃ | 4,5-Dihydroisoxazol-3-yl | H | S |
| 1870 | SO₂CH₃ | 4-Methyl-4,5-dihydroisoxazol-3-yl | H | S |
| 1871 | SO₂CH₃ | Isoxazol-3-yl | H | S |
| 1872 | SO₂CH₃ | 4-Methylisoxazol-3-yl | H | S |
| 1873 | SO₂CH₃ | 4,5-Dihydroisoxazol-4-yl | H | S |
| 1874 | SO₂CH₃ | 3-Methyl-4,5-dihydroisoxazol-4-yl | H | S |
| 1875 | SO₂CH₃ | Isoxazol-4-yl | H | S |
| 1876 | SO₂CH₃ | 3-Methylisoxazol-4-yl | H | S |
| 1877 | SO₂CH₃ | Phenyl | H | S |
| 1878 | SO₂CH₃ | Benzyl | H | S |
| 1879 | SO₂CH₃ | Benzoyl | H | S |
| 1880 | SO₂CH₃ | 2-Pyridyl | H | S |
| 1882 | SO₂CH₃ | CH₃ | CH₃ | S |
| 1883 | SO₂CH₃ | C₂H₅ | CH₃ | S |
| 1884 | SO₂CH₃ | n-C₃H₇ | CH₃ | S |
| 1885 | SO₂CH₃ | i-C₃H₇ | CH₃ | S |
| 1886 | SO₂CH₃ | n-C₄H₉ | CH₃ | S |
| 1887 | SO₂CH₃ | i-C₄H₉ | CH₃ | S |
| 1888 | SO₂CH₃ | s-C₄H₉ | CH₃ | S |
| 1889 | SO₂CH₃ | t-C₄H₉ | CH₃ | S |
| 1890 | SO₂CH₃ | CH₂OCH₃ | CH₃ | S |
| 1891 | SO₂CH₃ | CF₃ | CH₃ | S |
| 1892 | SO₂CH₃ | CF₂H | CH₃ | S |
| 1893 | SO₂CH₃ | CN | CH₃ | S |
| 1894 | SO₂CH₃ | OH | CH₃ | S |
| 1895 | SO₂CH₃ | OCH₃ | CH₃ | S |
| 1896 | SO₂CH₃ | NH₂ | CH₃ | S |
| 1897 | SO₂CH₃ | NHCH₃ | CH₃ | S |
| 1898 | SO₂CH₃ | N(CH₃)₂ | CH₃ | S |
| 1899 | SO₂CH₃ | CO₂CH₃ | CH₃ | S |
| 1900 | SO₂CH₃ | CO₂C₂H₅ | CH₃ | S |
| 1901 | SO₂CH₃ | C(O)CH₃ | CH₃ | S |
| 1902 | SO₂CH₃ | C(O)CF₃ | CH₃ | S |
| 1903 | SO₂CH₃ | C(=NOCH₃)CH₃ | CH₃ | S |
| 1904 | SO₂CH₃ | SO₂CH₃ | CH₃ | S |
| 1905 | SO₂CH₃ | SO₂CF₃ | CH₃ | S |
| 1906 | SO₂CH₃ | CH₂CO₂H | CH₃ | S |
| 1907 | SO₂CH₃ | CH₂COOCH₃ | CH₃ | S |
| 1908 | SO₂CH₃ | CH₂COOC₂H₅ | CH₃ | S |
| 1909 | SO₂CH₃ | Prop-1-en-3-yl | CH₃ | S |
| 1910 | SO₂CH₃ | trans-But-2-en-1-yl | CH₃ | S |
| 1911 | SO₂CH₃ | cis-But-2-en-1-yl | CH₃ | S |
| 1912 | SO₂CH₃ | cis-3-Methyl-but-2-en-1-yl | CH₃ | S |
| 1913 | SO₂CH₃ | Cyclopropyl | CH₃ | S |
| 1914 | SO₂CH₃ | Cyclopentyl | CH₃ | S |
| 1915 | SO₂CH₃ | Cyclohexyl | CH₃ | S |
| 1916 | SO₂CH₃ | 4,5-Dihydroisoxazol-3-yl | CH₃ | S |
| 1917 | SO₂CH₃ | 4-Methyl-4,5-dihydroisoxazol-3-yl | CH₃ | S |
| 1918 | SO₂CH₃ | Isoxazol-3-yl | CH₃ | S |
| 1919 | SO₂CH₃ | 4-Methylisoxazol-3-yl | CH₃ | S |
| 1920 | SO₂CH₃ | 4,5-Dihydroisoxazol-4-yl | CH₃ | S |
| 1921 | SO₂CH₃ | 3-Methyl-4,5-dihydroisoxazol-4-yl | CH₃ | S |
| 1922 | SO₂CH₃ | Isoxazol-4-yl | CH₃ | S |
| 1923 | SO₂CH₃ | 3-Methylisoxazol-4-yl | CH₃ | S |
| 1924 | SO₂CH₃ | Phenyl | CH₃ | S |
| 1925 | SO₂CH₃ | Benzyl | CH₃ | S |
| 1926 | SO₂CH₃ | Benzoyl | CH₃ | S |
| 1927 | SO₂CH₃ | 2-Pyridyl | CH₃ | S |
| 1929 | SO₂CH₃ | CH₃ | Cl | S |
| 1930 | SO₂CH₃ | C₂H₅ | Cl | S |
| 1931 | SO₂CH₃ | n-C₃H₇ | Cl | S |
| 1932 | SO₂CH₃ | i-C₃H₇ | Cl | S |
| 1933 | SO₂CH₃ | n-C₄H₉ | Cl | S |
| 1934 | SO₂CH₃ | i-C₄H₉ | Cl | S |
| 1935 | SO₂CH₃ | s-C₄H₉ | Cl | S |
| 1936 | SO₂CH₃ | t-C₄H₉ | Cl | S |
| 1937 | SO₂CH₃ | CH₂OCH₃ | Cl | S |
| 1938 | SO₂CH₃ | CF₃ | Cl | S |
| 1939 | SO₂CH₃ | CF₂H | Cl | S |
| 1940 | SO₂CH₃ | CN | Cl | S |
| 1941 | SO₂CH₃ | OH | Cl | S |
| 1942 | SO₂CH₃ | OCH₃ | Cl | S |
| 1943 | SO₂CH₃ | NH₂ | Cl | S |
| 1944 | SO₂CH₃ | NHCH₃ | Cl | S |
| 1945 | SO₂CH₃ | N(CH₃)₂ | Cl | S |
| 1946 | SO₂CH₃ | CO₂CH₃ | Cl | S |
| 1947 | SO₂CH₃ | CO₂C₂H₅ | Cl | S |
| 1948 | SO₂CH₃ | C(O)CH₃ | Cl | S |
| 1949 | SO₂CH₃ | C(O)CF₃ | Cl | S |
| 1950 | SO₂CH₃ | C(=NOCH₃)CH₃ | Cl | S |
| 1951 | SO₂CH₃ | SO₂CH₃ | Cl | S |
| 1952 | SO₂CH₃ | SO₂CF₃ | Cl | S |
| 1953 | SO₂CH₃ | CH₂CO₂H | Cl | S |
| 1954 | SO₂CH₃ | CH₂COOCH₃ | Cl | S |
| 1955 | SO₂CH₃ | CH₂COOC₂H₅ | Cl | S |
| 1956 | SO₂CH₃ | Prop-1-en-3-yl | Cl | S |
| 1957 | SO₂CH₃ | trans-But-2-en-1-yl | Cl | S |
| 1958 | SO₂CH₃ | cis-But-2-en-1-yl | Cl | S |
| 1959 | SO₂CH₃ | cis-3-Methyl-but-2-en-1-yl | Cl | S |
| 1960 | SO₂CH₃ | Cyclopropyl | Cl | S |
| 1961 | SO₂CH₃ | Cyclopentyl | Cl | S |
| 1962 | SO₂CH₃ | Cyclohexyl | Cl | S |
| 1963 | SO₂CH₃ | 4,5-Dihydroisoxazol-3-yl | Cl | S |
| 1964 | SO₂CH₃ | 4-Methyl-4,5-dihydroisoxazol-3-yl | Cl | S |
| 1965 | SO₂CH₃ | Isoxazol-3-yl | Cl | S |
| 1966 | SO₂CH₃ | 4-Methylisoxazol-3-yl | Cl | S |
| 1967 | SO₂CH₃ | 4,5-Dihydroisoxazol-4-yl | Cl | S |
| 1968 | SO₂CH₃ | 3-Methyl-4,5-dihydroisoxazol-4-yl | Cl | S |
| 1969 | SO₂CH₃ | Isoxazol-4-yl | Cl | S |
| 1970 | SO₂CH₃ | 3-Methylisoxazol-4-yl | Cl | S |
| 1971 | SO₂CH₃ | Phenyl | Cl | S |
| 1972 | SO₂CH₃ | Benzyl | Cl | S |
| 1973 | SO₂CH₃ | Benzoyl | Cl | S |
| 1974 | SO₂CH₃ | 2-Pyridyl | Cl | S |
| 1976 | CF₃ | CH₃ | H | S |
| 1977 | CF₃ | C₂H₅ | H | S |
| 1978 | CF₃ | n-C₃H₇ | H | S |
| 1979 | CF₃ | i-C₃H₇ | H | S |
| 1980 | CF₃ | n-C₄H₉ | H | S |
| 1981 | CF₃ | i-C₄H₉ | H | S |
| 1982 | CF₃ | s-C₄H₉ | H | S |
| 1983 | CF₃ | t-C₄H₉ | H | S |
| 1984 | CF₃ | CH₂OCH₃ | H | S |
| 1985 | CF₃ | CF₃ | H | S |
| 1986 | CF₃ | CF₂H | H | S |
| 1987 | CF₃ | CN | H | S |
| 1988 | CF₃ | OH | H | S |
| 1989 | CF₃ | OCH₃ | H | S |
| 1990 | CF₃ | NH₂ | H | S |
| 1991 | CF₃ | NHCH₃ | H | S |
| 1992 | CF₃ | N(CH₃)₂ | H | S |
| 1993 | CF₃ | CO₂CH₃ | H | S |
| 1994 | CF₃ | CO₂C₂H₅ | H | S |
| 1995 | CF₃ | C(O)CH₃ | H | S |
| 1996 | CF₃ | C(O)CF₃ | H | S |
| 1997 | CF₃ | C(=NOCH₃)CH₃ | H | S |
| 1998 | CF₃ | SO₂CH₃ | H | S |
| 1999 | CF₃ | SO₂CF₃ | H | S |
| 2000 | CF₃ | CH₂CO₂H | H | S |
| 2001 | CF₃ | CH₂COOCH₃ | H | S |
| 2002 | CF₃ | CH₂COOC₂H₅ | H | S |
| 2003 | CF₃ | Prop-1-en-3-yl | H | S |
| 2004 | CF₃ | trans-But-2-en-1-yl | H | S |
| 2005 | CF₃ | cis-But-2-en-1-yl | H | S |
| 2006 | CF₃ | cis-3-Methyl-but-2-en-1-yl | H | S |
| 2007 | CF₃ | Cyclopropyl | H | S |
| 2008 | CF₃ | Cyclopentyl | H | S |
| 2009 | CF₃ | Cyclohexyl | H | S |
| 2010 | CF₃ | 4,5-Dihydroisoxazol-3-yl | H | S |
| 2011 | CF₃ | 4-Methyl-4,5-dihydroisoxazol-3-yl | H | S |
| 2012 | CF₃ | Isoxazol-3-yl | H | S |
| 2013 | CF₃ | 4-Methylisoxazol-3-yl | H | S |
| 2014 | CF₃ | 4,5-Dihydroisoxazol-4-yl | H | S |
| 2015 | CF₃ | 3-Methyl-4,5-dihydroisoxazol-4-yl | H | |
| 2016 | CF₃ | Isoxazol-4-yl | H | S |
| 2017 | CF₃ | 3-Methylisoxazol-4-yl | H | S |
| 2018 | CF₃ | Phenyl | H | S |
| 2019 | CF₃ | Benzyl | H | S |
| 2020 | CF₃ | Benzoyl | H | S |
| 2021 | CF₃ | 2-Pyridyl | H | S |
| 2023 | CF₃ | CH₃ | CH₃ | S |
| 2024 | CF₃ | C₂H₅ | CH₃ | S |
| 2025 | CF₃ | n-C₃H₇ | CH₃ | S |
| 2026 | CF₃ | i-C₃H₇ | CH₃ | S |
| 2027 | CF₃ | n-C₄H₉ | CH₃ | S |
| 2028 | CF₃ | i-C₄H₉ | CH₃ | S |
| 2029 | CF₃ | s-C₄H₉ | CH₃ | S |
| 2030 | CF₃ | t-C₄H₉ | CH₃ | S |
| 2031 | CF₃ | CH₂OCH₃ | CH₃ | S |
| 2032 | CF₃ | CF₃ | CH₃ | S |
| 2033 | CF₃ | CF₂H | CH₃ | S |
| 2034 | CF₃ | CN | CH3 | S |
| 2035 | CF₃ | OH | CH₃ | S |
| 2036 | CF₃ | OCH₃ | CH₃ | S |
| 2037 | CF₃ | NH₂ | CH₃ | S |
| 2038 | CF₃ | NHCH₃ | CH₃ | S |
| 2039 | CF₃ | N(CH₃)₂ | CH₃ | S |
| 2040 | CF₃ | CO₂CH₃ | CH₃ | S |
| 2041 | CF₃ | CO₂C₂H₅ | CH₃ | S |
| 2042 | CF₃ | C(O)CH₃ | CH₃ | S |
| 2043 | CF₃ | C(O)CF₃ | CH₃ | S |
| 2044 | CF₃ | C(=NOCH₃)CH₃ | CH₃ | S |
| 2045 | CF₃ | SO₂CH₃ | CH₃ | S |
| 2046 | CF₃ | SO₂CF₃ | CH₃ | S |
| 2047 | CF₃ | CH₂CO₂H | CH₃ | S |
| 2048 | CF₃ | CH₂COOCH₃ | CH₃ | S |
| 2049 | CF₃ | CH₂COOC₂H₅ | CH₃ | S |
| 2050 | CF₃ | Prop-1-en-3-yl | CH₃ | S |
| 2051 | CF₃ | trans-But-2-en-1-yl | CH₃ | S |
| 2052 | CF₃ | cis-But-2-en-1-yl | CH₃ | S |
| 2053 | CF₃ | cis-3-Methyl-but-2-en-1-yl | CH₃ | S |
| 2054 | CF₃ | Cyclopropyl | CH₃ | S |
| 2055 | CF₃ | Cyclopentyl | CH₃ | S |
| 2056 | CF₃ | Cyclohexyl | CH₃ | S |
| 2057 | CF₃ | 4,5-Dihydroisoxazol-3-yl | CH₃ | S |
| 2058 | CF₃ | 4-Methyl-4,5-dihydroisoxazol-3-yl | CH₃ | S |
| 2059 | CF₃ | Isoxazol-3-yl | CH₃ | S |
| 2060 | CF₃ | 4-Methylisoxazol-3-yl | CH₃ | S |
| 2061 | CF₃ | 4,5-Dihydroisoxazol-4-yl | CH₃ | S |
| 2062 | CF₃ | 3-Methyl-4,5-dihydroisoxazol-4-yl | CH₃ | S |
| 2063 | CF3 | Isoxazol-4-yl | CH₃ | S |
| 2064 | CF₃ | 3-Methylisoxazol-4-yl | CH₃ | S |
| 2065 | CF₃ | Phenyl | CH₃ | S |
| 2066 | CF₃ | Benzyl | CH₃ | S |
| 2067 | CF₃ | Benzoyl | CH₃ | S |
| 2068 | CF₃ | 2-Pyridyl | CH₃ | S |
| 2070 | CF₃ | CH₃ | Cl | S |
| 2071 | CF₃ | C₂H₅ | Cl | S |
| 2072 | CF₃ | n-C₃H₇ | Cl | S |
| 2073 | CF₃ | i-C₃H₇ | Cl | S |
| 2074 | CF₃ | n-C₄H₉ | Cl | S |
| 2075 | C_{F3} | i-C₄H₉ | Cl | S |
| 2076 | CF₃ | s-C₄H₉ | Cl | S |
| 2077 | CF₃ | t-C₄H₉ | Cl | S |
| 2078 | CF₃ | CH₂OCH₃ | Cl | S |
| 2079 | CF₃ | CF₃ | Cl | S |
| 2080 | CF₃ | CF₂H | Cl | S |
| 2081 | CF₃ | CN | Cl | S |
| 2082 | CF₃ | OH | Cl | S |
| 2083 | CF₃ | OCH₃ | Cl | S |
| 2084 | CF₃ | NH₂ | Cl | S |
| 2085 | CF₃ | NHCH₃ | Cl | S |
| 2086 | CF₃ | N(CH₃)₂ | Cl | S |
| 2087 | CF₃ | CO₂CH₃ | Cl | S |
| 2088 | CF₃ | CO₂C₂H₅ | Cl | S |
| 2089 | CF₃ | C(O)CH₃ | Cl | S |
| 2090 | CF₃ | C(O)CF₃ | Cl | S |
| 2091 | CF₃ | C(=NOCH₃)CH₃ | Cl | S |
| 2092 | CF₃ | SO₂CH₃ | Cl | S |
| 2093 | CF₃ | SO₂CF₃ | Cl | S |
| 2094 | CF₃ | CH₂CO₂H | Cl | S |
| 2095 | CF₃ | CH₂COOCH₃ | Cl | S |
| 2096 | CF₃ | CH₂COOC₂H₅ | Cl | S |
| 2097 | CF₃ | Prop-1-en-3-yl | Cl | S |
| 2098 | CF₃ | trans-But-2-en-1-yl | Cl | S |
| 2099 | CF₃ | cis-But-2-en-1-yl | Cl | S |
| 2100 | CF₃ | cis-3-Methyl-but-2-en-1-yl | Cl | S |
| 2101 | CF₃ | Cyclopropyl | Cl | S |
| 2102 | CF₃ | Cyclopentyl | Cl | S |
| 2103 | CF₃ | Cyclohexyl | Cl | S |
| 2104 | CF₃ | 4,5-Dihydroisoxazol-3-yl | Cl | S |
| 2105 | CF₃ | 4-Methyl-4,5-dihydroisoxazol-3-yl | Cl | S |
| 2106 | CF₃ | Isoxazol-3-yl | Cl | S |
| 2107 | CF₃ | 4-Methylisoxazol-3-yl | Cl | S |
| 2108 | CF₃ | 4,5-Dihydroisoxazol-4-yl | Cl | S |
| 2109 | CF₃ | 3-Methyl-4,5-dihydroisoxazol-4-yl | Cl | S |
| 2110 | CF₃ | Isoxazol-4-yl | Cl | S |
| 2111 | CF₃ | 3-Methylisoxazol-4-yl | Cl | S |
| 2112 | CF₃ | Phenyl | Cl | S |
| 2113 | CF₃ | Benzyl | Cl | S |
| 2114 | CF₃ | Benzoyl | Cl | S |
| 2115 | CF₃ | 2-Pyridyl | Cl | S |
| 2117 | C₂H₅ | CH₃ | H | S |
| 2118 | C₂H₅ | C₂H₅ | H | S |
| 2119 | C₂H₅ | n-C₃H₇ | H | S |
| 2120 | C₂H₅ | i-C₃H₇ | H | S |
| 2121 | C₂H₅ | n-C₄H₉ | H | S |
| 2122 | C₂H₅ | i-C₄H₉ | H | S |
| 2123 | C₂H₅ | s-C₄H₉ | H | S |
| 2124 | C₂H₅ | t-C₄H₉ | H | S |
| 2125 | C₂H₅ | CH₂OCH₃ | H | S |
| 2126 | C₂H₅ | CF₃ | H | S |
| 2127 | C₂H₅ | CF₂H | H | S |
| 2128 | C₂H₅ | CN | H | S |
| 2129 | C₂H₅ | OH | H | S |
| 2130 | C₂H₅ | OCH₃ | H | S |
| 2131 | C₂H₅ | NH₂ | H | S |
| 2132 | C₂H₅ | NHCH₃ | H | S |
| 2133 | C₂H₅ | N(CH₃)₂ | H | S |
| 2134 | C₂H₅ | CO₂CH₃ | H | S |
| 2135 | C₂H₅ | CO₂C₂H₅ | H | S |
| 2136 | C₂H₅ | C(O)CH₃ | H | S |
| 2137 | C₂H₅ | C(O)CF₃ | H | S |
| 2138 | C₂H₅ | C(=NOCH₃)CH₃ | H | S |
| 2139 | C₂H₅ | SO₂CH₃ | H | S |
| 2140 | C₂H₅ | SO₂CF₃ | H | S |
| 2141 | C₂H₅ | CH₂CO₂H | H | S |
| 2142 | C_{Z}H_{S} | CH₂COOCH₃ | H | S |
| 2143 | C₂H₅ | CH₂COOC₂H₅ | H | S |
| 2144 | C₂H₅ | Prop-1-en-3-yl | H | S |
| 2145 | C₂H₅ | trans-But-2-en-1-yl | H | S |
| 2146 | C₂H₅ | cis-But-2-en-1-yl | H | S |
| 2147 | C₂H₅ | cis-3-Methyl-but-2-en-1-yl | H | S |
| 2148 | C₂H₅ | Cyclopropyl | H | S |
| 2149 | C₂H₅ | Cyclopentyl | H | S |
| 2150 | C₂H₅ | Cyclohexyl | H | S |
| 2151 | C₂H₅ | 4,5-Dihydroisoxazol-3-yl | H | S |
| 2152 | C₂H₅ | 4-Methyl-4,5-dihydroisoxazol-3-yl | H | S |
| 2153 | C₂H₅ | Isoxazol-3-yl | H | S |
| 2154 | C₂H₅ | 4-Methylisoxazol-3-yl | H | S |
| 2155 | C₂H₅ | 4,5-Dihydroisoxazol-4-yl | H | S |
| 2156 | C₂H₅ | 3-Methyl-4,5-dihydroisoxazol-4-yl | H | S |
| 2157 | C₂H₅ | Isoxazol-4-yl | H | S |
| 2158 | C₂H₅ | 3-Methylisoxazol-4-yl | H | S |
| 2159 | C₂H₅ | Phenyl | H | S |
| 2160 | C₂H₅ | Benzyl | H | S |
| 2161 | C₂H₅ | Benzoyl | H | S |
| 2162 | C₂H₅ | 2-Pyridyl | H | S |
| 2164 | C₂H₅ | CH₃ | CH₃ | S |
| 2165 | C₂H₅ | C₂H₅ | CH₃ | S |
| 2166 | C₂H₅ | n-C₃H₇ | CH₃ | S |
| 2167 | C₂H₅ | i-C₃H₇ | CH₃ | S |
| 2168 | C₂H₅ | n-C₄H₉ | CH₃ | S |
| 2169 | C₂H₅ | i-C₄H₉ | CH₃ | S |
| 2170 | C₂H₅ | s-C₄H₉ | CH₃ | S |
| 2171 | C₂H₅ | t-C₄H₉ | CH₃ | S |
| 2172 | C₂H₅ | CH₂OCH₃ | CH₃ | S |
| 2173 | C₂H₅ | CF₃ | CH₃ | S |
| 2174 | C₂H₅ | CF₂H | CH₃ | S |
| 2175 | C₂H₅ | CN | CH₃ | S |
| 2176 | C₂H₅ | OH | CH₃ | S |
| 2177 | C₂H₅ | OCH₃ | CH₃ | S |
| 2178 | C₂H₅ | NH₂ | CH₃ | S |
| 2179 | C₂H₅ | NHCH₃ | CH₃ | S |
| 2180 | C₂H₅ | N(CH₃)₂ | CH₃ | S |
| 2181 | C₂H₅ | CO₂CH₃ | CH₃ | S |
| 2182 | C₂H₅ | CO₂C₂H₅ | CH₃ | S |
| 2183 | C₂H₅ | C(O)CH₃ | CH₃ | S |
| 2184 | C₂H₅ | C(O)CF₃ | CH₃ | S |
| 2185 | C₂H₅ | C(=NOCH₃)CH₃ | CH₃ | S |
| 2186 | C₂H₅ | SO₂CH₃ | CH₃ | S |
| 2187 | C₂H₅ | SO₂CF₃ | CH₃ | S |
| 2188 | C₂H₅ | CH₂CO₂H | CH₃ | S |
| 2189 | C₂H₅ | CH₂COOCH₃ | CH₃ | S |
| 2190 | C₂H₅ | CH₂COOC₂H₅ | CH₃ | S |
| 2191 | C₂H₅ | Prop-1-en-3-yl | CH₃ | S |
| 2192 | C₂H₅ | trans-But-2-en-1-yl | CH₃ | S |
| 2193 | C₂H₅ | cis-But-2-en-1-yl | CH₃ | S |
| 2194 | C₂H₅ | cis-3-Methyl-but-2-en-1-yl | CH₃ | S |
| 2195 | C₂H₅ | Cyclopropyl | CH₃ | S |
| 2196 | C₂H₅ | Cyclopentyl | CH₃ | S |
| 2197 | C₂H₅ | Cyclohexyl | CH₃ | S |
| 2198 | C₂H₅ | 4,5-Dihydroisoxazol-3-yl | CH₃ | S |
| 2199 | C₂H₅ | 4-Methyl-4,5-dihydroisoxazol-3-yl | CH₃ | S |
| 2200 | C₂H₅ | Isoxazol-3-yl | CH₃ | S |
| 2201 | C₂H₅ | 4-Methylisoxazol-3-yl | CH₃ | S |
| 2202 | C₂H₅ | 4,5-Dihydroisoxazol-4-yl | CH₃ | S |
| 2203 | C₂H₅ | 3-Methyl-4,5-dihydroisoxazol-4-yl | CH₃ | S |
| 2204 | C₂H₅ | Isoxazol-4-yl | CH₃ | S |
| 2205 | C₂H₅ | 3-Methylisoxazol-4-yl | CH₃ | S |
| 2206 | C₂H₅ | Phenyl | CH₃ | S |
| 2207 | C₂H₅ | Benzyl | CH₃ | S |
| 2208 | C₂H₅ | Benzoyl | CH₃ | S |
| 2209 | C₂H₅ | 2-Pyridyl | CH₃ | S |
| 2211 | C₂H₅ | CH₃ | Cl | S |
| 2212 | C₁H₅ | C₂H₅ | Cl | S |
| 2213 | C₂H₅ | n-C₃H₇ | Cl | S |
| 2214 | C₂H₅ | i-C₃H₇ | Cl | S |
| 2215 | C₂H₅ | n-C₄H₉ | Cl | S |
| 2216 | C₂H₅ | i-C₄H₉ | Cl | S |
| 2217 | C₂H₅ | s-C₄H₉ | Cl | S |
| 2218 | C₂H₅ | t-C₄H₉ | Cl | S |
| 2219 | C₂H₅ | CH₂OCH₃ | Cl | S |
| 2220 | C₂H₅ | CF₃ | Cl | S |
| 2221 | C₂H₅ | CF₂H | Cl | S |
| 2222 | C₂H₅ | CN | Cl | S |
| 2223 | C₂H₅ | OH | Cl | S |
| 2224 | C₂H₅ | OCH₃ | Cl | S |
| 2225 | C₂H₅ | NH₂ | Cl | S |
| 2226 | C₂H₅ | NHCH₃ | Cl | S |
| 2227 | C₂H₅ | N(CH₃)₂ | Cl | S |
| 2228 | C₂H₅ | CO₂CH₃ | Cl | S |
| 2229 | C₂H₅ | CO₂C₂H₅ | Cl | S |
| 2230 | C₂H₅ | C(O)CH₃ | Cl | S |
| 2231 | C₂H₅ | C(O)CF₃ | Cl | S |
| 2232 | C₂H₅ | C(=NOCH₃)CH₃ | Cl | S |
| 2233 | C₂H₅ | SO₂CH₃ | Cl | S |
| 2234 | C₂H₅ | SO₂CF₃ | Cl | S |
| 2235 | C₂H₅ | CH₂CO₂H | Cl | S |
| 2236 | C₂H₅ | CH₂COOCH₃ | Cl | S |
| 2237 | C₂H₅ | CH₂COOC₂H₅ | Cl | S |
| 2238 | C₂H₅ | Prop-1-en-3-yl | Cl | S |
| 2239 | C₂H₅ | trans-But-2-en-1-yl | Cl | S |
| 2240 | C₂H₅ | cis-But-2-en-1-yl | Cl | S |
| 2241 | C₂H₅ | cis-3-Methyl-but-2-en-1-yl | Cl | S |
| 2242 | C₂H₅ | Cyclopropyl | Cl | S |
| 2243 | C₂H₅ | Cyclopentyl | Cl | S |
| 2244 | C₂H₅ | Cyclohexyl | Cl | S |
| 2245 | C₂H₅ | 4,5-Dihydroisoxazol-3-yl | Cl | S |
| 2246 | C₂H₅ | 4-Methyl-4,5-dihydroisoxazol-3-yl | Cl | S |
| 2247 | C₂H₅ | Isoxazol-3-yl | Cl | S |
| 2248 | C₂H₅ | 4-Methylisoxazol-3-yl | Cl | S |
| 2249 | C₂H₅ | 4,5-Dihydroisoxazol-4-yl | Cl | S |
| 2250 | C₂H₅ | 3-Methyl-4,5-dihydroisoxazol-4-yl | Cl | S |
| 2251 | C₂H₅ | Isoxazol-4-yl | Cl | S |
| 2252 | C₂H₅ | 3-Methylisoxazol-4-yl | Cl | S |
| 2253 | C₂H₅ | Phenyl | Cl | S |
| 2254 | C₂H₅ | Benzyl | Cl | S |
| 2255 | C₂H₅ | Benzoyl | Cl | S |
| 2256 | C₂H₅ | 2-Pyridyl | Cl | S |
| 2258 | CH₃ | CH₃ | H | NCH₃ |
| 2259 | CH₃ | C₂H₅ | H | NCH₃ |
| 2260 | CH₃ | n-C₃H₇ | H | NCH₃ |
| 2261 | CH₃ | i-C₃H₇ | H | NCH₃ |
| 2262 | CH₃ | n-C₄H₉ | H | NCH₃ |
| 2263 | CH₃ | i-C₄H₉ | H | NCH₃ |
| 2264 | CH₃ | s-C₄H₉ | H | NCH₃ |
| 2265 | CH₃ | t-C₄H₉ | H | NCH₃ |
| 2266 | CH₃ | CH₂OCH₃ | H | NCH₃ |
| 2267 | CH₃ | CF₃ | H | NCH₃ |
| 2268 | CH₃ | CF₂H | H | NCH₃ |
| 2269 | CH₃ | CN | H | NCH₃ |
| 2270 | CH₃ | OH | H | NCH₃ |
| 2271 | CH₃ | OCH₃ | H | NCH₃ |
| 2272 | CH₃ | NH₂ | H | NCH₃ |
| 2273 | CH₃ | NHCH₃ | H | NCH₃ |
| 2274 | CH₃ | N(CH₃)₂ | H | NCH₃ |
| 2275 | CH₃ | CO₂CH₃ | H | NCH₃ |
| 2276 | CH₃ | CO₂C₂H₅ | H | NCH₃ |
| 2277 | CH₃ | C(O)CH₃ | H | NCH₃ |
| 2278 | CH₃ | C(O)CF₃ | H | NCH₃ |
| 2279 | CH₃ | C(=NOCH₃)CH₃ | H | NCH₃ |
| 2280 | CH₃ | SO₂CH₃ | H | NCH₃ |
| 2281 | CH₃ | SO₂CF₃ | H | NCH₃ |
| 2282 | CH₃ | CH₂CO₂H | H | NCH₃ |
| 2283 | CH₃ | CH₂COOCH₃ | H | NCH₃ |
| 2284 | CH₃ | CH₂COOC₂H₅ | H | NCH₃ |
| 2285 | CH₃ | Prop-1-en-3-yl | H | NCH₃ |
| 2286 | CH₃ | trans-But-2-en-1-yl | H | NCH₃ |
| 2287 | CH₃ | cis-But-2-en-1-yl | H | NCH₃ |
| 2288 | CH₃ | cis-3-Methyl-but-2-en-1-yl | H | NCH₃ |
| 2289 | CH₃ | Cyclopropyl | H | NCH₃ |
| 2290 | CH₃ | Cyclopentyl | H | NCH₃ |
| 2291 | CH₃ | Cyclohexyl | H | NCH₃ |
| 2292 | CH₃ | 4,5-Dihydroisoxazol-3-yl | H | NCH₃ |
| 2293 | CH₃ | 4-Methyl-4,5-dihydroisoxazol-3-yl | H | NCH₃ |
| 2294 | CH₃ | Isoxazol-3-yl | H | NCH₃ |
| 2295 | CH₃ | 4-Methylisoxazol-3-yl | H | NCH₃ |
| 2296 | CH₃ | 4,5-Dihydroisoxazol-4-yl | H | NCH₃ |
| 2297 | CH₃ | 3-Methyl-4,5-dihydroisoxazol-4-yl | H | NCH₃ |
| 2298 | CH₃ | Isoxazol-4-yl | H | NCH₃ |
| 2299 | CH₃ | 3-Methylisoxazol-4-yl | H | NCH₃ |
| 2300 | CH₃ | Phenyl | H | NCH₃ |
| 2301 | CH₃ | Benzyl | H | NCH₃ |
| 2302 | CH₃ | Benzoyl | H | NCH₃ |
| 2303 | CH₃ | 2-Pyridyl | H | NCH₃ |
| 2305 | CH₃ | CH₃ | CH₃ | NCH₃ |
| 2306 | CH₃ | C₂H₅ | CH₃ | NCH₃ |
| 2307 | CH₃ | n-C₃H₇ | CH₃ | NCH₃ |
| 2308 | CH₃ | i-C₃H₇ | CH₃ | NCH₃ |
| 2309 | CH₃ | n-C₄H₉ | CH₃ | NCH₃ |
| 2310 | CH₃ | i-C₄H₉ | CH₃ | NCH₃ |
| 2311 | CH₃ | s-C₄H₉ | CH₃ | NCH₃ |
| 2312 | CH₃ | t-C₄H₉ | CH₃ | NCH₃ |
| 2313 | CH₃ | CH₂OCH₃ | CH₃ | NCH₃ |
| 2314 | CH₃ | CF₃ | CH₃ | NCH₃ |
| 2315 | CH₃ | CF₂H | CH₃ | NCH₃ |
| 2316 | CH₃ | CN | CH₃ | NCH₃ |
| 2317 | CH₃ | OH | CH₃ | NCH₃ |
| 2318 | CH₃ | OCH₃ | CH₃ | NCH₃ |
| 2319 | CH₃ | NH₂ | CH₃ | NCH₃ |
| 2320 | CH₃ | NHCH₃ | CH₃ | NCH₃ |
| 2321 | CH₃ | N(CH₃)₂ | CH₃ | NCH₃ |
| 2322 | CH₃ | CO₂CH₃ | CH₃ | NCH₃ |
| 2323 | CH₃ | CO₂C₂H₅ | CH₃ | NCH₃ |
| 2324 | CH₃ | C(O)CH₃ | CH₃ | NCH₃ |
| 2325 | CH₃ | C(O)CF₃ | CH₃ | NCH₃ |
| 2326 | CH₃ | C(=NOCH₃)CH₃ | CH₃ | NCH₃ |
| 2327 | CH₃ | SO₂CH₃ | CH₃ | NCH₃ |
| 2328 | CH₃ | SO₂CF₃ | CH₃ | NCH₃ |
| 2329 | CH₃ | CH₂CO₂H | CH₃ | NCH₃ |
| 2330 | CH₃ | CH₂COOCH₃ | CH₃ | NCH₃ |
| 2331 | CH₃ | CH₂COOC₂H₅ | CH₃ | NCH₃ |
| 2332 | CH₃ | Prop-1-en-3-yl | CH₃ | NCH₃ |
| 2333 | CH₃ | trans-But-2-en-1-yl | CH₃ | NCH₃ |
| 2334 | CH₃ | cis-But-2-en-1-yl | CH₃ | NCH₃ |
| 2335 | CH₃ | cis-3-Methyl-but-2-en-1-yl | CH₃ | NCH₃ |
| 2336 | CH₃ | Cyclopropyl | CH₃ | NCH₃ |
| 2337 | CH₃ | Cyclopentyl | CH₃ | NCH₃ |
| 2338 | CH₃ | Cyclohexyl | CH₃ | NCH₃ |
| 2339 | CH₃ | 4,5-Dihydroisoxazol-3-yl | CH₃ | NCH₃ |
| 2340 | CH₃ | 4-Methyl-4,5-dihydroisoxazol-3-yl | CH₃ | NCH₃ |
| 2341 | CH₃ | Isoxazol-3-yl | CH₃ | NCH₃ |
| 2342 | CH₃ | 4-Methylisoxazol-3-yl | CH₃ | NCH₃ |
| 2343 | CH₃ | 4,5-Dihydroisoxazol-4-yl | CH₃ | NCH₃ |
| 2344 | CH₃ | 3-Methyl-4,5-dihydroisoxazol-4-yl | CH₃ | NCH₃ |
| 2345 | CH₃ | Isoxazol-4-yl | CH₃ | NCH₃ |
| 2346 | CH₃ | 3-Methylisoxazol-4-yl | CH₃ | NCH₃ |
| 2347 | CH₃ | Phenyl | CH₃ | NCH₃ |
| 2348 | CH₃ | Benzyl | CH₃ | NCH₃ |
| 2349 | CH₃ | Benzoyl | CH₃ | NCH₃ |
| 2350 | CH₃ | 2-Pyridyl | CH₃ | NCH₃ |
| 2352 | CH₃ | CH₃ | Cl | NCH₃ |
| 2353 | CH₃ | C₂H₅ | Cl | NCH₃ |
| 2354 | CH₃ | n-C₃H₇ | Cl | NCH₃ |
| 2355 | CH₃ | i-C₃H₇ | Cl | NCH₃ |
| 2356 | CH₃ | n-C₄H₉ | Cl | NCH₃ |
| 2357 | CH₃ | i-C₄H₉ | Cl | NCH₃ |
| 2358 | CH₃ | s-C₄H₉ | Cl | NCH₃ |
| 2359 | CH₃ | t-C₄H₉ | Cl | NCH₃ |
| 2360 | CH₃ | CH₂OCH₃ | Cl | NCH₃ |
| 2361 | CH₃ | CF₃ | Cl | NCH₃ |
| 2362 | CH₃ | CF₂H | Cl | NCH₃ |
| 2363 | CH₃ | CN | Cl | NCH₃ |
| 2364 | CH₃ | OH | Cl | NCH₃ |
| 2365 | CH₃ | OCH₃ | Cl | NCH₃ |
| 2366 | CH₃ | NH₂ | Cl | NCH₃ |
| 2367 | CH₃ | NHCH₃ | Cl | NCH₃ |
| 2368 | CH₃ | N(CH₃)₂ | Cl | NCH₃ |
| 2369 | CH₃ | CO₂CH₃ | Cl | NCH₃ |
| 2370 | CH₃ | CO₂C₂H₅ | Cl | NCH₃ |
| 2371 | CH₃ | C(O)CH₃ | Cl | NCH₃ |
| 2372 | CH₃ | C(O)CF₃ | Cl | NCH₃ |
| 2373 | CH₃ | C(=NOCH₃)CH₃ | Cl | NCH₃ |
| 2374 | CH₃ | SO₂CH₃ | Cl | NCH₃ |
| 2375 | CH₃ | SO₂CF₃ | Cl | NCH₃ |
| 2376 | CH₃ | CH₂CO₂H | Cl | NCH₃ |
| 2377 | CH₃ | CH₂COOCH₃ | Cl | NCH₃ |
| 2378 | CH₃ | CH₂COOC₂H₅ | Cl | NCH₃ |
| 2379 | CH₃ | Prop-1-en-3-yl | Cl | NCH₃ |
| 2380 | CH₃ | trans-But-2-en-1-yl | Cl | NCH₃ |
| 2381 | CH₃ | cis-But-2-en-1-yl | Cl | NCH₃ |
| 2382 | CH₃ | cis-3-Methyl-but-2-en-1-yl | Cl | NCH₃ |
| 2383 | CH₃ | Cyclopropyl | Cl | NCH₃ |
| 2384 | CH₃ | Cyclopentyl | Cl | NCH₃ |
| 2385 | CH₃ | Cyclohexyl | Cl | NCH₃ |
| 2386 | CH₃ | 4,5-Dihydroisoxazol-3-yl | Cl | NCH₃ |
| 2387 | CH₃ | 4-Methyl-4,5-dihydroisoxazol-3-yl | Cl | NCH₃ |
| 2388 | CH₃ | Isoxazol-3-yl | Cl | NCH₃ |
| 2389 | CH₃ | 4-Methylisoxazol-3-yl | Cl | NCH₃ |
| 2390 | CH₃ | 4,5-Dihydroisoxazol-4-yl | Cl | NCH₃ |
| 2391 | CH₃ | 3-Methyl-4,5-dihydroisoxazol-4-yl | Cl | NCH₃ |
| 2392 | CH₃ | Isoxazol-4-yl | Cl | NCH₃ |
| 2393 | CH₃ | 3-Methylisoxazol-4-yl | Cl | NCH₃ |
| 2394 | CH₃ | Phenyl | Cl | NCH₃ |
| 2395 | CH₃ | Benzyl | Cl | NCH₃ |
| 2396 | CH₃ | Benzoyl | Cl | NCH₃ |
| 2397 | CH₃ | 2-Pyridyl | Cl | NCH₃ |
| 2399 | Cl | CH₃ | H | NCH₃ |
| 2400 | Cl | C₂H₅ | H | NCH₃ |
| 2401 | Cl | n-C₃H₇ | H | NCH₃ |
| 2402 | Cl | i-C₃H₇ | H | NCH₃ |
| 2403 | Cl | n-C₄H₉ | H | NCH₃ |
| 2404 | Cl | i-C₄H₉ | H | NCH₃ |
| 2405 | Cl | s-C₄H₉ | H | NCH₃ |
| 2406 | Cl | t-C₄H₉ | H | NCH₃ |
| 2407 | Cl | CH₂OCH₃ | H | NCH₃ |
| 2408 | Cl | CF₃ | H | NCH₃ |
| 2409 | Cl | CF₂H | H | NCH₃ |
| 2410 | Cl | CN | H | NCH₃ |
| 2411 | Cl | OH | H | NCH₃ |
| 2412 | Cl | OCH₃ | H | NCH₃ |
| 2413 | Cl | NH₂ | H | NCH₃ |
| 2414 | Cl | NHCH₃ | H | NCH₃ |
| 2415 | Cl | N(CH₃)₂ | H | NCH₃ |
| 2416 | Cl | CO₂CH₃ | H | NCH₃ |
| 2417 | Cl | CO₂C₂H₅ | H | NCH₃ |
| 2418 | Cl | C(O)CH₃ | H | NCH₃ |
| 2419 | Cl | C(O)CF₃ | H | NCH₃ |
| 2420 | Cl | C(=NOCH₃)CH₃ | H | NCH₃ |
| 2421 | Cl | SO₂CH₃ | H | NCH₃ |
| 2422 | Cl | SO₂CF₃ | H | NCH₃ |
| 2423 | Cl | CH₂CO₂H | H | NCH₃ |
| 2424 | Cl | CH₂COOCH₃ | H | NCH₃ |
| 2425 | Cl | CH₂COOC₂H₅ | H | NCH₃ |
| 2426 | Cl | Prop-1-en-3-yl | H | NCH₃ |
| 2427 | Cl | trans-But-2-en-1-yl | H | NCH₃ |
| 2428 | Cl | cis-But-2-en-1-yl | H | NCH₃ |
| 2429 | Cl | cis-3-Methyl-but-2-en-1-yl | H | NCH₃ |
| 2430 | Cl | Cyclopropyl | H | NCH₃ |
| 2431 | Cl | Cyclopentyl | H | NCH₃ |
| 2432 | Cl | Cyclohexyl | H | NCH₃ |
| 2433 | Cl | 4,5-Dihydroisoxazol-3-yl | H | NCH₃ |
| 2434 | Cl | 4-Methyl-4,5-dihydroisoxazol-3-yl | H | NCH₃ |
| 2435 | Cl | Isoxazol-3-yl | H | NCH₃ |
| 2436 | Cl | 4-Methylisoxazol-3-yl | H | NCH₃ |
| 2437 | Cl | 4,5-Dihydroisoxazol-4-yl | H | NCH₃ |
| 2438 | Cl | 3-Methyl-4,5-dihydroisoxazol-4-yl | H | NCH₃ |
| 2439 | Cl | Isoxazol-4-yl | H | NCH₃ |
| 2440 | Cl | 3-Methylisoxazol-4-yl | H | NCH₃ |
| 2441 | Cl | Phenyl | H | NCH₃ |
| 2442 | Cl | Benzyl | H | NCH₃ |
| 2443 | Cl | Benzoyl | H | NCH₃ |
| 2444 | Cl | 2-Pyridyl | H | NCH₃ |
| 2446 | Cl | CH₃ | CH₃ | NCH₃ |
| 2447 | Cl | C₂H₅ | CH₃ | NCH₃ |
| 2448 | Cl | n-C₃H₇ | CH₃ | NCH₃ |
| 2449 | Cl | i-C₃H₇ | CH₃ | NCH₃ |
| 2450 | Cl | n-C₄H₉ | CH₃ | NCH₃ |
| 2451 | Cl | i-C₄H₉ | CH₃ | NCH₃ |
| 2452 | Cl | s-C₄H₉ | CH₃ | NCH₃ |
| 2453 | Cl | t-C₄H₉ | CH₃ | NCH₃ |
| 2454 | Cl | CH₂OCH₃ | CH₃ | NCH₃ |
| 2455 | Cl | CF₃ | CH₃ | NCH₃ |
| 2456 | Cl | CF₂H | CH₃ | NCH₃ |
| 2457 | Cl | CN | CH₃ | NCH₃ |
| 2458 | Cl | OH | CH₃ | NCH₃ |
| 2459 | Cl | OCH₃ | CH₃ | NCH₃ |
| 2460 | Cl | NH₂ | CH₃ | NCH₃ |
| 2461 | Cl | NHCH₃ | CH₃ | NCH₃ |
| 2462 | Cl | N(CH₃)₂ | CH₃ | NCH₃ |
| 2463 | Cl | CO₂CH₃ | CH₃ | NCH₃ |
| 2464 | Cl | CO₂C₂H₅ | CH₃ | NCH₃ |
| 2465 | Cl | C(O)CH₃ | CH₃ | NCH₃ |
| 2466 | Cl | C(O)CF₃ | CH₃ | NCH₃ |
| 2467 | Cl | C(=NOCH₃)CH₃ | CH₃ | NCH₃ |
| 2468 | Cl | SO₂CH₃ | CH₃ | NCH₃ |
| 2469 | Cl | SO₂CF₃ | CH₃ | NCH₃ |
| 2470 | Cl | CH₂CO₂H | CH₃ | NCH₃ |
| 2471 | Cl | CH₂COOCH₃ | CH₃ | NCH₃ |
| 2472 | Cl | CH₂COOC₂H₅ | CH₃ | NCH₃ |
| 2473 | Cl | Prop-1-en-3-yl | CH₃ | NCH₃ |
| 2474 | Cl | trans-But-2-en-1-yl | CH₃ | NCH₃ |
| 2475 | Cl | cis-But-2-en-1-yl | CH₃ | NCH₃ |
| 2476 | Cl | cis-3-Methyl-but-2-en-1-yl | CH₃ | NCH₃ |
| 2477 | Cl | Cyclopropyl | CH₃ | NCH₃ |
| 2478 | Cl | Cyclopentyl | CH₃ | NCH₃ |
| 2479 | Cl | Cyclohexyl | CH₃ | NCH₃ |
| 2480 | Cl | 4,5-Dihydroisoxazol-3-yl | CH₃ | NCH₃ |
| 2481 | Cl | 4-Methyl-4,5-dihydroisoxazol-3-yl | CH₃ | NCH₃ |
| 2482 | Cl | Isoxazol-3-yl | CH₃ | NCH₃ |
| 2483 | Cl | 4-Methylisoxazol-3-yl | CH₃ | NCH₃ |
| 2484 | Cl | 4,5-Dihydroisoxazol-4-yl | CH₃ | NCH₃ |
| 2485 | Cl | 3-Methyl-4,5-dihydroisoxazol-4-yl | CH₃ | NCH₃ |
| 2486 | Cl | Isoxazol-4-yl | CH₃ | NCH₃ |
| 2487 | Cl | 3-Methylisoxazol-4-yl | CH₃ | NCH₃ |
| 2488 | Cl | Phenyl | CH₃ | NCH₃ |
| 2489 | Cl | Benzyl | CH₃ | NCH₃ |
| 2490 | Cl | Benzoyl | CH₃ | NCH₃ |
| 2491 | Cl | 2-Pyridyl | CH₃ | NCH₃ |
| 2493 | Cl | CH₃ | Cl | NCH₃ |
| 2494 | Cl | C₂H₅ | Cl | NCH₃ |
| 2495 | Cl | n-C₃H₇ | Cl | NCH₃ |
| 2496 | Cl | i-C₃H₇ | Cl | NCH₃ |
| 2497 | Cl | n-C₄H₉ | Cl | NCH₃ |
| 2498 | Cl | i-C₄H₉ | Cl | NCH₃ |
| 2499 | Cl | s-C₄H₉ | Cl | NCH₃ |
| 2500 | Cl | t-C₄H₉ | Cl | NCH₃ |
| 2501 | Cl | CH₂OCH₃ | Cl | NCH₃ |
| 2502 | Cl | CF₃ | Cl | NCH₃ |
| 2503 | Cl | CF₂H | Cl | NCH₃ |
| 2504 | Cl | CN | Cl | NCH₃ |
| 2505 | Cl | OH | Cl | NCH₃ |
| 2506 | Cl | OCH₃ | Cl | NCH₃ |
| 2507 | Cl | NH₂ | Cl | NCH₃ |
| 2508 | Cl | NHCH₃ | Cl | NCH₃ |
| 2509 | Cl | N(CH₃)₂ | Cl | NCH₃ |
| 2510 | Cl | CO₂CH₃ | Cl | NCH₃ |
| 2511 | Cl | CO₂C₂H₅ | Cl | NCH₃ |
| 2512 | Cl | C(O)CH₃ | Cl | NCH₃ |
| 2513 | Cl | C(O)CF₃ | Cl | NCH₃ |
| 2514 | Cl | C(=NOCH₃)CH₃ | Cl | NCH₃ |
| 2515 | Cl | SO₂CH₃ | Cl | NCH₃ |
| 2516 | Cl | SO₂CF₃ | Cl | NCH₃ |
| 2517 | Cl | CH₂CO₂H | Cl | NCH₃ |
| 2518 | Cl | CH₂COOCH₃ | Cl | NCH₃ |
| 2519 | Cl | CH₂COOC₂H₅ | Cl | NCH₃ |
| 2520 | Cl | Prop-1-en-3-yl | Cl | NCH₃ |
| 2521 | Cl | trans-But-2-en-1-yl | Cl | NCH₃ |
| 2522 | Cl | cis-But-2-en-1-yl | Cl | NCH₃ |
| 2523 | Cl | cis-3-Methyl-but-2-en-1-yl | Cl | NCH₃ |
| 2524 | Cl | Cyclopropyl | Cl | NCH₃ |
| 2525 | Cl | Cyclopentyl | Cl | NCH₃ |
| 2526 | Cl | Cyclohexyl | Cl | NCH₃ |
| 2527 | Cl | 4,5-Dihydroisoxazol-3-yl | Cl | NCH₃ |
| 2528 | Cl | 4-Methyl-4,5-dihydroisoxazol-3-yl | Cl | NCH₃ |
| 2529 | Cl | Isoxazol-3-yl | Cl | NCH₃ |
| 2530 | Cl | 4-Methylisoxazol-3-yl | Cl | NCH₃ |
| 2531 | Cl | 4,5-Dihydroisoxazol-4-yl | Cl | NCH₃ |
| 2532 | Cl | 3-Methyl-4,5-dihydroisoxazol-4-yl | Cl | NCH₃ |
| 2533 | Cl | Isoxazol-4-yl | Cl | NCH₃ |
| 2534 | Cl | 3-Methylisoxazol-4-yl | Cl | NCH₃ |
| 2535 | Cl | Phenyl | Cl | NCH₃ |
| 2536 | Cl | Benzyl | Cl | NCH₃ |
| 2537 | Cl | Benzoyl | Cl | NCH₃ |
| 2538 | Cl | 2-Pyridyl | Cl | NCH₃ |
| 2540 | OCH₃ | CH₃ | H | NCH₃ |
| 2541 | OCH₃ | C₂H₅ | H | NCH₃ |
| 2542 | OCH₃ | n-C₃H₇ | H | NCH₃ |
| 2543 | OCH₃ | i-C₃H₇ | H | NCH₃ |
| 2544 | OCH₃ | n-C₄H₉ | H | NCH₃ |
| 2545 | OCH₃ | i-C₄H₉ | H | NCH₃ |
| 2546 | OCH₃ | s-C₄H₉ | H | NCH₃ |
| 2547 | OCH₃ | t-C₄H₉ | H | NCH₃ |
| 2548 | OCH₃ | CH₂OCH₃ | H | NCH₃ |
| 2549 | OCH₃ | CF₃ | H | NCH₃ |
| 2550 | OCH₃ | CF₂H | H | NCH₃ |
| 2551 | OCH₃ | CN | H | NCH₃ |
| 2552 | OCH₃ | OH | H | NCH₃ |
| 2553 | OCH₃ | OCH₃ | H | NCH₃ |
| 2554 | OCH₃ | NH₂ | H | NCH₃ |
| 2555 | OCH₃ | NHCH₃ | H | NCH₃ |
| 2556 | OCH₃ | N(CH₃)₂ | H | NCH₃ |
| 2557 | OCH₃ | CO₂CH₃ | H | NCH₃ |
| 2558 | OCH₃ | CO₂C₂H₅ | H | NCH₃ |
| 2559 | OCH₃ | C(O)CH₃ | H | NCH₃ |
| 2560 | OCH₃ | C(O)CF₃ | H | NCH₃ |
| 2561 | OCH₃ | C(=NOCH₃)CH₃, | H | NCH₃ |
| 2562 | OCH₃ | SO₂CH₃ | H | NCH₃ |
| 2563 | OCH₃ | SO₂CF₃ | H | NCH₃ |
| 2564 | OCH₃ | CH₂CO₂H | H | NCH₃ |
| 2565 | OCH₃ | CH₂COOCH₃ | H | NCH₃ |
| 2566 | OCH₃ | CH₂COOC₂H₅ | H | NCH₃ |
| 2567 | OCH₃ | Prop-1-en-3-yl | H | NCH₃ |
| 2568 | OCH₃ | trans-But-2-en-1-yl | H | NCH₃ |
| 2569 | OCH₃ | cis-But-2-en-1-yl | H | NCH₃ |
| 2570 | OCH₃ | cis-3-Methyl-but-2-en-1-yl | H | NCH₃ |
| 2571 | OCH₃ | Cyclopropyl | H | NCH₃ |
| 2572 | OCH₃ | Cyclopentyl | H | NCH₃ |
| 2573 | OCH₃ | Cyclohexyl | H | NCH₃ |
| 2574 | OCH₃ | 4,5-Dihydroisoxazol-3-yl | H | NCH₃ |
| 2575 | OCH₃ | 4-Methyl-4,5-dihydroisoxazol-3-yl | H | NCH₃ |
| 2576 | OCH₃ | Isoxazol-3-yl | H | NCH₃ |
| 2577 | OCH₃ | 4-Methylisoxazol-3-yl | H | NCH₃ |
| 2578 | OCH₃ | 4,5-Dihydroisoxazol-4-yl | H | NCH₃ |
| 2579 | OCH₃ | 3-Methyl-4,5-dihydroisoxazol-4-yl | H | NCH₃ |
| 2580 | OCH₃ | Isoxazol-4-yl | H | NCH₃ |
| 2581 | OCH₃ | 3-Methylisoxazol-4-yl | H | NCH₃ |
| 2582 | OCH₃ | Phenyl | H | NCH₃ |
| 2583 | OCH₃ | Benzyl | H | NCH₃ |
| 2584 | OCH₃ | Benzoyl | H | NCH₃ |
| 2585 | OCH₃ | 2-Pyridyl | H | NCH₃ |
| 2587 | OCH₃ | CH₃ | CH₃ | NCH₃ |
| 2588 | OCH₃ | C₂H₅ | CH₃ | NCH₃ |
| 2589 | OCH₃ | n-C₃H₇ | CH₃ | NCH₃ |
| 2590 | OCH₃ | i-C₃H₇ | CH₃ | NCH₃ |
| 2591 | OCH₃ | n-C₄H₉ | CH₃ | NCH₃ |
| 2592 | OCH₃ | i-C₄H₉ | CH₃ | NCH₃ |
| 2593 | OCH₃ | s-C₄H₉ | CH₃ | NCH₃ |
| 2594 | OCH₃ | t-C₄H₉ | CH₃ | NCH₃ |
| 2595 | OCH₃ | CH₂OCH₃ | CH₃ | NCH₃ |
| 2596 | OCH₃ | CF₃ | CH₃ | NCH₃ |
| 2597 | OCH₃ | CF₂H | CH₃ | NCH₃ |
| 2598 | OCH₃ | CN | CH₃ | NCH₃ |
| 2599 | OCH₃ | OH | CH₃ | NCH₃ |
| 2600 | OCH₃ | OCH₃ | CH₃ | NCH₃ |
| 2601 | OCH₃ | NH₂ | CH₃ | NCH₃ |
| 2602 | OCH₃ | NHCH₃ | CH₃ | NCH₃ |
| 2603 | OCH₃ | N(CH₃)₂ | CH₃ | NCH₃ |
| 2604 | OCH₃ | CO₂CH₃ | CH₃ | NCH₃ |
| 2605 | OCH₃ | CO₂C₂H₅ | CH₃ | NCH₃ |
| 2606 | OCH₃ | C(O)CH₃ | CH₃ | NCH₃ |
| 2607 | OCH₃ | C(O)CF₃ | CH₃ | NCH₃ |
| 2608 | OCH₃ | C(=NOCH₃)CH₃ | CH₃ | NCH₃ |
| 2609 | OCH₃ | SO₂CH₃ | CH₃ | NCH₃ |
| 2610 | OCH₃ | SO₂CF₃ | CH₃ | NCH₃ |
| 2611 | OCH₃ | CH₂CO₂H | CH₃ | NCH₃ |
| 2612 | OCH₃ | CH₂COOCH₃ | CH₃ | NCH₃ |
| 2613 | OCH₃ | CH₂COOC₂H₅ | CH₃ | NCH₃ |
| 2614 | OCH₃ | Prop-1-en-3-yl | CH₃ | NCH₃ |
| 2615 | OCH₃ | trans-But-2-en-1-yl | CH₃ | NCH₃ |
| 2616 | OCH₃ | cis-But-2-en-1-yl | CH₃ | NCH₃ |
| 2617 | OCH₃ | cis-3-Methyl-but-2-en-1-yl | CH₃ | NCH₃ |
| 2618 | OCH₃ | Cyclopropyl | CH₃ | NCH₃ |
| 2619 | OCH₃ | Cyclopentyl | CH₃ | NCH₃ |
| 2620 | OCH₃ | Cyclohexyl | CH₃ | NCH₃ |
| 2621 | OCH₃ | 4,5-Dihydroisoxazol-3-yl | CH₃ | NCH₃ |
| 2622 | OCH₃ | 4-Methyl-4,5-dihydroisoxazol-3-yl | CH₃ | NCH₃ |
| 2623 | OCH₃ | Isoxazol-3-yl | CH₃ | NCH₃ |
| 2624 | OCH₃ | 4-Methylisoxazol-3-yl | CH₃ | NCH₃ |
| 2625 | OCH₃ | 4,5-Dihydroisoxazol-4-yl | CH₃ | NCH₃ |
| 2626 | OCH₃ | 3-Methyl-4,5-dihydroisoxazol-4-yl | CH₃ | NCH₃ |
| 2627 | OCH₃ | Isoxazol-4-yl | CH₃ | NCH₃ |
| 2628 | OCH₃ | 3-Methylisoxazol-4-yl | CH₃ | NCH₃ |
| 2629 | OCH₃ | Phenyl | CH₃ | NCH₃ |
| 2630 | OCH₃ | Benzyl | CH₃ | NCH₃ |
| 2631 | OCH₃ | Benzoyl | CH₃ | NCH₃ |
| 2632 | OCH₃ | 2-Pyridyl | CH₃ | NCH₃ |
| 2634 | OCH₃ | CH₃ | Cl | NCH₃ |
| 2635 | OCH₃ | C₂H₅ | Cl | NCH₃ |
| 2636 | OCH₃ | n-C₃H₇ | Cl | NCH₃ |
| 2637 | OCH₃ | i-C₃H₇ | Cl | NCH₃ |
| 2638 | OCH₃ | n-C₄H₉ | Cl | NCH₃ |
| 2639 | OCH₃ | i-C₄H₉ | Cl | NCH₃ |
| 2640 | OCH₃ | s-C₄H₉ | Cl | NCH₃ |
| 2641 | OCH₃ | t-C₄H₉ | Cl | NCH₃ |
| 2642 | OCH₃ | CH₂OCH₃ | Cl | NCH₃ |
| 2643 | OCH₃ | CF₃ | Cl | NCH₃ |
| 2644 | OCH₃ | CF₂H | Cl | NCH₃ |
| 2645 | OCH₃ | CN | Cl | NCH₃ |
| 2646 | OCH₃ | OH | Cl | NCH₃ |
| 2647 | OCH₃ | OCH₃ | Cl | NCH₃ |
| 2648 | OCH₃ | NH₂ | Cl | NCH₃ |
| 2649 | OCH₃ | NHCH₃ | Cl | NCH₃ |
| 2650 | OCH₃ | N(CH₃)₂ | Cl | NCH₃ |
| 2651 | OCH₃ | CO₂CH₃ | Cl | NCH₃ |
| 2652 | OCH₃ | CO₂C₂H₅ | Cl | NCH₃ |
| 2653 | OCH₃ | C(O)CH₃ | Cl | NCH₃ |
| 2654 | OCH₃ | C(O)CF₃ | Cl | NCH₃ |
| 2655 | OCH₃ | C(=NOCH₃)CH₃ | Cl | NCH₃ |
| 2656 | OCH₃ | SO₂CH₃ | Cl | NCH₃ |
| 2657 | OCH₃ | SO₂CF₃ | Cl | NCH₃ |
| 2658 | OCH₃ | CH₂CO₂H | Cl | NCH₃ |
| 2659 | OCH₃ | CH₂COOCH₃ | Cl | NCH₃ |
| 2660 | OCH₃ | CH₂COOC₂H₅ | Cl | NCH₃ |
| 2661 | OCH₃ | Prop-1-en-3-yl | Cl | NCH₃ |
| 2662 | OCH₃ | trans-But-2-en-1-yl | Cl | NCH₃ |
| 2663 | OCH₃ | cis-But-2-en-1-yl | Cl | NCH₃ |
| 2664 | OCH₃ | cis-3-Methyl-but-2-en-1-yl | Cl | NCH₃ |
| 2665 | OCH₃ | Cyclopropyl | Cl | NCH₃ |
| 2666 | OCH₃ | Cyclopentyl | Cl | NCH₃ |
| 2667 | OCH₃ | Cyclohexyl | Cl | NCH₃ |
| 2668 | OCH₃ | 4,5-Dihydroisoxazol-3-yl | Cl | NCH₃ |
| 2669 | OCH₃ | 4-Methyl-4,5-dihydroisoxazol-3-yl | Cl | NCH₃ |
| 2670 | OCH₃ | Isoxazol-3-yl | Cl | NCH₃ |
| 2671 | OCH₃ | 4-Methylisoxazol-3-yl | Cl | NCH₃ |
| 2672 | OCH₃ | 4,5-Dihydroisoxazol-4-yl | Cl | NCH₃ |
| 2673 | OCH₃ | 3-Methyl-4,5-dihydroisoxazol-4-yl | Cl | NCH₃ |
| 2674 | OCH₃ | Isoxazol-4-yl | Cl | NCH₃ |
| 2675 | OCH₃ | 3-Methylisoxazol-4-yl | Cl | NCH₃ |
| 2676 | OCH₃ | Phenyl | Cl | NCH₃ |
| 2677 | OCH₃ | Benzyl | Cl | NCH₃ |
| 2678 | OCH₃ | Benzoyl | Cl | NCH₃ |
| 2679 | OCH₃ | 2-Pyridyl | Cl | NCH₃ |
| 2681 | OCF₃ | CH₃ | H | NCH₃ |
| 2682 | OCF₃ | C₂H₅ | H | NCH₃ |
| 2683 | OCF₃ | n-C₃H₇ | H | NCH₃ |
| 2684 | OCF₃ | i-C₃H₇ | H | NCH₃ |
| 2685 | OCF₃ | n-C₄H₉ | H | NCH₃ |
| 2686 | OCF₃ | i-C₄H₉ | H | NCH₃ |
| 2687 | OCF₃ | s-C₄H₉ | H | NCH₃ |
| 2688 | OCF₃ | t-C₄H₉ | H | NCH₃ |
| 2689 | OCF₃ | CH₂OCH₃ | H | NCH₃ |
| 2690 | OCF₃ | CF₃ | H | NCH₃ |
| 2691 | OCF₃ | CF₂H | H | NCH₃ |
| 2692 | OCF₃ | CN | H | NCH₃ |
| 2693 | OCF₃ | OH | H | NCH₃ |
| 2694 | OCF₃ | OCH₃ | H | NCH₃ |
| 2695 | OCF₃ | NH₂ | H | NCH₃ |
| 2696 | OCF₃ | NHCH₃ | H | NCH₃ |
| 2697 | OCF₃ | N(CH₃)₂ | H | NCH₃ |
| 2698 | OCF₃ | CO₂CH₃ | H | NCH₃ |
| 2699 | OCF₃ | CO₂C₂H₅ | H | NCH₃ |
| 2700 | OCF₃ | C(O)CH₃ | H | NCH₃ |
| 2701 | OCF₃ | C(O)CF₃ | H | NCH₃ |
| 2702 | OCF₃ | C(=NOCH₃)CH₃ | H | NCH₃ |
| 2703 | OCF₃ | SO₂CH₃ | H | NCH₃ |
| 2704 | OCF₃ | SO₂CF₃ | H | NCH₃ |
| 2705 | OCF₃ | CH₂CO₂H | H | NCH₃ |
| 2706 | OCF₃ | CH₂COOCH₃ | H | NCH₃ |
| 2707 | OCF₃ | CH₂COOC₂H₅ | H | NCH₃ |
| 2708 | OCF₃ | Prop-1-en-3-yl | H | NCH₃ |
| 2709 | OCF₃ | trans-But-2-en-1-yl | H | NCH₃ |
| 2710 | OCF₃ | cis-But-2-en-1-yl | H | NCH₃ |
| 2711 | OCF₃ | cis-3-Methyl-but-2-en-1-yl | H | NCH₃ |
| 2712 | OCF₃ | Cyclopropyl | H | NCH₃ |
| 2713 | OCF₃ | Cyclopentyl | H | NCH₃ |
| 2714 | OCF₃ | Cyclohexyl | H | NCH₃ |
| 2715 | OCF₃ | 4,5-Dihydroisoxazol-3-yl | H | NCH₃ |
| 2716 | OCF₃ | 4-Methyl-4,5-dihydroisoxazol-3-yl | H | NCH₃ |
| 2717 | OCF₃ | Isoxazol-3-yl | H | NCH₃ |
| 2718 | OCF₃ | 4-Methylisoxazol-3-yl | H | NCH₃ |
| 2719 | OCF₃ | 4,5-Dihydroisoxazol-4-yl | H | NCH₃ |
| 2720 | OCF₃ | 3-Methyl-4,5-dihydroisoxazol-4-yl | H | NCH₃ |
| 2721 | OCF₃ | Isoxazol-4-yl | H | NCH₃ |
| 2722 | OCF₃ | 3-Methylisoxazol-4-yl | H | NCH₃ |
| 2723 | OCF₃ | Phenyl | H | NCH₃ |
| 2724 | OCF₃ | Benzyl | H | NCH₃ |
| 2725 | OCF₃ | Benzoyl | H | NCH₃ |
| 2726 | OCF₃ | 2-Pyridyl | H | NCH₃ |
| 2728 | OCF₃ | CH₃ | CH₃ | NCH₃ |
| 2729 | OCF₃ | C₂H₅ | CH₃ | NCH₃ |
| 2730 | OCF₃ | n-C₃H₇ | CH₃ | NCH₃ |
| 2731 | OCF₃ | i-C₃H₇ | CH₃ | NCH₃ |
| 2732 | OCF₃ | n-C₄H₉ | CH₃ | NCH₃ |
| 2733 | OCF₃ | i-C₄H₉ | CH₃ | NCH₃ |
| 2734 | OCF₃ | s-C₄H₉ | CH₃ | NCH₃ |
| 2735 | OCF₃ | t-C₄H₉ | CH₃ | NCH₃ |
| 2736 | OCF₃ | CH₂OCH₃ | CH₃ | NCH₃ |
| 2737 | OCF₃ | CF₃ | CH₃ | NCH₃ |
| 2738 | OCF₃ | CF₂H | CH₃ | NCH₃ |
| 2739 | OCF₃ | CN | CH₃ | NCH₃ |
| 2740 | OCF₃ | OH | CH₃ | NCH₃ |
| 2741 | OCF₃ | OCH₃ | CH₃ | NCH₃ |
| 2742 | OCF₃ | NH₂ | CH₃ | NCH₃ |
| 2743 | OCF₃ | NHCH₃ | CH₃ | NCH₃ |
| 2744 | OCF₃ | N(CH₃)₂ | CH₃ | NCH₃ |
| 2745 | OCF₃ | CO₂CH₃ | CH₃ | NCH₃ |
| 2746 | OCF₃ | CO₂C₂H₅ | CH₃ | NCH₃ |
| 2747 | OCF₃ | C(O)CH₃ | CH₃ | NCH₃ |
| 2748 | OCF₃ | C(O)CF₃ | CH₃ | NCH₃ |
| 2749 | OCF₃ | C(=NOCH₃)CH₃ | CH₃ | NCH₃ |
| 2750 | OCF₃ | SO₂CH₃ | CH₃ | NCH₃ |
| 2751 | OCF₃ | SO₂CF₃ | CH₃ | NCH₃ |
| 2752 | OCF₃ | CH₂CO₂H | CH₃ | NCH₃ |
| 2753 | OCF₃ | CH₂COOCH₃ | CH₃ | NCH₃ |
| 2754 | OCF₃ | CH₂COOC₂H₅ | CH₃ | NCH₃ |
| 2755 | OCF₃ | Prop-1-en-3-yl | CH₃ | NCH₃ |
| 2756 | OCF₃ | trans-But-2-en-1-yl | CH₃ | NCH₃ |
| 2757 | OCF₃ | cis-But-2-en-1-yl | CH₃ | NCH₃ |
| 2758 | OCF₃ | cis-3-Methyl-but-2-en-1-yl | CH₃ | NCH₃ |
| 2759 | OCF₃ | Cyclopropyl | CH₃ | NCH₃ |
| 2760 | OCF₃ | Cyclopentyl | CH₃ | NCH₃ |
| 2761 | OCF₃ | Cyclohexyl | CH₃ | NCH₃ |
| 2762 | OCF₃ | 4,5-Dihydroisoxazol-3-yl | CH₃ | NCH₃ |
| 2763 | OCF₃ | 4-Methyl-4,5-dihydroisoxazol-3-yl | CH₃ | NCH₃ |
| 2764 | OCF₃ | Isoxazol-3-yl | CH₃ | NCH₃ |
| 2765 | OCF₃ | 4-Methylisoxazol-3-yl | CH₃ | NCH₃ |
| 2766 | OCF₃ | 4,5-Dihydroisoxazol-4-yl | CH₃ | NCH₃ |
| 2767 | OCF₃ | 3-Methyl-4,5-dihydroisoxazol-4-yl | CH₃ | NCH₃ |
| 2768 | OCF₃ | Isoxazol-4-yl | CH₃ | NCH₃ |
| 2769 | OCF₃ | 3-Methylisoxazol-4-yl | CH₃ | NCH₃ |
| 2770 | OCF₃ | Phenyl | CH₃ | NCH₃ |
| 2771 | OCF₃ | Benzyl | CH₃ | NCH₃ |
| 2772 | OCF₃ | Benzoyl | CH₃ | NCH₃ |
| 2773 | OCF₃ | 2-Pyridyl | CH₃ | NCH₃ |
| 2775 | OCF₃ | CH₃ | Cl | NCH₃ |
| 2776 | OCF₃ | C₂H₅ | Cl | NCH₃ |
| 2777 | OCF₃ | n-C₃H₇ | Cl | NCH₃ |
| 2778 | OCF₃ | i-C₃H₇ | Cl | NCH₃ |
| 2779 | OCF₃ | n-C₄H₉ | Cl | NCH₃ |
| 2780 | OCF₃ | i-C₄H₉ | Cl | NCH₃ |
| 2781 | OCF₃ | s-C₄H₉ | Cl | NCH₃ |
| 2782 | OCF₃ | t-C₄H₉ | Cl | NCH₃ |
| 2783 | OCF₃ | CH₂OCH₃ | Cl | NCH₃ |
| 2784 | OCF₃ | CF₃ | Cl | NCH₃ |
| 2785 | OCF₃ | CF₂H | Cl | NCH₃ |
| 2786 | OCF₃ | CN | Cl | NCH₃ |
| 2787 | OCF₃ | OH | Cl | NCH₃ |
| 2788 | OCF₃ | OCH₃ | Cl | NCH₃ |
| 2789 | OCF₃ | NH₂ | Cl | NCH₃ |
| 2790 | OCF₃ | NHCH₃ | Cl | NCH₃ |
| 2791 | OCF₃ | N(CH₃)₂ | Cl | NCH₃ |
| 2792 | OCF₃ | CO₂CH₃ | Cl | NCH₃ |
| 2793 | OCF₃ | CO₂C₂H₅ | Cl | NCH₃ |
| 2794 | OCF₃ | C(O)CH₃ | Cl | NCH₃ |
| 2795 | OCF₃ | C(O)CF₃ | Cl | NCH₃ |
| 2796 | OCF₃ | C(=NOCH₃)CH₃ | Cl | NCH₃ |
| 2797 | OCF₃ | SO₂CH₃ | Cl | NCH₃ |
| 2798 | OCF₃ | SO₂CF₃ | Cl | NCH₃ |
| 2799 | OCF₃ | CH₂CO₂H | Cl | NCH₃ |
| 2800 | OCF₃ | CH₂COOCH₃ | Cl | NCH₃ |
| 2801 | OCF₃ | CH₂COOC₂H₅ | Cl | NCH₃ |
| 2802 | OCF₃ | Prop-1-en-3-yl | Cl | NCH₃ |
| 2803 | OCF₃ | trans-But-2-en-1-yl | Cl | NCH₃ |
| 2804 | OCF₃ | cis-But-2-en-1-yl | Cl | NCH₃ |
| 2805 | OCF₃ | cis-3-Methyl-but-2-en-1-yl | Cl | NCH₃ |
| 2806 | OCF₃ | Cyclopropyl | Cl | NCH₃ |
| 2807 | OCF₃ | Cyclopentyl | Cl | NCH₃ |
| 2808 | OCF₃ | Cyclohexyl | Cl | NCH₃ |
| 2809 | OCF₃ | 4,5-Dihydroisoxazol-3-yl | Cl | NCH₃ |
| 2810 | OCF₃ | 4-Methyl-4,5-dihydroisoxazol-3-yl | Cl | NCH₃ |
| 2811 | OCF₃ | Isoxazol-3-yl | Cl | NCH₃ |
| 2812 | OCF₃ | 4-Methylisoxazol-3-yl | Cl | NCH₃ |
| 2813 | OCF₃ | 4,5-Dihydroisoxazol-4-yl | Cl | NCH₃ |
| 2814 | OCF₃ | 3-Methyl-4,5-dihydroisoxazol-4-yl | Cl | NCH₃ |
| 2815 | OCF₃ | Isoxazol-4-yl | Cl | NCH₃ |
| 2816 | OCF₃ | 3-Methylisoxazol-4-yl | Cl | NCH₃ |
| 2817 | OCF₃ | Phenyl | Cl | NCH₃ |
| 2818 | OCF₃ | Benzyl | Cl | NCH₃ |
| 2819 | OCF₃ | Benzoyl | Cl | NCH₃ |
| 2820 | OCF₃ | 2-Pyridyl | Cl | NCH₃ |
| 2822 | SCH₃ | CH₃ | H | NCH₃ |
| 2823 | SCH₃ | C₂H₅ | H | NCH₃ |
| 2824 | SCH₃ | n-C₃H₇ | H | NCH₃ |
| 2825 | SCH₃ | i-C₃H₇ | H | NCH₃ |
| 2826 | SCH₃ | n-C₄H₉ | H | NCH₃ |
| 2827 | SCH₃ | i-C₄H₉ | H | NCH₃ |
| 2828 | SCH₃ | s-C₄H₉ | H | NCH₃ |
| 2829 | SCH₃ | t-C₄H₉ | H | NCH₃ |
| 2830 | SCH₃ | CH₂OCH₃ | H | NCH₃ |
| 2831 | SCH₃ | CF₃ | H | NCH₃ |
| 2832 | SCH₃ | CF₂H | H | NCH₃ |
| 2833 | SCH₃ | CN | H | NCH₃ |
| 2834 | SCH₃ | OH | H | NCH₃ |
| 2835 | SCH₃ | OCH₃ | H | NCH₃ |
| 2836 | SCH₃ | NH₂ | H | NCH₃ |
| 2837 | SCH₃ | NHCH₃ | H | NCH₃ |
| 2838 | SCH₃ | N(CH₃)₂ | H | NCH₃ |
| 2839 | SCH₃ | CO₂CH₃ | H | NCH₃ |
| 2840 | SCH₃ | CO₂C₂H₅ | H | NCH₃ |
| 2841 | SCH₃ | C(O)CH₃ | H | NCH₃ |
| 2842 | SCH₃ | C(O)CF₃ | H | NCH₃ |
| 2843 | SCH₃ | C(=NOCH₃)CH₃ | H | NCH₃ |
| 2844 | SCH₃ | SO₂CH₃ | H | NCH₃ |
| 2845 | SCH₃ | SO₂CF₃ | H | NCH₃ |
| 2846 | SCH₃ | CH₂CO₂H | H | NCH₃ |
| 2847 | SCH₃ | CH₂COOCH₃ | H | NCH₃ |
| 2848 | SCH₃ | CH₂COOC₂H₅ | H | NCH₃ |
| 2849 | SCH₃ | Prop-1-en-3-yl | H | NCH₃ |
| 2850 | SCH₃ | trans-But-2-en-1-yl | H | NCH₃ |
| 2851 | SCH₃ | cis-But-2-en-1-yl | H | NCH₃ |
| 2852 | SCH₃ | cis-3-Methyl-but-2-en-1-yl | H | NCH₃ |
| 2853 | SCH₃ | Cyclopropyl | H | NCH₃ |
| 2854 | SCH₃ | Cyclopentyl | H | NCH₃ |
| 2855 | SCH₃ | Cyclohexyl | H | NCH₃ |
| 2856 | SCH₃ | 4,5-Dihydroisoxazol-3-yl | H | NCH₃ |
| 2857 | SCH₃ | 4-Methyl-4,5-dihydroisoxazol-3-yl | H | NCH₃ |
| 2858 | SCH₃ | Isoxazol-3-yl | H | NCH₃ |
| 2859 | SCH₃ | 4-Methylisoxazol-3-yl | H | NCH₃ |
| 2860 | SCH₃ | 4,5-Dihydroisoxazol-4-yl | H | NCH₃ |
| 2861 | SCH₃ | 3-Methyl-4,5-dihydroisoxazol-4-yl | H | NCH₃ |
| 2862 | SCH₃ | Isoxazol-4-yl | H | NCH₃ |
| 2863 | SCH₃ | 3-Methylisoxazol-4-yl | H | NCH₃ |
| 2864 | SCH₃ | Phenyl | H | NCH₃ |
| 2865 | SCH₃ | Benzyl | H | NCH₃ |
| 2866 | SCH₃ | Benzoyl | H | NCH₃ |
| 2867 | SCH₃ | 2-Pyridyl | H | NCH₃ |
| 2869 | SCH₃ | CH₃ | CH₃ | NCH₃ |
| 2870 | SCH₃ | C₂H₅ | CH₃ | NCH₃ |
| 2871 | SCH₃ | n-C₃H₇ | CH₃ | NCH₃ |
| 2872 | SCH₃ | i-C₃H₇ | CH₃ | NCH₃ |
| 2873 | SCH₃ | n-C₄H₉ | CH₃ | NCH₃ |
| 2874 | SCH₃ | i-C₄H₉ | CH₃ | NCH₃ |
| 2875 | SCH₃ | s-C₄H₉ | CH₃ | NCH₃ |
| 2876 | SCH₃ | t-C₄H₉ | CH₃ | NCH₃ |
| 2877 | SCH₃ | CH₂OCH₃ | CH₃ | NCH₃ |
| 2878 | SCH₃ | CF₃ | CH₃ | NCH₃ |
| 2879 | SCH₃ | CF₂H | CH₃ | NCH₃ |
| 2880 | SCH₃ | CN | CH₃ | NCH₃ |
| 2881 | SCH₃ | OH | CH₃ | NCH₃ |
| 2882 | SCH₃ | OCH₃ | CH₃ | NCH₃ |
| 2883 | SCH₃ | NH₂ | CH₃ | NCH₃ |
| 2884 | SCH₃ | NHCH₃ | CH₃ | NCH₃ |
| 2885 | SCH₃ | N(CH₃)₂ | CH₃ | NCH₃ |
| 2886 | SCH₃ | CO₂CH₃ | CH₃ | NCH₃ |
| 2887 | SCH₃ | CO₂C₂H₅ | CH₃ | NCH₃ |
| 2888 | SCH₃ | C(O)CH₃ | CH₃ | NCH₃ |
| 2889 | SCH₃ | C(O)CF₃ | CH₃ | NCH₃ |
| 2890 | SCH₃ | C(=NOCH₃)CH₃ | CH₃ | NCH₃ |
| 2891 | SCH₃ | SO₂CH₃ | CH₃ | NCH₃ |
| 2892 | SCH₃ | SO₂CF₃ | CH₃ | NCH₃ |
| 2893 | SCH₃ | CH₂CO₂H | CH₃ | NCH₃ |
| 2894 | SCH₃ | CH₂COOCH₃ | CH₃ | NCH₃ |
| 2895 | SCH₃ | CH₂COOC₂H₅ | CH₃ | NCH₃ |
| 2896 | SCH₃ | Prop-1-en-3-yl | CH₃ | NCH₃ |
| 2897 | SCH₃ | trans-But-2-en-1-yl | CH₃ | NCH₃ |
| 2898 | SCH₃ | cis-But-2-en-1-yl | CH₃ | NCH₃ |
| 2899 | SCH₃ | cis-3-Methyl-but-2-en-1-yl | CH₃ | NCH₃ |
| 2900 | SCH₃ | Cyclopropyl | CH₃ | NCH₃ |
| 2901 | SCH₃ | Cyclopentyl | CH₃ | NCH₃ |
| 2902 | SCH₃ | Cyclohexyl | CH₃ | NCH₃ |
| 2903 | SCH₃ | 4,5-Dihydroisoxazol-3-yl | CH₃ | NCH₃ |
| 2904 | SCH₃ | 4-Methyl-4,5-dihydroisoxazol-3-yl | CH₃ | NCH₃ |
| 2905 | SCH₃ | Isoxazol-3-yl | CH₃ | NCH₃ |
| 2906 | SCH₃ | 4-Methylisoxazol-3-yl | CH₃ | NCH₃ |
| 2907 | SCH₃ | 4,5-Dihydroisoxazol-4-yl | CH₃ | NCH₃ |
| 2908 | SCH₃ | 3-Methyl-4,5-dihydroisoxazol-4-yl | CH₃ | NCH₃ |
| 2909 | SCH₃ | Isoxazol-4-yl | CH₃ | NCH₃ |
| 2910 | SCH₃ | 3-Methylisoxazol-4-yl | CH₃ | NCH₃ |
| 2911 | SCH₃ | Phenyl | CH₃ | NCH₃ |
| 2912 | SCH₃ | Benzyl | CH₃ | NCH₃ |
| 2913 | SCH₃ | Benzoyl | CH₃ | NCH₃ |
| 2914 | SCH₃ | 2-Pyridyl | CH₃ | NCH₃ |
| 2916 | SCH₃ | CH₃ | Cl | NCH₃ |
| 2917 | SCH₃ | C₂H₅ | Cl | NCH₃ |
| 2918 | SCH₃ | n-C₃H₇ | Cl | NCH₃ |
| 2919 | SCH₃ | i-C₃H₇ | Cl | NCH₃ |
| 2920 | SCH₃ | n-C₄H₉ | Cl | NCH₃ |
| 2921 | SCH₃ | i-C₄H₉ | Cl | NCH₃ |
| 2922 | SCH₃ | s-C₄H₉ | Cl | NCH₃ |
| 2923 | SCH₃ | t-C₄H₉ | Cl | NCH₃ |
| 2924 | SCH₃ | CH₂OCH₃ | Cl | NCH₃ |
| 2925 | SCH₃ | CF₃ | Cl | NCH₃ |
| 2926 | SCH₃ | CF₂H | Cl | NCH₃ |
| 2927 | SCH₃ | CN | Cl | NCH₃ |
| 2928 | SCH₃ | OH | Cl | NCH₃ |
| 2929 | SCH₃ | OCH₃ | Cl | NCH₃ |
| 2930 | SCH₃ | NH₂ | Cl | NCH₃ |
| 2931 | SCH₃ | NHCH₃ | Cl | NCH₃ |
| 2932 | SCH₃ | N(CH₃)₂ | Cl | NCH₃ |
| 2933 | SCH₃ | CO₂CH₃ | Cl | NCH₃ |
| 2934 | SCH₃ | CO₂C₂H₅ | Cl | NCH₃ |
| 2935 | SCH₃ | C(O)CH₃ | Cl | NCH₃ |
| 2936 | SCH₃ | C(O)CF₃ | Cl | NCH₃ |
| 2937 | SCH₃ | C(=NOCH₃)CH₃ | Cl | NCH₃ |
| 2938 | SCH₃ | SO₂CH₃ | Cl | NCH₃ |
| 2939 | SCH₃ | SO₂CF₃ | Cl | NCH₃ |
| 2940 | SCH₃ | CH₂CO₂H | Cl | NCH₃ |
| 2941 | SCH₃ | CH₂COOCH₃ | Cl | NCH₃ |
| 2942 | SCH₃ | CH₂COOC₂H₅ | Cl | NCH₃ |
| 2943 | SCH₃ | Prop-1-en-3-yl | Cl | NCH₃ |
| 2944 | SCH₃ | trans-But-2-en-1-yl | Cl | NCH₃ |
| 2945 | SCH₃ | cis-But-2-en-1-yl | Cl | NCH₃ |
| 2946 | SCH₃ | cis-3-Methyl-but-2-en-1-yl | Cl | NCH₃ |
| 2947 | SCH₃ | Cyclopropyl | Cl | NCH₃ |
| 2948 | SCH₃ | Cyclopentyl | Cl | NCH₃ |
| 2949 | SCH₃ | Cyclohexyl | Cl | NCH₃ |
| 2950 | SCH₃ | 4,5-Dihydroisoxazol-3-yl | Cl | NCH₃ |
| 2951 | SCH₃ | 4-Methyl-4,5-dihydroisoxazol-3-yl | Cl | NCH₃ |
| 2952 | SCH₃ | Isoxazol-3-yl | Cl | NCH₃ |
| 2953 | SCH₃ | 4-Methylisoxazol-3-yl | Cl | NCH₃ |
| 2954 | SCH₃ | 4,5-Dihydroisoxazol-4-yl | Cl | NCH₃ |
| 2955 | SCH₃ | 3-Methyl-4,5-dihydroisoxazol-4-yl | Cl | NCH₃ |
| 2956 | SCH₃ | Isoxazol-4-yl | Cl | NCH₃ |
| 2957 | SCH₃ | 3-Methylisoxazol-4-yl | Cl | NCH₃ |
| 2958 | SCH₃ | Phenyl | Cl | NCH₃ |
| 2959 | SCH₃ | Benzyl | Cl | NCH₃ |
| 2960 | SCH₃ | Benzoyl | Cl | NCH₃ |
| 2961 | SCH₃ | 2-Pyridyl | Cl | NCH₃ |
| 2963 | SO₂CH₃ | CH₃ | H | NCH₃ |
| 2964 | SO₂CH₃ | C₂H₅ | H | NCH₃ |
| 2965 | SO₂CH₃ | n-C₃H₇ | H | NCH₃ |
| 2966 | SO₂CH₃ | i-C₃H₇ | H | NCH₃ |
| 2967 | SO₂CH₃ | n-C₄H₉ | H | NCH₃ |
| 2968 | SO₂CH₃ | i-C₄H₉ | H | NCH₃ |
| 2969 | SO₂CH₃ | s-C₄H₉ | H | NCH₃ |
| 2970 | SO₂CH₃ | t-C₄H₉ | H | NCH₃ |
| 2971 | SO₂CH₃ | CH₂OCH₃ | H | NCH₃ |
| 2972 | SO₂CH₃ | CF₃ | H | NCH₃ |
| 2973 | SO₂CH₃ | CF₂H | H | NCH₃ |
| 2974 | SO₂CH₃ | CN | H | NCH₃ |
| 2975 | SO₂CH₃ | OH | H | NCH₃ |
| 2976 | SO₂CH₃ | OCH₃ | H | NCH₃ |
| 2977 | SO₂CH₃ | NH₂ | H | NCH₃ |
| 2978 | SO₂CH₃ | NHCH₃ | H | NCH₃ |
| 2979 | SO₂CH₃ | N(CH₃)₂ | H | NCH₃ |
| 2980 | SO₂CH₃ | CO₂CH₃ | H | NCH₃ |
| 2981 | SO₂CH₃ | CO₂C₂H₅ | H | NCH₃ |
| 2982 | SO₂CH₃ | C(O)CH₃ | H | NCH₃ |
| 2983 | SO₂CH₃ | C(O)CF₃ | H | NCH₃ |
| 2984 | SO₂CH₃ | C(=NOCH₃)CH₃ | H | NCH₃ |
| 2985 | SO₂CH₃ | SO₂CH₃ | H | NCH₃ |
| 2986 | SO₂CH₃ | SO₂CF₃ | H | NCH₃ |
| 2987 | SO₂CH₃ | CH₂CO₂H | H | NCH₃ |
| 2988 | SO₂CH₃ | CH₂COOCH₃ | H | NCH₃ |
| 2989 | SO₂CH₃ | CH₂COOC₂H₅ | H | NCH₃ |
| 2990 | SO₂CH₃ | Prop-1-en-3-yl | H | NCH₃ |
| 2991 | SO₂CH₃ | trans-But-2-en-1-yl | H | NCH₃ |
| 2992 | SO₂CH₃ | cis-But-2-en-1-yl | H | NCH₃ |
| 2993 | SO₂CH₃ | cis-3-Methyl-but-2-en-1-yl | H | NCH₃ |
| 2994 | SO₂CH₃ | Cyclopropyl | H | NCH₃ |
| 2995 | SO₂CH₃ | Cyclopentyl | H | NCH₃ |
| 2996 | SO₂CH₃ | Cyclohexyl | H | NCH₃ |
| 2997 | SO₂CH₃ | 4,5-Dihydroisoxazol-3-yl | H | NCH₃ |
| 2998 | SO₂CH₃ | 4-Methyl-4,5-dihydroisoxazol-3-yl | H | NCH₃ |
| 2999 | SO₂CH₃ | Isoxazol-3-yl | H | NCH₃ |
| 3000 | SO₂CH₃ | 4-Methylisoxazol-3-yl | H | NCH₃ |
| 3001 | SO₂CH₃ | 4,5-Dihydroisoxazol-4-yl | H | NCH₃ |
| 3002 | SO₂CH₃ | 3-Methyl-4,5-dihydroisoxazol-4-yl | H | NCH₃ |
| 3003 | SO₂CH₃ | Isoxazol-4-yl | H | NCH₃ |
| 3004 | SO₂CH₃ | 3-Methylisoxazol-4-yl | H | NCH₃ |
| 3005 | SO₂CH₃ | Phenyl | H | NCH₃ |
| 3006 | SO₂CH₃ | Benzyl | H | NCH₃ |
| 3007 | SO₂CH₃ | Benzoyl | H | NCH₃ |
| 3008 | SO₂CH₃ | 2-Pyridyl | H | NCH₃ |
| 3010 | SO₂CH₃ | CH₃ | CH₃ | NCH₃ |
| 3011 | SO₂CH₃ | C₂H₅ | CH₃ | NCH₃ |
| 3012 | SO₂CH₃ | n-C₃H₇ | CH₃ | NCH₃ |
| 3013 | SO₂CH₃ | i-C₃H₇ | CH₃ | NCH₃ |
| 3014 | SO₂CH₃ | n-C₄H₉ | CH₃ | NCH₃ |
| 3015 | SO₂CH₃ | i-C₄H₉ | CH₃ | NCH₃ |
| 3016 | SO₂CH₃ | s-C₄H₉ | CH₃ | NCH₃ |
| 3017 | SO₂CH₃ | t-C₄H₉ | CH₃ | NCH₃ |
| 3018 | SO₂CH₃ | CH₂OCH₃ | CH₃ | NCH₃ |
| 3019 | SO₂CH₃ | CF₃ | CH₃ | NCH₃ |
| 3020 | SO₂CH₃ | CF₂H | CH₃ | NCH₃ |
| 3021 | SO₂CH₃ | CN | CH₃ | NCH₃ |
| 3022 | SO₂CH₃ | OH | CH₃ | NCH₃ |
| 3023 | SO₂CH₃ | OCH₃ | CH₃ | NCH₃ |
| 3024 | SO₂CH₃ | NH₂ | CH₃ | NCH₃ |
| 3025 | SO₂CH₃ | NHCH₃ | CH₃ | NCH₃ |
| 3026 | SO₂CH₃ | N(CH₃)₂ | CH₃ | NCH₃ |
| 3027 | SO₂CH₃ | CO₂CH₃ | CH₃ | NCH₃ |
| 3028 | SO₂CH₃ | CO₂C₂H₅ | CH₃ | NCH₃ |
| 3029 | SO₂CH₃ | C(O)CH₃ | CH₃ | NCH₃ |
| 3030 | SO₂CH₃ | C(O)CF₃ | CH₃ | NCH₃ |
| 3031 | SO₂CH₃ | C(=NOCH₃)CH₃ | CH₃ | NCH₃ |
| 3032 | SO₂CH₃ | SO₂CH₃ | CH₃ | NCH₃ |
| 3033 | SO₂CH₃ | SO₂CF₃ | CH₃ | NCH₃ |
| 3034 | SO₂CH₃ | CH₂CO₂H | CH₃ | NCH₃ |
| 3035 | SO₂CH₃ | CH₂COOCH₃ | CH₃ | NCH₃ |
| 3036 | SO₂CH₃ | CH₂COOC₂H₅ | CH₃ | NCH₃ |
| 3037 | SO₂CH₃ | Prop-1-en-3-yl | CH₃ | NCH₃ |
| 3038 | SO₂CH₃ | trans-But-2-en-1-yl | CH₃ | NCH₃ |
| 3039 | SO₂CH₃ | cis-But-2-en-1-yl | CH₃ | NCH₃ |
| 3040 | SO₂CH₃ | cis-3-Methyl-but-2-en-1-yl | CH₃ | NCH₃ |
| 3041 | SO₂CH₃ | Cyclopropyl | CH₃ | NCH₃ |
| 3042 | SO₂CH₃ | Cyclopentyl | CH₃ | NCH₃ |
| 3043 | SO₂CH₃ | Cyclohexyl | CH₃ | NCH₃ |
| 3044 | SO₂CH₃ | 4,5-Dihydroisoxazol-3-yl | CH₃ | NCH₃ |
| 3045 | SO₂CH₃ | 4-Methyl-4,5-dihydroisoxazol-3-yl | CH₃ | NCH₃ |
| 3046 | SO₂CH₃ | Isoxazol-3-yl | CH₃ | NCH₃ |
| 3047 | SO₂CH₃ | 4-Methylisoxazol-3-yl | CH₃ | NCH₃ |
| 3048 | SO₂CH₃ | 4,5-Dihydroisoxazol-4-yl | CH₃ | NCH₃ |
| 3049 | SO₂CH₃ | 3-Methyl-4,5-dihydroisoxazol-4-yl | CH₃ | NCH₃ |
| 3050 | SO₂CH₃ | Isoxazol-4-yl | CH₃ | NCH₃ |
| 3051 | SO₂CH₃ | 3-Methylisoxazol-4-yl | CH₃ | NCH₃ |
| 3052 | SO₂CH₃ | Phenyl | CH₃ | NCH₃ |
| 3053 | SO₂CH₃ | Benzyl | CH₃ | NCH₃ |
| 3054 | SO₂CH₃ | Benzoyl | CH₃ | NCH₃ |
| 3055 | SO₂CH₃ | 2-Pyridyl | CH₃ | NCH₃ |
| 3057 | SO₂CH₃ | CH₃ | Cl | NCH₃ |
| 3058 | SO₂CH₃ | C₂H₅ | Cl | NCH₃ |
| 3059 | SO₂CH₃ | n-C₃H₇ | Cl | NCH₃ |
| 3060 | SO₂CH₃ | i-C₃H₇ | Cl | NCH₃ |
| 3061 | SO₂CH₃ | n-C₄H₉ | Cl | NCH₃ |
| 3062 | SO₂CH₃ | i-C₄H₉ | Cl | NCH₃ |
| 3063 | SO₂CH₃ | s-C₄H₉ | Cl | NCH₃ |
| 3064 | SO₂CH₃ | t-C₄H₉ | Cl | NCH₃ |
| 3065 | SO₂CH₃ | CH₂OCH₃ | Cl | NCH₃ |
| 3066 | SO₂CH₃ | CF₃ | Cl | NCH₃ |
| 3067 | SO₂CH₃ | CF₂H | Cl | NCH₃ |
| 3068 | SO₂CH₃ | CN | Cl | NCH₃ |
| 3069 | SO₂CH₃ | OH | Cl | NCH₃ |
| 3070 | SO₂CH₃ | OCH₃ | Cl | NCH₃ |
| 3071 | SO₂CH₃ | NH₂ | Cl | NCH₃ |
| 3072 | SO₂CH₃ | NHCH₃ | Cl | NCH₃ |
| 3073 | SO₂CH₃ | N(CH₃)₂ | Cl | NCH₃ |
| 3074 | SO₂CH₃ | CO₂CH₃ | Cl | NCH₃ |
| 3075 | SO₂CH₃ | CO₂C₂H₅ | Cl | NCH₃ |
| 3076 | SO₂CH₃ | C(O)CH₃ | Cl | NCH₃ |
| 3077 | SO₂CH₃ | C(O)CF₃ | Cl | NCH₃ |
| 3078 | SO₂CH₃ | C(=NOCH₃)CH₃ | Cl | NCH₃ |
| 3079 | SO₂CH₃ | SO₂CH₃ | Cl | NCH₃ |
| 3080 | SO₂CH₃ | SO₂CF₃ | Cl | NCH₃ |
| 3081 | SO₂CH₃ | CH₂CO₂H | Cl | NCH₃ |
| 3082 | SO₂CH₃ | CH₂COOCH₃ | Cl | NCH₃ |
| 3083 | SO₂CH₃ | CH₂COOC₂H₅ | Cl | NCH₃ |
| 3084 | SO₂CH₃ | Prop-1-en-3-yl | Cl | NCH₃ |
| 3085 | SO₂CH₃ | trans-But-2-en-1-yl | Cl | NCH₃ |
| 3086 | SO₂CH₃ | cis-But-2-en-1-yl | Cl | NCH₃ |
| 3087 | SO₂CH₃ | cis-3-Methyl-but-2-en-1-yl | Cl | NCH₃ |
| 3088 | SO₂CH₃ | Cyclopropyl | Cl | NCH₃ |
| 3089 | SO₂CH₃ | Cyclopentyl | Cl | NCH₃ |
| 3090 | SO₂CH₃ | Cyclohexyl | Cl | NCH₃ |
| 3091 | SO₂CH₃ | 4,5-Dihydroisoxazol-3-yl | Cl | NCH₃ |
| 3092 | SO₂CH₃ | 4-Methyl-4,5-dihydroisoxazol-3-yl | Cl | NCH₃ |
| 3093 | SO₂CH₃ | Isoxazol-3-yl | Cl | NCH₃ |
| 3094 | SO₂CH₃ | 4-Methylisoxazol-3-yl | Cl | NCH₃ |
| 3095 | SO₂CH₃ | 4,5-Dihydroisoxazol-4-yl | Cl | NCH₃ |
| 3096 | SO₂CH₃ | 3-Methyl-4,5-dihydroisoxazol-4-yl | Cl | NCH₃ |
| 3097 | SO₂CH₃ | Isoxazol-4-yl | Cl | NCH₃ |
| 3098 | SO₂CH₃ | 3-Methylisoxazol-4-yl | Cl | NCH₃ |
| 3099 | SO₂CH₃ | Phenyl | Cl | NCH₃ |
| 3100 | SO₂CH₃ | Benzyl | Cl | NCH₃ |
| 3101 | SO₂CH₃ | Benzoyl | Cl | NCH₃ |
| 3102 | SO₂CH₃ | 2-Pyridyl | Cl | NCH₃ |
| 3104 | CF₃ | CH₃ | H | NCH₃ |
| 3105 | CF₃ | C₂H₅ | H | NCH₃ |
| 3106 | CF₃ | n-C₃H₇ | H | NCH₃ |
| 3107 | CF₃ | i-C₃H₇ | H | NCH₃ |
| 3108 | CF₃ | n-C₄H₉ | H | NCH₃ |
| 3109 | CF₃ | i-C₄H₉ | H | NCH₃ |
| 3110 | CF₃ | s-C₄H₉ | H | NCH₃ |
| 3111 | CF₃ | t-C₄H₉ | H | NCH₃ |
| 3112 | CF₃ | CH₂OCH₃ | H | NCH₃ |
| 3113 | CF₃ | CF₃ | H | NCH₃ |
| 3114 | CF₃ | CF₂H | H | NCH₃ |
| 3115 | CF₃ | CN | H | NCH₃ |
| 3116 | CF₃ | OH | H | NCH₃ |
| 3117 | CF₃ | OCH₃ | H | NCH₃ |
| 3118 | CF₃ | NH₂ | H | NCH₃ |
| 3119 | CF₃ | NHCH₃ | H | NCH₃ |
| 3120 | CF₃ | N(CH₃)₂ | H | NCH₃ |
| 3121 | CF₃ | CO₂CH₃ | H | NCH₃ |
| 3122 | CF₃ | CO₂C₂H₅ | H | NCH₃ |
| 3123 | CF₃ | C(O)CH₃ | H | NCH₃ |
| 3124 | CF₃ | C(O)CF₃ | H | NCH₃ |
| 3125 | CF₃ | C(=NOCH₃)CH₃ | H | NCH₃ |
| 3126 | CF₃ | SO₂CH₃ | H | NCH₃ |
| 3127 | CF₃ | SO₂CF₃ | H | NCH₃ |
| 3128 | CF₃ | CH₂CO₂H | H | NCH₃ |
| 3129 | CF₃ | CH₂COOCH₃ | H | NCH₃ |
| 3130 | CF₃ | CH₂COOC₂H₅ | H | NCH₃ |
| 3131 | CF₃ | Prop-1-en-3-yl | H | NCH₃ |
| 3132 | CF₃ | trans-But-2-en-1-yl | H | NCH₃ |
| 3133 | CF₃ | cis-But-2-en-1-yl | H | NCH₃ |
| 3134 | CF₃ | cis-3-Methyl-but-2-en-1-yl | H | NCH₃ |
| 3135 | CF₃ | Cyclopropyl | H | NCH₃ |
| 3136 | CF₃ | Cyclopentyl | H | NCH₃ |
| 3137 | CF₃ | Cyclohexyl | H | NCH₃ |
| 3138 | CF₃ | 4,5-Dihydroisoxazol-3-yl | H | NCH₃ |
| 3139 | CF₃ | 4-Methyl-4,5-dihydroisoxazol-3-yl | H | NCH₃ |
| 3140 | CF₃ | Isoxazol-3-yl | H | NCH₃ |
| 3141 | CF₃ | 4-Methylisoxazol-3-yl | H | NCH₃ |
| 3142 | CF₃ | 4,5-Dihydroisoxazol-4-yl | H | NCH₃ |
| 3143 | CF₃ | 3-Methyl-4,5-dihydroisoxazol-4-yl | H | NCH₃ |
| 3144 | CF₃ | Isoxazol-4-yl | H | NCH₃ |
| 3145 | CF₃ | 3-Methylisoxazol-4-yl | H | NCH₃ |
| 3146 | CF₃ | Phenyl | H | NCH₃ |
| 3147 | CF₃ | Benzyl | H | NCH₃ |
| 3148 | CF₃ | Benzoyl | H | NCH₃ |
| 3149 | CF₃ | 2-Pyridyl | H | NCH₃ |
| 3151 | CF₃ | CH₃ | CH₃ | NCH₃ |
| 3152 | CF₃ | C₂H₅ | CH₃ | NCH₃ |
| 3153 | CF₃ | n-C₃H₇ | CH₃ | NCH₃ |
| 3154 | CF₃ | i-C₃H₇ | CH₃ | NCH₃ |
| 3155 | CF₃ | n-C₄H₉ | CH₃ | NCH₃ |
| 3156 | CF₃ | i-C₄H₉ | CH₃ | NCH₃ |
| 3157 | CF₃ | s-C₄H₉ | CH₃ | NCH₃ |
| 3158 | CF₃ | t-C₄H₉ | CH₃ | NCH₃ |
| 3159 | CF₃ | CH₂OCH₃ | CH₃ | NCH₃ |
| 3160 | CF₃ | CF₃ | CH₃ | NCH₃ |
| 3161 | CF₃ | CF₂H | CH₃ | NCH₃ |
| 3162 | CF₃ | CN | CH₃ | NCH₃ |
| 3163 | CF₃ | OH | CH₃ | NCH₃ |
| 3164 | CF₃ | OCH₃ | CH₃ | NCH₃ |
| 3165 | CF₃ | NH₂ | CH₃ | NCH₃ |
| 3166 | CF₃ | NHCH₃ | CH₃ | NCH₃ |
| 3167 | CF₃ | N(CH₃)₂ | CH₃ | NCH₃ |
| 3168 | CF₃ | CO₂CH₃ | CH₃ | NCH₃ |
| 3169 | CF₃ | CO₂C₂H₅ | CH₃ | NCH₃ |
| 3170 | CF₃ | C(O)CH₃ | CH₃ | NCH₃ |
| 3171 | CF₃ | C(O)CF₃ | CH₃ | NCH₃ |
| 3172 | CF₃ | C(=NOCH₃)CH₃ | CH₃ | NCH₃ |
| 3173 | CF₃ | SO₂CH₃ | CH₃ | NCH₃ |
| 3174 | CF₃ | SO₂CF₃ | CH₃ | NCH₃ |
| 3175 | CF₃ | CH₂CO₂H | CH₃ | NCH₃ |
| 3176 | CF₃ | CH₂COOCH₃ | CH₃ | NCH₃ |
| 3177 | CF₃ | CH₂COOC₂H₅ | CH₃ | NCH₃ |
| 3178 | CF₃ | Prop-1-en-3-yl | CH₃ | NCH₃ |
| 3179 | CF₃ | trans-But-2-en-1-yl | CH₃ | NCH₃ |
| 3180 | CF₃ | cis-But-2-en-1-yl | CH₃ | NCH₃ |
| 3181 | CF₃ | cis-3-Methyl-but-2-en-1-yl | CH₃ | NCH₃ |
| 3182 | CF₃ | Cyclopropyl | CH₃ | NCH₃ |
| 3183 | CF₃ | Cyclopentyl | CH₃ | NCH₃ |
| 3184 | CF₃ | Cyclohexyl | CH₃ | NCH3 |
| 3185 | CF₃ | 4,5-Dihydroisoxazol-3-yl | CH₃ | NCH₃ |
| 3186 | CF₃ | 4-Methyl-4,5-dihydroisoxazol-3-yl | CH₃ | NCH₃ |
| 3187 | CF₃ | Isoxazol-3-yl | CH₃ | NCH₃ |
| 3188 | CF₃ | 4-Methylisoxazol-3-yl | CH₃ | NCH₃ |
| 3189 | CF₃ | 4,5-Dihydroisoxazol-4-yl | CH₃ | NCH₃ |
| 3190 | CF₃ | 3-Methyl-4,5-dihydroisoxazol-4-yl | CH₃ | NCH₃ |
| 3191 | CF₃ | Isoxazol-4-yl | CH₃ | NCH₃ |
| 3192 | CF₃ | 3-Methylisoxazol-4-yl | CH₃ | NCH₃ |
| 3193 | CF₃ | Phenyl | CH₃ | NCH₃ |
| 3194 | CF₃ | Benzyl | CH₃ | NCH₃ |
| 3195 | CF₃ | Benzoyl | CH₃ | NCH₃ |
| 3196 | CF₃ | 2-Pyridyl | CH₃ | NCH₃ |
| 3198 | CF₃ | CH₃ | Cl | NCH₃ |
| 3199 | CF₃ | C₂H₅ | Cl | NCH₃ |
| 3200 | CF₃ | n-C₃H₇ | Cl | NCH₃ |
| 3201 | CF₃ | i-C₃H₇ | Cl | NCH₃ |
| 3202 | CF₃ | n-C₄H₉ | Cl | NCH₃ |
| 3203 | CF₃ | i-C₄H₉ | Cl | NCH₃ |
| 3204 | CF₃ | s-C₄H₉ | Cl | NCH₃ |
| 3205 | CF₃ | t-C₄H₉ | Cl | NCH₃ |
| 3206 | CF₃ | CH₂OCH₃ | Cl | NCH₃ |
| 3207 | CF₃ | CF₃ | Cl | NCH₃ |
| 3208 | CF₃ | CF₂H | Cl | NCH₃ |
| 3209 | CF₃ | CN | Cl | NCH₃ |
| 3210 | CF₃ | OH | Cl | NCH₃ |
| 3211 | CF₃ | OCH₃ | Cl | NCH₃ |
| 3212 | CF₃ | NH₂ | Cl | NCH₃ |
| 3213 | CF₃ | NHCH₃ | Cl | NCH₃ |
| 3214 | CF₃ | N(CH₃)₂ | Cl | NCH₃ |
| 3215 | CF₃ | CO₂CH₃ | Cl | NCH₃ |
| 3216 | CF₃ | CO₂C₂H₅ | Cl | NCH₃ |
| 3217 | CF₃ | C(O)CH₃ | Cl | NCH₃ |
| 3218 | CF₃ | C(O)CF₃ | Cl | NCH₃ |
| 3219 | CF₃ | C(=NOCH₃)CH₃ | Cl | NCH₃ |
| 3220 | CF₃ | SO₂CH₃ | Cl | NCH₃ |
| 3221 | CF₃ | SO₂CF₃ | Cl | NCH₃ |
| 3222 | CF₃ | CH₂CO₂H | Cl | NCH₃ |
| 3223 | CF₃ | CH₂COOCH₃ | Cl | NCH₃ |
| 3224 | CF₃ | CH₂COOC₂H₅ | Cl | NCH₃ |
| 3225 | CF₃ | Prop-1-en-3-yl | Cl | NCH₃ |
| 3226 | CF₃ | trans-But-2-en-1-yl | Cl | NCH₃ |
| 3227 | CF₃ | cis-But-2-en-1-yl | Cl | NCH₃ |
| 3228 | CF₃ | cis-3-Methyl-but-2-en-1-yl | Cl | NCH₃ |
| 3229 | CF₃ | Cyclopropyl | Cl | NCH₃ |
| 3230 | CF₃ | Cyclopentyl | Cl | NCH₃ |
| 3231 | CF₃ | Cyclohexyl | Cl | NCH₃ |
| 3232 | CF₃ | 4,5-Dihydroisoxazol-3-yl | Cl | NCH₃ |
| 3233 | CF₃ | 4-Methyl-4,5-dihydroisoxazol-3-yl | Cl | NCH₃ |
| 3234 | CF₃ | Isoxazol-3-yl | Cl | NCH₃ |
| 3235 | CF₃ | 4-Methylisoxazol-3-yl | Cl | NCH₃ |
| 3236 | CF₃ | 4,5-Dihydroisoxazol-4-yl | Cl | NCH₃ |
| 3237 | CF₃ | 3-Methyl-4,5-dihydroisoxazol-4-yl | Cl | NCH₃ |
| 3238 | CF₃ | Isoxazol-4-yl | Cl | NCH₃ |
| 3239 | CF₃ | 3-Methylisoxazol-4-yl | Cl | NCH₃ |
| 3240 | CF₃ | Phenyl | Cl | NCH₃ |
| 3241 | CF₃ | Benzyl | Cl | NCH₃ |
| 3242 | CF₃ | Benzoyl | Cl | NCH₃ |
| 3243 | CF₃ | 2-Pyridyl | Cl | NCH₃ |
| 3245 | C₂H₅ | CH₃ | H | NCH₃ |
| 3246 | C₂H₅ | C₂H₅ | H | NCH₃ |
| 3247 | C₂H₅ | n-C₃H₇ | H | NCH₃ |
| 3248 | C₂H₅ | i-C₃H₇ | H | NCH₃ |
| 3249 | C₂H₅ | n-C₄H₉ | H | NCH₃ |
| 3250 | C₂H₅ | i-C₄H₉ | H | NCH₃ |
| 3251 | C₂H₅ | s-C₄H₉ | H | NCH₃ |
| 3252 | C₂H₅ | t-C₄H₉ | H | NCH₃ |
| 3253 | C₂H₅ | CH₂OCH₃ | H | NCH₃ |
| 3254 | C₂H₅ | CF₃ | H | NCH₃ |
| 3255 | C₂H₅ | CF₂H | H | NCH₃ |
| 3256 | C₂H₅ | CN | H | NCH₃ |
| 3257 | C₂H₅ | OH | H | NCH₃ |
| 3258 | C₂H₅ | OCH₃ | H | NCH₃ |
| 3259 | C₂H₅ | NH₂ | H | NCH₃ |
| 3260 | C₂H₅ | NHCH₃ | H | NCH₃ |
| 3261 | C₂H₅ | N(CH₃)₂ | H | NCH₃ |
| 3262 | C₂H₅ | CO₂CH₃ | H | NCH₃ |
| 3263 | C₂H₅ | CO₂C₂H₅ | H | NCH₃ |
| 3264 | C₂H₅ | C(O)CH₃ | H | NCH₃ |
| 3265 | C₂H₅ | C(O)CF₃ | H | NCH₃ |
| 3266 | C₂H₅ | C(=NOCH₃)CH₃ | H | NCH₃ |
| 3267 | C₂H₅ | SO₂CH₃ | H | NCH₃ |
| 3268 | C₂H₅ | SO₂CF₃ | H | NCH₃ |
| 3269 | C₂H₅ | CH₂CO₂H | H | NCH₃ |
| 3270 | C₂H₅ | CH₂COOCH₃ | H | NCH₃ |
| 3271 | C₂H₅ | CH₂COOC₂H₅ | H | NCH₃ |
| 3272 | C₂H₅ | Prop-1-en-3-yl | H | NCH₃ |
| 3273 | C₂H₅ | trans-But-2-en-1-yl | H | NCH₃ |
| 3274 | C₂H₅ | cis-But-2-en-1-yl | H | NCH₃ |
| 3275 | C₂H₅ | cis-3-Methyl-but-2-en-1-yl | H | NCH₃ |
| 3276 | C₂H₅ | Cyclopropyl | H | NCH₃ |
| 3277 | C₂H₅ | Cyclopentyl | H | NCH₃ |
| 3278 | C₂H₅ | Cyclohexyl | H | NCH₃ |
| 3279 | C₂H₅ | 4,5-Dihydroisoxazol-3-yl | H | NCH₃ |
| 3280 | C₂H₅ | 4-Methyl-4,5-dihydroisoxazol-3-yl | H | NCH₃ |
| 3281 | C₂H₅ | Isoxazol-3-yl | H | NCH₃ |
| 3282 | C₂H₅ | 4-Methylisoxazol-3-yl | H | NCH₃ |
| 3283 | C₂H₅ | 4,5-Dihydroisoxazol-4-yl | H | NCH₃ |
| 3284 | C₂H₅ | 3-Methyl-4,5-dihydroisoxazol-4-yl | H | NCH₃ |
| 3285 | C₂H₅ | Isoxazol-4-yl | H | NCH₃ |
| 3286 | C₂H₅ | 3-Methylisoxazol-4-yl | H | NCH₃ |
| 3287 | C₂H₅ | Phenyl | H | NCH₃ |
| 3288 | C₂H₅ | Benzyl | H | NCH₃ |
| 3289 | C₂H₅ | Benzoyl | H | NCH₃ |
| 3290 | C₂H₅ | 2-Pyridyl | H | NCH₃ |
| 3292 | C₂H₅ | CH₃ | CH₃ | NCH₃ |
| 3293 | C₂H₅ | C₂H₅ | CH₃ | NCH₃ |
| 3294 | C₂H₅ | n-C₃H₇ | CH₃ | NCH₃ |
| 3295 | C₂H₅ | i-C₃H₇ | CH₃ | NCH₃ |
| 3296 | C₂H₅ | n-C₄H₉ | CH₃ | NCH₃ |
| 3297 | C₂H₅ | i-C₄H₉ | CH₃ | NCH₃ |
| 3298 | C₂Hₛ | s-C₄H₉ | CH₃ | NCH₃ |
| 3299 | C₂H₅ | t-C₄H₉ | CH₃ | NCH₃ |
| 3300 | C₂H₅ | CH₂OCH₃ | CH₃ | NCH₃ |
| 3301 | C₂H₅ | CF₃ | CH₃ | NCH₃ |
| 3302 | C₂H₅ | CF₂H | CH₃ | NCH₃ |
| 3303 | C₂H₅ | CN | CH₃ | NCH₃ |
| 3304 | C₂H₅ | OH | CH₃ | NCH₃ |
| 3305 | C₂H₅ | OCH₃ | CH₃ | NCH₃ |
| 3306 | C₂H₅ | NH₂ | CH₃ | NCH₃ |
| 3307 | C₂H₅ | NHCH₃ | CH₃ | NCH₃ |
| 3308 | C₂H₅ | N(CH₃)₂ | CH₃ | NCH₃ |
| 3309 | C₂H₅ | CO₂CH₃ | CH₃ | NCH₃ |
| 3310 | C₂H₅ | CO₂C₂H₅ | CH₃ | NCH₃ |
| 3311 | C₂H₅ | C(O)CH₃ | CH₃ | NCH₃ |
| 3312 | C₂H₅ | C(O)CF₃ | CH₃ | NCH₃ |
| 3313 | C₂H₅ | C(=NOCH₃)CH₃ | CH₃ | NCH₃ |
| 3314 | C₂H₅ | SO₂CH₃ | CH₃ | NCH₃ |
| 3315 | C₂H₅ | SO₂CF₃ | CH₃ | NCH₃ |
| 3316 | C₂H₅ | CH₂CO₂H | CH₃ | NCH₃ |
| 3317 | C₂H₅ | CH₂COOCH₃ | CH₃ | NCH₃ |
| 3318 | C₂H₅ | CH₂COOC₂H₅ | CH₃ | NCH₃ |
| 3319 | C₂H₅ | Prop-1-en-3-yl | CH₃ | NCH₃ |
| 3320 | C₂H₅ | trans-But-2-en-1-yl | CH₃ | NCH₃ |
| 3321 | C₂H₅ | cis-But-2-en-1-yl | CH₃ | NCH₃ |
| 3322 | C₂H₅ | cis-3-Methyl-but-2-en-1-yl | CH₃ | NCH₃ |
| 3323 | C₂H₅ | Cyclopropyl | CH₃ | NCH₃ |
| 3324 | C₂H₅ | Cyclopentyl | CH₃ | NCH₃ |
| 3325 | C₂H₅ | Cyclohexyl | CH₃ | NCH₃ |
| 3326 | C₂H₅ | 4,5-Dihydroisoxazol-3-yl | CH₃ | NCH₃ |
| 3327 | C₂H₅ | 4-Methyl-4,5-dihydroisoxazol-3-yl | CH₃ | NCH₃ |
| 3328 | C₂H₅ | Isoxazol-3-yl | CH₃ | NCH₃ |
| 3329 | C₂H₅ | 4-Methylisoxazol-3-yl | CH₃ | NCH₃ |
| 3330 | C₂H₅ | 4,5-Dihydroisoxazol-4-yl | CH₃ | NCH₃ |
| 3331 | C₂H₅ | 3-Methyl-4,5-dihydroisoxazol-4-yl | CH₃ | NCH₃ |
| 3332 | C₂H₅ | Isoxazol-4-yl | CH₃ | NCH₃ |
| 3333 | C₂H₅ | 3-Methylisoxazol-4-yl | CH₃ | NCH₃ |
| 3334 | C₂H₅ | Phenyl | CH₃ | NCH₃ |
| 3335 | C₂H₅ | Benzyl | CH₃ | NCH₃ |
| 3336 | C₂H₅ | Benzoyl | CH₃ | NCH₃ |
| 3337 | C₂H₅ | 2-Pyridyl | CH₃ | NCH₃ |
| 3339 | C₂H₅ | CH₃ | Cl | NCH₃ |
| 3340 | C₂H₅ | C₂H₅ | Cl | NCH₃ |
| 3341 | C₂H₅ | n-C₃H₇ | Cl | NCH₃ |
| 3342 | C₂H₅ | i-C₃H₇ | Cl | NCH₃ |
| 3343 | C₂H₅ | n-C₄H₉ | Cl | NCH₃ |
| 3344 | C₂H₅ | i-C₄H₉ | Cl | NCH₃ |
| 3345 | C₂H₅ | s-C₄H₉ | Cl | NCH₃ |
| 3346 | C₂H₅ | t-C₄H₉ | Cl | NCH₃ |
| 3347 | C₂H₅ | CH₂OCH₃ | Cl | NCH₃ |
| 3348 | C₂H₅ | CF₃ | Cl | NCH₃ |
| 3349 | C₂H₅ | CF₂H | Cl | NCH₃ |
| 3350 | C₂H₅ | CN | Cl | NCH₃ |
| 3351 | C₂H₅ | OH | Cl | NCH₃ |
| 3352 | C₂H₅ | OCH₃ | Cl | NCH₃ |
| 3353 | C₂H₅ | NH₂ | Cl | NCH₃ |
| 3354 | C₂H₅ | NHCH₃ | Cl | NCH₃ |
| 3355 | C₂H₅ | N(CH₃)₂ | Cl | NCH₃ |
| 3356 | C₂H₅ | CO₂CH₃ | Cl | NCH₃ |
| 3357 | C₂H₅ | CO₂C₂H₅ | Cl | NCH₃ |
| 3358 | C₂H₅ | C(O)CH₃ | Cl | NCH₃ |
| 3359 | C₂H₅ | C(O)CF₃ | Cl | NCH₃ |
| 3360 | C₂H₅ | C(=NOCH₃)CH₃ | Cl | NCH₃ |
| 3361 | C₂H₅ | SO₂CH₃ | Cl | NCH₃ |
| 3362 | C₂H₅ | SO₂CF₃ | Cl | NCH₃ |
| 3363 | C₂H₅ | CH₂CO₂H | Cl | NCH₃ |
| 3364 | C₂H₅ | CH₂COOCH₃ | Cl | NCH₃ |
| 3365 | C₂H₅ | CH₂COOC₂H₅ | Cl | NCH₃ |
| 3366 | C₂H₅ | Prop-1-en-3-yl | Cl | NCH₃ |
| 3367 | C₂H₅ | trans-But-2-en-1-yl | Cl | NCH₃ |
| 3368 | C₂H₅ | cis-But-2-en-1-yl | Cl | NCH₃ |
| 3369 | C₂H₅ | cis-3-Methyl-but-2-en-l-yl | Cl | NCH₃ |
| 3370 | C₂H₅ | Cyclopropyl | Cl | NCH₃ |
| 3371 | C₂H₅ | Cyclopentyl | Cl | NCH₃ |
| 3372 | C₂H₅ | Cyclohexyl | Cl | NCH₃ |
| 3373 | C₂H₅ | 4,5-Dihydroisoxazol-3-yl | Cl | NCH₃ |
| 3374 | C₂H₅ | 4-Methyl-4,5-dihydroisoxazol-3-yl | Cl | NCH₃ |
| 3375 | C₂H₅ | Isoxazol-3-yl | Cl | NCH₃ |
| 3376 | C₂H₅ | 4-Methylisoxazol-3-yl | Cl | NCH₃ |
| 3377 | C₂H₅ | 4,5-Dihydroisoxazol-4-yl | Cl | NCH₃ |
| 3378 | C₂H₅ | 3-Methyl-4,5-dihydroisoxazol-4-yl | Cl | NCH₃ |
| 3379 | C₂H₅ | Isoxazol-4-yl | Cl | NCH₃ |
| 3380 | C₂H₅ | 3-Methylisoxazol-4-yl | Cl | NCH₃ |
| 3381 | C₂H₅ | Phenyl | Cl | NCH₃ |
| 3382 | C₂H₅ | Benzyl | Cl | NCH₃ |
| 3383 | C₂H₅ | Benzoyl | Cl | NCH₃ |
| 3384 | C₂H₅ | 2-Pyridyl | Cl | NCH₃ |
| 3385 | CH₃ | n-Pentyl | H | O |
| 3386 | CH₃ | CH₂-C≡C-CH₂-CH₃ | H | O |
| 3387 | CH₃ | CH₂-CH₂-CH=CH₂ | H | O |
| 3388 | CH₃ | CH₂-CH₂-CH₂-CH=CH₂ | H | O |
| 3389 | CH₃ | CH₂-[(3-OCH₃)C₆H₄] | H | O |
| 3390 | CH₃ | CH₂-C≡C-CH₃ | H | O |
| 3391 | CH₃ | CH₂-CH₂-(1,3-dioxolan-2-yl) | H | O |
| 3392 | CH₃ | CH₂-(1,3-dioxolan-2-yl) | H | O |
| 3393 | CH₃ | n-Pentyl | CH₃ | O |
| 3394 | CH₃ | CH₂-C≡C-CH₂-CH₃ | CH₃ | O |
| 3395 | CH₃ | CH₂-CH₂-CH=CH₂ | CH₃ | O |
| 3396 | CH₃ | CH₂-CH₂-CH₂-CH=CH₂ | CH₃ | O |
| 3397 | CH₃ | CH₂-[(3-OCH₃)C₆H₄] | CH₃ | O |
| 3398 | CH₃ | CH₂-C≡C-CH₃ | CH₃ | O |
| 3399 | CH₃ | CH₂-CH₂-(1,3-dioxolan-2-yl) | CH₃ | O |
| 3400 | CH₃ | CH₂-(1,3-dioxolan-2-yl) | CH₃ | O |
| 3401 | CH₃ | n-Pentyl | Cl | O |
| 3402 | CH₃ | CH₂-C≡C-CH₂-CH₃ | Cl | O |
| 3403 | CH₃ | CH₂-CH₂-CH=CH₂ | Cl | O |
| 3404 | CH₃ | CH₂-CH₂-CH₂-CH=CH₂ | Cl | O |
| 3405 | CH₃ | CH₂-[(3-OCH₃)C₆H₄] | Cl | O |
| 3406 | CH₃ | CH₂-C≡C-CH₃ | Cl | O |
| 3407 | CH₃ | CH₂-CH₂-(1,3-dioxolan-2-yl) | Cl | O |
| 3408 | CH₃ | CH₂-(1,3-dioxolan-2-yl) | Cl | O |
| 3409 | Cl | n-Pentyl | H | O |
| 3410 | Cl | CH₂-C≡C-CH₂-CH₃ | H | O |
| 3411 | Cl | CH₂-CH₂-CH=CH₂ | H | O |
| 3412 | Cl | CH₂-CH₂-CH₂-CH=CH₂ | H | O |
| 3413 | Cl | CH₂-[(3-OCH₃)C₆H₄] | H | O |
| 3414 | Cl | CH₂-C≡C-CH₃ | H | O |
| 3415 | Cl | CH₂-CH₂-(1,3-dioxolan-2-yl) | H | O |
| 3416 | Cl | CH₂-(1,3-dioxolan-2-yl) | H | O |
| 3417 | Cl | n-Pentyl | CH₃ | O |
| 3418 | Cl | CH₂-C≡C-CH₂-CH₃ | CH₃ | O |
| 3419 | Cl | CH₂-CH₂-CH=CH₂ | CH₃ | O |
| 3420 | Cl | CH₂-CH₂-CH₂-CH=CH₂ | CH₃ | O |
| 3421 | Cl | CH₂-[(3-OCH₃)C₆H₄] | CH₃ | O |
| 3422 | Cl | CH₂-C≡C-CH₃ | CH₃ | O |
| 3423 | Cl | CH₂-CH₂-(1,3-dioxolan-2-yl) | CH₃ | O |
| 3424 | Cl | CH₂-(1,3-dioxolan-2-yl) | CH₃ | O |
| 3425 | Cl | n-Pentyl | Cl | O |
| 3426 | Cl | CH₂-C≡C-CH₂-CH₃ | Cl | O |
| 3427 | Cl | CH₂-CH₂-CH=CH₂ | Cl | O |
| 3428 | Cl | CH₂-CH₂-CH₂-CH=CH₂ | Cl | O |
| 3429 | Cl | CH₂-[(3-OCH₃)C₆H₄) | Cl | O |
| 3430 | Cl | CH₂-C≡C-CH₃ | Cl | O |
| 3431 | Cl | CH₂-CH₂-(1,3-dioxolan-2-yl) | Cl | O |
| 3432 | Cl | CH₂-(1,3-dioxolan-2-yl) | Cl | O |
| 3433 | OCH₃ | n-Pentyl | H | O |
| 3434 | OCH₃ | CH₂-C≡C-CH₂-CH₃ | H | O |
| 3435 | OCH₃ | CH₂-CH₂-CH=CH₂ | H | O |
| 3436 | OCH₃ | CH₂-CH₂-CH₂-CH=CH₂ | H | O |
| 3437 | OCH₃ | CH₂-[(3-OCH₃)C₆H₄] | H | O |
| 3438 | OCH₃ | CH₂-C≡C-CH₃ | H | O |
| 3439 | OCH₃ | CH₂-CH₂-(1,3-dioxolan-2-yl) | H | O |
| 3440 | OCH₃ | CH₂-(1,3-dioxolan-2-yl) | H | O |
| 3441 | OCH₃ | n-Pentyl | CH₃ | O |
| 3442 | OCH₃ | CH₂-C≡C-CH₂-CH₃ | CH₃ | O |
| 3443 | OCH₃ | CH₂-CH₂-CH=CH₂ | CH₃ | O |
| 3444 | OCH₃ | CH₂-CH₂-CH₂-CH=CH₂ | CH₃ | O |
| 3445 | OCH₃ | CH₂-[(3-OCH₃)C₆H₄] | CH₃ | O |
| 3446 | OCH₃ | CH₂-C≡C-CH₃ | CH₃ | O |
| 3447 | OCH₃ | CH₂-CH₂-(1,3-dioxolan-2-yl) | CH₃ | O |
| 3448 | OCH₃ | CH₂-(1,3-dioxolan-2-yl) | CH₃ | O |
| 3449 | OCH₃ | n-Pentyl | Cl | O |
| 3450 | OCH₃ | CH₂-C≡C-CH₂-CH₃ | Cl | O |
| 3451 | OCH₃ | CH₂-CH₂-CH=CH₂ | Cl | O |
| 3452 | OCH₃ | CH₂-CH₂-CH₂-CH=CH₂ | Cl | O |
| 3453 | OCH₃ | CH₂-[(3-OCH₃)C₆H₄] | Cl | O |
| 3954 | OCH₃ | CH₂-C≡C-CH₃ | Cl | O |
| 3455 | OCH₃ | CH₂-CH₂-(1,3-dioxolan-2-yl) | Cl | O |
| 3456 | OCH₃ | CH₂-(1,3-dioxolan-2-yl) | Cl | O |
| 3457 | OCF₃ | n-Pentyl | H | O |
| 3458 | OCF₃ | CH₂-C≡C-CH₂-CH₃ | H | O |
| 3459 | OCF₃ | CH₂-CH₂-CH=CH₂ | H | O |
| 3460 | OCF₃ | CH₂-CH₂-CH₂-CH=CH₂ | H | O |
| 3461 | OCF₃ | CH₂-[(3-OCH₃)C₆H₄] | H | O |
| 3462 | OCF₃ | CH₂-C≡C-CH₃ | H | O |
| 3463 | OCF₃ | CH₂-CH₂-(1,3-dioxolan-2-yl) | H | O |
| 3464 | OCF₃ | CH₂-(1,3-dioxolan-2-yl) | H | O |
| 3465 | OCF₃ | n-Pentyl | CH₃ | O |
| 3466 | OCF₃ | CH₂-C≡C-CH₂-CH₃ | CH₃ | O |
| 3467 | OCF₃ | CH₂-CH₂-CH=CH₂ | CH₃ | O |
| 3468 | OCF₃ | CH₂-CH₂-CH₂-CH=CH₂ | CH₃ | O |
| 3469 | OCF₃ | CH₂-[(3-OCH₃)C₆H₄] | CH₃ | O |
| 3470 | OCF₃ | CH₂-C≡C-CH₃ | CH₃ | O |
| 3471 | OCF₃ | CH₂-CH₂-(1,3-dioxolan-2-yl) | CH₃ | O |
| 3472 | OCF₃ | CH₂-(1,3-dioxolan-2-yl) | CH₃ | O |
| 3473 | OCF₃ | n-Pentyl | Cl | O |
| 3474 | OCF₃ | CH₂-C≡C-CH₂-CH₃ | Cl | O |
| 3475 | OCF₃ | CH₂-CH₂-CH=CH₂ | Cl | O |
| 3476 | OCF₃ | CH₂-CH₂-CH₂-CH=CH₂ | Cl | O |
| 3477 | OCF₃ | CH₂-[(3-OCH₃) C₆H₄] | Cl | O |
| 3478 | OCF₃ | CH₂-C≡C-CH₃ | Cl | O |
| 3479 | OCF₃ | CH₂-CH₂-(1,3-dioxolan-2-yl) | Cl | O |
| 3480 | OCF₃ | CH₂-(1,3-dioxolan-2-yl) | Cl | O |
| 3481 | SCH₃ | n-Pentyl | H | O |
| 3482 | SCH₃ | CH₂-C≡C-CH₂-CH₃ | H | O |
| 3483 | SCH₃ | CH₂-CH₂-CH=CH₂ | H | O |
| 3484 | SCH₃ | CH₂-CH₂-CH₂-CH=CH₂ | H | O |
| 3485 | SCH₃ | CH₂-[(3-OCH₃)C₆H₄] | H | O |
| 3486 | SCH₃ | CH₂-C≡C-CH₃ | H | O |
| 3487 | SCH₃ | CH₂-CH₂-(1,3-dioxolan-2-yl) | H | O |
| 3488 | SCH₃ | CH₂-(1,3-dioxolan-2-yl) | H | O |
| 3489 | SCH₃ | n-Pentyl | CH₃ | O |
| 3490 | SCH₃ | CH₂-C≡C-CH₂-CH₃ | CH₃ | O |
| 3491 | SCH₃ | CH₂-CH₂-CH=CH₂ | CH₃ | O |
| 3492 | SCH₃ | CH₂-CH₂-CH₂-CH=CH₂ | CH₃ | O |
| 3493 | SCH₃ | CH₂-[(3-OCH₃)C₆H₄] | CH₃ | O |
| 3494 | SCH₃ | CH₂-C≡C-CH₃ | CH₃ | O |
| 3495 | SCH₃ | CH₂-CH₂-(1,3-dioxolan-2-yl) | CH₃ | O |
| 3496 | SCH₃ | CH₂-(1,3-dioxolan-2-yl) | CH₃ | O |
| 3497 | SCH₃ | n-Pentyl | Cl | O |
| 3498 | SCH₃ | CH₂-C≡C-CH₂-CH₃ | Cl | O |
| 3499 | SCH₃ | CH₂-CH₂-CH=CH₂ | Cl | O |
| 3500 | SCH₃ | CH₂-CH₂-CH₂-CH=CH₂ | Cl | O |
| 3501 | SCH₃ | CH₂-[(3-OCH₃)C₆H₄] | Cl | O |
| 3502 | SCH₃ | CH₂-C≡C-CH₃ | Cl | O |
| 3503 | SCH₃ | CH₂-CH₂-(1,3-dioxolan-2-yl) | Cl | O |
| 3504 | SCH₃ | CH₂-(1,3-dioxolan-2-yl) | Cl | O |
| 3505 | SO₂CH₃ | n-Pentyl | H | O |
| 3506 | SO₂CH₃ | CH₂-C≡C-CH₂-CH₃ | H | O |
| 3507 | SO₂CH₃ | CH₂-CH₂-CH=CH₂ | H | O |
| 3508 | SO₂CH₃ | CH₂-CH₂-CH₂-CH=CH₂ | H | O |
| 3509 | SO₂CH₃ | CH₂-[(3-OCH₃)C₆H₄] | H | O |
| 3510 | SO₂CH₃ | CH₂-C≡C-CH₃ | H | O |
| 3511 | SO₂CH₃ | CH₂-CH₂-(1,3-dioxolan-2-yl) | H | O |
| 3512 | SO₂CH₃ | CH₂-(1,3-dioxolan-2-yl) | H | O |
| 3513 | SO₂CH₃ | n-Pentyl | CH₃ | O |
| 3514 | SO₂CH₃ | CH₂-C≡C-CH₂-CH₃ | CH₃ | O |
| 3515 | SO₂CH₃ | CH₂-CH₂-CH=CH₂ | CH₃ | O |
| 3516 | SO₂CH₃ | CH₂-CH₂-CH₂-CH=CH₂ | CH₃ | O |
| 3517 | SO₂CH₃ | CH₂-[(3-OCH₃)C₆H₄] | CH₃ | O |
| 3518 | SO₂CH₃ | CH₂-C≡C-CH₃ | CH₃ | O |
| 3519 | SO₂CH₃ | CH₂-CH₂-(1,3-dioxolan-2-yl) | CH₃ | O |
| 3520 | SO₂CH₃ | CH₂-(1,3-dioxolan-2-yl) | CH₃ | O |
| 3521 | SO₂CH₃ | n-Pentyl | Cl | O |
| 3522 | SO₂CH₃ | CH₂-C≡C-CH₂-CH₃ | Cl | O |
| 3523 | SO₂CH₃ | CH₂-CH₂-CH=CH₂ | Cl | O |
| 3524 | SO₂CH₃ | CH₂-CH₂-CH₂-CH=CH₂ | Cl | O |
| 3525 | SO₂CH₃ | CH₂-[(3-OCH₃)C₆H₄] | Cl | O |
| 3526 | SO₂CH₃ | CH₂-C≡C-CH₃ | Cl | O |
| 3527 | SO₂CH₃ | CH₂-CH₂-(1,3-dioxolan-2-yl) | Cl | O |
| 3528 | SO₂CH₃ | CH₂-(1,3-dioxolan-2-yl) | Cl | O |
| 3529 | CF₃ | n-Pentyl | H | O |
| 3530 | CF₃ | CH₂-C≡C-CH₂-CH₃ | H | O |
| 3531 | CF₃ | CH₂-CH₂-CH=CH₂ | H | O |
| 3532 | CF₃ | CH₂-CH₂-CH₂-CH=CH₂ | H | O |
| 3533 | CF₃ | CH₂-[(3-OCH₃)C₆H₄] | H | O |
| 3534 | CF₃ | CH₂-C≡C-CH₃ | H | O |
| 3535 | CF₃ | CH₂-CH₂-(1,3-dioxolan-2-yl) | H | O |
| 3536 | CF₃ | CH₂-(1,3-dioxolan-2-yl) | H | O |
| 3537 | CF₃ | n-Pentyl | CH₃ | O |
| 3538 | CF₃ | CH₂-C≡C-CH₂-CH₃ | CH₃ | O |
| 3539 | CF₃ | CH₂-CH₂-CH=CH₂ | CH₃ | O |
| 3540 | CF₃ | CH₂-CH₂-CH₂-CH=CH₂ | CH₃ | O |
| 3541 | CF₃ | CH₂-[(3-OCH₃)C₆H₄] | CH₃ | O |
| 3542 | CF₃ | CH₂-C≡C-CH₃ | CH₃ | O |
| 3543 | CF₃ | CH₂-CH₂-(1,3-dioxolan-2-yl) | CH₃ | O |
| 3544 | CF₃ | CH₂-(1,3-dioxolan-2-yl) | CH₃ | O |
| 3545 | CF₃ | n-Pentyl | Cl | O |
| 3546 | CF₃ | CH₂-C≡C-CH₂-CH₃ | Cl | O |
| 3547 | CF₃ | CH₂-CH₂-CH=CH₂ | Cl | O |
| 3548 | CF₃ | CH₂-CH₂-CH₂-CH=CH₂ | Cl | O |
| 3549 | CF₃ | CH₂-[(3-OCH₃)C₆H₄] | Cl | O |
| 3550 | CF₃ | CH₂-C≡C-CH₃ | Cl | O |
| 3551 | CF₃ | CH₂-CH₂-(1,3-dioxolan-2-yl) | Cl | O |
| 3552 | CF₃ | CH₂-(1,3-dioxolan-2-yl) | Cl | O |
| 3553 | C₂H₅ | n-Pentyl | H | O |
| 3554 | C₂H₅ | CH₂-C≡C-CH₂-CH₃ | H | O |
| 3555 | C₂H₅ | CH₂-CH₂-CH=CH₂ | H | O |
| 3556 | C₂H₅ | CH₂-CH₂-CH₂-CH=CH₂ | H | O |
| 3557 | C₂H₅ | CH₂-[(3-OCH₃)C₆H₄] | H | O |
| 3558 | C₂H₅ | CH2-C≡C-CH₃ | H | O |
| 3559 | C₂H₅ | CH₂-CH₂-(1,3-dioxolan-2-yl) | H | O |
| 3560 | C₂H₅ | CH₂-(1,3-dioxolan-2-yl) | H | O |
| 3561 | C₂H₅ | n-Pentyl | CH₃ | O |
| 3562 | C₂H₅ | CH₂-C≡C-CH₂-CH₃ | CH₃ | O |
| 3563 | C₂H₅ | CH₂-CH₂-CH=CH₂ | CH₃ | O |
| 3564 | C₂H₅ | CH₂-CH₂-CH₂-CH=CH₂ | CH₃ | O |
| 3565 | C₂H₅ | CH₂-[(3-OCH₃)C₆H₄] | CH₃ | O |
| 3566 | C₂H₅ | CH₂-C≡C-CH₃ | CH₃ | O |
| 3567 | C₂H₅ | CH₂-CH₂-(1,3-dioxolan-2-yl) | CH₃ | O |
| 3568 | C₂H₅ | CH₂-(1,3-dioxolan-2-yl) | CH₃ | O |
| 3569 | C₂H₅ | n-Pentyl | Cl | O |
| 3570 | C₂H₅ | CH₂-C≡C-CH₂-CH₃ | Cl | O |
| 3571 | C₂H₅ | CH₂-CH₂-CH=CH₂ | Cl | O |
| 3572 | C₂H₅ | CH₂-CH₂-CH₂-CH=CH₂ | Cl | O |
| 3573 | C₂H₅ | CH₂-[(3-OCH₃)C₆H₄] | Cl | O |
| 3574 | C₂H₅ | CH₂-C≡C-CH₃ | Cl | O |
| 3575 | C₂H₅ | CH₂-CH₂-(1,3-dioxolan-2-yl) | Cl | O |
| 3576 | C₂H₅ | CH₂-(1,3-dioxolan-2-yl) | Cl | O |
| 3577 | CH₃ | n-Pentyl | H | S |
| 3578 | CH₃ | CH₂-C≡C-CH₂-CH₃ | H | S |
| 3579 | CH₃ | CH₂-CH₂-CH=CH₂ | H | S |
| 3580 | CH₃ | CH₂-CH₂-CH₂-CH=CH₂ | H | S |
| 3581 | CH₃ | CH₂-[(3-OCH₃)C₆H₄] | H | S |
| 3582 | CH₃ | CH₂-C≡C-CH₃ | H | S |
| 3583 | CH₃ | CH₂-CH₂-(1,3-dioxolan-2-yl) | H | S |
| 3584 | CH₃ | CH₂-(1,3-dioxolan-2-yl) | H | S |
| 3585 | CH₃ | n-Pentyl | CH₃ | S |
| 3586 | CH₃ | CH₂-C≡C-CH₂-CH₃ | CH₃ | S |
| 3587 | CH₃ | CH₂-CH₂-CH=CH₂ | CH₃ | S |
| 3588 | CH₃ | CH₂-CH₂-CH₂-CH=CH₂ | CH₃ | S |
| 3589 | CH₃ | CH₂-[(3-OCH₃)C₆H₄] | CH₃ | S |
| 3590 | CH₃ | CH₂-C≡C-CH₃ | CH₃ | S |
| 3591 | CH₃ | CH₂-CH₂-(1,3-dioxolan-2-yl) | CH₃ | S |
| 3592 | CH₃ | CH₂-(1,3-dioxolan-2-yl) | CH₃ | S |
| 3593 | CH₃ | n-Pentyl | Cl | S |
| 3594 | CH₃ | CH₂-C≡C-CH₂-CH₃ | Cl | S |
| 3595 | CH₃ | CH₂-CH₂-CH=CH₂ | Cl | S |
| 3596 | CH₃ | CH₂-CH₂-CH₂-CH=CH₂ | Cl | S |
| 3597 | CH₃ | CH₂-[(3-OCH₃)C₆H₄] | Cl | S |
| 3598 | CH₃ | CH₂-C≡C-CH₃ | Cl | S |
| 3599 | CH₃ | CH₂-CH₂-(1,3-dioxolan-2-yl) | Cl | S |
| 3600 | CH₃ | CH₂-(1,3-dioxolan-2-yl) | Cl | S |
| 3601 | Cl | n-Pentyl | H | S |
| 3602 | Cl | CH₂-C≡C-CH₂-CH₃ | H | S |
| 3603 | Cl | CH₂-CH₂-CH=CH₂ | H | S |
| 3604 | Cl | CH₂-CH₂-CH₂-CH=CH₂ | H | S |
| 3605 | Cl | CH₂-[(3-OCH₃)C₆H₄] | H | S |
| 3606 | Cl | CH₂-C≡C-CH₃ | H | S |
| 3607 | Cl | CH₂-CH₂-(1,3-dioxolan-2-yl) | H | S |
| 3608 | Cl | CH₂-(1,3-dioxolan-2-yl) | H | S |
| 3609 | Cl | n-Pentyl | CH₃ | S |
| 3610 | Cl | CH₂-C≡C-CH₂-CH₃ | CH₃ | S |
| 3611 | Cl | CH₂-CH₂-CH=CH₂ | CH₃ | S |
| 3612 | Cl | CH₂-CH₂-CH₂-CH=CH₂ | CH₃ | S |
| 3613 | Cl | CH₂-[(3-OCH₃)C₆H₄] | CH₃ | S |
| 3614 | Cl | CH₂-C≡C-CH₃ | CH₃ | S |
| 3615 | Cl | CH₂-CH₂-(1,3-dioxolan-2-yl) | CH₃ | S |
| 3616 | Cl | CH₂-(1,3-dioxolan-2-yl) | CH₃ | S |
| 3617 | Cl | n-Pentyl | Cl | S |
| 3618 | Cl | CH₂-C≡C-CH₂-CH₃ | Cl | S |
| 3619 | Cl | CH₂-CH₂-CH=CH₂ | Cl | S |
| 3620 | Cl | CH₂-CH₂-CH₂-CH=CH₂ | Cl | S |
| 3621 | Cl | CH₂-[(3-OCH₃)C₆H₄] | Cl | S |
| 3622 | Cl | CH₂-C≡C-CH₃ | Cl | S |
| 3623 | Cl | CH₂-CH₂-(1,3-dioxolan-2-yl) | Cl | S |
| 3624 | Cl | CH₂-(1,3-dioxolan-2-yl) | Cl | S |
| 3625 | OCH₃ | n-Pentyl | H | S |
| 3626 | OCH₃ | CH₂-C≡C-CH₂-CH₃ | H | S |
| 3627 | OCH₃ | CH₂-CH₂-CH=CH₂ | H | S |
| 3628 | OCH₃ | CH₂-CH₂-CH₂-CH=CH₂ | H | S |
| 3629 | OCH₃ | CH₂-[(3-OCH₃)C₆H₄] | H | S |
| 3630 | OCH₃ | CH₂-C≡C-CH₃ | H | S |
| 3631 | OCH₃ | CH₂-CH₂-(1,3-dioxolan-2-yl) | H | S |
| 3632 | OCH₃ | CH₂-(1,3-dioxolan-2-yl) | H | S |
| 3633 | OCH₃ | n-Pentyl | CH₃ | S |
| 3634 | OCH₃ | CH₂-C≡C-CH₂-CH₃ | CH₃ | S |
| 3635 | OCH₃ | CH₂-CH₂-CH=CH₂ | CH₃ | S |
| 3636 | OCH₃ | CH₂-CH₂-CH₂-CH=CH₂ | CH₃ | S |
| 3637 | OCH₃ | CH₂-[(3-OCH₃)C₆H₄] | CH₃ | S |
| 3638 | OCH₃ | CH₂-C≡C-CH₃ | CH₃ | S |
| 3639 | OCH₃ | CH₂-CH₂-(1,3-dioxolan-2-yl) | CH₃ | S |
| 3640 | OCH₃ | CH₂-(1,3-dioxolan-2-yl) | CH₃ | S |
| 3641 | OCH₃ | n-Pentyl | Cl | S |
| 3642 | OCH₃ | CH₂-C≡C-CH₂-CH₃ | Cl | S |
| 3643 | OCH₃ | CH₂-CH₂-CH=CH₂ | Cl | S |
| 3644 | OCH₃ | CH₂-CH₂-CH₂-CH=CH₂ | Cl | S |
| 3645 | OCH₃ | CH₂-[(3-OCH₃)C₆H₄] | Cl | S |
| 3646 | OCH₃ | CH₂-C≡C-CH₃ | Cl | S |
| 3647 | OCH₃ | CH₂-CH₂-(1,3-dioxolan-2-yl) | Cl | S |
| 3648 | OCH₃ | CH₂-(1,3-dioxolan-2-yl) | Cl | S |
| 3649 | OCF₃ | n-Pentyl | H | S |
| 3650 | OCF₃ | CH₂-C≡C-CH₂-CH₃ | H | S |
| 3651 | OCF₃ | CH₂-CH₂-CH=CH₂ | H | S |
| 3652 | OCF₃ | CH₂-CH₂-CH₂-CH=CH₂ | H | S |
| 3653 | OCF₃ | CH₂-[(3-OCH₃)C₆H₄] | H | S |
| 3654 | OCF₃ | CH₂-C≡C-CH₃ | H | S |
| 3655 | OCF₃ | CH₂-CH₂-(1,3-dioxolan-2-yl) | H | S |
| 3656 | OCF₃ | CH₂-(1,3-dioxolan-2-yl), | H | S |
| 3657 | OCF₃ | n-Pentyl | CH₃ | S |
| 3658 | OCF₃ | CH₂-C≡C-CH₂-CH₃ | CH₃ | S |
| 3659 | OCF₃ | CH₂-CH₂-CH=CH₂ | CH₃ | S |
| 3660 | OCF₃ | CH₂-CH₂-CH₂-CH=CH₂ | CH₃ | S |
| 3661 | OCF₃ | CH₂-[(3-OCH₃)C₆H₄] | CH₃ | S |
| 3662 | OCF₃ | CH₂-C≡C-CH₃ | CH₃ | S |
| 3663 | OCF₃ | CH₂-CH₂-(1,3-dioxolan-2-yl) | CH₃ | S |
| 3664 | OCF₃ | CH₂-(1,3-dioxolan-2-yl) | CH₃ | S |
| 3665 | OCF₃ | n-Pentyl | Cl | S |
| 3666 | OCF₃ | CH₂-C≡C-CH₂-CH₃ | Cl | S |
| 3667 | OCF₃ | CH₂-CH₂-CH=CH₂ | Cl | S |
| 3668 | OCF₃ | CH₂-CH₂-CH₂-CH=CH₂ | Cl | S |
| 3669 | OCF₃ | CH₂-[(3-OCH₃)C₆H₄] | Cl | S |
| 3670 | OCF₃ | CH₂-C≡C-CH₃ | Cl | S |
| 3671 | OCF₃ | CH₂-CH₂-(1,3-dioxolan-2-yl) | Cl | S |
| 3672 | OCF₃ | CH₂-(1,3-dioxolan-2-yl) | Cl | S |
| 3673 | SCH₃ | n-Pentyl | H | S |
| 3674 | SCH₃ | CH₂-C≡C-CH₂-CH₃ | H | S |
| 3675 | SCH₃ | CH₂-CH₂-CH=CH₂ | H | S |
| 3676 | SCH₃ | CH₂-CH₂-CH₂-CH=CH₂ | H | S |
| 3677 | SCH₃ | CH₂-[(3-OCH₃)C₆H₄] | H | S |
| 3678 | SCH₃ | CH₂-C≡C-CH₃ | H | S |
| 3679 | SCH₃ | CH₂-CH₂-(1,3-dioxolan-2-yl) | H | S |
| 3680 | SCH₃ | CH₂-(1,3-dioxolan-2-yl) | H | S |
| 3681 | SCH₃ | n-Pentyl | CH₃ | S |
| 3682 | SCH₃ | CH₂-C≡C-CH₂-CH₃ | CH₃ | S |
| 3683 | SCH₃ | CH₂-CH₂-CH=CH₂ | CH₃ | S |
| 3684 | SCH₃ | CH₂-CH₂-CH₂-CH=CH₂ | CH₃ | S |
| 3685 | SCH₃ | CH₂-[(3-OCH₃)C₆H₄] | CH₃ | S |
| 3686 | SCH₃ | CH₂-C≡C-CH₃ | CH₃ | S |
| 3687 | SCH₃ | CH₂-CH₂-(1,3-dioxolan-2-yl) | CH₃ | S |
| 3688 | SCH₃ | CH₂-(1,3-dioxolan-2-yl) | CH₃ | S |
| 3689 | SCH₃ | n-Pentyl | Cl | S |
| 3690 | SCH₃ | CH₂-C≡C-CH₂-CH₃ | Cl | S |
| 3691 | SCH₃ | CH₂-CH₂-CH=CH₂ | Cl | S |
| 3692 | SCH₃ | CH₂-CH₂-CH₂-CH=CH₂ | Cl | S |
| 3693 | SCH₃ | CH₂-[(3-OCH₃)C₆H₄] | Cl | S |
| 3694 | SCH₃ | CH₂-C≡C-CH₃ | Cl | S |
| 3695 | SCH₃ | CH₂-CH₂-(1,3-dioxolan-2-yl) | Cl | S |
| 3696 | SCH₃ | CH₂-(1,3-dioxolan-2-yl) | Cl | S |
| 3697 | SO₂CH₃ | n-Pentyl | H | S |
| 3698 | SO₂CH₃ | CH₂-C≡C-CH₂-CH₃ | H | S |
| 3699 | SO₂CH₃ | CH₂-CH₂-CH=CH₂ | H | S |
| 3700 | SO₂CH₃ | CH₂-CH₂-CH₂-CH=CH₂ | H | S |
| 3701 | SO₂CH₃ | CH₂-[(3-OCH₃)C₆H₄] | H | S |
| 3702 | SO₂CH₃ | CH₂-C≡C-CH₃ | H | S |
| 3703 | SO₂CH₃ | CH₂-CH₂-(1,3-dioxolan-2-yl) | H | S |
| 3704 | SO₂CH₃ | CH₂-(1,3-dioxolan-2-yl) | H | S |
| 3705 | SO₂CH₃ | n-Pentyl | CH₃ | S |
| 3706 | SO₂CH₃ | CH₂-C≡C-CH₂-CH₃ | CH₃ | S |
| 3707 | SO₂CH₃ | CH₂-CH₂-CH=CH₂ | CH₃ | S |
| 3708 | SO₂CH₃ | CH₂-CH₂-CH₂-CH=CH₂ | CH₃ | S |
| 3709 | SO₂CH₃ | CH₂-[(3-OCH₃)C₆H₄] | CH₃ | S |
| 3710 | SO₂CH₃ | CH₂-C≡C-CH₃ | CH₃ | S |
| 3711 | SO₂CH₃ | CH₂-CH₂-(1,3-dioxolan-2-yl) | CH₃ | S |
| 3712 | SO₂CH₃ | CH₂-(1,3-dioxolan-2-yl) | CH₃ | S |
| 3713 | SO₂CH₃ | n-Pentyl | Cl | S |
| 3714 | SO₂CH₃ | CH₂-C≡C-CH₂-CH₃ | Cl | S |
| 3715 | SO₂CH₃ | CH₂-CH₂-CH=CH₂ | Cl | S |
| 3716 | SO₂CH₃ | CH₂-CH₂-CH₂-CH=CH₂ | Cl | S |
| 3717 | SO₂CH₃ | CH₂-[(3-OCH₃)C₆H₄] | Cl | S |
| 3718 | SO₂CH₃ | CH₂-C≡C-CH₃ | Cl | S |
| 3719 | SO₂CH₃ | CH₂-CH₂-(1,3-dioxolan-2-yl) | Cl | S |
| 3720 | SO₂CH₃ | CH₂-(1,3-dioxolan-2-yl) | Cl | S |
| 3721 | CF₃ | n-Pentyl | H | S |
| 3722 | CF₃ | CH₂-C≡C-CH₂-CH₃ | H | S |
| 3723 | CF₃ | CH₂-CH₂-CH=CH₂ | H | S |
| 3724 | CF₃ | CH₂-CH₂-CH₂-CH=CH₂ | H | S |
| 3725 | CF₃ | CH₂-[(3-OCH₃)C₆H₄] | H | S |
| 3726 | CF₃ | CH₂-C≡C-CH₃ | H | S |
| 3727 | CF₃ | CH₂-CH₂-(1,3-dioxolan-2-yl) | H | S |
| 3728 | CF₃ | CH₂-(1,3-dioxolan-2-yl) | H | S |
| 3729 | CF₃ | n-Pentyl | CH₃ | S |
| 3730 | CF₃ | CH₂-C≡C-CH₂-CH₃ | CH₃ | S |
| 3731 | CF₃ | CH₂-CH₂-CH=CH₂ | CH₃ | S |
| 3732 | CF₃ | CH₂-CH₂-CH₂-CH=CH₂ | CH₃ | S |
| 3733 | CF₃ | CH₂-[(3-OCH₃)C₆H₄] | CH₃ | S |
| 3734 | CF₃ | CH₂-C≡C-CH₃ | CH₃ | S |
| 3735 | CF₃ | CH₂-CH₂-(1,3-dioxolan-2-yl) | CH₃ | S |
| 3736 | CF₃ | CH₂-(1,3-dioxolan-2-yl) | CH₃ | S |
| 3737 | CF₃ | n-Pentyl | Cl | S |
| 3738 | CF₃ | CH₂-C≡C-CH₂-CH₃ | Cl | S |
| 3739 | CF₃ | CH₂-CH₂-CH=CH₂ | Cl | S |
| 3740 | CF₃ | CH₂-CH₂-CH₂-CH=CH₂ | Cl | S |
| 3741 | CF₃ | CH₂-[(3-OCH₃)C₆H₄] | Cl | S |
| 3742 | CF₃ | CH₂-C≡C-CH₃ | Cl | S |
| 3743 | CF₃ | CH₂-CH₂-(1,3-dioxolan-2-yl) | Cl | S |
| 3744 | CF₃ | CH₂-(1,3-dioxolan-2-yl) | Cl | S |
| 3745 | C₂H₅ | n-Pentyl | H | S |
| 3746 | C₂H₅ | CH₂-C≡C-CH₂-CH₃ | H | S |
| 3747 | C₂H₅ | CH₂-CH₂-CH=CH₂ | H | S |
| 3748 | C₂H₅ | CH₂-CH₂-CH₂-CH=CH₂ | H | S |
| 3749 | C₂H₅ | CH₂-[(3-OCH₃)C₆H₄] | H | S |
| 3750 | C₂H₅ | CH₂-C≡C-CH₃ | H | S |
| 3751 | C₂H₅ | CH₂-CH₂-(1,3-dioxolan-2-yl) | H | S |
| 3752 | C₂H₅ | CH₂-(1,3-dioxolan-2-yl) | H | S |
| 3753 | C₂H₅ | n-Pentyl | CH₃ | S |
| 3754 | C₂H₅ | CH₂-C≡C-CH₂-CH₃ | CH₃ | S |
| 3755 | C₂H₅ | CH₂-CH₂-CH=CH₂ | CH₃ | S |
| 3756 | C₂H₅ | CH₂-CH₂-CH₂-CH=CH₂ | CH₃ | S |
| 3757 | C₂H₅ | CH₂-[(3-OCH₃)C₆H₄] | CH₃ | S |
| 3758 | C₂H₅ | CH₂-C≡C-CH₃ | CH₃ | S |
| 3759 | C₂H₅ | CH₂-CH₂-(1,3-dioxolan-2-yl) | CH₃ | s |
| 3760 | C₂H₅ | CH₂-(1,3-dioxolan-2-yl) | CH₃ | S |
| 3761 | C₂H₅ | n-Pentyl | Cl | S |
| 3762 | C₂H₅ | CH₂-C≡C-CH₂-CH₃ | Cl | S |
| 3763 | C₂H₅ | CH₂-CH₂-CH=CH₂ | Cl | S |
| 3764 | C₂H₅ | CH₂-CH₂-CH₂-CH=CH₂ | Cl | S |
| 3765 | C₂H₅ | CH₂-[(3-OCH₃)C₆H₄] | Cl | S |
| 3766 | C₂H₅ | CH₂-C≡C-CH₃ | Cl | S |
| 3767 | C₂H₅ | CH₂-CH₂-(1,3-dioxolan-2-yl) | Cl | S |
| 3768 | C₂H₅ | CH₂-(1,3-dioxolan-2-yl) | Cl | S |
| 3769 | CH₃ | n-Pentyl | H | NCH₃ |
| 3770 | CH₃ | CH₂-C≡C-CH₂-CH₃ | H | NCH₃ |
| 3771 | CH₃ | CH₂-CH₂-CH=CH₂ | H | NCH₃ |
| 3772 | CH₃ | CH₂-CH₂-CH₂-CH=CH₂ | H | NCH₃ |
| 3773 | CH₃ | CH₂-[(3-OCH₃)C₆H₄] | H | NCH₃ |
| 3774 | CH₃ | CH₂-C≡C-CH₃ | H | NCH₃ |
| 3775 | CH₃ | CH₂-CH₂-(1,3-dioxolan-2-yl) | H | NCH₃ |
| 3776 | CH₃ | CH₂-(1,3-dioxolan-2-yl) | H | NCH₃ |
| 3777 | CH₃ | n-Pentyl | CH₃ | NCH₃ |
| 3778 | CH₃ | CH₂-C≡C-CH₂-CH₃ | CH₃ | NCH₃ |
| 3779 | CH₃ | CH₂-CH₂-CH=CH₂ | CH₃ | NCH₃ |
| 3780 | CH₃ | CH₂-CH₂-CH₂-CH=CH₂ | CH₃ | NCH₃ |
| 3781 | CH₃ | CH₂-[(3-OCH₃)C₆H₄] | CH₃ | NCH₃ |
| 3782 | CH₃ | CH₂-C≡C-CH₃ | CH₃ | NCH₃ |
| 3783 | CH₃ | CH₂-CH₂-(1,3-dioxolan-2-yl) | CH₃ | NCH₃ |
| 3784 | CH₃ | CH₂-(1,3-dioxolan-2-yl) | CH₃ | NCH₃ |
| 3785 | CH₃ | n-Pentyl | Cl | NCH₃ |
| 3786 | CH₃ | CH₂-C≡C-CH₂-CH₃ | Cl | NCH₃ |
| 3787 | CH₃ | CH₂-CH₂-CH=CH₂ | Cl | NCH₃ |
| 3788 | CH₃ | CH₂-CH₂-CH₂-CH=CH₂ | Cl | NCH₃ |
| 3789 | CH₃ | CH₂-[(3-OCH₃)C₆H₄] | Cl | NCH₃ |
| 3790 | CH₃ | CH₂-C≡C-CH₃ | Cl | NCH₃ |
| 3791 | CH₃ | CH₂-CH₂-(1,3-dioxolan-2-yl) | Cl | NCH₃ |
| 3792 | CH₃ | CH₂-(1,3-dioxolan-2-yl) | Cl | NCH₃ |
| 3793 | Cl | n-Pentyl | H | NCH₃ |
| 3794 | Cl | CH₂-C≡C-CH₂-CH₃ | H | NCH₃ |
| 3795 | Cl | CH₂-CH₂-CH=CH₂ | H | NCH₃ |
| 3796 | Cl | CH₂-CH₂-CH₂-CH=CH₂ | H | NCH₃ |
| 3797 | Cl | CH₂-[(3-OCH₃)C₆H₄] | H | NCH₃ |
| 3798 | Cl | CH₂-C≡C-CH₃ | H | NCH₃ |
| 3799 | Cl | CH₂-CH₂-(1,3-dioxolan-2-yl) | H | NCH₃ |
| 3800 | Cl | CH₂-(1,3-dioxolan-2-yl) | H | NCH₃ |
| 3801 | Cl | n-Pentyl | CH₃ | NCH₃ |
| 3802 | Cl | CH₂-C≡C-CH₂-CH₃ | CH₃ | NCH₃ |
| 3803 | Cl | CH₂-CH₂-CH=CH₂ | CH₃ | NCH₃ |
| 3804 | Cl | CH₂-CH₂-CH₂-CH=CH₂ | CH₃ | NCH₃ |
| 3805 | Cl | CH₂-[(3-OCH₃)C₆H₄] | CH₃ | NCH₃ |
| 3806 | Cl | CH₂-C≡C-CH₃ | CH₃ | NCH₃ |
| 3807 | Cl | CH₂-CH₂-(1,3-dioxolan-2-yl) | CH₃ | NCH₃ |
| 3808 | Cl | CH₂-(1,3-dioxolan-2-yl) | CH₃ | NCH₃ |
| 3809 | Cl | n-Pentyl | Cl | NCH₃ |
| 3810 | Cl | CH₂-C≡C-CH₂-CH₃ | Cl | NCH₃ |
| 3811 | Cl | CH₂-CH₂-CH=CH₂ | Cl | NCH₃ |
| 3812 | Cl | CH₂-CH₂-CH₂-CH=CH₂ | Cl | NCH₃ |
| 3813 | Cl | CH₂-[(3-OCH₃)C₆H₄] | Cl | NCH₃ |
| 3814 | Cl | CH₂-C≡C-CH₃ | Cl | NCH₃ |
| 3815 | Cl | CH₂-CH₂-(1,3-dioxolan-2-yl) | Cl | NCH₃ |
| 3816 | Cl | CH₂-(1,3-dioxolan-2-yl) | Cl | NCH₃ |
| 3817 | OCH₃ | n-Pentyl | H | NCH₃ |
| 3818 | OCH₃ | CH₂-C≡C-CH₂-CH₃ | H | NCH₃ |
| 3819 | OCH₃ | CH₂-CH₂-CH=CH₂ | H | NCH₃ |
| 3820 | OCH₃ | CH₂-CH₂-CH₂-CH=CH₂ | H | NCH₃ |
| 3821 | OCH₃ | CH₂-[(3-OCH₃)C₆H₄] | H | NCH₃ |
| 3822 | OCH₃ | CH₂-C≡C-CH₃ | H | NCH₃ |
| 3823 | OCH₃ | CH₂-CH₂-(1,3-dioxolan-2-yl) | H | NCH₃ |
| 3824 | OCH₃ | CH₂-(1,3-dioxolan-2-yl) | H | NCH₃ |
| 3825 | OCH₃ | n-Pentyl | CH₃ | NCH₃ |
| 3826 | OCH₃ | CH₂-C≡C-CH₂-CH₃ | CH₃ | NCH₃ |
| 3827 | OCH₃ | CH₂-CH₂-CH=CH₂ | CH₃ | NCH₃ |
| 3828 | OCH₃ | CH₂-CH₂-CH₂-CH=CH₂ | CH₃ | NCH₃ |
| 3829 | OCH₃ | CH₂-[(3-OCH₃)C₆H₄] | CH₃ | NCH₃ |
| 3830 | OCH₃ | CH₂-C≡C-CH₃ | CH₃ | NCH₃ |
| 3831 | OCH₃ | CH₂-CH₂-(1,3-dioxolan-2-yl) | CH₃ | NCH3 |
| 3832 | OCH₃ | CH₂-(1,3-dioxolan-2-yl) | CH₃ | NCH₃ |
| 3833 | OCH₃ | n-Pentyl | Cl | NCH₃ |
| 3834 | OCH₃ | CH₂-C≡C-CH₂-CH₃ | Cl | NCH₃ |
| 3835 | OCH₃ | CH₂-CH₂-CH=CH₂ | Cl | NCH₃ |
| 3836 | OCH₃ | CH₂-CH₂-CH₂-CH=CH₂ | Cl | NCH₃ |
| 3837 | OCH₃ | CH₂-[(3-OCH₃)C₆H₄) | Cl | NCH₃ |
| 3838 | OCH₃ | CH₂-C≡C-CH₃ | Cl | NCH₃ |
| 3839 | OCH₃ | CH₂-CH₂-(1,3-dioxolan-2-yl) | Cl | NCH₃ |
| 3840 | OCH₃ | CH₂-(1,3-dioxolan-2-yl) | Cl | NCH₃ |
| 3841 | OCF₃ | n-Pentyl | H | NCH₃ |
| 3842 | OCF₃ | CH₂-C≡C-CH₂-CH₃ | H | NCH₃ |
| 3843 | OCF₃ | CH₂-CH₂-CH=CH₂ | H | NCH₃ |
| 3844 | OCF₃ | CH₂-CH₂-CH₂-CH=CH₂ | H | NCH₃ |
| 3845 | OCF₃ | CH₂-[(3-OCH₃)C₆H₄] | H | NCH₃ |
| 3846 | OCF₃ | CH₂-C≡C-CH₃ | H | NCH₃ |
| 3847 | OCF₃ | CH₂-CH₂-(1,3-dioxolan-2-yl) | H | NCH₃ |
| 3848 | OCF₃ | CH₂-(1,3-dioxolan-2-yl) | H | NCH₃ |
| 3849 | OCF₃ | n-Pentyl | CH₃ | NCH₃ |
| 3850 | OCF₃ | CH₂-C≡C-CH₂-CH₃ | CH₃ | NCH₃ |
| 3851 | OCF₃ | CH₂-CH₂-CH=CH₂ | CH₃ | NCH₃ |
| 3852 | OCF₃ | CH₂-CH₂-CH₂-CH=CH₂ | CH₃ | NCH₃ |
| 3853 | OCF₃ | CH₂₋[(3-OCH₃)C₆H₄] | CH₃ | NCH₃ |
| 3854 | OCF₃ | CH₂-C≡C-CH₃ | CH₃ | NCH₃ |
| 3855 | OCF₃ | CH₂-CH₂-(1,3-dioxolan-2-yl) | CH₃ | NCH₃ |
| 3856 | OCF₃ | CH₂-(1,3-dioxolan-2-yl) | CH₃ | NCH₃ |
| 3857 | OCF₃ | n-Pentyl | Cl | NCH₃ |
| 3858 | OCF₃ | CH₂-C≡C-CH₂-CH₃ | Cl | NCH₃ |
| 3859 | OCF₃ | CH₂-CH₂-CH=CH₂ | Cl | NCH₃ |
| 3860 | OCF₃ | CH₂-CH₂-CH₂-CH=CH₂ | Cl | NCH₃ |
| 3861 | OCF₃ | CH₂-[(3-OCH₃)C₆H₄] | Cl | NCH₃ |
| 3862 | OCF₃ | CH₂-C≡C-CH₃ | Cl | NCH₃ |
| 3863 | OCF₃ | CH₂-CH₂-(1,3-dioxolan-2-yl) | Cl | NCH₃ |
| 3864 | OCF₃ | CH₂-(1,3-dioxolan-2-yl) | Cl | NCH₃ |
| 3865 | SCH₃ | n-Pentyl | H | NCH₃ |
| 3866 | SCH₃ | CH₂-C≡C-CH₂-CH₃ | H | NCH₃ |
| 3867 | SCH₃ | CH₂-CH₂-CH=CH₂ | H | NCH₃ |
| 3868 | SCH₃ | CH₂-CH₂-CH₂-CH=CH₂ | H | NCH₃ |
| 3869 | SCH₃ | CH₂-[(3-OCH₃)C₆H₄] | H | NCH₃ |
| 3870 | SCH₃ | CH₂-C≡C-CH₃ | H | NCH₃ |
| 3871 | SCH₃ | CH₂-CH₂-(1,3-dioxolan-2-yl) | H | NCH₃ |
| 3872 | SCH₃ | CH₂-(1,3-dioxolan-2-yl) | H | NCH₃ |
| 3873 | SCH₃ | n-Pentyl | CH₃ | NCH₃ |
| 3874 | SCH₃ | CH₂-C≡C-CH₂-CH₃ | CH₃ | NCH₃ |
| 3875 | SCH₃ | CH₂-CH₂-CH=CH₂ | CH₃ | NCH₃ |
| 3876 | SCH₃ | CH₂-CH₂-CH₂-CH=CH₂ | CH₃ | NCH₃ |
| 3877 | SCH₃ | CH₂-[(3-OCH₃)C₆H₄] | CH₃ | NCH₃ |
| 3878 | SCH₃ | CH₂-C≡C-CH₃ | CH₃ | NCH₃ |
| 3879 | SCH₃ | CH₂-CH₂-(1,3-dioxolan-2-yl) | CH₃ | NCH₃ |
| 3880 | SCH₃ | CH₂-(1,3-dioxolan-2-yl) | CH₃ | NCH₃ |
| 3881 | SCH₃ | n-Pentyl | Cl | NCH₃ |
| 3882 | SCH₃ | CH₂-C≡C-CH₂-CH₃ | Cl | NCH₃ |
| 3883 | SCH₃ | CH₂-CH₂-CH=CH₂ | Cl | NCH₃ |
| 3884 | SCH₃ | CH₂-CH₂-CH₂-CH=CH₂ | Cl | NCH₃ |
| 3885 | SCH₃ | CH₂-[(3-OCH₃)C₆H₄] | Cl | NCH₃ |
| 3886 | SCH₃ | CH₂-C≡C-CH₃ | Cl | NCH₃ |
| 3887 | SCH₃ | CH₂-CH₂-(1,3-dioxolan-2-yl) | Cl | NCH₃ |
| 3888 | SCH₃ | CH₂-(1,3-dioxolan-2-yl) | Cl | NCH₃ |
| 3889 | SO₂CH₃ | n-Pentyl | H | NCH₃ |
| 3890 | SO₂CH₃ | CH₂-C≡C-CH₂-CH₃ | H | NCH₃ |
| 3891 | SO₂CH₃ | CH₂-CH₂-CH=CH₂ | H | NCH₃ |
| 3892 | SO₂CH₃ | CH₂-CH₂-CH₂-CH=CH₂ | H | NCH₃ |
| 3893 | SO₂CH₃ | CH₂-[(3-OCH₃)C₆H₄] | H | NCH₃ |
| 3894 | SO₂CH₃ | CH₂-C≡C-CH₃ | H | NCH₃ |
| 3895 | SO₂CH₃ | CH₂-CH₂-(1,3-dioxolan-2-yl) | H | NCH₃ |
| 3896 | SO₂CH₃ | CH₂-(1,3-dioxalan-2-yl) | H | NCH₃ |
| 3897 | SO₂CH₃ | n-Pentyl | CH₃ | NCH₃ |
| 3898 | SO₂CH₃ | CH₂-C≡C-CH₂-CH₃ | CH₃ | NCH₃ |
| 3899 | SO₂CH₃ | CH₂-CH₂-CH=CH₂ | CH₃ | NCH₃ |
| 3900 | SO₂CH₃ | CH₂-CH₂-CH₂-CH=CH₂ | CH₃ | NCH₃ |
| 3901 | SO₂CH₃ | CH₂-[(3-OCH₃)C₆H₄] | CH₃ | NCH₃ |
| 3902 | SO₂CH₃ | CH₂-C≡C-CH₃ | CH₃ | NCH₃ |
| 3903 | SO₂CH₃ | CH₂-CH₂-(1,3-dioxolan-2-yl) | CH₃ | NCH₃ |
| 3904 | SO₂CH₃ | CH₂-(1,3-dioxolan-2-yl) | CH₃ | NCH₃ |
| 3905 | SO₂CH₃ | n-Pentyl | Cl | NCH₃ |
| 3906 | SO₂CH₃ | CH₂-C≡C-CH₂-CH₃ | Cl | NCH₃ |
| 3907 | SO₂CH₃ | CH₂-CH₂-CH=CH₂ | Cl | NCH₃ |
| 3908 | SO₂CH₃ | CH₂-CH₂-CH₂-CH=CH₂ | Cl | NCH₃ |
| 3909 | SO₂CH₃ | CH₂-[(3-OCH₃)C₆H₄] | Cl | NCH₃ |
| 3910 | SO₂CH₃ | CH₂-C≡C-CH₃ | Cl | NCH₃ |
| 3911 | SO₂CH₃ | CH₂-CH₂-(1,3-dioxolan-2-yl) | Cl | NCH₃ |
| 3912 | SO₂CH₃ | CH₂-(1,3-dioxolan-2-yl) | Cl | NCH₃ |
| 3913 | CF₃ | n-Pentyl | H | NCH₃ |
| 3914 | CF₃ | CH₂-C≡C-CH₂-CH₃ | H | NCH₃ |
| 3915 | CF₃ | CH₂-CH₂-CH=CH₂ | H | NCH₃ |
| 3916 | CF₃ | CH₂₋CH₂₋CH₂-CH=CH₂ | H | NCH₃ |
| 3917 | CF₃ | CH₂-[(3-OCH₃)C₆H₄] | H | NCH₃ |
| 3918 | CF₃ | CH₂-C≡C-CH₃ | H | NCH₃ |
| 3919 | CF₃ | CH₂-CH₂-(1,3-dioxolan-2-yl) | H | NCH₃ |
| 3920 | CF₃ | CH₂-(1,3-dioxolan-2-yl) | H | NCH₃ |
| 3921 | CF₃ | n-Pentyl | CH₃ | NCH₃ |
| 3922 | CF₃ | CH₂-C≡C-CH₂-CH₃ | CH₃ | NCH₃ |
| 3923 | CF₃ | CH₂-CH₂-CH=CH₂ | CH₃ | NCH₃ |
| 3924 | CF₃ | CH₂-CH₂-CH₂-CH=CH₂ | CH₃ | NCH₃ |
| 3925 | CF₃ | CH₂-[(3-OCH₃)C₆H₄] | CH₃ | NCH₃ |
| 3926 | CF₃ | CH₂-C≡C-CH₃ | CH₃ | NCH₃ |
| 3927 | CF₃ | CH₂-CH₂-(1,3-dioxolan-2-yl) | CH₃ | NCH₃ |
| 3928 | CF₃ | CH₂-(1,3-dioxolan-2-yl) | CH₃ | NCH₃ |
| 3929 | CF₃ | n-Pentyl | Cl | NCH₃ |
| 3930 | CF₃ | CH₂-C≡C-CH₂-CH₃ | Cl | NCH₃ |
| 3931 | CF₃ | CH₂-CH₂-CH=CH₂ | Cl | NCH₃ |
| 3932 | CF₃ | CH₂-CH₂-CH₂-CH=CH₂ | Cl | NCH₃ |
| 3933 | CF₃ | CH₂-[(3-OCH₃)C₆H₄] | Cl | NCH₃ |
| 3934 | CF₃ | CH₂-C≡C-CH₃ | Cl | NCH₃ |
| 3935 | CF₃ | CH₂-CH₂-(1,3-dioxolan-2-yl) | Cl | NCH₃ |
| 3936 | CF₃ | CH₂-(1,3-dioxolan-2-yl) | Cl | NCH₃ |
| 3937 | C₂H₅ | n-Pentyl | H | NCH₃ |
| 3938 | C₂H₅ | CH₂-C≡C-CH₂-CH₃ | H | NCH₃ |
| 3939 | C₂H₅ | CH₂-CH₂-CH=CH₂ | H | NCH₃ |
| 3940 | C₂H₅ | CH₂-CH₂-CH₂-CH=CH₂ | H | NCH₃ |
| 3941 | C₂H₅ | CH₂-[(3-OCH₃)C₆H₄] | H | NCH₃ |
| 3942 | C₂H₅ | CH₂-C≡C-CH₃ | H | NCH₃ |
| 3943 | C₂H₅ | CH₂-CH₂-(1,3-dioxolan-2-yl) | H | NCH₃ |
| 3944 | C₂H₅ | CH₂-(1,3-dioxolan-2-yl) | H | NCH₃ |
| 3945 | C₂H₅ | n-Pentyl | CH₃ | NCH₃ |
| 3946 | C₂H₅ | CH₂-C≡C-CH₂-CH₃ | CH₃ | NCH₃ |
| 3947 | C₂H₅ | CH₂-CH₂-CH=CH₂ | CH₃ | NCH₃ |
| 3948 | C₂H₅ | CH₂-CH₂-CH₂-CH=CH₂ | CH₃ | NCH₃ |
| 3949 | C₂H₅ | CH₂-[(3-OCH₃)C₆H₄] | CH₃ | NCH₃ |
| 3950 | C₂H₅ | CH₂-C≡C-CH₃ | CH₃ | NCH₃ |
| 3951 | C₂H₅ | CH₂-CH₂-(1,3-dioxolan-2-yl) | CH₃ | NCH₃ |
| 3952 | C₂H₅ | CH₂-(1,3-dioxolan-2-yl) | CH₃ | NCH₃ |
| 3953 | C₂H₅ | n-Pentyl | Cl | NCH₃ |
| 3954 | C₂H₅ | CH₂-C≡C-CH₂-CH₃ | Cl | NCH₃ |
| 3955 | C₂H₅ | CH₂-CH₂-CH=CH₂ | Cl | NCH₃ |
| 3956 | C₂H₅ | CH₂-CH₂-CH₂-CH=CH₂ | Cl | NCH₃ |
| 3957 | C₂H₅ | CH₂-[(3-OCH₃)C₆H₄] | Cl | NCH₃ |
| 3958 | C₂H₅ | CH₂-C≡C-CH₃ | Cl | NCH₃ |
| 3959 | C₂H₅ | CH₂-CH₂-(1,3-dioxolan-2-yl) | Cl | NCH₃ |
| 3960 | C₂H₅ | CH₂-(1,3-dioxolan-2-yl) | Cl | NCH₃ |

Die Herstellung der Verbindungen der Formel I, worin R⁷ für Hydroxy steht, erfolgt durch Umsetzung einer aktivierten Carbonsäure IVb oder einer Carbonsäure IVa, die vorzugsweise in situ aktiviert wird, mit 5-Hydroxypyrazol der Formel III zu dem Acylierungsprodukt und anschließender Umlagerung.

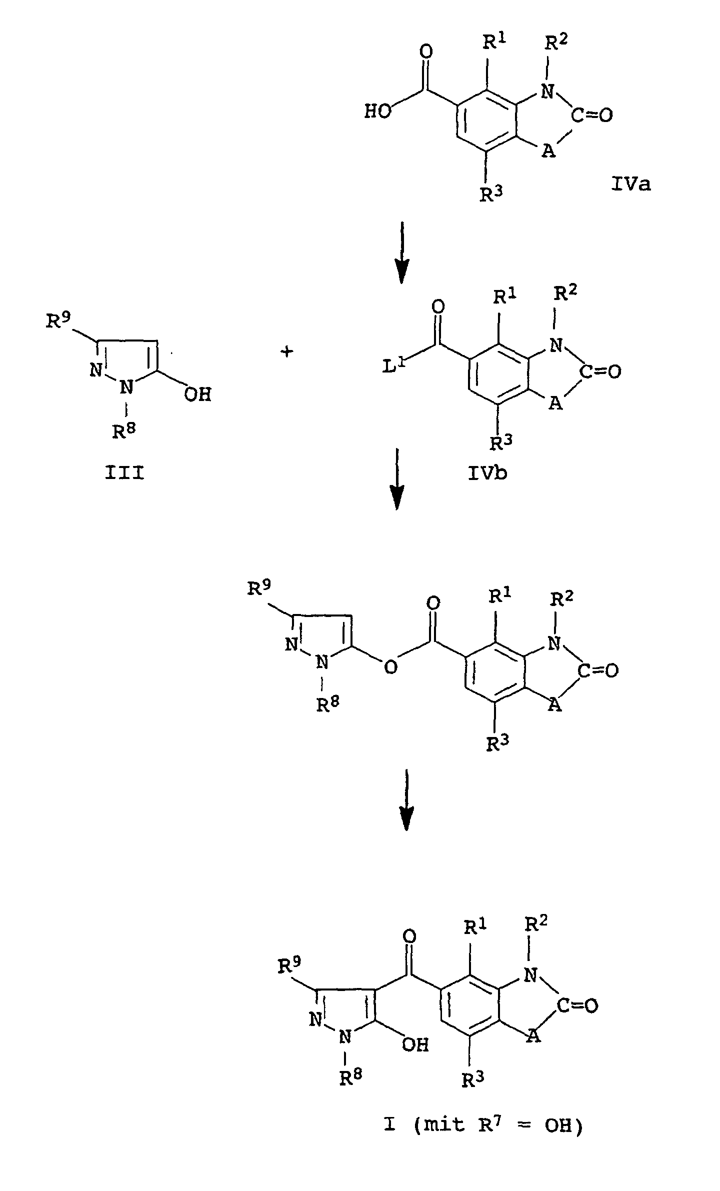

L¹ steht für eine nucleophil verdrängbare Abgangsgruppe, wie Halogen z. B. Brom oder Chlor, Hetaryl, z.B. Imidazolyl oder Pyridyl, Carboxylat, z.B. Acetat oder Trifluoracetat etc.

Die aktivierte Carbonsäure IVb kann direkt eingesetzt werden, wie im Fall der Benzoylhalogenide oder in situ erzeugt werden, z.B. mit einem Carbodiimid wie Ethyl-(3'-dimethylaminopropyl)carbodiimid, Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuss der Hilfsbase z. B. 1,2 bis 1,5 Moläquivalente, bezogen auf Iva bzw. IVb, kann unter Umständen vorteilhaft sein.

Als Hilfsbasen eignen sich tertiäre Alkylamine, Pyridin, 4-Dimethylaminopyridin oder Alkalimetallcarbonate. Als Lösungsmittel können z. B. chlorierte Kohlenwasserstoffe, wie Methylenchlorid oder 1,2-Dichlorethan, aromatische Kohlenwasserstoffe, wie Toluol, Xylol oder Chlorbenzol, Ether, wie Diethylether, Methyltert.-butylether, Tetrahydrofuran oder Dioxan, polare aprotische Lösungsmittel, wie Acetonitril, Dimethylformamid oder Dimethylsulfoxid oder Ester wie Essigsäureethylester oder Gemische hiervon verwendet werden.

Werden Halogenide als aktivierte Carbonsäurekomponente eingesetzt, so kann es zweckmäßig sein, bei Zugabe dieses Reaktionspartners die Reaktionsmischung auf 0 °C bis 10 °C abzukühlen. Anschließend rührt man bei 20 °C bis 100 °C, vorzugsweise bei 25 °C bis 50 °C, bis die Umsetzung vollständig ist. Die Aufarbeitung erfolgt in üblicher Weise, z. B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 °C bis 100 °C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, aromatische Amine wie Pyridin oder Alkalicarbonate, wie Natriumcarbonat oder Kaliumcarbonat, die vorzugsweise in äquimolarer Menge oder bis zu einem vierfachen Überschuss, bezogen auf den Ester, eingesetzt werden. Bevorzugt werden Triethylamin oder Alkalicarbonat verwendet, vorzugsweise in doppelt äquimolaren Verhältnis in Bezug auf den Ester.

Als Cyanoverbindungen kommen anorganische Cyanide, wie Natriumcyanid oder Kaliumcyanid und organische Cyanoverbindungen, wie Acetoncyanhydrin oder Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise etwa 10 Molprozent, bezogen auf den Ester, eingesetzt.

Die Aufarbeitung kann in an sich bekannter Weise erfolgen. Das Reaktionsgemisch wird z. B. mit verdünnter Mineralsäure, wie 5 %ige Salzsäure oder Schwefelsäure, angesäuert, mit einem organischen Lösungsmittel, z. B. Methylenchlorid oder Essigsäureethylester extrahiert. Der organische Extrakt kann mit 5-10%iger Alkalicarbonatlösung, z. B. Natriumcarbonat- oder Kaliumcarbonatlösung extrahiert werden. Die wässrige Phase wird angesäuert und der sich bildende Niederschlag abgesaugt und/oder mit Methylenchlorid oder Essigsäureethylester extrahiert, getrocknet und eingeengt.

B. Die Darstellung von Verbindungen der Formel I mit R⁷ = Halogen erfolgt durch Umsetzung von Pyrazol-Derivaten der Formel I (mit R⁷ = Hydroxy) mit Halogenierungsmitteln: 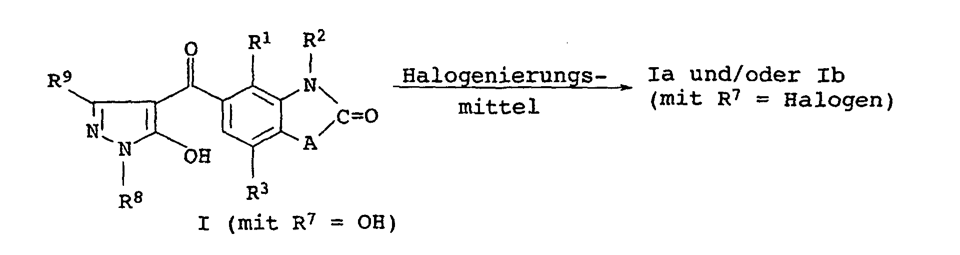 Hier und im folgenden steht "Verbindung 1a" für eine Verbindung der allgemeinen Formel I, worin Pz für einen Pyrazolylrest der allgemeinen Formel IIa steht und Verbindung 1b entsprechend für eine Verbindung der allgemeinen Formel I, worin Pz für einen Rest IIb steht.

Als Halogenierungsmittel eignen sich beispielsweise Phosgen, Diphosgen, Triphosgen, Thionylchlorid, Oxalylchlorid, Phosphoroxychlorid, Phosphorpentachlorid, Mesylchlorid, Chlormethylen-N,N-dimethylammoniumchlorid, Oxalylbromid, Phosphoroxybromid etc.

C. Die Darstellung von Verbindungen der Formel I mit R⁷ = OR¹⁰, OSO₂R¹¹, OPOR¹²R¹³ oder OPSR¹²R¹³ durch Umsetzung von Pyrazol-Derivaten der Formel I (mit R⁷ = Hydroxy) mit Alkylierungs-, Sulfonylierungs- bzw. Phosphonylierungsmitteln Vα, Vβ, Vγ beziehungsweise Vδ. 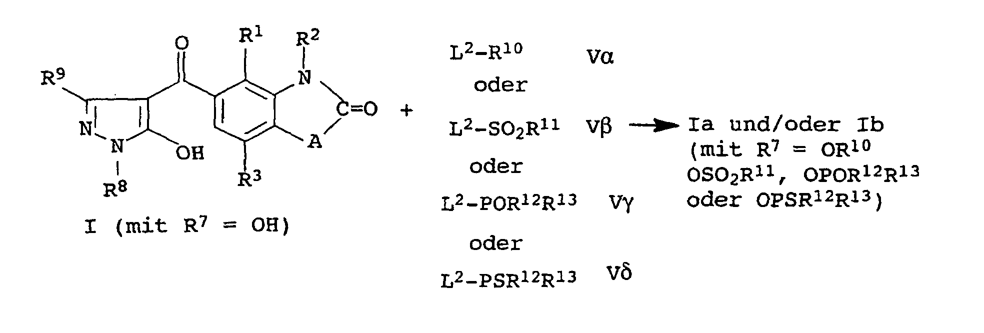 L² steht für eine nucleophil verdrängbare Abgangsgruppe, wie Halogen, z.B. Chlor oder Brom, Hetaryl, z. B. Imidazolyl, Carboxylat, z. B. Acetat, oder Sulfonat, z. B. Mesylat oder Triflat etc.

Bei der Herstellung von Verbindungen der Formel I mit R⁷ = OR¹⁰ aus Verbindungen der Formel I mit R⁷ = OH arbeitet man vorzugsweise in Gegenwart einer Base.

Die Reaktanden und die Base werden dabei zweckmäßigerweise in äquimolaren Mengen eingesetzt. Ein geringer Überschuß der Base, z. B. 1,1 - 1,5 Moläquivalente, bezogen auf I kann unter Umständen vorteilhaft sein.

Als Basen eignen sich tertiäre Amine, Pyridine, Alkalimetallcarbonate oder Alkalimetallhydride. Als Lösungsmittel eignen sich z. B. chlorierte Kohlenwasserstoffe wie Methylenchlorid oder 1,2-Dichlorethan, aromatische Kohlenwasserstoffe wie Toluol, Xylol oder Chlorbenzol, Ether wie Diethylether, Methyl-tert.-butylether, Tetrahydrofuran, Dioxan oder Dimethoxyethan, polare aprotische Lösungsmittel wie Acetonitril, Dimethylformamid oder Dimethylsulfoxid oder Ester wie Essigsäureethylester und Gemische hiervon.

Werden anstelle des Alkohols I (R⁷ = OH) Halogenide (R⁷ = Halogen) oder aktivierte Alkohole wie Mesylate oder Tosylate (R⁷ = OSO₂CH₃ oder OSO₂-Tolyl) zur Derivatisierung eingesetzt, so kann es zweckmäßig sein, bei Zugabe des Reaktionspartners die Reaktionsmischung auf 0 °C bis 10°C abzukühlen. Anschließend rührt man bei 20 °C bis 100°C, vorzugsweise bei 20°C bis 75 °C bis die Umsetzung vollständig ist.

Die Aufarbeitung erfolgt in üblicher Weise, z. B. wird das Reaktionsgemisch auf Wasser gegossen, das Wertprodukt extrahiert. Als Lösungsmittel eignen sich hierfür besonders Lösungsmittel wie Methylenchlorid, Essigsäureethylester, Methyl-tert-butylether oder Diethylether. Nach Trocknen der organischen Phase und Entfernen des Lösungsmitel kann das Rohprohdukt gegebenenfalls noch durch säulenchromatographie an Kieselgel gereinigt werden. Als Eluenten eignen sich Lösungsmittel wie Methylenchlorid, Essigsäureethylester, Cyclohexan, Petrolether, Methanol, Aceton oder Chloroform und Gemische hiervon.

Die Verbindungen der Formel Vα, Vβ, Vγ oder Vδ können direkt eingesetzt werden wie z. B. im Fall der Carbonsäurehalogenide oder in situ erzeugt werden, z.B. aktivierte Carbonsäuren (mit Carbonsäure und Dicyclohexylcarbodiimid etc.).

D. Die Darstellung von Verbindungen der Formel I mit R⁷ = OR¹⁰, SR¹⁰, POR¹²R¹³, NR¹⁴R¹⁵, ONR¹⁴R¹⁵ oder N-gebundenes Heterocyclyl erfolgt durch Umsetzung von Verbindungen der Formel I mit R⁷ = Halogen, OSO₂R¹¹ mit Verbindungen der Formel VIα, VIβ, VIγ, VIδ, VIε oder VIη, gegebenenfalls in Gegenwart einer Base oder unter vorangehender Salzbildung.

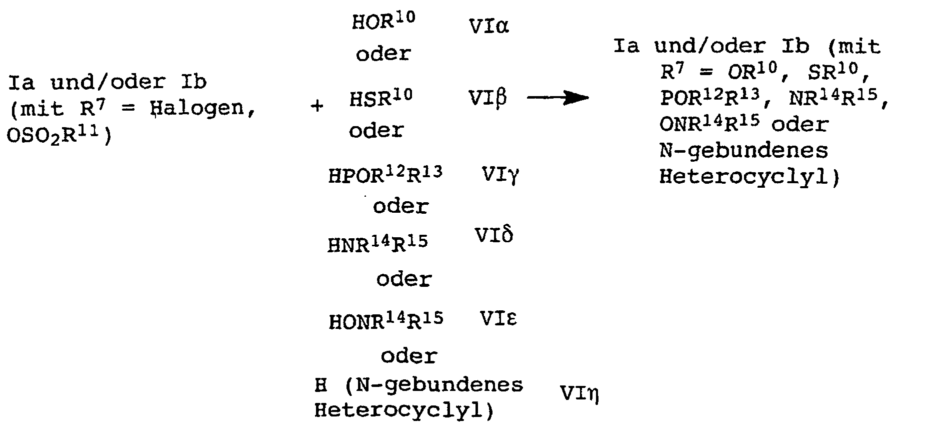

E. Die Darstellung von Verbindungen der Formel I mit R⁷ = SOR¹¹, SO₂R¹¹ erfolgt beispielsweise durch Umsetzung von Verbindungen der Formel I mit R⁷ = SR¹¹ mit einem Oxidationsmittel.

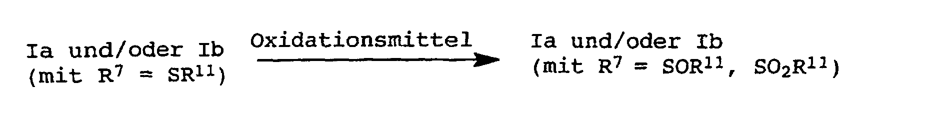

Als Oxidationsmittel kommen beispielsweise m-Chlorperbenzoesäure, Peroxyessigsäure, Trifluorperoxyessigsäure, Wasserstoffperoxid, ggf. in Gegenwart eines Katalysators wie Wolframat, in Betracht.

Für die unter den Punkten B bis E genannten Reaktionen gelten folgende Bedingungen:

Die Ausgangsverbindungen werden in der Regel im äquimolaren Verhältnis eingesetzt. Es kann aber auch von vorteil sein, die eine oder andere Komponente im Überschuss einzusetzen.

Gegebenenfalls kann es von Vorteil sein, die Umsetzungen in Gegenwart einer Base durchzuführen. Die Reaktanden und die Base werden dabei zweckmäßigerweise in äquimolaren Mengen eingesetzt.

Im Hinblick auf die Verfahren C und D kann es unter Umständen vorteilhaft sein, ein Überschuss der Base z. B. 1,5 bis 3 Moläquivalente jeweils bezogen auf das Edukt einzusetzen.

Als Basen eignen sich tertiäre Alkylamine, wie Triethylamin, aromatische Amine, wie Pyridin, Alkalimetallcarbonate, z. B. Natriumcarbonat oder Kaliumcarbonat, Alkalimetallhydrogencarbonate, wie Natriumhydrogencarbonat und Kaliumhydrogencarbonat, Alkalimetallalkoholate wie Natriummethanolat, Natriumethanolat, Kalium-tert.-butanolat oder Alkalimetallhydride, z. B. Natriumhydrid. Bevorzugt verwendet werden Triethylamin oder Pyridin.

Als Lösungsmittel kommen z. B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

In Abhängigkeit von den Reaktionsbedingungen können bei den Verfahren B bis D die Verbindungen Ia, Ib oder Gemische hiervon gebildet werden. Letztere können durch klassische Trennmethoden, wie z. B. Kristallisation, Chromatographie etc., getrennt werden.

F. Die Darstellung von Verbindungen der Formel I, worin Pz für eine Gruppe der allgemeinen Formel IIa steht, kann auch durch Umsetzung eines metallierten Pyrazol-Derivats der Formel VII mit einem Carbonsäure-Derivat der Formel IVb erfolgen:

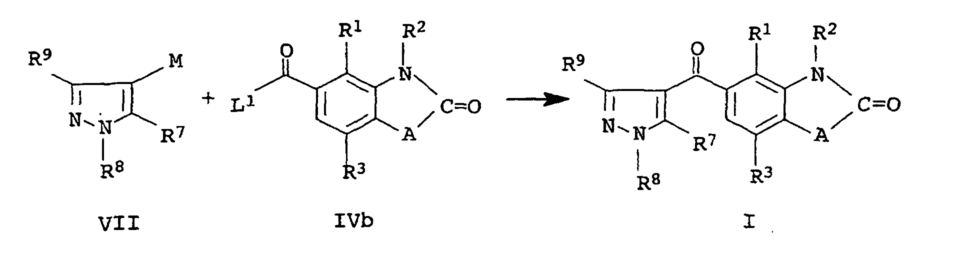

M steht hierbei für ein Metall, insbesondere für ein Alkalimetall wie Lithium oder Natrium, ein Erdalkalimetall wie z. B. Magnesium oder ein Übergangsmetall wie Palladium, Nickel etc. und L¹ für eine nucleophil verdrängbare Abgangsgruppe wie Halogen, z.B. Chlor oder Brom, Alkylsulfonat wie Mesylat, Halogenalkylsulfonat wie Triflat oder Cyanid. R⁷ weist vorzugsweise keine aziden Wasserstoffatome auf.

Die Umsetzung wird in der Regel bei Temperaturen von -100 °C bis Rückflußtemperatur des Reaktionsgemisches durchgeführt. Als Lösungsmittel eignen sich inerte aprotische Lösungsmittel, wie Ether, z.B. Diethylether, Tetrahydrofuran. Die Verbindungen der Formel IVb werden in der Regel im Überschuss eingesetzt, es kann aber auch von Vorteil sein, diese in äquimolaren Mengen oder im Unterschuß einzusetzen. Die Aufarbeitung erfolgt zum Produkt hin.

Die metallierten Pyrazol-Derivate der Formel VII können auf an sich bekannte Art und Weise durch Umsetzung von in 4-Position halogenierten Pyrazolen mit Metallen wie Lithium, Natrium, Magnesium etc. oder mit metallorganischen Verbindungen wie z. B. Butyllithium gebildet werden. Es ist aber auch möglich Pyrazole, die in 4-Position mit Wasserstoff verknüpft sind, direkt zu metallieren, z. B. mit den voranstehend genannten Metallen bzw. metallorganischen Verbindungen. Die Umsetzungen werden in der Regel in einem inerten aprotischen Lösungsmittel durchgeführt, bevorzugt in Ether wie Diethylether, Tetrahydrofuran etc.. Die Reaktionstemperatur liegt im Bereich von -100°C bis zur Höhe des Siedepunktes des Reaktionsgemisches. Die Verbindungen der Formel VII werden vorzugsweise in situ erzeugt und direkt umgesetzt.

Die als Ausgangsmaterialien verwendeten 5-Hydroxypyrazole der Formel III sind bekannt oder können an sich nach bekannten Verfahren hergestellt werden, wie sie beispielsweise in der EP-A 240 001, in J. Chem. Soc. 315, S.383 (1997), J. Prakt. Chem. 315, S. 382 (1973) beschrieben sind (siehe auch Übersichten in Advances Heterocycle. Chem. 48, S. 223-299 (1990) und Katritzky, Rees (Hrsg.), Comprehensive Heterocyclic Chem. Vol. 5, Pergamon Press 1984, Oxford, S. 167-343 und dort zitierte Literatur). 1,3-Dimethyl-5-hydroxypyrazol ist überdies eine käufliche Verbindung.

Die Alkylierungsmittel Vα, Sulfonylierungsmittel Vβ, Phosphonylierungsmittel Vγ beziehungsweise Vδ, sowie die Verbindungen VIα, VIβ, VIγ, VIδ und VIε sind ebenfalls bekannt oder können nach bekannten Verfahren hergestellt werden.

Die Carbonsäuren der allgemeinen Formel IVa beziehungsweise ihre aktivierten Derivate IVb sind neu und ebenfalls Gegenstand der vorliegenden Erfindung.

Schema 1 veranschaulicht einen allgemeinen Zugang zur Herstellung von Verbindungen der Formel IVa, worin A für Sauerstoff oder NR⁶ steht.

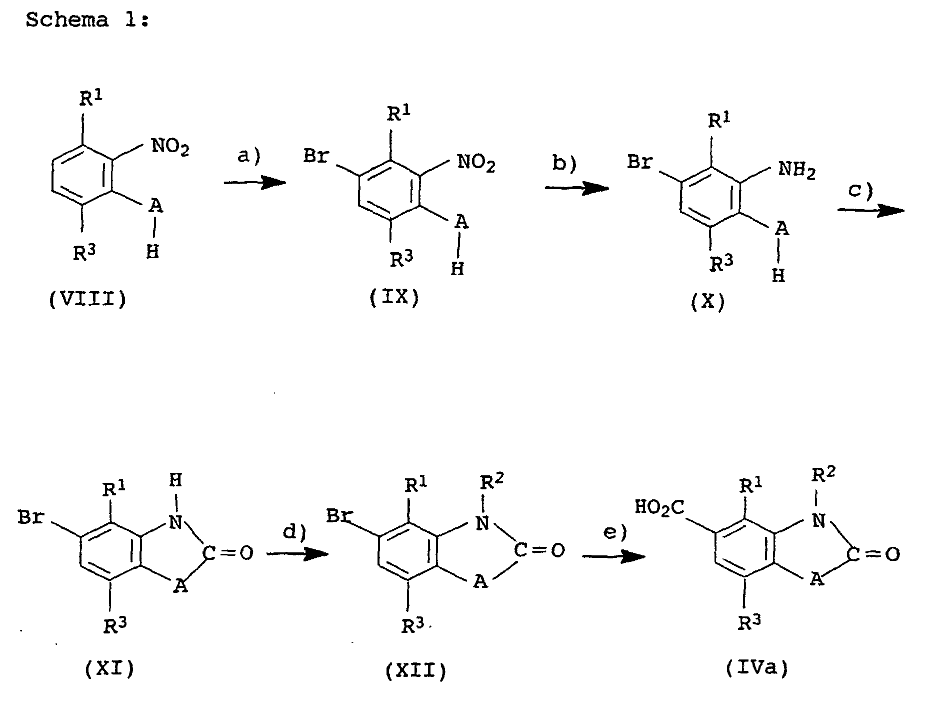

Benzazoloncarbonsäuren der allgemeinen Formel IVa (A = O oder NR⁶) können gemäß Schema 1 ausgehend von in 3-Position substituierten 2-Nitroanilinen bzw. 2-Nitrophenolen der Formel VIII hergestellt werden. In Schritt a) erfolgt zunächst die Bromierung der Nitroverbindungen VIII in meta-Position zur Nitrogruppe. Übliche Bromierungsmittel für diesen Zweck sind Brom, N-Bromsuccinimid, N-Bromhydantoin oder Pyridiniumperbromid, die gegebenenfalls zusammen mit einer Lewis-Säure wie FeBr₃ eingesetzt werden. Üblicherweise führt man die Bromierung in einem inerten Lösungsmittel durch. Geeignete Lösungsmittel sind aliphatische oder cycloaliphatische Kohlenwasserstoffe, beispielsweise n-Hexan oder Cyclohexan, halogenierte Kohlenwasserstoffe, beispielsweise Dichlormethan, Trichlormethan, Tetrachlormethan, Trichlorethan, Trichlorethylen, Heteroaromate wie Pyridin oder wasserfreie anorganische oder organische Säuren, wie Essigsäure. Übliche Reaktionstemperaturen liegen im Bereich von -15 °C bis 154°C, vorzugsweise im Bereich von -15 °C bis 100°C. Verfahren zur Bromierung von Nitroverbindungen sind bekannt, z. B. aus Organikum, 16. Aufl., 1986, S. 315.

Anschließend wird im Schritt b) die Nitrogruppe der Verbindung IX zur Aminogruppe reduziert. Als Reduktionsmittel kommen beispielsweise Hydrazine, Metallhydride wie Aluminiumhydrid und davon abgeleitete komplexe Hydride wie Lithiumaluminiumhydrid, Diisobutylaluminiumhydrid, oder Borane, sowie nascierender Wasserstoff, z. B. Eisen, Zink oder Zinn in Gegenwart von Säuren, wie Salzsäure oder Carbonsäuren, wie Essigsäure, in Betracht. Ein weiteres geeignetes Reduktionsmittel ist Wasserstoff in Gegenwart katalytischer Mengen an Übergangsmetallen wie Nickel, Palladium, Platin, Ruthenium oder Rhodium. Die Übergangsmetalle können als solche oder in geträgerter Form, beispielsweise auf Aktivkohle, in Form aktivierter Metalle, z. B. Raney-Nickel, oder in Form löslicher Komplexverbindungen eingesetzt werden. Vorzugsweise führt man die Umsetzung in einem Lösungsmittel durch. Geeignete Lösungsmittel für die Reduktion sind abhängig von der Löslichkeit des zu hydrierenden Substrates und dem gewählten Reduktionsmittel beispielsweise C₁-C₄-Alkohole, wie Methanol, Ethanol, n-Propanol, Isopropanol oder n-Butanol, halogenierte C₁-C₆-Rohlenwasserstoffe, wie Dichlormethan, Trichlormethan, Trichlorethan, Trichlorethylen, aromatische Kohlenwasserstoffe, wie Benzol, Toluol, Xylole, Chlorbenzol, wässrige Lösungen anorganischer Säuren, wie wässrige Salzsäure oder organische Säuren und deren Mischungen mit Wasser. Üblicherweise erfolgt die Reduktion bei Temperaturen im Bereich von -15 °C bis +100 °C, vorzugsweise im Bereich von 0 °C bis 40 °C. Die Reduktion mit Wasserstoff erfolgt üblicherweise bei einem Wasserstoffdruck im Bereich von 1 bis 50 bar. Vorzugsweise erfolgen die katalytischen Hydrierungen mit Wasserstoff im Bereich von 1 bis 10 bar. Zur katalytischen Hydrierung aromatischer Nitrogruppen siehe beispielsweise Rylander in "Catalytic Hydrogenation over Platinum Metals", Academic Press, New York, 1967, 168-202; Furst et al., Chem. Rev. 1965, 65, 52; Tepko et al., J. Org. Chem. 1980, 45, 4992.

Die Kondensation des 3-Bromanilins X zum Heterocyclus in Schritt c) gelingt durch Umsetzung der Verbindung X mit einem Kohlensäureäquivalent, wie Chlorameisensäuremethylester, Phosgen oder dessen Syntheseäquivalente wie Diphosgen oder Triphosgen, unter den für die Phosgenierung üblichen Bedingungen. Die Cyclisierung erfolgt in der Regel unter neutralen bis sauren Reaktionsbedingungen bei Temperaturen im Bereich von 0 °C bis 150 °C und vorzugsweise im Bereich von 20 °C bis 120 °C. Üblicherweise führt man die Phosgenierung in einem Lösungsmittel durch. Als Lösungsmittel kommen insbesondere aliphatische oder cycloaliphatische Kohlenwasserstoffe, wie n-Hexan oder Cyclohexan, halogenierte Kohlenwasserstoffe, wie Dichlormethan, Trichlormethan, Trichlorethan, Trichlorethylen, aromatische Kohlenwasserstoffe, wie Benzol, aliphatische Ether, wie Diethylether, Methyl-tert.-butylether, oder cyclische Ether, wie Tetrahydrofuran oder Dioxan in Betracht. Verfahren zur Phosgenierung sind bekannt, siehe beispielsweise Justus Liebigs Ann. Chem., 1978, Nr. 2, 193-213; J. Med. Chem., 1987, 30, N 7, 1166-1176, J. Heterocycl. Chem. 1991, 28 (8), 1937-1939; J. Nat. Prod. 1995, 58 (3), 456-458.

Die Einführung des Substituenten R² im Schritt d) gelingt beispielsweise durch Umsetzung des in Schritt c) erhaltenen 5-Brombenzazol-2-ons XI mit R²-L oder dessen Vorstufe, wobei L für eine nucleophil verdrängbare Abgangsgruppe steht. Geeignete Abgangsgruppen sind beispielsweise Halogen wie Chlor, Brom oder Iod, Carboxylat wie Acetat oder Trifluoracetat oder Sulfonate wie Tosylat, Mesylat oder Triflat. Die Umsetzung erfolgt vorzugsweise in Gegenwart einer Hilfsbase, z. B. Alkali- oder Erdalkalicarbonate, wie Natrium-, Kalium-, Magnesium- oder Calciumcarbonat, Alkali- oder Erdalkalihydride, beispielsweise Natriumhydrid, tertiäre Alkylamine, beispielsweise Triethylamin, aromatische Amine, beispielsweise Pyridin, DMPU. Die Umsetzung erfolgt in der Regel bei Temperaturen im Bereich von -15 °C bis 150 °C und vorzugsweise bei 0 °C bis 100 °C. Üblicherweise führt man die Reaktion in einem Lösungsmittel durch. Geeignete Lösungsmittel sind beispielsweise die vorgenannten inerten Kohlenwasserstoffe, die vorgenannten halogenierten Kohlenwasserstoffe, aromatische Kohlenwasserstoffe, wie Benzol, Toluol, Xylol oder Chlorbenzol, die vorgenannten acyclischen oder cyclischen Ether, ferner polar aprotische Lösungsmittel wie Dimethylformamid, Acetonitril oder Dimethylsulfoxid. Verfahren zur Einführung eines Substituenten an einem heterocyclischen Amid-Stickstoffatom sind bekannt, siehe auch Eur. J. Med. Chem. 1995, 30 (9), 715-719; Tetrahedron 1998, 54 (9), 1763-1772.

Die sukzessive Umsetzung von XII mit Magnesium oder Alkylmagnesiumhalogeniden zur entsprechenden Grignard-Verbindung und die anschließende Umsetzung der Grignard-Verbindung mit Kohlendioxid ergibt dann die Carbonsäure IVa (Schritt e)). Übliche Reaktionstemperaturen liegen im Bereich von -15 °C bis 150 °C, vorzugsweise im Bereich von -15 °C bis 100 °C. Geeignete Lösungsmittel sind wasserfreie Lösungsmittel, insbesondere die vorgenannten inerten cyclischen oder acyclischen Kohlenwasserstoffe, die vorgenannten aromatischen Kohlenwasserstoffe oder die vorgenannten acyclischen oder cyclischen Ether. Durch Einleiten von trockenem Kohlendioxid in die erhaltene Lösung der zu XII korrespondierenden Grignard-Verbindung und anschließende wässrige Aufarbeitung erhält man die Benzazolon-5-carbonsäure IVa. Der Kohlendioxiddruck beträgt üblicherweise 1 bis 6 bar.

Alternativ kann XII durch Halogen-Metallaustausch mit einem Alkalimetallalkyl, z.B. einem Lithiumalkyl, wie Methyllithium, n-Butyllithium oder tert-Butyllithium, und anschließende Umsetzung des Lithiierungsproduktes mit CO₂ in die Carbonsäure IVa überführt werden. Übliche Reaktionstemperaturen liegen im Bereich von -100 °C bis 0 °C, vorzugsweise im Bereich von -78 °C bis -50 °C. Geeignete Lösungsmittel sind wasserfreie Lösungsmittel, insbesondere die vorgenannten inerten Kohlenwasserstoffe, die vorgenannten aromatischen Kohlenwasserstoffe oder die vorgenannten acyclischen oder cyclischen Ether. Einleiten von trockenem Kohlendioxid in die Lösung des Lithiierungsproduktes von XII liefert die Benzazoloncarbonsäure IVa. Der Kohlendioxiddruck beträgt üblicherweise 1 bis 6 bar.

Reaktionsschritt e) in Schema 1 kann auch durch Umsetzung von XII mit Kohlenmonoxid, einer Base und Wasser unter erhöhtem Druck in Gegenwart eines Palladium-, Nickel-, Cobalt- oder Rhodium-Katalysators realisiert werden.

Die Katalysatoren Nickel, Cobalt, Rhodium und insbesondere Palladium können metallisch oder in Form üblicher Salze wie in Form von Halogenverbindungen, z.B. Palladium(II)-chlorid, Rhodium(III)-chlorid Hydrat, Acetaten, z. B. Palladium(II)-acetat, Cyaniden usw. in den bekannten Wertigkeitsstufen eingesetzt werden. Ferner können Metallkomplexe mit tertiären Phosphinen, Metallalkylcarbonyle, Metallcarbonyle, z. B. CO₂(CO)₈, Ni(CO)₄, Metallcarbonyl-Komplexe mit tertiären Phosphinen, z. B. (PPh₃)₂Ni(CO)₂, oder mit tertiären Phosphinen komplexierte Übergangsmetallsalze eingesetzt werden. Die letztgenannte Ausführungsform ist insbesondere im Fall von Palladium als Katalysator bevorzugt. Die Art der Phosphinliganden ist dabei von untergeordneter Bedeutung. Geeignet sind z. B. Liganden der allgemeinen Formeln: 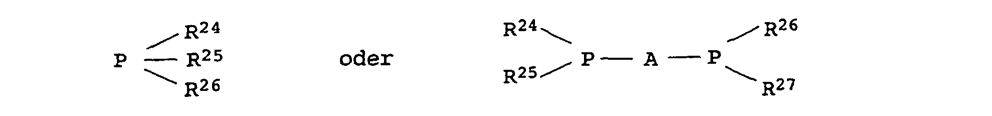 wobei die Reste R²⁴ bis R²⁶ für niedermolekulares Alkyl, z.B. C₁-C₆-Alkyl, Aryl, C₁-C₄-Alkylaryl, z. B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste. A steht für einen zweiwertigen organischen Rest, z. B. C₁-C₄-Alkylen, 1,2-Cycloalkylen, α,α'-Ferrocendiyl, α,α-Biphenyl oder vergleichbare bifunktionelle Gruppen.

Die Herstellung der Phosphinkomplexe kann in an sich bekannter Weise, z. B. wie in den eingangs genannten Dokumenten beschrieben erfolgen. Beispielsweise geht man von üblichen kommerziell erhältlichen Metallsalzen wie Palladium(II)-chlorid oder Palladium(II)-acetat aus und fügt das Phosphin z.B. P(C₆H₅)₃, P(n-C₄H₉)₃, PCH₃(C₆H₅)₂, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Moläquivalente, besonders bevorzugt 1 bis 5 Moläquivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff IVa einsetzen.

Zur Herstellung der Benzazoloncarbonsäure IVa führt man die Umsetzung mit Kohlenmonoxid und mindestens äquimolaren Mengen an Wasser, bezogen auf das in Schritt d) erhaltene Bromid durch. Der Reaktionspartner Wasser kann gleichzeitig auch als Lösungsmittel dienen, d. h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether, z.B. Methyl-tert.-butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base, im Überschuss, so dass kein zusätzliches Lösungsmittel erforderlich ist.

Für das Verfahren geeignete Basen sind alle inerten Basen, die den bei der Umsetzung freiwerdenden Iodwasserstoff bzw. Bromwasserstoff zu binden vermögen. Beispielsweise sind hier tertiäre Amine wie tert.-Alkylamine, z. B. Trialkylamine wie Triethylamin, cyclische Amine wie N-Methylpiperidin oder N,N'-Dimethylpiperazin, Pyridin, Alkali- oder -hydrogencarbonate, oder tetraalkylsubstituierte Harnstoffderivate wie Tetra-C₁-C₄-alkylharnstoff, z. B. Tetramethylharnstoff, zu nennen.

Die Menge an Base ist nicht kritisch. Üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf das Bromid vorliegt. Vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 °C bis 250 °C, insbesondere bei 30 °C bis 150 °C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Selbstverständlich kann auch zunächst der Reaktionsschritt e) und anschließend der Reaktionsschritt d) durchgeführt werden.

Die als Ausgangsmaterialien für die Synthese der Benzazoloncarbonsäuren IVa verwendeten in 3-Position substituierten 2-Nitroaniline bzw. 2-Nitrophenole der Formel VIII sind bekannt und können nach an sich bekannten Verfahren hergestellt werden. Alternativ können die Verbindungen IX, worin A für Sauerstoff steht, auf die später im Schema 7 aufgezeigte Weise hergestellt werden.

Ein weiterer allgemeiner Zugang zu Benzazoloncarbonsäuren der Formel IVa wird in Schema 2 gezeigt.

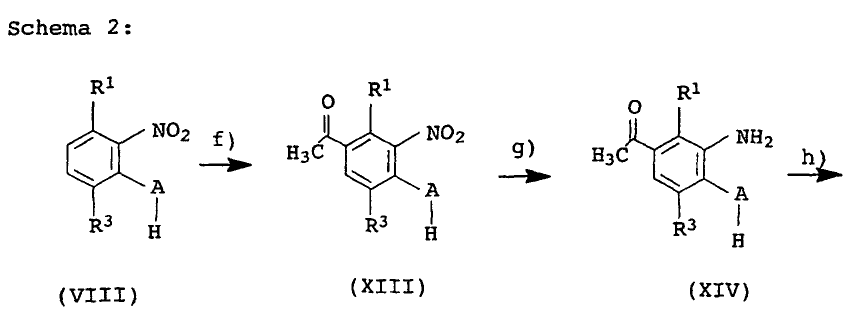

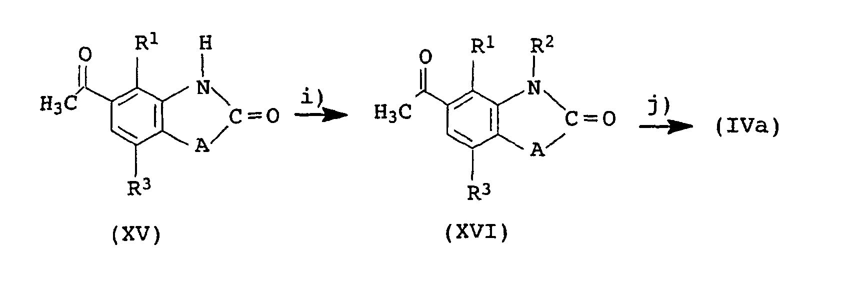

Ausgehend von den in 3-Position substituierten 2-Nitroanilinen bzw. 2-Nitrophenolen der Formel VIII lässt sich durch Friedel-Crafts-Acylierung in Schritt f) das substituierte Acetophenon XIII herstellen. Die Acetylgruppe lässt sich in bekannter Weise durch Umsetzung der Verbindung VIII mit Essigsäure oder aktivierter Essigsäure, wie Essigsäureanhydrid oder Acetylchlorid, in Gegenwart einer Lewis-Säure wie Aluminiumtrichlorid, Bortrifluorid oder Trifluoressigsäure unter wasserfreien Bedingungen einführen. Üblicherweise benötigt man mehr als 1 Mol Lewis-Säure pro Mol entstehendes Keton, da das entstehende Keton die Lewis-Säure komplex bindet. Nach beendeter Reaktion wird dieser Komplex hydrolytisch gespalten. Üblicherweise führt man Friedel-Crafts-Acylierungen in einem Lösungsmittel durch. Geeignete Lösungsmittel sind die vorgenannten cyclischen und acyclischen Kohlenwasserstoffe, die vorgenannten Halogenkohlenwasserstoffe, aromatische Kohlenwasserstoffe wie Nitrobenzol oder die vorgenannten Ether. Die Reaktionstemperaturen liegen in der Regel im Bereich von 0 °C bis 150 °C und vorzugsweise im Bereich von 20 °C bis 120 °C. Verfahren zur Einführung von Acylgruppen sind bekannt, siehe beispielsweise Organikum, 16. Aufl. 1986, S. 325.

Das substituierte Acetophenon XIII wird dann in Schritt g) zur Aminoverbindung XIV reduziert. Die Reduktion erfolgt auf die in Schritt b) im Schema 1 beschriebene Weise. Danach wird das Anilin-Derivat XIV in Schritt h) auf die in Schritt c) in Schema 1 beschriebene Weise phosgeniert. Der Substituent R² lässt sich analog zu schritt d) in Schema 1 einführen (Schritt i) in Schema 2).

Die Umwandlung der in Schritt i) in Schema 2 erhaltenen Verbindung XVI in die Benzazoloncarbonsäure IVa gelingt mit Hilfe der Haloform-Reaktion. Hierfür lässt man ein Halogenierungsmittel, wie Hypohalogenit, beispielsweise Hypochlorit, oder Chlor in alkalischer Lösung auf die Verbindung XVI einwirken. Es bildet sich zunächst ein Trihalogenmethylcarbonyl-Derivat, das unter den alkalischen Reaktionsbedingungen unter Bildung der erwünschten Benzazoloncarbonsäure IVa hydrolytisch gespalten wird. Geeignete Basen sind insbesondere Alkalihydroxide, wie Natrium- oder Kaliumhydroxid. Üblicherweise führt man die Reaktion in Lösung durch. Als Lösungsmittel kommen insbesondere Wasser, Gemische aus Wasser und organischen Lösungsmitteln, wie C₁-C₄-Alkoholen, beispielsweise Methanol, Ethanol, Propanol, Butanol, oder die vorgenannten Ether in Betracht. Übliche Reaktionstemperaturen liegen im Bereich von 0 °C bis 150 °C, vorzugsweise im Bereich von 20 °C bis 120 °C. Zur Haloform-Reaktion, siehe beispielsweise Organikum, 16. Aufl. 1986, S. 375.

Sofern A in Formel IVa für Schwefel steht, können die Benzazoloncarbonsäuren der allgemeinen Formel IVa auch auf die in Schema 3 aufgezeigte Weise hergestellt werden.

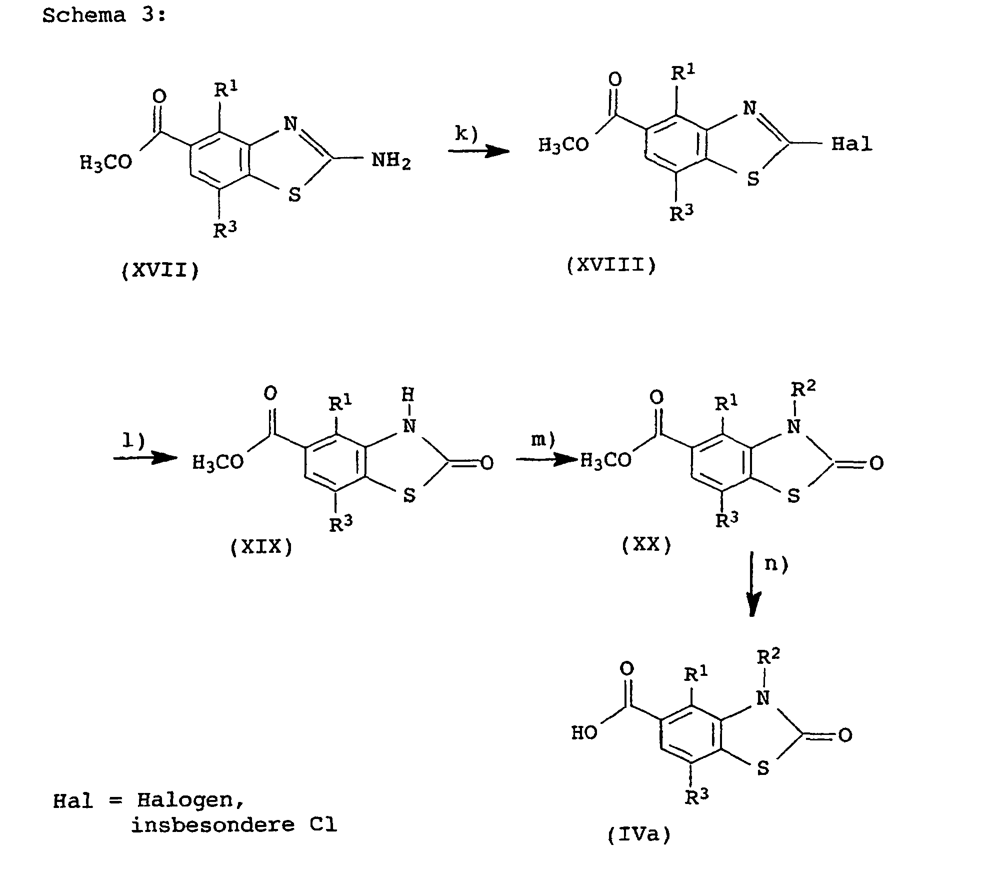

Die Benzazoloncarbonsäuren der allgemeinen Formel IVa mit A = Schwefel lassen sich gemäß Schema 3 ausgehend von den Aminobenzothiazolen XVII herstellen. Die Überführung des 2-Aminobenzothiazols XVII in die 2-Halogenbenzothiazolverbindung XVIII gelingt in bekannter Weise unter Sandmeyer-Bedingungen (Schritt k)). Auf diese Weise lassen sich weitere Funktionalitäten in der 2-Position des Benzothiazolrings einführen.

Hierfür setzt man zunächst das 2-Aminobenzothiazol XVII mit anorganischem oder organischem Nitrit, wie Natriumnitrit in Gegenwart von Säure, wie Salzsäure oder mit tert.-Butylnitrit um. Das dabei erhaltene Diazoniumsalz wird dann mit einem anorganischen Halogenid, wie Natriumchlorid unter Zusatz von Kupfer oder mit einem Cu(I)-Halogenid, wie Cu(I)-chlorid umgesetzt. Die Umsetzung erfolgt in der Regel bei Temperaturen im Bereich von 0 °C bis 150°C und vorzugsweise im Bereich von 20°C bis 100°C. Geeignete Lösungsmittel sind insbesondere Wasser oder Gemische aus Wasser mit organischen Lösungsmitteln wie die vorgenannten Alkohole oder Ether. Zur Herstellung aromatischer Halogenide, insbesondere Chloride, nach Sandmeyer siehe auch Organikum, 16. Aufl. 1986, S. 545.

Die 2-Halogenbenzothiazolverbindung XVIII kann man anschließend in Schritt 1) unter sauren oder alkalischen Bedingungen zum Benzothiazolon hydrolysieren. Hierfür versetzt man die Verbindung XVIII mit einer Base, wie Alkali- oder Erdalkalihydroxid, beispielsweise Natrium-, Kalium- oder Magnesiumhydroxid, oder Alkalialkoholat wie Natrium- oder Kaliummethylat oder mit einer Säure wie Salzsäure. Üblicherweise führt man die Hydrolyse in einem Lösungsmittel durch. Geeignete Lösungsmittel sind in Abhängigkeit von der verwendeten Base polar aprotische Lösungsmittel, wie Dimethylformamid, Dimethylsulfoxid, N-Methylpyrrolidon oder Acetonitril, Ether wie Tetrahydrofuran oder Dioxan, Wasser und Gemische aus Wasser mit den vorgenannten Alkoholen, Ethern oder polar aprotischen Lösungsmitteln. Die Hydrolyse erfolgt in der Regel bei 0 °C bis 150 °C und vorzugsweise bei 20 °C bis 120 °C. Zur Hydrolyse von Halogen-Heteroaromaten zu Ketoverbindungen siehe auch J. Med. Chem. 1977, 20, No. 6, 791-796.

Die Einführung des Substituenten R² in die Verbindung XIX im Schritt m) erfolgt wie im Schritt d) in Schema 1 beschrieben. Für die Verseifung im Schritt n) setzt man beispielsweise den in Schritt m) erhaltenen Benzazolonmethylester XX mit Alkalihydroxid, z. B. Lithium-, Natrium- oder Kaliumhydroxid, mit Erdalkalihydroxid wie Magnesiumhydroxid oder mit Alkaliiodiden wie Natriumiodid in einem geeigneten Lösungsmittel um, wobei vorzugsweise in Abwesenheit von Sauerstoff gearbeitet wird. Übliche Reaktionstemperaturen liegen im Bereich von 0 °C bis 200 °C und insbesondere im Bereich von 20 °C bis 180 °C. Geeignete Lösungsmittel sind die vorgenannten aliphatischen oder cycloaliphatischen Kohlenwassserstoffe, die halogenierten Kohlenwasserstoffen, die aromatischen Kohlenwasserstoffe, die vorgenannten Ether und Alkohole, wässrige, einphasige Systeme sowie Pyridin. Zur Verseifung siehe beispielsweise Organikum, 16, Aufl. 1986, S. 415, Mc Murry, Org. React. 1976, 24, 187; Taschner et al., Rocz. Chem. 1956, 30, 323; Houben-Weyl: "Methoden der organischen Chemie", Band E 8 b 1994; S. 1010 f.; J.Chem. Soc. Perkin Trans., Part 1, 1976, No.12, 1291-1296; insbesondere A. R. Katritzky et al., J. Heterocycl. Chem., 30 (1) 135-139, 1993. Die Synthese des Ausgangsmaterials XVII ist in der PCT/EP 00/04042 und PCT/EP 00/04040 beschrieben.

Ein weiterer Zugang zu den Verbindungen der allgemeinen Formel IVa ist in Schema 4 aufgezeigt.

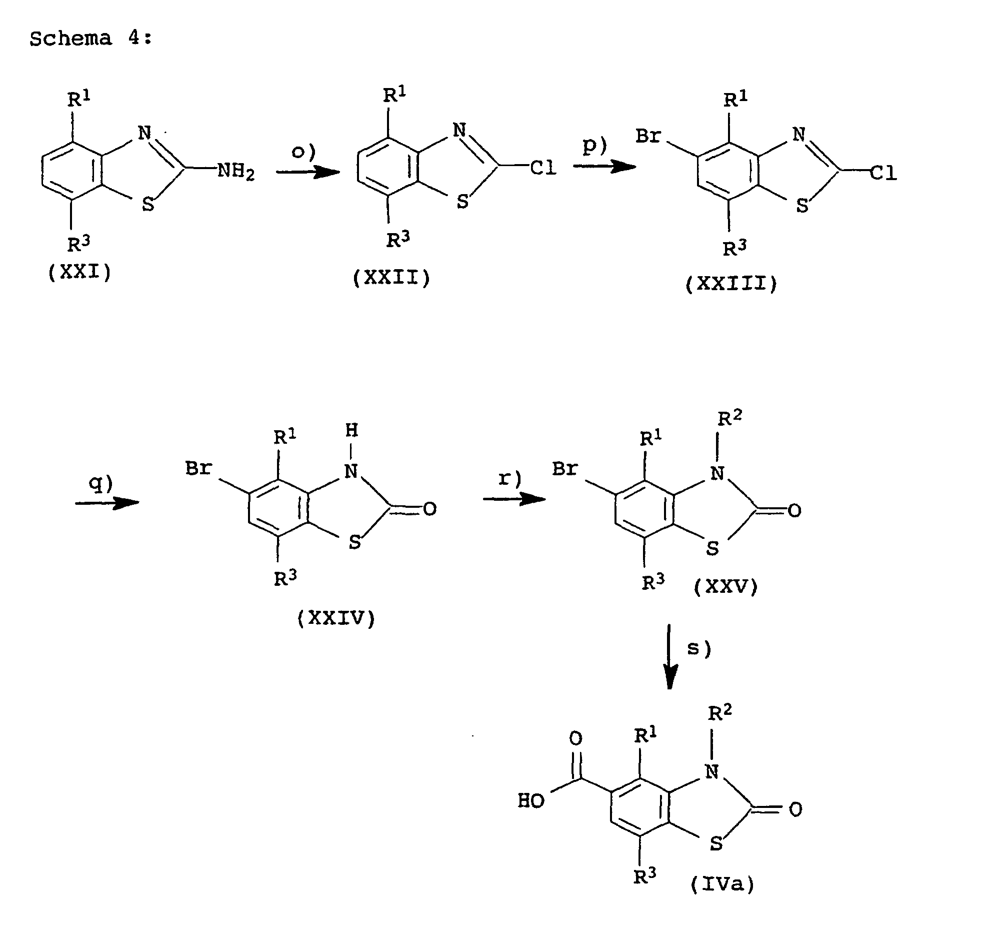

Ausgehend von den an sich bekannten 2-Aminobenzothiazolen der allgemeinen Formel XXI lassen sich in einer Sandmeyer-Reaktion im Schritt o) die 2-Chlorbenzothiazolverbindungen XXII herstellen.

Die hierfür erforderlichen Reaktionsbedingungen entsprechen denen von Schritt k) in Schema 3. Das 2-Chlorbenzothiazol XXII lässt sich in analoger Weise zu Schritt a) in Schema 1 bromieren, wobei der Bromsubstituent selektiv in ortho-Position zu R¹ eintritt (Schritt p)). Die Verbindung XXIII wird anschließend unter den für Schritt l) in Schema 3 angegebenen Bedingungen basisch hydrolysiert (Schritt q)). Im Schritt r) lässt sich der Substituent R² unter den für Schritt d) in Schema 1 beschriebenen Bedingungen in XXIV einführen. Die Einführung der Carboxygruppe (Schritt s)) erfolgt gemäß den in Schritt e) in Schema 1 beschriebenen Reaktionsbedingungen.

Eine Variante zur Herstellung der Benzothiazoloncarbonsäure IVa (A = S) ist in Schema 5 dargestellt.

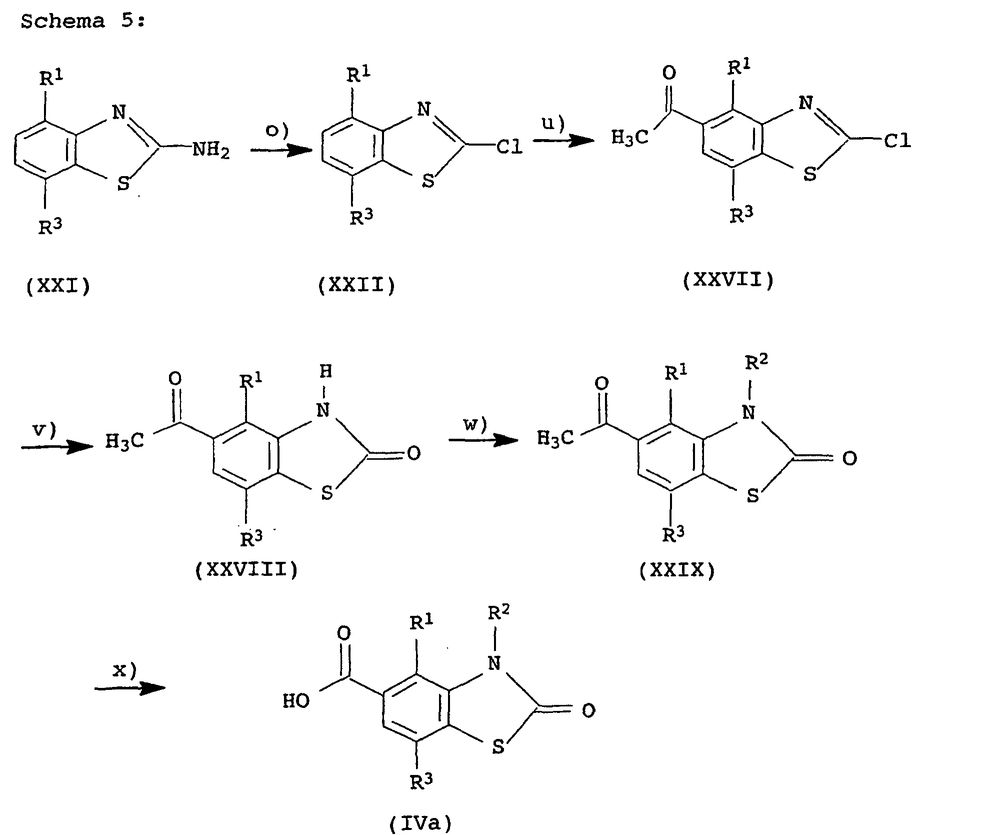

Wiederum geht man zunächst von substituierten 2-Aminobenzothiazolen XXI aus, die in der zuvor beschriebenen Weise in die 2-Chlorbenzothiazole XXII überführt werden (Schritt o), siehe Schema 3). Die 2-Chlorbenzothiazole XXII werden danach in Schritt u) unter den in Schritt f) in Schema 2 angegebenen Bedingungen einer Friedel-Crafts-Acylierung unterworfen, wobei man die Verbindung XXVII erhält. Die Hydrolyse von XXVII erfolgt in anologer Weise wie für Reaktionsschritt q) in Schema 4 beschrieben. Die Einführung des Substituenten R² in XXVIII in Schritt w) erfolgt in bekannter Weise, wie beispielsweise in Schritt d) in Schema 1 beschrieben. Die abschließende Haloform-Reaktion in Schritt x) unter den in Schritt j) in Schema 2 angegebenen Bedingungen überführt das substituierte Acetophenon XXIX unter Verlust eines Kohlenstoffatoms in die erwünschte Benzazoloncarbonsäure IVa.

Ein anderer Zugang zu Benzazoloncarbonsäuren der allgemeinen Formel IVa (A = Schwefel) ist in Schema 6 angegeben.

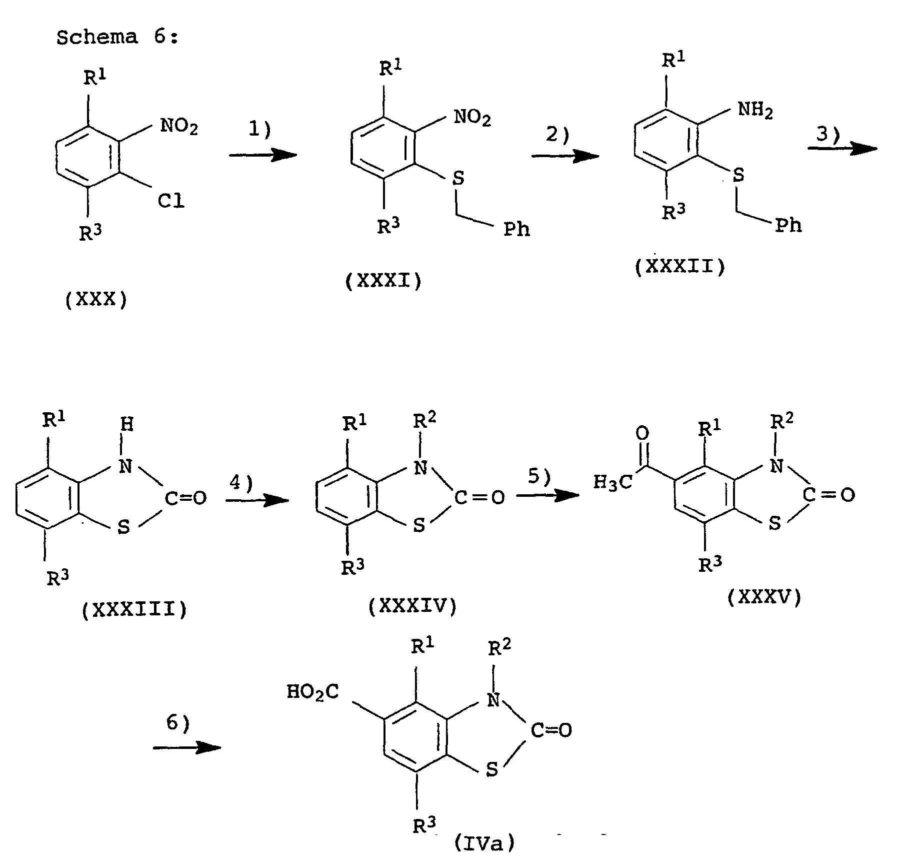

Die an sich bekannten o-Chlornitrobenzole der allgemeinen Formel XXX werden zunächst mit Alkalisalzen des Benzylmercaptans in die entsprechenden Thioether XXXI überführt (Schritt 1)). Die Substitution erfolgt üblicherweise in einem der vorgenannten aliphatischen oder cycloaliphatischen Kohlenwasserstoffe, halogenierten Kohlenwasserstoffe, aromatischen Kohlenwasserstoffe, Ether, Dimethylformamid, NMP, Sulfolan oder Dimethylsulfoxid. Übliche Reaktionstemperaturen liegen im Bereich von 0 °C bis 250°C und vorzugsweise im Bereich von 50°C bis 175°C. Verfahren für die nucleophile Substitution sind bekannt, siehe auch A. Bagno et al., J. Chem. Soc. Perkin Trans II, 1991 (5), 651-655; J. R. Beck et al., J. Org. Chem., 1978, 43, No. 10, 2048-2052.

Die Verbindung XXXI wird danach in Schritt 2) zur Aminoverbindung XXXII reduziert. Die hierfür erforderlichen Reaktionsbedingungen entsprechen denen für Schritt b) in Schema 1. Anschließend wird die Aminoverbindung XXXII unter den in Schritt c) in Schema 1 beschriebenen Reaktionsbedingungen mit Kohlensäureäquivalenten zum Benzothiazolon XXXIII cyclisiert (Schritt 3)). Die anschließende Einführung des Substituenten R² gelingt unter den in Schritt d) in Schema 1 beschriebenen Reaktionsbedingungen (Schritt 4)). Durch Friedel-Crafts-Acylierung der Verbindung XXXIV gelingt die Einführung der Acetylgruppe in ortho-Position zum Substituenten R¹ (Schritt 5)). Die hierfür erforderlichen Reaktionsbedingungen entsprechen denen des Schritts f) in Schema 2. Die erwünschte Benzazoloncarbonsäure IVa ist aus der Verbindung XXXV durch Haloform-Reaktion erhältlich (Schritt 6)). Die herfür erforderlichen Reaktionsbedingungen entsprechen denen in Schritt j) in Schema 2.

In ähnlicher Weise wie in Schema 1 können in einer Variante dieses Verfahrens Verbindungen der allgemeinen Formel IVa mit A = Schwefel ausgehend von den o-Chlornitrobenzolen XXX erhalten werden, wenn man den in Schritt 1) erhaltenen Thioether XXXI zunächst bromiert. Die hierfür erforderlichen Reaktionsbedingungen entsprechen denen des Schritts a) in Schema 1. Die anschließende Reduktion der Nitrogruppe zur Aminogruppe erfolgt unter den in Schritt b) in Schema 1 genannten Bedingungen. Die Kondensation mit Kohlensäureäquivalenten liefert die Benzothiazolone. Die hierfür erforderlichen Reaktionsbedingungen entsprechen denen für Schritt c) in Schema 1. Der Substituent R² wird auf die übliche Weise, analog zu Schritt d) in Schema 1, eingeführt. Die Einführung der Carboxygruppe erfolgt auf die in Schritt e) in Schema 1 beschriebene Vorgehensweise.

Ein weiterer Zugang für Benzazoloncarbonsäuren mit A = Sauerstoff wird in Schema 7 gezeigt.

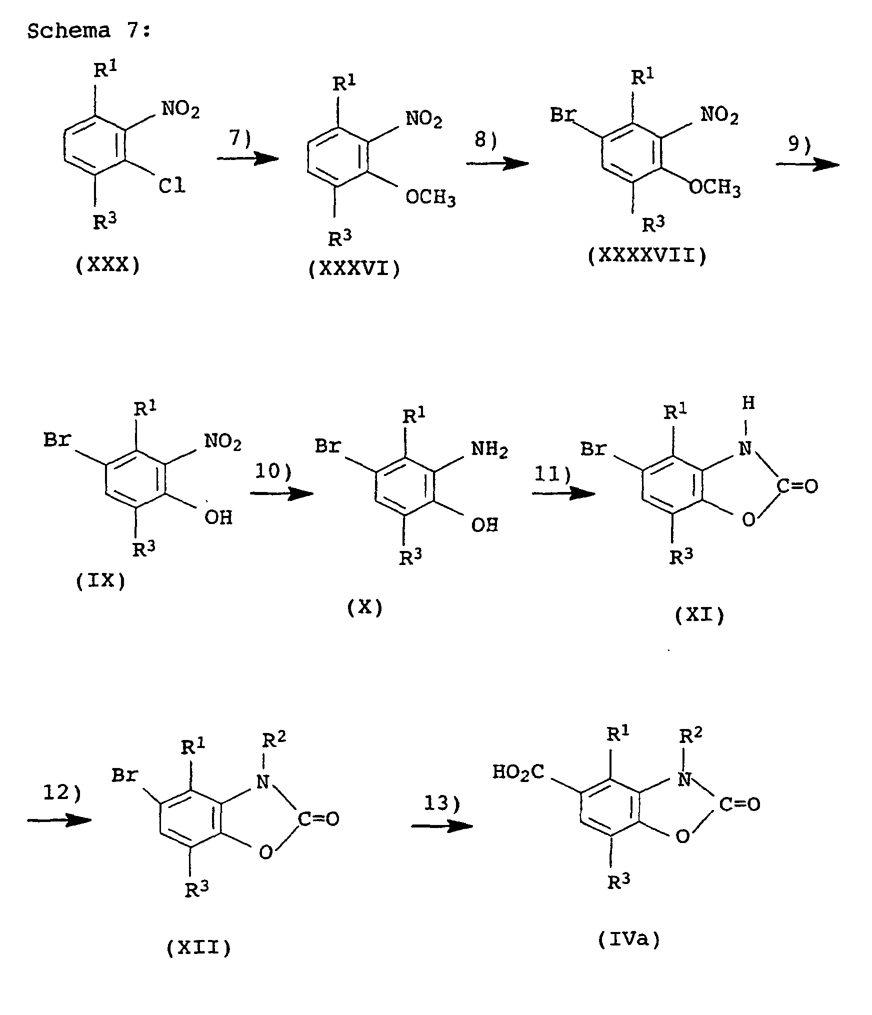

Gemäß Schema 7 kann man beispielsweise o-Chlornitrobenzole der allgemeinen Formel XXX mit Alkalisalzen von Alkoholaten in die entsprechenden o-Nitroether XXXVI umwandeln (Schritt 7)). Diese nucleophile Substitution am Aromaten erfolgt in der Regel unter Ausschluss von Wasser und wird üblicherweise in einem Lösungsmittel durchgeführt. Geeignete Lösungsmittel sind insbesondere die vorgenannten aliphatischen oder cycloaliphatischen Kohlenwasserstoffe, halogenierte Kohlenwasserstoffe, aromatischen Kohlenwasserstoffe, Ether, DMF, NMP, Sulfolan oder Dimethylsulfoxid. In der Regel liegen die erforderlichen Reaktionstemperaturen im Bereich von 0 °C bis 250 °C und vorzugsweise bei 50 °C bis 175 °C. Verfahren zur Herstellung von aromatischen Ethern aus o-Nitrochlorverbindungen sind bekannt, siehe auch A. Bagno et al., J.Chem. Soc. Perkin Trans II, 1991 (5), 651-655; J. R. Beck et al., J. Org. Chem., 1978, 43, No. 10, 2048-2052.

Das erhaltene o-Nitroanisol XXXVI wird unter den in Schritt a) in Schema 1 angegebenen Reaktionsbedingungen bromiert, wobei das Bromatom selektiv in para-Position zur Methoxygruppe eintritt. (Schritt 8)).

Anschließend wird die Hydroxygruppe durch Etherspaltung in Schritt 9) unter sauren Reaktionsbedingungen in Gegenwart einer Lewis-Säure, wie Aluminiumtrichlorid, Aluminiumtribromid oder Halogenwasserstoffsäuren, wie Iodwasserstoffsäure oder Bromwasserstoffsäure freigesetzt, wobei man 4-Brom-2-nitrophenole der Formel IX erhält. Üblicherweise führt man die Etherspaltung in einem Lösungsmittel durch. Geeignete Lösungsmittel sind die vorgenannten acylischen und cyclischen Kohlenwasserstoffe, halogenierte Kohlenwasserstoffe, aromatische Kohlenwasserstoffe oder Säuren, wie Essigsäure. In der Regel erfolgt die Etherspaltung bei Temperaturen im Bereich von -15 °C bis 150 °C, vorzugsweise im Bereich von 0 °C bis 100 °C. Zur Spaltung von Phenolethern siehe auch Organikum, 16. Aufl. 1986, S. 192.

Die weiteren Reaktionsschritte 10) bis 13) in Schema 7 entsprechen den Reaktionsschritten b) bis e) in Schema 1.

Die folgenden Beispiele sollen die Erfindung erläutern.

### Beispiel 1: Herstellung von 3,4-Dimethyl-5-(5'-hydroxy-1'-methyl-pyrazol-4-yl)-carbonyl-benzothiazol-2-on (Verbindung I-1a.1129)

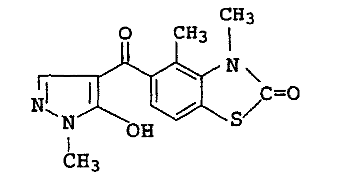

### 1.1 2-Chlor-4-methylbenzothiazol-5-carbonsäuremethylester

Zu einer Lösung von 5 g (22,5 mmol) 2-Amino-4-methylbenzothiazol-5-carbonsäuremethylester in 2 L Acetonitril gab man 10 mL Wasser, 4,5 g (44,8 mmol) Kupfer(I)-chlorid, 6,6 g Natriumchlorid (110 mmol) und 2 mL 15-Krone-5. Anschließend tropfte man unter Rühren eine Lösung von 3 g (29 mmol) tert.-Butylnitrit zu, erhitzte die Lösung 15 h zum Rückfluss, filtrierte den ausgefallenen Niederschlag ab und engte die Lösung im Vakuum ein. Anschließend extrahierte man den Rückstand dreimal mit je 500 mL Essigester. Hierzu erhitzte man das LÖsungsmittel bis zum Siedepunkt und filtrierte die Lösung heiß. Die Extrakte wurden im Vakuum eingeengt. Den Rückstand reinigte man durch Ausrühren mit n-Hexan/Diethylether. Man isolierte 4,2 g (17,4 mmol, 77 % Ausbeute) 2-Chlor-4-methylbenzothiazol-5-carbonsäuremethylester mit Schmp. 112 °C.
¹H-NMR (CDCl₃): δ (ppm) = 2,98 (s, 3H), 3,94 (s, 3H), 7,64 (d, 1H), 7,95 (d, 1H).

### 1.2 4-Methylbenzothiazol-2-on-5-carbonsäuremethylester

Zu einer Lösung von 22,5 g (93 mmol) 2-Chlor-4-methyl-benzothiazol-5-carbonsäuremethylester in 360 mL N-Methylpyrrolidon gab man 13,6 g (186 mmol) Kaliummethylat und erwärmte 5 Stunden auf 100 °C. Nach dem Abkühlen saugte man den ausgefallenen Niederschlag ab und versetzte das Filtrat mit Wasser. Anschließend stellte man den pH-Wert der Reaktionslösung auf pH 1 ein, extrahierte die wässrige Lösung dreimal mit Ethylacetat, wusch und trocknete die vereinigten organischen Phasen. Man entfernte das Lösungsmittel im Vakuum und rührte zur Reinigung den Rückstand mit Methylenchlorid/n-Hexan aus. Man erhielt in 55%iger Ausbeute 11,38 g (51 mmol) 4-Methylbenzothiazol-2-on-5-carbonsäuremethylester mit Schmp. 262°C bis 266 °C.
¹H-NMR (CDCl₃): δ (ppm) = 2,64 (s, 3H), 3,91 (s, 3H), 7,28 (d, 1H), 7,74 (d, 1H), 9,94 (br s, 1 H, NH).

### 1.3 3,4-Dimethylbenzothiazol-2-on-5-carbonsäuremethylester

Zu einer Lösung von 11,38 g (51 mmol) 4-Methylbenzothiazol-2-on-5-carbonsäuremethylester in 400 mL Aceton fügte man 14,18 g Kaliumcarbonat. Anschließend tropfte man unter Rühren 5,1 mL (54 mmol) Dimethylsulfat zu und rührte 78 Stunden bei 23 °C. Man filtrierte vom ausgefallenen Feststoff ab und entfernte das Lösungsmittel im Vakuum. Umkristallisation aus Ether ergab in 84%iger Ausbeute 10,15 g (43 mmol) 3,4-Dimethylbenzothiazol-2-on-5-carbonsäuremethylester mit Schmp. 97 °C bis 100 °C.
¹H-NMR (CDCl₃): δ (ppm) = 2,78 (s, 3H), 3,77 (s, 3H), 3,92 (s, 3H), 7,28 (d, 1H), 7,36 (d, 1H).

### 1.4 3,4-Dimethylbenzothiazol-2-on-5-carbonsäure

Man versetzte eine Lösung von 10,15 g (43 mmol) 3,4-Dimethylbenzothiazol-2-on-5-carbonsäuremethylester in 100 mL Tetrahydrofuran und 100 mL Wasser mit 2,06 g (86 mmol) Lithiumhydroxid. Anschließend erhitzte man die Lösung 2 Stunden zum Rückfluss. Nach dem Abkühlen entfernte man das Tetrahydrofuran im Vakuum, säuerte die verbliebene wässrige Lösung an, saugte den ausgefallenen Niederschlag ab und trocknete ihn. Weiteres Produkt wurde nach Extraktion der Mutterlauge mit Essigester erhalten. Insgesamt isolierte man 8 g (36 mmol, 83 % Ausbeute) 3,4-Dimethylbenzothiazol-2-on-5-carbonsäure mit Schmp. 235 °C bis 239 °C.
¹H-NMR (DMSO-D₆): δ (ppm) = 2,77 (s, 3H), 3,66 (s, 3H), 7,42 (d, 1H), 7,56 (d, 1H).

### 1.5 3,4-Dimethyl-benzothiazol-2-on-5-carbonsäure-1'-methyl-pyrazol-5'-yl-ester

Man löste 0,94 g (4 mmol) 3,4-Dimethylbenzothiazol-2-on-5-carbonsäure, 0,43 g 5-Hydroxy-1-methylpyrazol und 0,81 g Ethyl-(3'-dimethylaminopropyl)carbodiimid in 40 mL Acetonitril und rührte die Lösung 10 Stunden bei 23 °C. Nach beendeter Reaktion filtrierte man vom ausgefallenen Niederschlag ab und engte die Lösung im Vakuum ein. Man nahm den Rückstand in Wasser auf und extrahierte mit Methylenchlorid. Nach Waschen und Trocknen der organischen Phase entfernte man das Lösungsmittel. Man isolierte 0,72 g (2,4 mmol, 56 % Ausbeute) 3,4-Dimethylbenzothiazol-2-on-5-carbonsäure-1'-methyl-pyrazol-5'-ylester mit Schmp. 168 °C bis 174 °C.
¹H-NMR (CDCl₃): δ (ppm) = 2,88 (s, 3H), 3,80 (s, 3H), 3,82 (s, 3H), 6,23 (s, 1H), 7,39 (d, 1H), 7,47 (s, 1H), 7,82 (d, 1H).

### 1.6 3,4-Dimethyl-5-(5'-hydroxy-1'-methyl-pyrazol-4-yl)-carbonylbenzothiazol-2-on

Zu einer Lösung von 0,61 g (2 mmol) 3,4-Dimethylbenzothiazol-2-on-5-carbonsäure-1'-methylpyrazol-5'-yl-ester in 40 mL Dimethoxyethan gab man unter Rühren 1,41 g Kaliumcarbonat. Man erwärmte die Reaktionsmischung 72 Stunden unter Rückfluss. Nach dem Entfernen des Lösungsmittels im Vakuum nahm man den Rückstand mit Wasser auf. Die wässrige Phase wurde zunächst bei pH 10,8 und 7,0 mit Methylenchlorid extrahiert. Nach Ansäuern auf pH 1 konnte das Produkt durch Extraktion mit Methylenchlorid erhalten werden. Nach dem Entfernen des Lösungsmittels erhielt man 0,52 g (1,7 mmol, 87 % Ausbeute) der Titelverbindung mit Schmp. 192°C bis 195 °C.
¹H-NMR (CDCl₃): δ (ppm) = 2,66 (s, 3H), 3,73 (s, 3H), 3,79 (s, 3H), 4,60 (m, 1H), 7,26 (d, 1H), 7,36 (d, 1H), 7,38 (s, 1H).

### Beispiel 2: Herstellung von 3,4-Dimethyl-5-(5'-hydroxy-1'-ethyl-pyrazol-4-yl)-carbonyl-benzothiazol-2-on (Verbindung I-1b.1129)

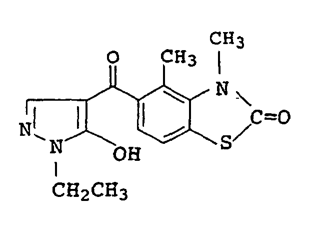

### 2.1 3,4-Dimethylbenzothiazol-2-on-5-carbonsäure-1'-ethyl-pyrazol-5'-yl-ester

Man führte die Umsetzung analog zu der im Beispiel 1.5 beschriebenen Vorgehensweise um, setzte aber 1,1 g (5 mmol) 3,4-Dimethylbenzothiazol-2-on-5-carbonsäure, 0,58 g Hydroxy-1-ethylpyrazol und 0,94 g Ethyl-(3'-dimethylaminopropyl)carbodiimid ein.

Man isolierte 0,55 g (1,7 mmol, 34 % Ausbeute) 3,4-Dimethylbenzothiazol-2-on-5-carbonsäure-1'-ethyl-pyrazol-5'-yl-ester mit Schmp. 122°C bis 124 °C.
¹H-NMR (CDCl₃): δ (ppm) = 1,42 (t, 3H), 2,88 (s, 3H), 3,90 (s, 3H), 4,12 (q, 2H), 6,23 (s, 1N), 7,39 (d, 1N), 7,50 (s, 1N), 7,79 (d, 1N).

### 2.2 3,4-Dimethyl-5-(5'-hydroxy-1'-ethyl-pyrazol-4-yl)-carbonylbenzothiazol-2-on

Man führte die Umsetzung analog zu der in Beispiel 1.6 beschriebenen Vorgehensweise aus, nur setzte man anstelle von 0,62 g (2 mmol) 3,4-Dimethylbenzothiazol-2-on-5-carbonsäure-1'-methylpyrazol-5'-ylester 0,53 g (1,7 mmol) 3,4-Dimethyl-benzothiazol-2-on-5-carbonsäure-1'-ethylpyrazol-5'-ylester ein.

Zur Reinigung der Titelverbindung rührte man das erhaltenen Rohprodukt mit Ether aus. Man isolierte 0,17 g (0,5 mmol, 32 % Ausbeute) mit Schmp. 147 °C bis 149 °C.
¹H-NMR (CDCl₃): δ (ppm) = 1,44 (t, 3H), 2,66 (s, 3H), 3,79 (s, 3H), 4,09 (q , 2H), 7,26 (d, 1H), 7,36 (d, 1H), 7,38 (s, 1H).

### Beispiel 3: Herstellung von 3,4-Dimethyl-5-(5'-hydroxy-1'-isopropylpyrazol-4-yl)-carbonylbenzothiazol-2-on (Verbindung I-1c.1129)

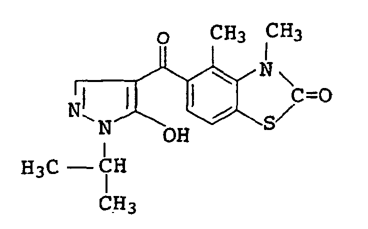

### 3.1 3,4-Dimethylbenzothiazol-2-on-5-carbonsäure-1'-isopropylpyrazol-5'-yl-ester

Man führte die Umsetzung analog zu der in Beispiel 1.5 beschriebenen Vorgehensweise aus.

Ausgehend von 1,1 g (5 mmol) 3,4-Dimethylbenzothiazol-2-on-5-carbonsäure, 0,65 g 5-Hydroxy-1-isopropylpyrazol und 0,94 g Ethyl-(3'-dimethylaminopropyl)carbodiimid isolierte man 0,65 g (2 mmol, 40 % Ausbeute) 3,4-Dimethylbenzothiazol-2-on-5-carbonsäure-1'-isopropylpyrazol-5'-ylester mit Schmp. 137°C bis 140 °C.
¹H-NMR (CDCl₃): δ (ppm) = 1,48 (d, 6H), 2,88 (s, 3H), 3,90 (s, 3H), 4,52 (m, 1H), 6,21 (s, 1H), 7,40 (d, 1H), 7,51 (s, 1H), 7,80 (d, 1H).

### 3.2 3,4-Dimethyl-5-(5'-hydroxy-1'-isopropylpyrazol-4-yl)-carbonylbenzothiazol-2-on

Man führte die Umsetzung analog zu der Vorgehensweise in Beispiel 1.6 aus, setzte aber anstelle von 3,4-Dimethylbenzothiazol-2-on-5-carbonsäure-1'-methyl-pyrazol-5_{'}-yl-ester 0,63 g (1,9 mmol) 3,4-Dimethylbenzothiazol-2-on-5-carbonsäure-1'-isopropylpyrazol-5'-ylester ein.

Man isolierte 0,39 g (1,2 mmol, 65 % Ausbeute) der Titelverbindung mit Schmp. 175°C bis 177°C.
¹H-NMR (CDCl₃): δ (ppm) = 1,68 (d, 6H), 2,66 (s, 3H), 3,79 (s, 3H), 4,60 (m, 1H), 7,26 (d, 1H), 7,36 (d, 1H), 7,38 (s, 1H).

In analoger Weise wurden die Verbindungen I der Beispiele 4 bis 47 durch analoge Umsetzung der jeweiligen Carbonsäure IVa mit dem 5-Hydroxypyrazol III und gegebenenfalls nachfolgende Derivatisierung der dabei erhaltenen Verbindung I hergestellt.

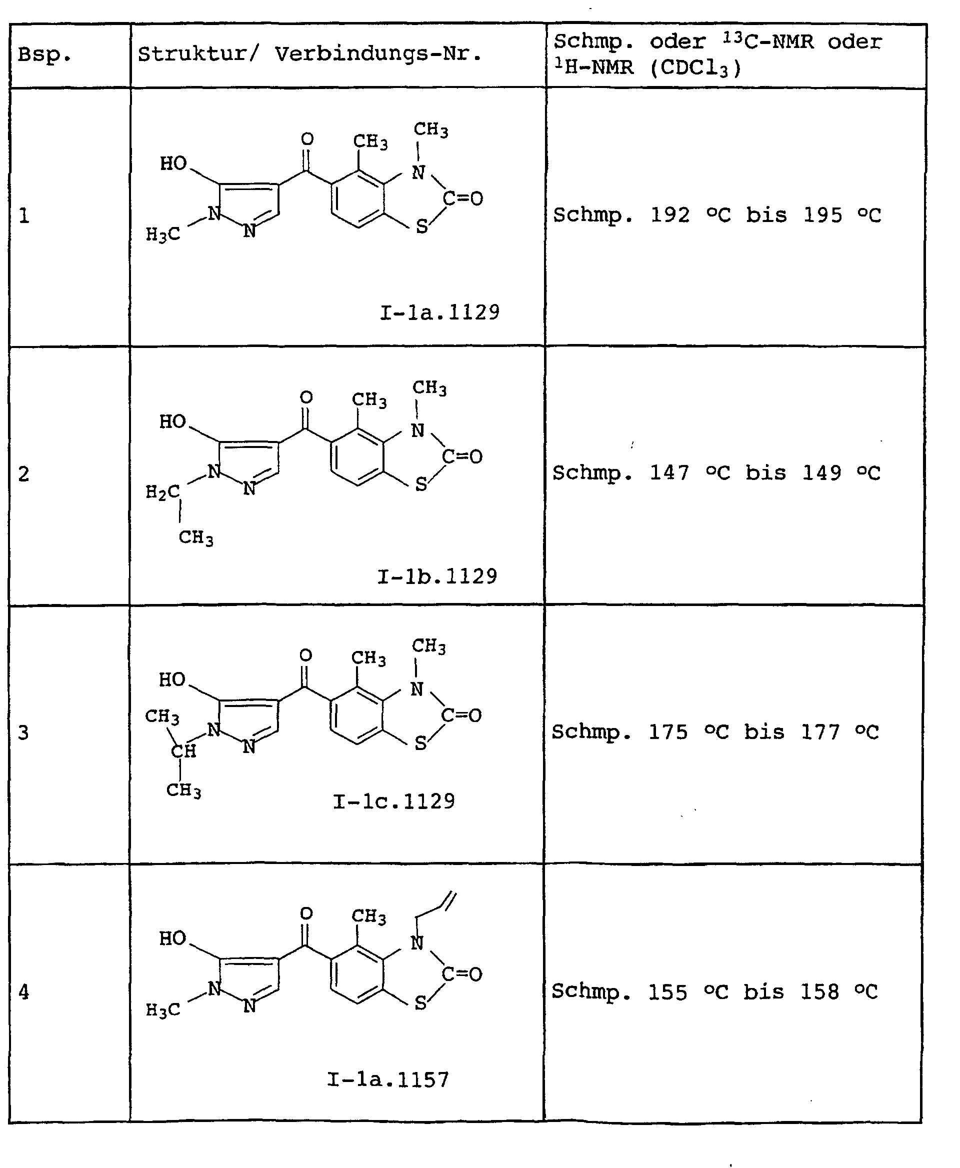

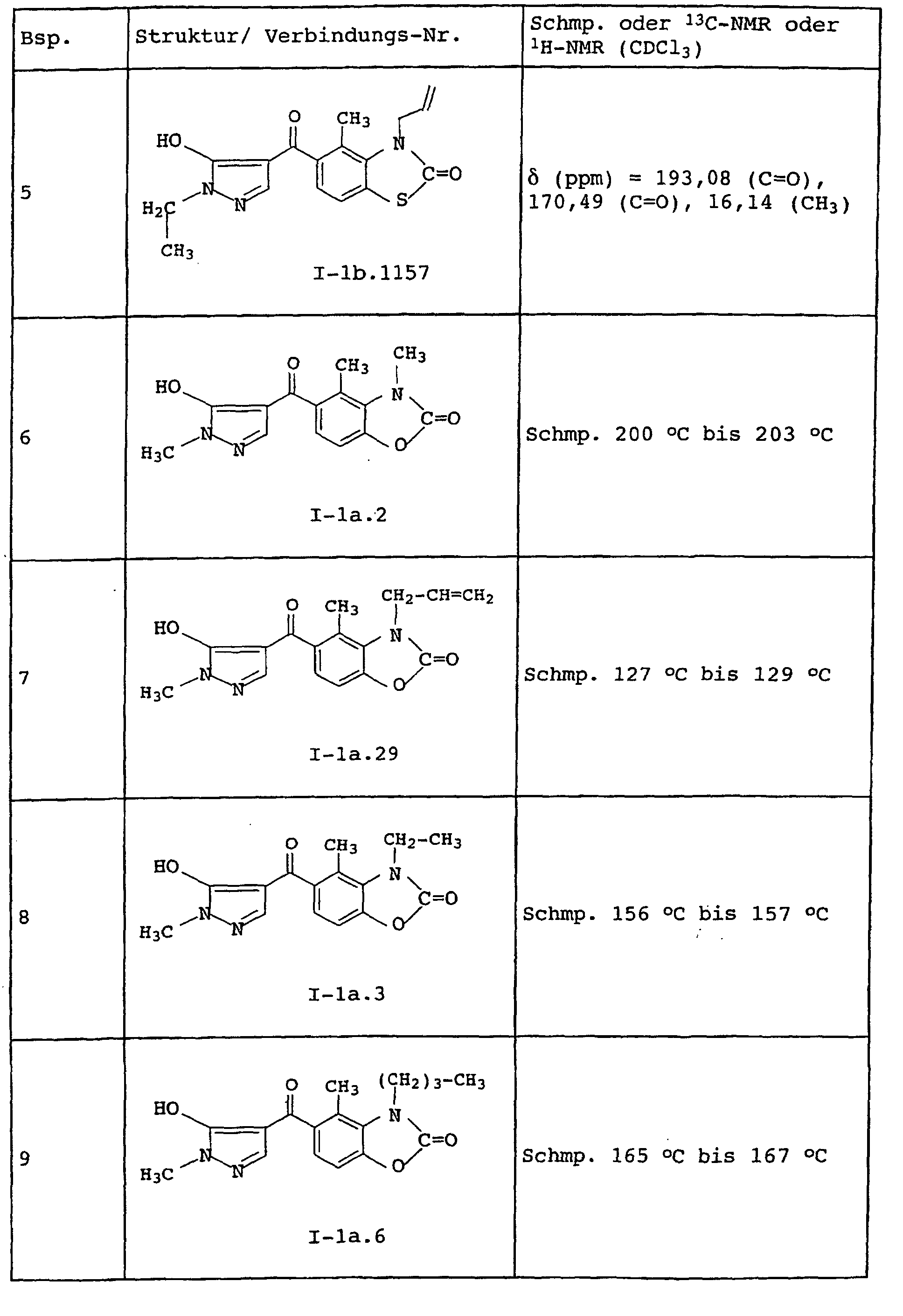

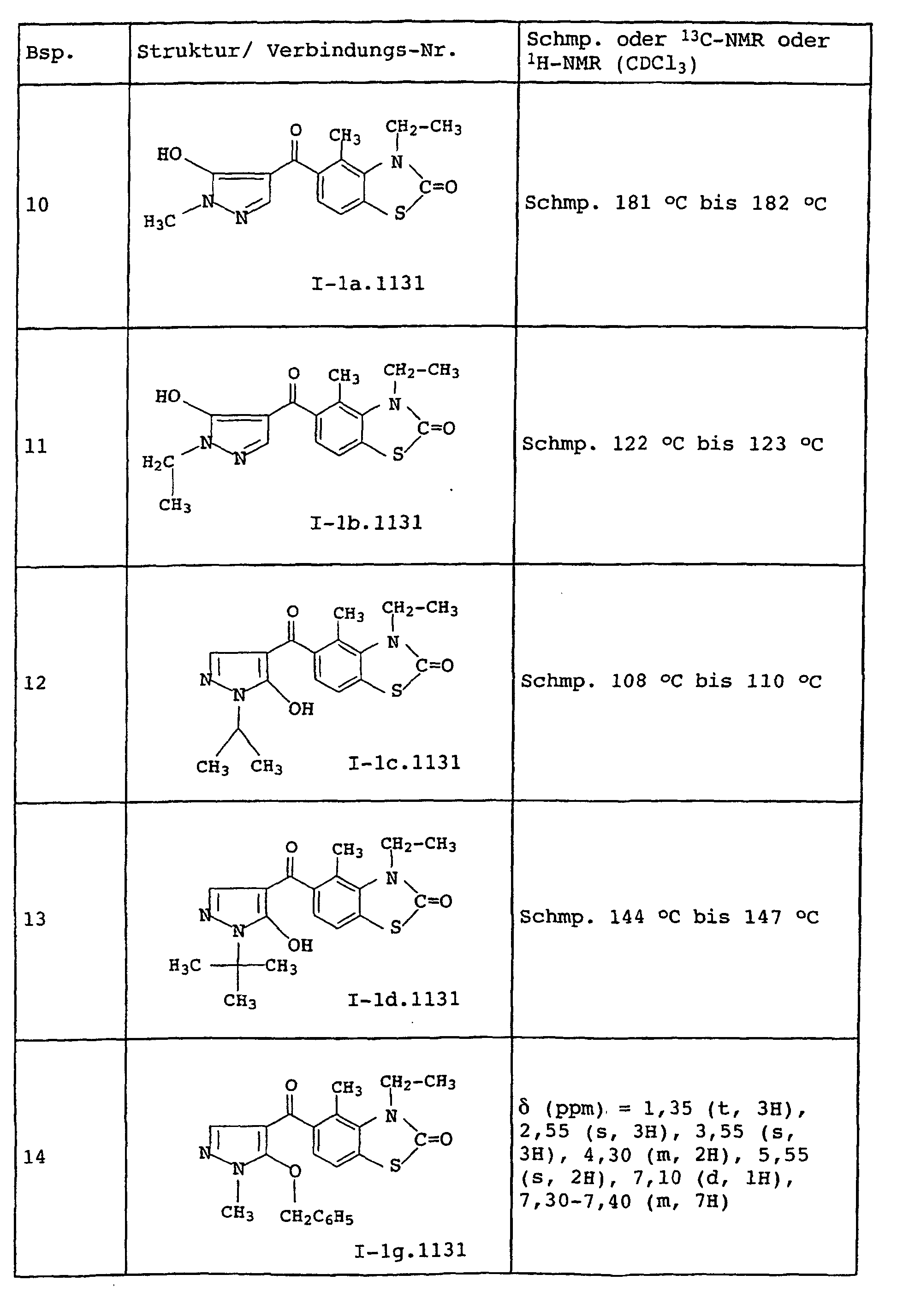

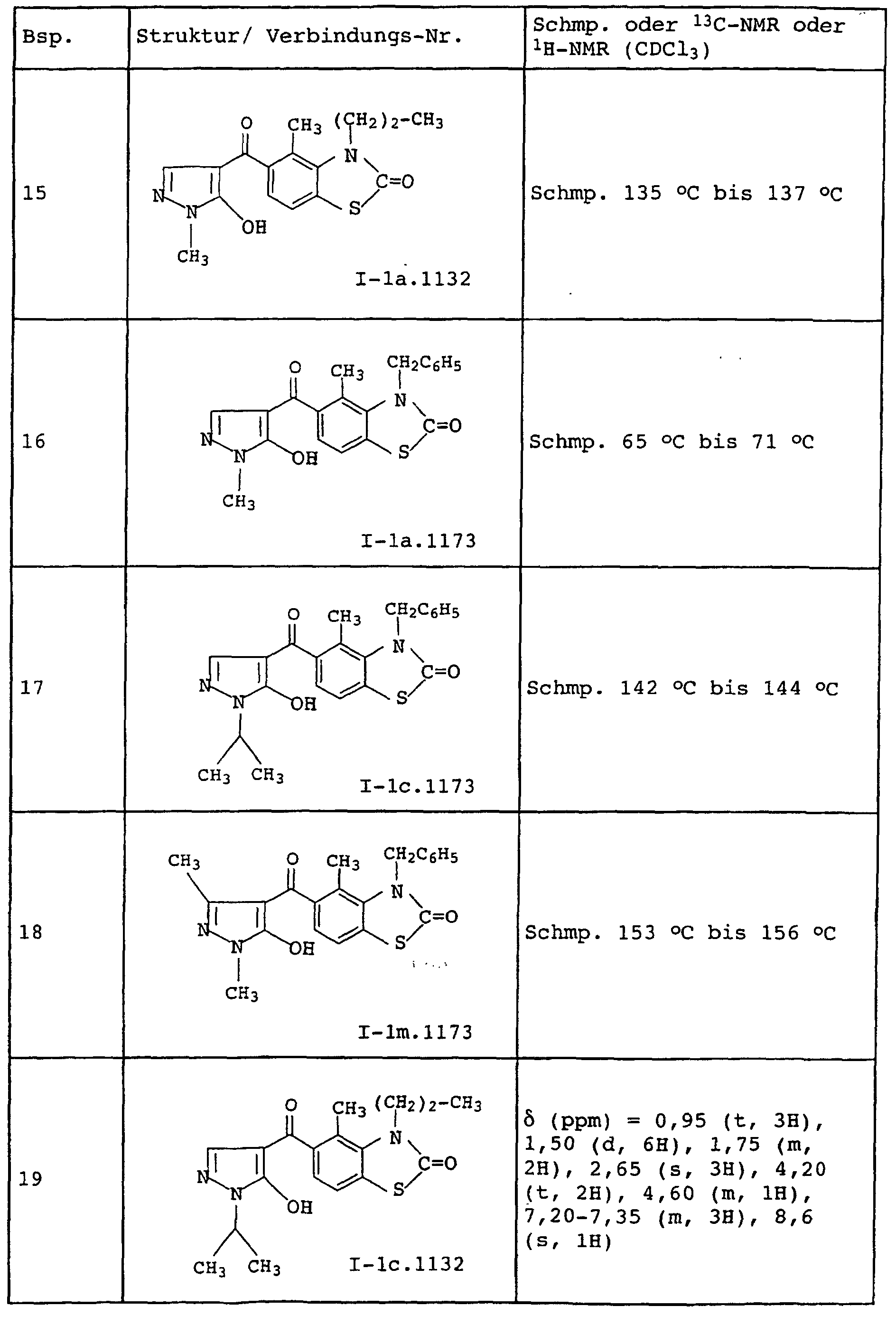

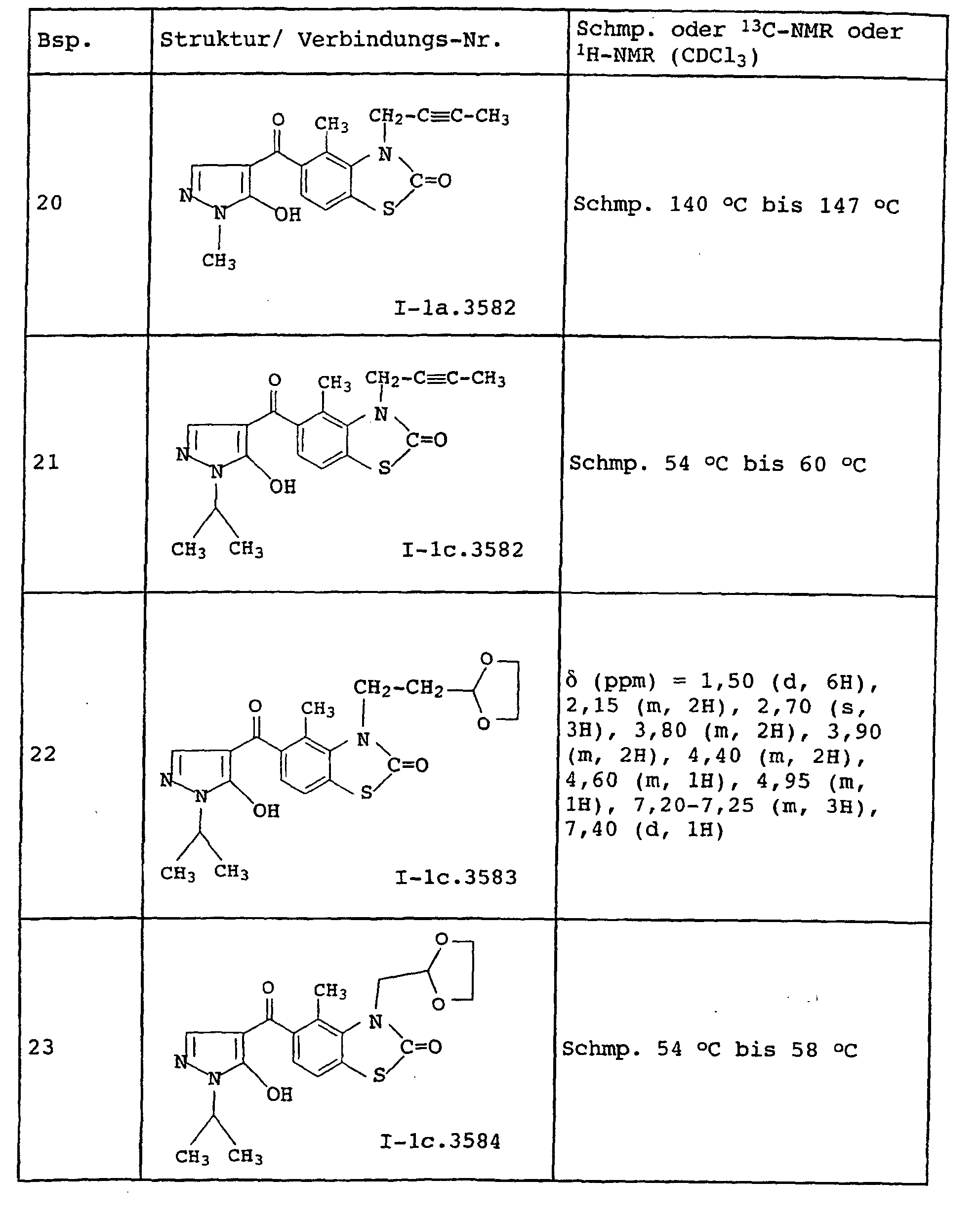

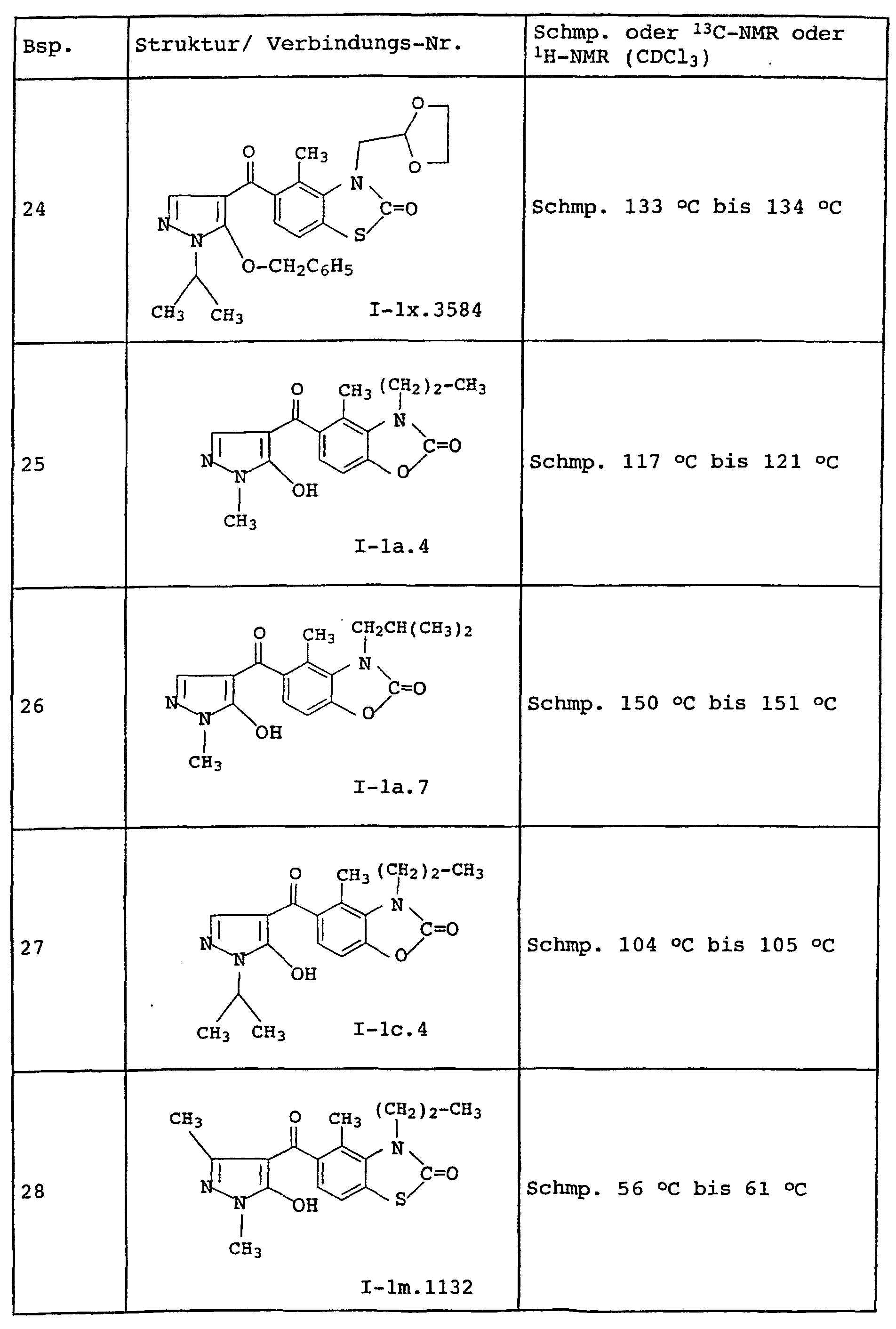

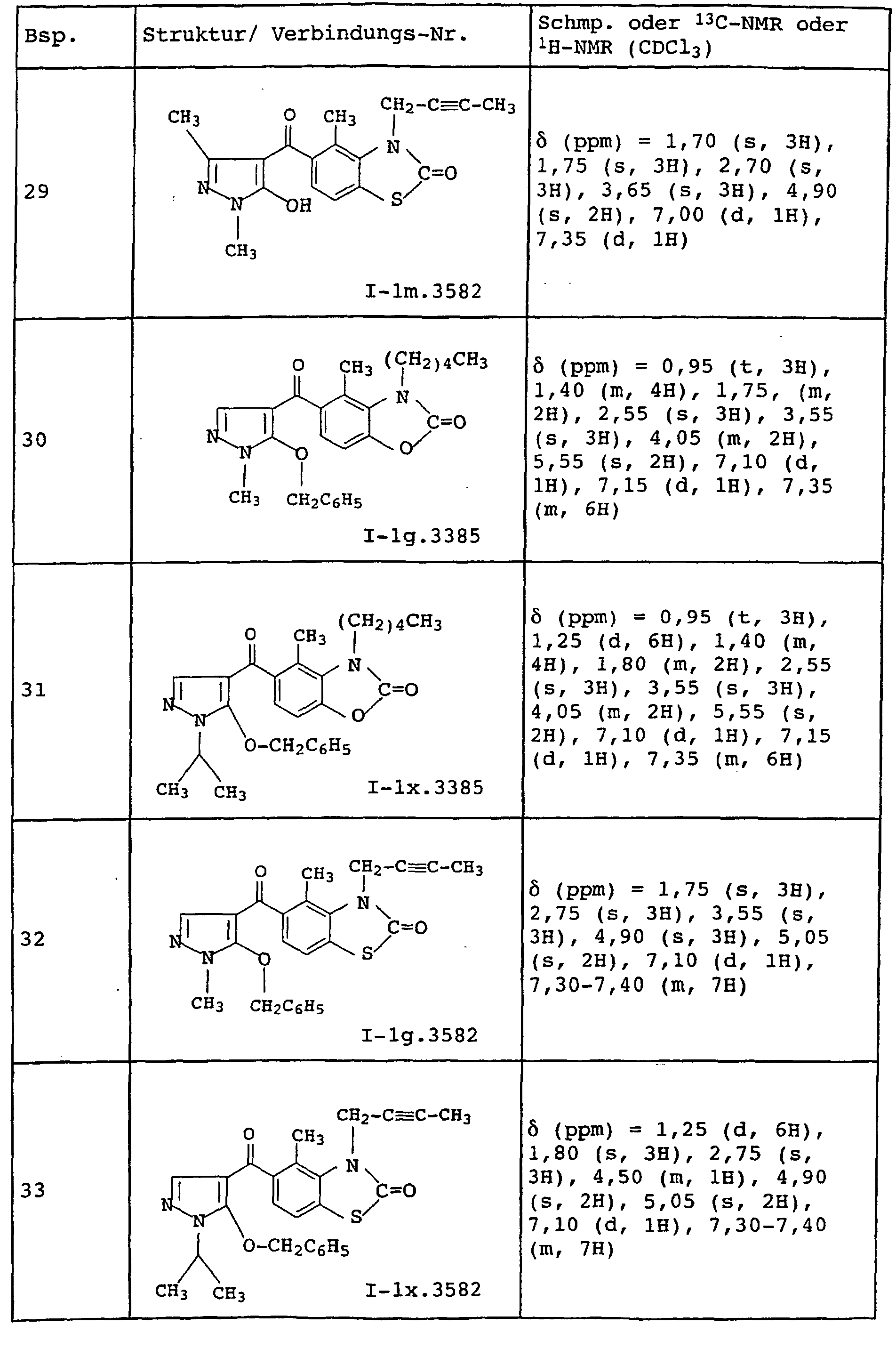

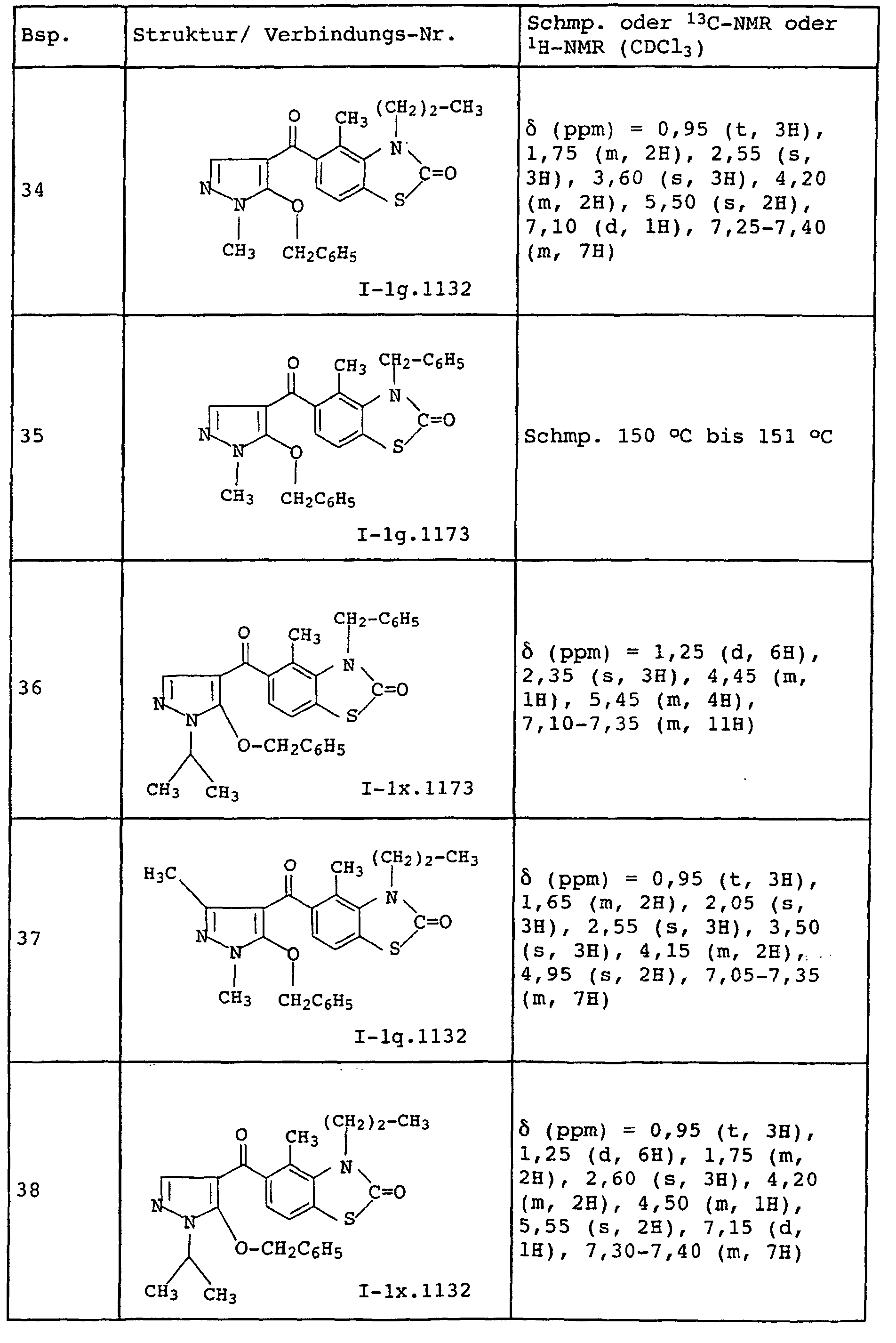

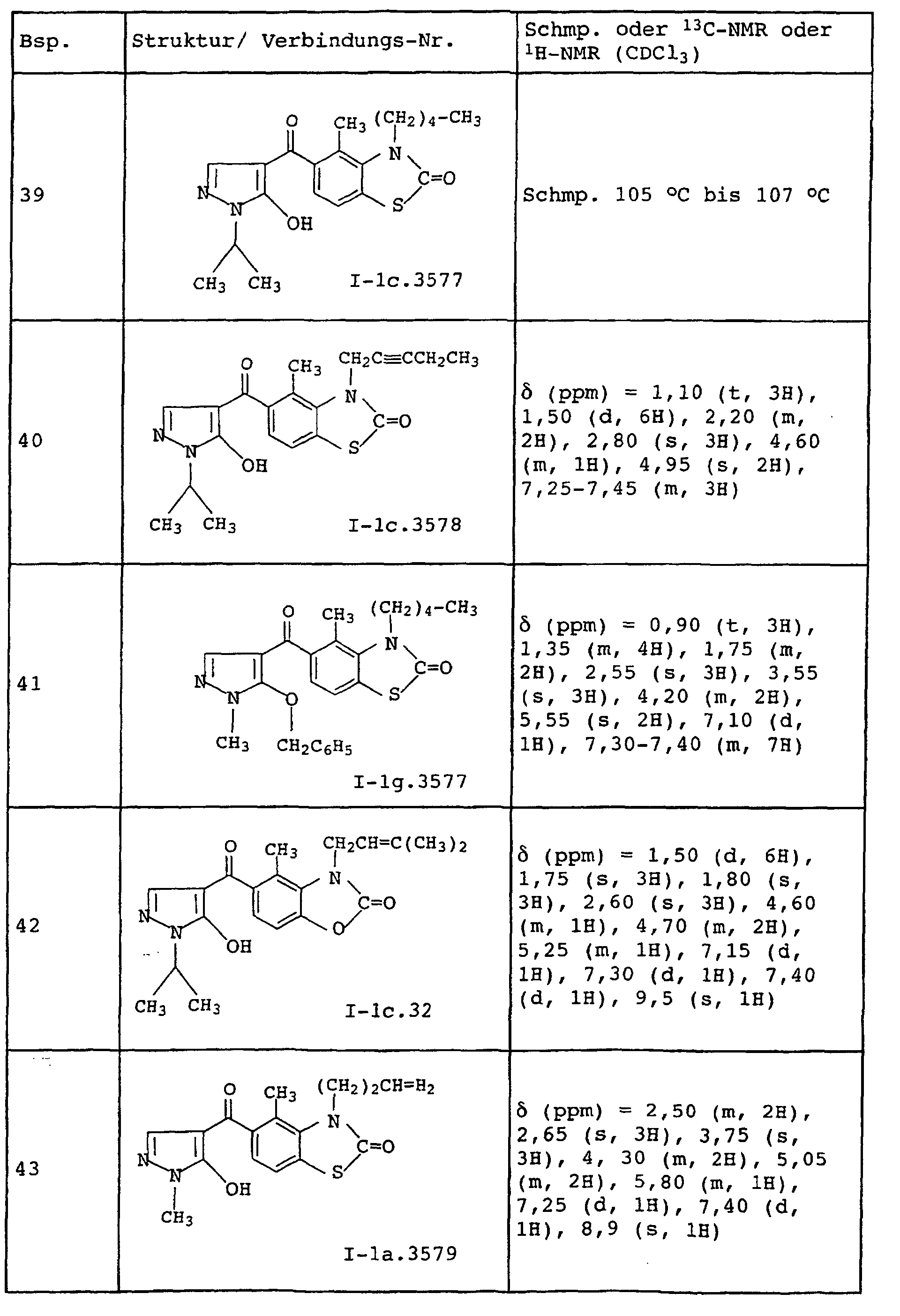

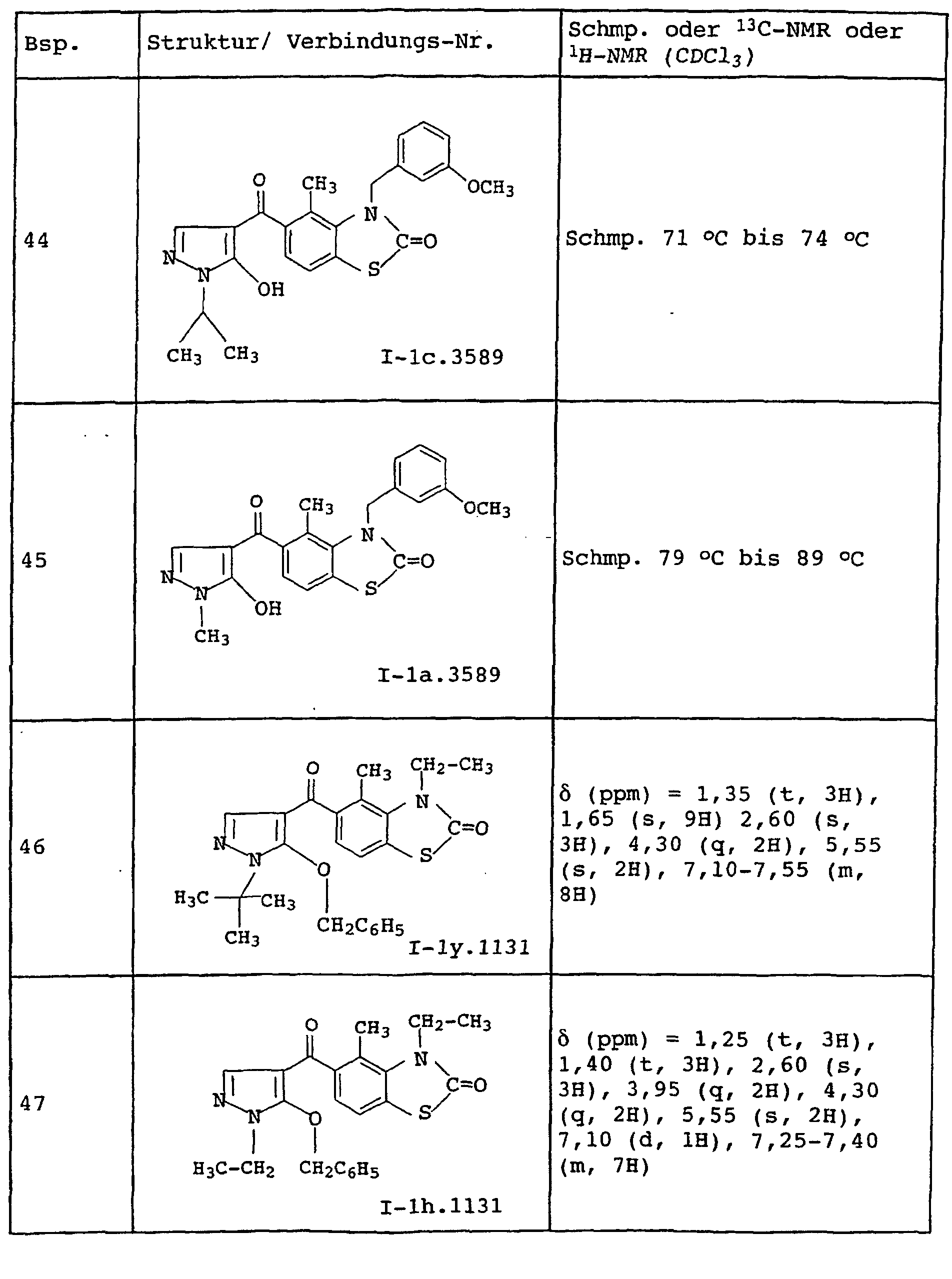

Die Verbindungen I und deren landwirtschaftlich brauchbaren Salze eignen sich - sowohl als Isomerengemische als auch in Form der reinen Isomere - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleisten. Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsstoffe.

Als inerte Zusatzstoffe kommen im Wesentlichen in Betracht:
- Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:
I 20 Gewichtsteile der Verbindung der allgemeinen Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II 20 Gewichtsteile der Verbindung der allgemeinen Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III 20 Gewichtsteile des Wirkstoffs der allgemeinen Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV 20 Gewichtsteile des Wirkstoffs der allgemeinen Formel I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V 3 Gewichtsteile des Wirkstoffs der allgemeinen Formel I werden mit 97 Gewichtsteilen feinteiligem Kaolin vermischt. Man erhält auf diese Weise ein Stäubemittel, das 3 Gew.-% des Wirkstoffs enthält.
VI 20 Gewichtsteile des Wirkstoffs der allgemeinen Formel I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
VII 1 Gewichtsteil der Verbindung der allgemeinen Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII1 Gewichtsteil der Verbindung der allgemeinen Formel I wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Die Applikation der herbiziden Mittel bzw. der Wirkstoffe kann im vorauflauf-, im Nachauflaufverfahren oder zusammen mit dem Saatgut einer Kulturpflanze erfolgen. Es besteht auch die Möglichkeit, die herbiziden Mittel bzw. Wirkstoffe dadurch zu applizieren, daß mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.).

Zur Verbreiterung des Wirkungsspektrums und zur Erzielung synergistischer Effekte können die Verbindungen I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, 2-Hetaroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximether-Derivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der Benzazolone der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 31,3, 62,5, 125 und/oder 250 g/ha aktive Substanz (a. S.).

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Eine mindestens 95 %ige Schädigung entspricht einer sehr guten herbiziden Wirkung.

Die in den Gewächshausversuchen verwendeten Pflanzen setzten sich aus folgenden Arten zusammen:

| Bayercode | Deutscher Name | Englischer Name |
|---|---|---|
| AMARE | Zurückgekrümmter Fuchsschwanz | redroot pigweed |
| BIDPI | Zweizahn, behaarter | hairy beggarticks |
| BRAPL | | marmelade grass |
| CHEAL | Weißer Gänsefuß | lambsquarters (goosefoot) |
| ECHCG | Hühnerhirse | barnyardgrass |
| LAMAM | Taubnessel,stengelumfassende | henbit |
| PAPRH | Klatschmohn | cornpoppy |
| PHBPU | purpurne Trichterwinde | morningglory, common |
| POLPE | Flohknöterich | ladysthumb |
| SETFA | Grosse Borstenhirse | giant foxtail |
| STEME | Vogelsternmiere | chickweed |
| THLAR | Pfennigkraut | pennycress |

Die folgenden Ergebnisse wurden im Nachlaufverfahren erhalten.

Bei Aufwandmengen von 31,3 bzw. 62,5 g/ha (a.S.) zeigte die Verbindung I-1a.1129 aus Beispiel 1 im Nachauflauf eine sehr gute herbizide Wirkung gegen CHEAL, SETFA, STEME und THLAR.

Bei Aufwandmengen von 31,3 bzw. 62,5 g/ha (a.S.) zeigte die Verbindung I-1b.1129 aus Beispiel 2 im Nachauflauf eine sehr gute herbizide Wirkung gegen CHEAL, ECHCG, PARPH, STEME und THLAR.

Bei Aufwandmengen von 31,3 bzw. 62,5 g/ha (a.s.) zeigte die Verbindung I-1c.1129 aus Beispiel 3 im Nachauflauf eine sehr gute herbizide Wirkung gegen AMARE, CHEAL, ECHCG und POLPE.

Bei Aufwandmengen von 125 bzw. 250 g/ha (a.S.) zeigte die Verbindung I-1a.1131 aus Beispiel 10 im Nachauflauf eine sehr gute herbizide Wirkung gegen BRAPL. AMARE, CHEAL, ECHCG und SETFA.

Bei Aufwandmengen von 125 bzw. 250 g/ha (a.S.) zeigte die Verbindung I-1b.1131 aus Beispiel 11 im Nachauflauf eine sehr gute herbizide Wirkung gegen BRAPL. AMARE, CHEAL, ECHCG und SETFA.

Bei Aufwandmengen von 125 bzw. 250 g/ha (a.S.) zeigte die Verbindung I-1c.1131 aus Beispiel 12 im Nachauflauf eine sehr gute herbizide Wirkung gegen BRAPL. AMARE, CHEAL, ECHCG und SETFA.

Bei Aufwandmengen von 125 bzw. 250 g/ha (a.S.) zeigte die Verbindung I-1d.1131 aus Beispiel 13 im Nachauflauf eine sehr gute herbizide Wirkung gegen BRAPL. AMARE, CHEAL, ECHCG und SETFA.

Bei Aufwandmengen von 125 bzw. 250 g/ha (a.S.) zeigte die Verbindung I-1h.1131 aus Beispiel 47 im Nachauflauf eine sehr gute herbizide Wirkung gegen BRAPL. AMARE, CHEAL, ECHCG und SETFA.

Bei Aufwandmengen von 125 bzw. 250 g/ha (a.S.) zeigte die Verbindung I-1y.1131 aus Beispiel 46 im Nachauflauf eine sehr gute herbizide Wirkung gegen BRAPL. PHBPU, CHEAL, ECHCG und SETFA.

Bei Aufwandmengen von 125 bzw. 250 g/ha (a.S.) zeigte die Verbindung I-1g.1131 aus Beispiel 14 im Nachauflauf eine sehr gute herbizide Wirkung gegen BRAPL. AMARE, CHEAL, ECHCG und SETFA.

Bei Aufwandmengen von 125 bzw. 62,5 g/ha (a.S.) zeigte die Verbindung I-1a.4 aus Beispiel 25 im Nachauflauf eine sehr gute herbizide Wirkung gegen BRAPL. AMARE, CHEAL, ECHCG und SETFA.

Bei Aufwandmengen von 125 bzw. 250 g/ha (a.S.) zeigte die Verbindung I-1a.6 aus Beispiel 9 im Nachauflauf eine sehr gute herbizide Wirkung gegen BIDPI, CHEAL, POLPE und SETFA.

## Patentansprüche

1. Pyrazolylbenzazolone der allgemeinen Formel I, 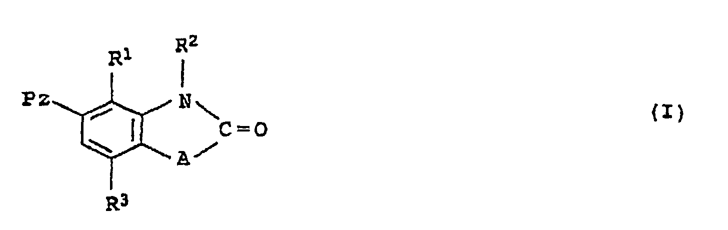 worin A, R¹, R², R³ und Pz die nachfolgend angegebenen Bedeutungen aufweisen,
A O, S, SO, SO₂ oder NR⁶;
R¹ Halogen, C₁-C₄-Alkyl, C₁-C₄-Halogenalkyl, C₁-C₄-Hydroxyalkyl, C₁-C₄-Alkoxy-C₁-C₄-alkyl, C₁-C₄-Halogenalkoxy-C₁-C₄-alkyl, C₁-C₄-Halogenalkylthio-C₁-C₄-alkyl, C₁-C₄-Alkylthio-C₁-C₄-alkyl, C₁-C₄-Alkylsulfonyl-C₁-C₄-alkyl, C₁-C₄-Halogenalkylsulfonyl-C₁-C₄-alkyl, C₁-C₄-Alkoxy, C₁-C₄-Hydroxyalkoxy, C₁-C₄-Halogenalkoxy, C₁-C₄-Alkoxy-C₁-C₄-alkoxy, C₁-C₄-Halogenalkoxy-C₁-C₄-alkoxy, C₁-C₄-Alkylthio, C₁-C₄-Halogenalkylthio, C₁-C₄-Alkylsulfonyl, C₁-C₄-Halogenalkylsulfonyl;
R² Hydroxy, Nitro, Amino, C₁-C₆-Alkylamino, Di-(C₁-C₆-alkyl)amino, Cyano, CHO, C₁-C₆-Alkoxy, C₁-C₆-Alkyl, C₁-C₆-Hydroxyalkyl, C₁-C₆-Halogenalkyl, C₁-C₆-Alkoxy-C₁-C₆-alkyl, C₁-C₆-Halogenalkoxy-C₁-C₆-alkyl, C₂-C₆-Alkenyl, C₂-C₆-Halogenalkenyl, C₁-C₆-Halogenalkoxy-C₂-C₆-alkenyl, C₁-C₆-Alkoxy-C₂-C₆-alkenyl, C₂-C₆-Alkinyl, C₂-C₆-Halogenalkinyl, C₁-C₆-Halogenalkoxy-C₂-C₆-alkinyl, C₁-C₆-Alkoxy-C₂-C₆-alkinyl, C₁-C₆-Alkylsulfonyl, C₁-C₆-Halogenalkylsulfonyl, (C₁-C₆-Alkyl)carbonyl, C₁-C₆-Halogenalkylcarbonyl, Hydroxycarbonyl-C₁-C₄-alkyl, C₁-C₆-Alkoxycarbonyl-C₁-C₆-alkyl, ein Rest der Formel C(O)OR⁴, CON(R⁵)₂ oder C(=NOR^{4a})R^{4b}, Aryl, Aryl-C₁-C₄-alkyl, Arylsulfonyl, Arylcarbonyl, C₃-C₆-Cycloalkyl, C₃-C₆-Cycloalkenyl, 3-, 4-, 5-, 6- oder 7-gliedriges Heterocyclyl, 3-, 4-, 5-, 6- oder 7-gliedriges Heterocyclyl-C₁-C₆-alkyl, wobei jeder Aryl-, Cycloalkyl-, Cycloalkenyl- und jeder Heterocyclylrest unsubstituiert sein kann oder ein, zwei, drei oder vier Substituenten tragen kann, jeweils ausgewählt unter Halogen, C₁-C₄-Halogenalkyl, C₁-C₄-Alkoxy und C₁-C₄-Halogenalkoxy;
R³ Wasserstoff, C₁-C₄-Alkyl, C₁-C₄-Halogenalkyl, C₁-C₄-Alkoxy-C₁-C₄-alkyl, C₁-C₄-Halogenalkoxy-C₁-C₄-alkyl oder Halogen;
worin
R⁴ C₁-C₆-Alkyl, C₁-C₆-Halogenalkyl, C₁-C₆-Alkoxy-C₁-C₆-alkyl, C₁-C₆-Halogenalkoxy-C₁-C₆-alkyl, C₂-C₆-Alkenyl, C₂-C₆-Halogenalkenyl, C₁-C₆-Halogenalkoxy-C₂-C₆-alkenyl, C₁-C₆-Alkoxy-C₂-C₆-alkenyl, C₂-C₆-Alkinyl, C₂-C₆-Halogenalkinyl, C₁-C₆-Halogenalkoxy-C₂-C₆-alkinyl, C₁-C₆-Alkoxy-C₂-C₆-alkinyl bedeutet;
R^{4a}, R4b unabhängig voneinander die für R⁴ genannten Bedeutungen aufweisen können und R^{4b} für Wasserstoff stehen kann;
R⁵ unabhängig voneinander für Wasserstoff, C₁-C₆-Alkyl, C₁-C₆-Halogenalkyl, C₁-C₆-Alkoxy-C₁-C₆-alkyl, (C₁-C₆-Halogenalkoxy)-C₁-C₆-alkyl, C₂-C₆-Alkenyl, C₂-C₆-Halogenalkenyl, C₁-C₆-Halogenalkyl-C₂-C₆-alkenyl, C₁-C₆-Halogenalkoxy-C₂-C₆-alkenyl, C₁-C₆-Alkoxy-C₂-C₆-alkenyl, C₂-C₆-Alkinyl, C₂-C₆-Halogenalkinyl, C₁-C₆-Halogenalkyl-C₂-C₆-alkinyl, C₁-C₆-Halogenalkoxy-C₂-C₆-alkinyl, C₁-C₆-Alkoxy-C₂-C₆-alkinyl stehen, oder zusammen einen 3- bis 7-gliedrigen Heterocyclus bilden, der gegebenenfalls partiell oder vollständig halogeniert ist und/oder einen, zwei oder drei Substituenten, ausgewählt unter C₁-C₆-Alkyl, C₁-C₆-Halogenalkyl, C₁-C₆-Alkoxy, C₁-C₆-Halogenalkoxy tragen können;
R⁶ Wasserstoff, C₁-C₄-Alkyl, C₁-C₄-Halogenalkyl, C₁-C₄-Haloalkoxy-C₁-C₄-alkyl, C₁-C₄-Alkoxy-C₁-C₄-alkyl bedeutet; und
Pz für einen Rest der Formel IIa oder IIb steht, 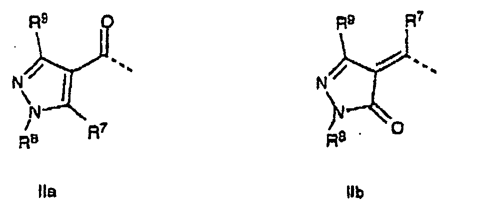
worin die Variablen R⁷, R⁸ und R⁹ folgende Bedeutung haben:
R⁷ Hydroxy, Mercapto, Halogen, OR¹⁰, SR¹⁰, SOR¹¹, SO₂R¹¹, OSO₂R¹¹, P(O)R¹²R¹³, OP(O)R¹²R¹³, P(S)R¹²R¹³, OP(S)R¹²R¹³, NR¹⁴R¹⁵, ONR¹⁴R¹⁵ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
R⁸ Wasserstoff, C₁-C₆-Alkyl, C₁-C₆-Halogenalkyl, C₃-C₆-Cycloalkyl, Hydroxy, C₁-C₆-Alkoxy oder C₁-C₆-Halogenalkoxy;
R⁹ Wasserstoff, Halogen, C₁-C₆-Alkyl, C₁-C₆-Halogenalkyl, Hydroxy, C₁-C₆-Alkoxy, C₁-C₆-Halogenalkoxy, C₁-C₆-Alkylthio oder C₁-C₆-Halogenalkylthio;
wobei
R¹⁰ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl oder C₁-C₆-Alkoxyimino-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Phenyl-C₁-C₆-alkyl, Phenylcarbonyl-C₁-C₆-alkyl, Phenylcarbonyl, Phenoxycarbonyl, Phenyloxythiocarbonyl, Phenylaminocarbonyl, N-(C₁-C₆-Alkyl)-N-(phenyl)-aminocarbonyl, Phenyl-C₂-C₆-alkenylcarbonyl, Heterocyclyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, Heterocyclylaminocarbonyl, N-(C₁-C₆-Alkyl)-N-(heterocyclyl)-aminocarbonyl, oder Heteroryclyl-C₂-C₆-alkenylcarbonyl, wobei der Phenyl- und der Heterocyclyl-Rest der 18 letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
R¹¹ C₁-C₆-Alkyl, C₃-C₆-Alkenyl, C₃-C₆-Alkinyl oder C₃-C₆-Cycloalkyl, wobei die vier genannten Reste partiell oder vollständig halogeniert sein können und/oder eine, zwei oder drei der folgenden Gruppen tragen können: Cyano, C₁-C₄-Alkoxy, C₁-C₄-Halogenalkoxy, C₁-C₄-Alkylthio, C₁-C₄-Halogenalkylthio, C₁-C₄-Alkylcarbonyl, C₁-C₄-Alkoxycarbonyl oder C₁-C₄Halogenalkoxycarbonyl; Phenyl, Phenyl-C₁-C₆-alkyl, Heterocyclyl oder Heterocyclyl-C₁-C₆-alkyl, wobei der Phenyl- und der Heterocyclyl-Rest der letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄ Alkoxy, C₁-C₄-Halogenalkoxy oder C₁-C₄-Alkoxycarbonyl;
R¹², R¹³ unabhängig voneinander Wasserstoff, Hydroxy, C₁-C₆-Alkyl, C₁-C₆-Alkoxy, C₁-C₆-Alkylthio, Phenyl, Phenyl-C₁-C₄-alkyl oder Phenoxy, wobei die drei letztgenannten Substituenten partiell oder vollständig halogeniert sein können und/oder einen, zwei oder drei der folgenden Reste trägen können: Nitro, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl;
R¹⁴ Wasserstoff, C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-alkyl)-amino oder C₁-C₆-Alkylcarbonylamino, wobei die genannten Alkyl-, Cycloalkyl- und Alkoxyreste partiell oder vollständig halogeniert sein können und/oder einen, zwei oder drei der folgenden Reste tragen können: Cyano, C₁-C₄-Alkoxycarbonyl, C₁-C₄-Alkylaminocarbonyl, Di-(C₁-C₄-alkyl)-aminocarbonyl oder C₃-C₆-Cycloalkyl;
Phenyl, Phenyl-C₁-C₄-alkyl, Phenylcarbonyl, Heterocyclyl, Heterocyclyl-C₁-C₄-alkyl oder Heterocyclylcarbonyl, wobei der Phenyl- oder Heterocyclyl-Rest der sechs letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy; und
R¹⁵ Wasserstoff, C₁-C₆-Alkyl oder C₃-C₆-Alkenyl, C₃-C₆-Alkinyl; bedeuten;
sowie deren landwirtschaftlich brauchbaren Salze.

2. Benzazolone gemäß Anspruch 1, worin A in Formel I für Sauerstoff oder NR⁶ steht.

3. Benzazolone gemäß Anspruch 1, worin A in Formel I für Schwefel oder SO₂ steht.

4. Benzazolone gemäß einem der vorhergehenden Ansprüche, worin R¹ in Formel I ausgewählt ist unter C₁-C₄-Alkyl, Halogen und C₁-C₄-Alkoxy.

5. Benzazolone nach einem der vorhergehenden Ansprüche, worin R² in Formel I für einen Heterocyclus, ausgewählt unter 4,5-Dihydroisoxazol-3-yl, 4,5-Dihydroisoxazol-4-yl, Isoxazol-3-yl, Isoxazol-4-yl, 1,3-Dioxolan-2-yl, 1,3-Dithiolan-2-yl, 1,3-Dioxan-2-yl, 1,3-Dithian-2-yl, Oxazolin-2-yl und Oxazolidin-2-yl steht, wobei die vorgenannten Heterocyclen 1-, 2- oder 3-fach mit C₁-C₄-Alkyl substituiert sein können.

6. Benzazolone nach einem der Ansprüche 1 bis 4, worin R² in Formel I für Cyano, C₁-C₄-Alkyl, C₃-C₆-Alkenyl, C₁-C₄-(Halogen)alkylcarbonyl, C₁-C₄-Alkoxycarbonyl-C₁-C₄-alkyl oder C₁-C₄-Alkoxycarbonyl steht.

7. Benzazolone nach einem der vorhergehenden Ansprüche, worin R³ in Formel I für Wasserstoff, C₁-C₄-Alkyl oder Halogen steht.

8. Benzazolone gemäß einem der vorhergehenden Ansprüche, worin Pz in Formel I für einen Rest der Formel IIa steht, worin R⁷ ausgewählt ist unter Hydroxy, OR¹⁰ und OSO₂R¹¹ mit den für R¹⁰ und R¹¹ in Anspruch 1 angegebenen Bedeutungen, wobei R⁸ und R⁹ die in Anspruch 1 angegebenen Bedeutungen haben.

9. Benzazolone gemäß Anspruch 8, wobei in Formel IIa
R⁷ für Hydroxy, C₁-C₄-Alkyloxy, Acetoxy, O-CH₂-Phenyl, Phenylcarbonyloxy, 2-, 3- oder 4-Fluorphenylcarbonyloxy, Cyclopropylcarbonyloxy, C₁-C₄-Sulfonyloxy, Phenylsulfonyloxy und 2-, 3- oder 4-Methylphenylsulfonyloxy;
R⁸ für C₁-C₄-Alkyl oder Cyclopropyl und
R⁹ für Wasserstoff oder C₁-C₄-Alkyl stehen.

10. Benzazoloncarbonsäuren der allgemeinen Formel IVa 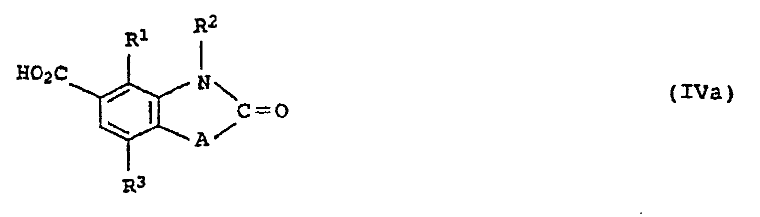 worin
A, R¹, R² und R³ die in Anspruch 1 angegebene Bedeutung aufweisen.

11. Mittel, enthaltend mindestens ein Pyrazol-Derivat der Formel I oder ein landwirtschaftlich brauchbares Salz von I gemäß einem der Ansprüche 1 bis 9, und übliche Hilfsmittel.

12. Verfahren zur Bekämpfung von unerwünschtem Pflanzenwuchs, **dadurch gekennzeichnet, daß** man eine herbizid wirksame Menge mindestens eines Pyrazol-Derivates der Formel I oder eines landwirtschaftlich brauchbaren Salzes von I gemäß einem der Ansprüche 1 bis 9, auf Pflanzen, deren Lebensraum und/oder auf Samen einwirken läßt.

13. Verwendung von Pyrazol-Derivaten der Formel I oder deren landwirtschaftlich brauchbaren Salzen gemäß einem der Ansprüche 1 bis 9 als Herbizide.

## Claims

1. A pyrazolylbenzazolone of the formula I 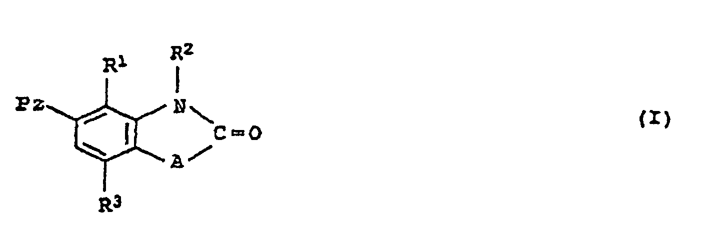 in which A, R¹, R², R³ and Pz are as defined below:
A is O, S, SO, SO₂ or NR⁶;
R¹ is halogen, C₁-C₄-alkyl, C₁-C₄-haloalkyl, C₁-C₄-hydroxy-alkyl, C₁-C₄-alkoxy-C₁-C₄-alkyl, C₁-C₄-haloalkoxy-C₁-C₄-alkyl, C₁-C₄-haloalkylthio-C₁-C₄-alkyl, C₁-C₄-alkylthio-C₁-C₄-alkyl, C₁-C₄-alkylsulfonyl-C₁-C₄-alkyl, C₁-C₄-haloalkylsulfonyl-C₁-C₄-alkyl, C₁-C₄-alkoxy, C₁-C₄-hydroxyalkoxy, C₁-C₄-haloalkoxy, C₁-C₄-alkoxy-C₁-C₄-alkoxy, C₁-C₄-haloalkoxy-C₁-C₄-alkoxy, C₁-C₄-alkylthio, C₁-C₄-haloalkylthio, C₁-C₄-alkylsulfonyl,C₁-C₄-haloalkylsulfonyl;
R² is hydroxyl, nitro, amino, C₁-C₆-alkylamino, di(C₁-C₆-alkyl)amino, cyano, CHO, C₁-C₆-alkoxy, C₁-C₆-alkyl, C₁-C₆-hydroxyalkyl, C₁-C₆-haloalkyl, C₁-C₆-alkoxy-C₁-C₆-alkyl, C₁-C₆-haloalkoxy-C₁-C₆-alkyl, C₂-C₆-alkenyl, C₂-C₆-haloalkenyl, C₁-C₆-haloalkoxy-C₂-C₆-alkenyl, C₁-C₆-alkoxy-C₂-C₆-alkenyl, C₂-C₆-alkynyl, C₂-C₆-haloalkynyl, C₁-C₆-haloalkoxy-C₂-C₆-alkynyl, C₁-C₆-alkoxy-C₂-C₆-alkynyl, C₁-C₆-alkylsulfonyl, C₁-C₆-haloalkylsulfonyl, (C₁-C₆-alkyl) carbonyl, C₁-C₆-haloalkylcarbonyl, hydroxycarbonyl-C₁-C₄-alkyl, C₁-C₆-alkoxycarbonyl-C₁-C₆-alkyl,
a radical of the formula C (O) OR⁴, CON (R⁵)₂ or C(=NOR^{4a})R^{4b}, aryl, aryl-C₁-C₄-alkyl, arylsulfonyl, arylcarbonyl, C₃-C₆-cycloalkyl, C₃-C₆-cycloalkenyl,
3-, 4-, 5-, 6- or 7-membered heterocyclyl,3-, 4-, 5-, 6- or 7-membered heterocyclyl-C₁-C₆-alkyl, where each aryl, cycloalkyl, cycloalkenyl and each heterocyclyl radical may be unsubstituted or may carry one, two, three or four substituents, in each case selected from the group consisting of halogen, C₁-C₄-haloalkyl, C₁-C₄-alkoxy and C₁-C₄-haloalkoxy;
R³ is hydrogen, C₁-C₄-alkyl, C₁-C₄-haloalkyl, C₁-C₄-alkoxy-C₁-C₄-alkyl, C₁-C₄-haloalkoxy-C₁-C₄-alkyl or halogen;
in which
R⁴ is C₁-C₆-alkyl, C₁-C₆-haloalkyl, C₁-C₆-alkoxy-C₁-C₆-alkyl, C₁-C₆-haloalkoxy-C₁-C₆-alkyl, C₂-C₆-alkenyl, C₂-C₆-haloalkenyl, C₁-C₆-haloalkoxy-C₂-C₆-alkenyl, C₁-C₆-alkoxy-C₂-C₆-alkenyl, C₂-C₆-alkynyl, C₂-C₆-haloalkynyl, C₁-C₆-haloalkoxy-C₂-C₆-alkynyl, C₁-C₆-alkoxy-C₂-C₆-alkynyl;
R^{4a}, R^{4b} independently of one another may have the meanings mentioned for R⁴, and R^{4b} may be hydrogen;
R⁵ independently of one another are hydrogen, C₁-C₆-alkyl, C₁-C₆-haloalkyl, C₁-C₆-alkoxy-C₁-C₆-alkyl, (C₁-C₆-haloalkoxy) -C₁-C₆-alkyl, C₂-C₆-alkenyl, C₂-C₆-haloalkenyl, C₁-C₆-haloalkyl-C₂-C₆-alkenyl, C₁-C₆-haloalkoxy-C₂-C₆-alkenyl, C₁-C₆-alkoxy-C₂-C₆-alkenyl, C₂-C₆-alkynyl, C₂-C₆-haloalkynyl, C₁-C₆-haloalkyl-C₂-C₆-alkynyl, C₁-C₆-haloalkoxy-C₂-C₆-alkynyl, C₁-C₆-alkoxy-C₂-C₆-alkynyl, or together form a 3- to 7-membered heterocycle which may be partially or fully halogenated and/or may carry one, two or three substituents selected from the group consisting of C₁-C₆-alkyl, C₁-C₆-haloalkyl, C₁-C₆-alkoxy, C₁-C₆-haloalkoxy;
R⁶ is hydrogen, C₁-C₄-alkyl, C₁-C₄-haloalkyl, C₁-C₄-haloalkoxy-C₁-C₄-alkyl, C₁-C₄-alkoxy-C₁-C₄-alkyl ; and
Pz is a radical of the formula IIa or IIb, 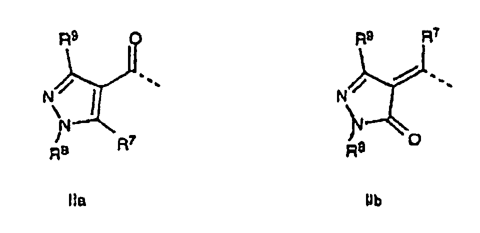
in which the variables R⁷, R⁸ and R⁹ are as defined below:
R⁷ is hydroxyl, mercapto, halogen, OR¹⁰, SR¹⁰, SOR¹¹, SO₂R¹¹, OSO₂R¹¹, P(O)R¹²R¹³, OP(O)R¹²R¹³, P(S)R¹²R¹³, OP(S)R¹²R¹³, NR¹⁴R¹⁵, ONR¹⁴R¹⁵ or N-bonded heterocyclyl which may be partially or fully halogenated and/or may carry one, two or three of the following radicals: nitro, cyano, C₁-C₄-alkyl, C₁-C₄-haloalkyl, C₁-C₄-alkoxy or C₁-C₄-haloalkoxy;
R⁸ is hydrogen, C₁-C₆-alkyl, C₁-C₆-haloalkyl, C₃-C₆-cycloalkyl, hydroxyl, C₁-C₆-alkoxy or C₁-C₆-haloalkoxy;
R⁹ is hydrogen, halogen, C₁-C₆-alkyl, C₁-C₆-haloalkyl, hydroxyl, C₁-C₆-alkoxy, C₁-C₆-haloalkoxy, C₁-C₆-alkylthio or C₁-C₆-haloalkylthio;
where
R¹⁰ is C₁-C₆-alkyl, C₃-C₆-alkenyl, C₃-C₆-haloalkenyl, C₃-C₆-alkynyl, C₃-C₆-haloalkynyl, C₃-C₆-cycloalkyl, C₁-C₆-alkylcarbonyl, C₂-C₆-alkenylcarbonyl, C₂-C₆-alkynylcarbonyl, C₃-C₆-cycloalkylcarbonyl, C₁-C₆-alkoxycarbonyl, C₃-C₆-alkenyloxycarbonyl, C₃-C₆-alkynyloxycarbonyl, C₁-C₆-alkylthiocarbonyl, C₁-C₆-alkylaminocarbonyl, C₃-C₆-alkenylaminocarbonyl, C₃-C₆-alkynylaminocarbonyl, N,N-di(C₁-C₆-alkyl) amino-carbonyl, N- (C₃-C₆-alkenyl) -N-(C₁-C₆-alkyl)amino-carbonyl, N- (C₃-C₆-alkynyl)-N- (C₁-C₆-alkyl) amino-carbonyl, N-(C₁-C₆-alkoxy) -N- (C₁-C₆-alkyl) amino-carbonyl, N- (C₃-C₆-alkenyl) -N- (C₁-C₆-alkoxy) amino-carbonyl, N- (C₃-C₆-alkynyl) -N- (C₁-C₆-alkoxy) amino-carbonyl, di (C₁-C₆-alkyl)aminothiocarbonyl or C₁-C₆-alkoxyimino-C₁-C₆-alkyl, where the alkyl, cycloalkyl and alkoxy radicals mentioned may be partially or fully halogenated and/or may carry one to three of the following groups: cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, C₁-C₄-alkylaminocarbonyl, di(C₁-C₄-alkyl)aminocarbonyl, aminocarbonyl, C₁-C₄-alkylcarbonyloxy or C₃-C₆-cycloalkyl;
is phenyl, phenyl-C₁-C₆-alkyl, phenyl-carbonyl-C₁-C₆-alkyl, phenylcarbonyl, phenoxycarbonyl, phenyloxythiocarbonyl, phenylaminocarbonyl, N-(C₁-C₆-alkyl )-N-(phenyl)aminocarbonyl, phenyl-C₂-C₆-alkenylcarbonyl, heterocyclyl, heterocyclyl-C₁-C₆-alkyl, heterocyclylcarbonyl-C₁-C₆-alkyl, heterocyclylcarbonyl, heterocyclyloxycarbonyl, heterocyclyloxythiocarbonyl, heterocyclylamino-carbonyl, N-(C₁-C₆-alkyl)-N-(heterocyclyl)-aminocarbonyl, or heterocyclyl-C₂-C₆-alkenylcarbonyl, where the phenyl and the heterocyclyl radical of the 18 lastmentioned substituents may be partially or fully halogenated and/or may carry one, two or three of the following radicals: nitro, cyano, C₁-C₄-alkyl, C₁-C₄-haloalkyl, C₁-C₄-alkoxy or C₁-C₄-haloalkoxy;
R¹¹ is C₁-C₆-alkyl, C₃-C₆-alkenyl, C₃-C₆-alkynyl or C₃-C₆-cycloalkyl, where the four radicals mentioned may be partially or fully halogenated and/or may carry one, two or three of the following groups: cyano, C₁-C₄-alkoxy, C₁-C₄-haloalkoxy, C₁-C₄-alkylthio, C₁-C₄-haloalkylthio, C₁-C₄-alkylcarbonyl, C₁-C₄-alkoxycarbonyl or C₁-C₄-haloalkoxycarbonyl;
is phenyl, phenyl-C₁-C₆-alkyl, heterocyclyl or heterocyclyl-C₁-C₆-alkyl, where the phenyl and the heterocyclyl radical of the lastmentioned substituents may be partially or fully halogenated and/or may carry one, two or three of the following radicals: nitro, cyano, C₁-C₄-alkyl, C₁-C₄-haloalkyl, C₁-C₄-alkoxy, C₁-C₄-haloalkoxy or C₁-C₄-alkoxycarbonyl;
R¹², R¹³ independently of one another are hydrogen, hydroxyl, C₁-C₆-alkyl, C₁-C₆-alkoxy, C₁-C₆-alkylthio, phenyl, phenyl-C₁-C₉-alkyl or phenoxy, where the three lastmentioned substituents may be partially or fully halogenated and/or may carry one, two or three of the following radicals: nitro, cyano, C₁-C₄-alkyl, C₁-C₄-haloalkyl, C₁-C₄-alkoxy, C₁-C₄-haloalkoxy or C₁-C₄-alkoxycarbonyl;
R¹⁴ is hydrogen, C₁-C₆-alkyl, C₃-C₆-alkenyl, C₃-C₆-haloalkenyl, C₃-C₆-alkynyl, C₃-C₆-haloalkynyl , C₃-C₆-cycloalkyl, C₁-C₆-alkylcarbonyl, hydroxyl, C₁-C₆-alkoxy, C₃-C₆-alkenyloxy, C₃-C₆-alkynyloxy, amino, C₁-C₆-alkylamino, di (C₁-C₆-alkyl) amino or C₁-C₆-alkylcarbonylamino, where the alkyl, cycloalkyl and alkoxy radicals mentioned may be partially or fully halogenated and/or may carry one, two or three of the following radicals: cyano, C₁-C₄-alkoxy-carbonyl, C₁-C₄-alkylaminocarbonyl, di (C₁-C₄-alkyl)-aminocarbonyl or C₃-C₆-cycloalkyl;
is phenyl, phenyl-C₁-C₄-alkyl, phenyl-carbonyl, heterocyclyl, heterocyclyl-C₁-C₄-alkyl or heterocyclylcarbonyl, where the phenyl or heterocyclyl radical of the six lastmentioned substituents may be partially or fully halogenated and/or may carry one, two or three of the following radicals: nitro, cyano, C₁-C₄-alkyl, C₁-C₄-haloalkyl, C₁-C₄-alkoxy or C₁-C₄-haloalkoxy; and
R¹⁵ is hydrogen, C₁-C₆-alkyl or C₃-C₆-alkenyl, C₃-C₆-alkynyl;
and its agriculturally useful salts.

2. A benzazolone as claimed in claim 1 where A in the formula I is oxygen or NR⁶.

3. A benzazolone as claimed in claim 1 where A in the formula I is sulfur or SO₂.

4. A benzazolone as claimed in any of the preceding claims where R¹ in the formula I is selected from the group consisting of C₁-C₄-alkyl, halogen and C₁-C₄-alkoxy.

5. A benzazolone as claimed in any of the preceding claims where R² in the formula I is a heterocycle selected from the group consisting of 4,5-dihydroisoxazol-3-yl, 4,5-dihydro-isoxazol-4-yl, isoxazol-3-yl, isoxazol-4-yl, 1,3-dioxolan-2-yl, 1,3-dithiolan-2-yl, 1,3-dioxan-2-yl, 1,3-dithian-2-yl, oxazolin-2-yl and oxazolidin-2-yl, where the abovementioned heterocycles may be mono-, di- or trisubstituted by C₁-C₄-alkyl.

6. A benzazolone as claimed in any of claims 1 to 4, where R² in the formula I is cyano, C₁-C₄-alkyl, C₃-C₆-alkenyl, C₁-C₄- (halo) alkylcarbonyl, C₁-C₄-alkoxycarbonyl-C₁-C₄-alkyl or C₁-C₄-alkoxycarbonyl.

7. A benzazolone as claimed in any of the preceding claims where R³ in the formula I is hydrogen, C₁-C₄-alkyl or halogen.

8. A benzazolone as claimed in any of the preceding claims where Pz in the formula I is a radical of the formula IIa where R⁷ is selected from the group consisting of hydroxyl, OR¹⁰ and OSO₂R¹¹, where R¹⁰ and R¹¹ are as defined in claim 1, R⁸ and R⁹ being as defined in claim 1.

9. A benzazolone as claimed in claim 8 where in the formula IIa
R⁷ is hydroxyl, C₁-C₄-alkyloxy, acetoxy, O-CH₂-phenyl, phenylcarbonyloxy, 2-, 3- or 4-fluorophenyl-carbonyloxy, cyclopropylcarbonyloxy, C₁-C₄-sulfonyloxy, phenylsulfonyloxy or 2-, 3- or 4-methylphenyl-sulfonyloxy;
R⁸ is C₁-C₄-alkyl or cyclopropyl and
R⁹ is hydrogen or C₁-C₄-alkyl.

10. A benzazolonecarboxylic acid of the formula IVa 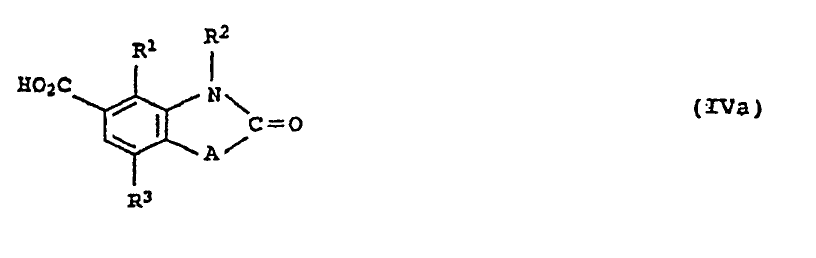 where
A, R¹, R² and R³ are as defined in claim 1.

11. A composition, comprising at least one pyrazole derivative of the formula I or an agriculturally useful salt of I as claimed in any of claims 1 to 9, and customary auxiliaries.

12. A method for controlling undesirable vegetation, which comprises allowing a herbicidally effective amount of at least one pyrazole derivative of the formula I or an agriculturally useful salt of I as claimed in any of claims 1 to 9 to act on plants, their habitat and/or on seed.

13. The use of pyrazole derivatives of the formula I or their agriculturally useful salts as claimed in any of claims 1 to 9 as herbicides.

## Revendications

1. Pyrazolylbenzazolones de formule générale I 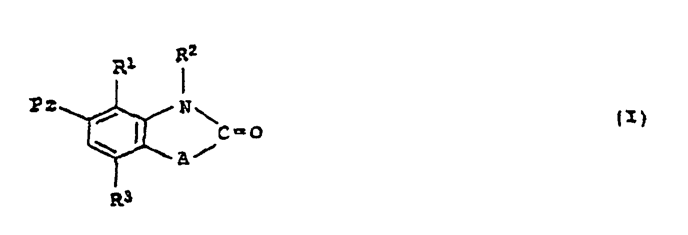 dans laquelle A, R¹, R², R³ et Pz ont les significations indiquées ci-après :
A O, S, SO, SO₂ ou NR⁶ ;
R¹ un atome d'halogène, un groupe alkyle en C₁-C₄, halogéno(alkyle en C₁-C₄), hydroxy(alkyle en C₁-C₄), (alcoxy en C₁-C₄)-(alkyle en C₁-C₄), halogéno(alcoxy en C₁-C₄)(alkyle en C₁-C₄), halogéno(alkyle en C₁-C₄)thio-(alkyle en C₁-C₄), (alkyle en C₁-C₄)thio-(alkyle en C₁-C₄), (alkyle en C₁-C₄)sulfonyl-(alkyle en C₁-C₄), halogéno-(alkyle en C₁-C₄)sulfonyl-(alkyle en C₁-C₄), alcoxy en C₁-C₄, hydroxy(alcoxy en C₁-C₄), halogéno(alcoxy en C₁-C₄), (alcoxy en C₁-C₄)-(alcoxy en C₁-C₄), halogéno(alcoxy en C₁-C₄)(alcoxy en C₁-C₄), (alkyle en C₁-C₄)thio, halogéno(alkyle en C₁C-₄)thio, (alkyle en C₁-C₄)sulfonyle, halogéno(alkyle en C₁-C₄)sulfonyle;
R² un groupe hydroxy, nitro, amino, (alkyle en C₁-C₆)amino, di(alkyle en C₁-C₆)amino, cyano, CHO, alcoxy en C₁-C₆, alkyle en C₁-C₆, hydroxy(alkyle en C₁-C₆), halogéno(alkyle en C₁-C₆), (alcoxy en C₁-C₆)-(alkyle en C₁-C₆), halogéno(alcoxy en C₁-C₆)-(alkyle en C₁-C₆), alcényle en C₂-C₆, halogéno(alcényle en C₂-C₆), halogéno(alcoxy en C₁-C₆)-(alcényle en C₂-C₆), (alcoxy en C₁-C₆)-(alcényle en C₂-C₆), alcynyle en C₂-C₆, halogéno(alcynyle en C₂-C₆), halogéno(alcoxy en C₁-C₆)-(alcynyle en C₂-C₆), (alcoxy en C₁-C₆)-(alcynyle en C₂-C₆), (alkyle en C₁-C₆)sulfonyle, halogéno(alkyle en C₁-C₆)sulfonyle, (alkyle en C₁-C₆)carbonyle, halogéno(alkyle en C₁-C₆)carbonyle, hydroxycarbonyl-(alkyle en C₁-C₄), (alcoxy en C₁-C₆)carbonyl-(alkyle en C₁-C₆).
un groupe de formule C(O)OR⁴, CON(R⁵)₂ ou C(=NOR^{4a})R^{4b}, aryle, aryl-(alkyle en C₁-C₄), arylsulfonyle, arylcarbonyle, cycloalkyle en C₃-C₆, cycloalcényle en C₃-C₆, hétérocyclyle à 3, 4, 5, 6 ou 7 chaînons, (hétérocyclyle à 3, 4, 5, 6 ou 7 chaînons)-(alkyle en C₁-C₆), chaque groupe aryle, cycloalkyle, cycloalcényle et chaque groupe hétérocyclyle pouvant être non substitué ou porter un, deux, trois ou quatre substituants, choisis parmi un atome d'halogène, un groupe halogéno(alkyle en C₁-C₄), (alcoxy en C₁-C₄) et halogéno(alcoxy en C₁-C₄),
R³ un atome d'hydrogène, un groupe alkyle en C₁-C₄, halogéno(alkyle en C₁-C₄), (alcoxy en C₁-C₄)-(alkyle en C₁-C₄), halogéno(alcoxy en C₁-C₄)-(alkyle en C₁-C₄) ou un atome d'halogène ;
où
R⁴ signifie un groupe alkyle en C₁-C₆, halogéno(alkyle en C₁-C₆), (alcoxy en C₁-C₆)-(alkyle en C₁-C₆), halogéno(alcoxy en C₁-C₆)-(alkyle en C₁-C₆), alcényle en C₂-C₆, halogéno-(alcényle en C₂-C₆), halogéno(alcoxy en C₁-C₆)-(alcényle en C₂-C₆), (alcoxy en C₁-C₆)-(alcényle en C₂-C₆), alcynyle en C₂-C₆, halogéno(alcynyle en C₂-C₆), halogéno(alcoxy en C₁-C₆)-(alcynyle en C₂-C₆), (alcoxy en C₁-C₆)-(alcynyle en C₂-C₆) ;
R^{4a}, R^{ab} ont indépendamment les significations mentionnées pour R⁴, et R^{4b} peut représenter un atome d'hydrogène ;
R⁵ représentent indépendamment un atome d'hydrogène, un groupe alkyle en C₁-C₆, halogéno(alkyle en C₁-C₆), (alcoxy en C₁-C₆)(alkyle en C₁-C₆), halogéno(alcoxy en C₁-C₆)-(alkyle en C₁-C₆), alcényle en C₂-C₆, halogéno(alcényle en C₂-C₆), halogéno(alkyle en C₁-C₆)-(alcényle en C₂-C₆), halogéno(alcoxy en C₁-C₆)-(alcényle en C₂-C₆), (alcoxy en C₁-C₆)-(alcényle en C₂-C₆), alcynyle en C₂-C₆, halogéno(alcynyle en C₂-C₆), halogéno(alkyle en C₁-C₆)-(alcynyle en C₂-C₆), halogéno(alcoxy en C₁-C₆)-(alcynyle en C₂-C₆), (alcoxy en C₁-C₆)-(alcynyle en C₂-C₆), ou forment ensemble un hétérocycle ayant 3 à 7 chaînons, qui peut être éventuellement partiellement ou totalement halogéné, et/ou porte un, deux ou trois substituants choisis parmi les groupes alkyle en C₁-C₆, halogéno(alkyle en C₁-C₆), alcoxy en C₁-C₆, halogéno(alcoxy en C₁-C₆);
R⁶ représente un atome d'hydrogène, un groupe alkyle en C₁-C₄, halogéno(alkyle en C₁-C₄), halogéno(alcoxy en C₁-C₄)-(alkyle en C₁-C₄), (alcoxy en C₁-C₄)-(alkyle en C₁-C₄) ; et
Pz représente un groupe de formule IIa ou IIb, 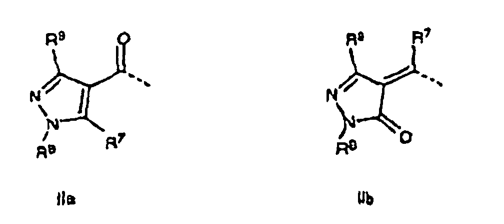 où les variables R⁷, R⁸ et R⁹ ont les significations suivantes :
R⁷ représente un groupe hydroxy, mercapto, halogéno, OR¹⁰, SR¹⁰, SOR¹¹, SO₂R¹¹, OSO₂R¹¹, P(O)R¹²R¹³, OP(O)R¹²R¹³, P(S)R¹²R¹³, OP(S)R¹²R¹³, NR¹⁴R¹⁵, ONR¹⁴R¹⁵ ou hétérocyclyle lié à N, qui peut être partiellement ou totalement halogéné et/ou porter un, deux ou trois des groupes suivants : nitro, cyano, alkyle en C₁-C₄, halogéno(alkyle en C₁-C₄), alcoxy en C₁-C₄ ou halogéno(alcoxy en C₁-C₄) ;
R⁸ représente un atome d'hydrogène, un groupe alkyle en C₁-C₆, halogéno(alkyle en C₁-C₆), cycloalkyle en C₃-C₆, hydroxy, alcoxy en C₁-C₆ ou halogéno(alcoxy en C₁-C₆);
R⁹ représente un atome d'hydrogène, un atome d'halogène, un groupe alkyle en C₁-C₆, halogéno(alkyle en C₁-C₆), un groupe hydroxy, un groupe alcoxy en C₁-C₆, halogéno(alcoxy en C₁-C₆), (alkyle en C₁-C₆)thio ou halogéno(alkyle en C₁-C₆)thio ;
où
R¹⁰ représente un groupe alkyle en C₁-C₆, alcényle en C₃-C₆, halogéno(alcényle en C₃-C₆), alcynyle en C₃-C₆, halogéno(alcynyle en C₃-C₆), cycloalkyle en C₃-C₆, (alkyle en C₁-C₆)carbonyle, (alcényle en C₂-C₆)carbonyle, (alcynyle en C₂-C₆)carbonyle, (cycloalkyle en C₃-C₆)carbonyle, (alcoxy en C₁-C₆)carbonyle, (alcényle en C₃-C₆)oxycarbonyle, (alcynyle en C₃-C₆)oxycarbonyle, (alkyle en C₁-C₆)thiocarbonyle, (alkyle en C₁-C₆)aminocarbonyle, (alcényle en C₃-C₆)aminocarbonyle, (alcynyle en C₃-C₆)aminocarbonyle, N,N-di-(alkyle en C₁-C₆)aminocarbonyle, N-(alcényle en C₃-C₆)-N-(alkyle en C₁-C₆)-aminocarbonyle, N-(alcynyle en C₃-C₆)-N-(alkyle en C₁-C₆)-aminocarbonyle, N-(alcoxy en C₁-C₆)-N-(alkyle en C₁-C₆)-aminocarbonyle, N-(alcényle en C₃-C₆)-N-(alcoxy en C₁-C₆)-aminocarbonyle, N-(alcynyle en C₃-C₆)-N-(alcoxy en C₁-C₆)-aminocarbonyle, di(alkyle en C₁-C₆)aminothiocarbonyle ou (alcoxy en C₁-C₆)-imino-(alkyle en C₁-C₆), les groupes alkyle, cycloalkyle et alcoxy mentionnés pouvant être partiellement ou totalement halogénés et/ou porter de un à trois des groupes suivants : cyano, alcoxy en C₁-C₄, (alkyle en C₁-C₄)thio, di(alkyle en C₁-C₄)amino, (alkyle en C₁-C₄)carbonyle, (alcoxy en C₁-C₄)carbonyle, (alcoxy en C₁-C₄)-(alcoxy en C₁-C₄)carbonyle, (alkyle en C₁-C₄)aminocarbonyle, di(alkyle en C₁-C₄)aminocarbonyle, aminocarbonyle, (alkyle en C₁-C₄)carbonyloxy ou cycloalkyle en C₃-C₆;
phényle, phényl(alkyle en C₁-C₆), phénylcarbonyl(alkyle en C₁-C₆), phénylcarbonyle, phénoxycarbonyle, phényloxythiocarbonyle, phénylamino-carbonyle, N-(alkyle en C₁-C₆)-N-(phényl)aminocarbonyle, phényl(alcényle en C₂-C₆)carbonyle, hétérocyclyle, hétérocyclyl(alkyle en C₁-C₆), hétérocyclylcarbonyl(alkyle en C₁-C₆), hétérocyclylcarbonyle, hétérocyclyloxy-carbonyle, hétérocyclyloxythiocarbonyle, hétérocyclylaminocarbonyle, N-(alkyle en C₁-C₆)-N-(hétérocyclyl)aminocarbonyle ou hétérocyclyl(alcényle en C₂-C₆)carbonyle, les groupes phényle et hétérocyclyle des 18 substituants mentionnés en derniers pouvant être partiellement ou totalement halogénés et/ou porter un, deux ou trois des groupes suivants : nitro, cyano, (alkyle en C₁-C₄), halogéno(alkyle en C₁-C₄), alcoxy en C₁-C₄ ou halogéno(alcoxy en C₁-C₄) ;
R¹¹ représente un groupe alkyle en C₁-C₆, alcényle en C₃-C₆, alcynyle en C₃-C₆ ou cycloalkyle en C₃-C₆, les quatre groupes mentionnés pouvant être partiellement ou totalement halogénés et/ou porter un, deux ou trois des groupes suivants : cyano, alcoxy en C₁-C₄, halogéno(alcoxy en C₁-C₄), (alkyle en C₁-C₄)thio, halogéno(alkyle en C₁-C₄)thio, (alkyle en C₁-C₄)carbonyle, (alcoxy en C₁-C₄)carbonyle ou halogéno(alcoxy en C₁-C₄)carbonyle ;
phényle, phényl(alkyle en C₁-C₆), hétérocyclyle ou hétérocyclyl(alkyle en C₁-C₆), les groupes phényle et hétérocyclyle des substituants mentionnés en derniers pouvant être partiellement ou totalement halogénés et/ou porter un, deux ou trois des groupes suivants : nitro, cyano, alkyle en C₁-C₄, halogéno(alkyle en C₁-C₄), alcoxy en C₁-C₄, halogéno(alcoxy en C₁-C₄), ou (alcoxy en C₁-C₄)carbonyle ;
R¹², R¹³ représentent indépendamment un atome d'hydrogène, un groupe hydroxy, alkyle en C₁-C₆, alcoxy en C₁-C₆, (alkyle en C₁-C₆)thio, phényle, phényl(alkyle en C₁-C₄) ou phénoxy, les trois substituants mentionnés en derniers pouvant être partiellement ou totalement halogénés et/ou porter un, deux ou trois des groupes suivants : nitro, cyano, alkyle en C₁-C₄, halogéno(alkyle en C₁-C₄), alcoxy en C₁-C₄, halogéno(alcoxy en C₁-C₄), ou (alcoxy en C₁-C₄)carbonyle ;
R¹⁴ représente un atome d'hydrogène, un groupe alkyle en C₁-C₆, alcényle en C₃-C₆, halogéno(alcényle en C₃-C₆), alcynyle en C₃-C₆, halogéno(alcynyle en C₃-C₆), cycloalkyle en C₃-C₆, (alkyle en C₁-C₆)carbonyle, hydroxy, alcoxy en C₁-C₆, (alcényle en C₃-C₆)oxy, (alcynyle en C₃-C₆)oxy, amino, (alkyle en C₁-C₆)amino, di(alkyle en C₁-C₆)amino ou(alkyle en C₁-C₆)carbonylamino, les groupes alkyle, cycloalkyle et alcoxy mentionnés en derniers pouvant être partiellement ou totalement halogénés et/ou porter un, deux ou trois des groupes suivants : cyano, (alcoxy en C₁-C₄)carbonyle, (alkyle en C₁-C₄)aminocarbonyle, di(alkyle en C₁-C₄)aminocarbonyle, ou cycloalkyle en C₃-C₆ ;
phényle, phényl(alkyle en C₁-C₄), phénylcarbonyle, hétérocyclyle, hétérocyclyl(alkyle en C₁-C₄) ou hétérocyclylcarbonyle, les groupes phényle ou hétérocyclyle des six substituants mentionnés en derniers pouvant être partiellement ou totalement halogénés et/ou porter un, deux ou trois des groupes suivants : nitro, cyano, alkyle en C₁-C₄, halogéno(alkyle en C₁-C₄), alcoxy en C₁-C₄ ou halogéno(alcoxy en C₁-C₄) ; et
R¹⁵ représente un atome d'hydrogène, un groupe alkyle en C₁-C₆, alcényle en C₃-C₆ ou alcynyle en C₃-C₆ ;
ainsi que leurs sels utilisables en agriculture.

2. Benzazolones selon la revendication 1, dans lesquelles A dans la formule I représente un atome d'oxygène ou un groupe NR⁶.

3. Benzazolones selon la revendication 1, dans lesquelles A dans la formule I représente un atome de soufre ou un groupe SO₂.

4. Benzazolones selon l'une quelconque des revendications précédentes, dans lesquelles R¹ dans la formule 1 est choisi parmi un groupe alkyle en C₁-C₄, un atome d'halogène et un groupe alcoxy en C₁-C₄.

5. Benzazolones selon l'une quelconque des revendications précédentes, dans lesquelles R² dans la formule I représente un hétérocycle choisi parmi les groupes 4,5-di-hydroisoxazol-3-yle, 4,5-di-hydroisoxazol-4-yle, isoxazol-3-yle, isoxazol-4-yle, 1,3-dioxolane-2-yle, 1,3-dithiolane-2-yle, 1,3-dioxane-2-yle, 1,3-dithiane-2-yle, oxazoline-2-yle et oxazolidine-2-yle, les hétérocyles mentionnés pouvant être substitués une, deux ou trois fois par un groupe alkyle en C₁-C₄.

6. Benzazolones selon l'une quelconque des revendications 1 à 4, dans lesquelles R² dans la formule I représente un groupe cyano, alkyle en C₁-C₄, alcényle en C₃-C₆, halogéno(alkyle en C₁-C₄)carbonyle, (alcoxy en C₁-C₄)carbonyle (alkyle en C₁-C₄) ou (alcoxy en C₁-C₄)carbonyle.

7. Benzazolones selon l'une quelconque des revendications précédentes, dans lesquelles R³ dans la formule I représente un atome d'hydrogène, un groupe alkyle en C₁-C₄ ou un atome d'halogène.

8. Benzazolones selon l'une quelconque des revendications précédentes, dans lesquelles Pz dans la formule I représente un groupe de formule IIa, R⁷ étant choisi parmi un groupe hydroxy, un groupe OR¹⁰ ou un groupe OSO₂R¹¹, R¹⁰ et R¹¹ ainsi que R⁸ et R⁹ ayant les significations mentionnées à la revendication 1.

9. Benzazolones selon la revendication 8, dans lesquelles, dans la formule IIa,
R⁷ représente un groupe hydroxy, alkyloxy en C₁-C₄, acétoxy, O-CH₂-phényle, phénylcarbonyloxy, 2-, 3- ou 4-fluorophénylcarbonyloxy, cyclopropyl-carbonyloxy, (sulfonyle en C₁-C₄)oxy, phénylsulfonyloxy et 2-, 3- ou 4-méthylphénylsulfonyloxy ;
R⁸ représente un groupe alkyle en C₁-C₄ ou cyclopropyle et
R⁹ représente un atome d'hydrogène ou un groupe alkyle en C₁-C₄.

10. Acides benzolonecarboxyliques de formule générale IVa 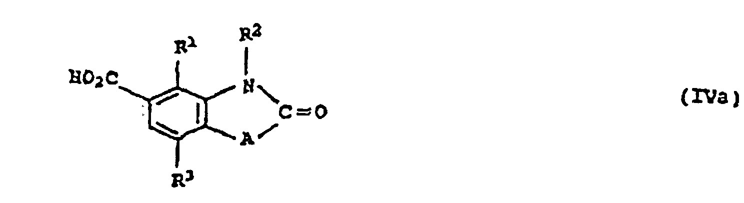 dans laquelle
A, R¹, R² et R³ ont les significations indiquées à la revendication 1.

11. Agent contenant au moins un dérivé de pyrazole de formule I ou un sel de I utilisable en agriculture selon l'une quelconque des revendications 1 à 9, et des adjuvants usuels.

12. Procédé de lutte contre des végétations indésirables, **caractérisé en ce que** l'on applique sur des plantes, leur espace vital et/ou leurs semences une quantité ayant une activité herbicide d'au moins un dérivé de pyrazole de formule I ou d'un sel de I utilisable en agriculture selon l'une quelconque des revendications 1 à 9.

13. Utilisation, en tant qu'herbicide, de dérivés de pyrazole de formule I ou d'un de leurs sels utilisables en agriculture selon l'une quelconque des revendications 1 à 9.
